# EUROPEAN PATENT APPLICATION

(11) **EP 2 465 936 A1**
(43) Date of publication of application: **20.06.2012**
(21) Application number: 10015774.2
(22) Date of filing: 20.12.2010
(51) Int. Cl.: C12P 17/06, C12P 17/16

(54) **Enzymatic synthesis of statins and intermediates thereof**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention discloses a process for preparing an active pharmaceutical ingredient (API) or intermediates thereof, notably particular step in the synthesis of an intermdiate useful for example in the preparation of statins, by using an enzyme capable of catalyzing oxidation or dehydrogenation. The invention further provides an expression system effectively translating said enzyme. In addition, the invention relates to a specific use of such enzyme for preparing API or intermediate thereof, and in particular for preparing statin or interemediate thereof.

SEQUENCE LISTING

<110>  Lek Pharmaceuticals d.d.

<120>  Enzymatic synthesis of active pharmaceutical ingredient and
       intermediates thereof

<130>  Enzymatic synthesis of active pharmaceutical ingredient and
       intermediates thereof

<160>  82

<170>  PatentIn version 3.3

<210>  1
<211>  1134
<212>  DNA
<213>  Artificial

<220>
<223>  GDH 01; Sequence based on nucleotide sequence encoding aldose
       sugar dehydrogenase YliI (originates from Escherichia coli)

<400>  1
gcgccatatg catcgacaat cctttttcct tgtgcccctt atttgtcttt cttccgctct      60

ctgggcggct cctgcaacgg taaatgtcga agtactgcaa gacaaactcg accatccctg     120

ggcactggcc tttttacccg ataatcacgg tatgttaatc actctgcgcg gcggcgagtt     180

gcgtcactgg caagcaggaa aaggattatc tgcgccgctt tccggagttc cggacgtttg     240

ggcgcacggg cagggcggcc tgctggacgt ggttttagcg cctgattttg ctcagtctcg     300

ccgcatctgg ttaagttatt ccgaagttgg cgatgatggc aaagccggaa ctgctgtggg     360

ttatggccgc ttaagtgatg atctctcaaa agtgaccgac ttccgcaccg tcttccgcca     420

gatgccaaaa ctgtctaccg gcaaccattt tggcgggcgg ctggtattcg acggtaaagg     480

ttatcttttt attgctctgg gcgaaaacaa tcagcgcccg acggcgcagg atctggataa     540

attacagggc aaactggtgc gtctgaccga ccagggcgaa atcccggatg ataatccttt     600

tataaaggaa tccggtgtgc gcgccgagat ctggtcttat ggcattcgta atccgcaagg     660

aatggcgatg aatccgtgga gtaatgcact gtggctgaat gaacatggcc cgcgcggtgg     720

tgatgaaatt aatatcccgc aaaaaggcaa aaactacggc tggccgctgg caacctgggg     780

aatcaactat tcaggcttta agataccgga agcgaaaggg gagatcgtcg ccgggaccga     840

gcaacctgtt ttttactgga aagattcgcc cgctgtgagc ggcatggcct tctataacag     900

cgataaattc ccccagtggc agcaaaaatt atttattggc gcgctgaaag ataaagatgt     960

cattgtgatg agcgtcaacg gcgacaaagt gacagaagat ggccgtattt taacggacag    1020

agggcagcga attcgtgatg ttcgcactgg acccgacggt tatttatacg ttctcaccga    1080

cgagtccagt ggggaattac ttaaagttag cccacgcaat tagcctgagc gcgc          1134

<210>  2
<211>  371
<212>  PRT
<213>  Escherichia coli

<400>  2

Met His Arg Gln Ser Phe Phe Leu Val Pro Leu Ile Cys Leu Ser Ser
1               5                   10                  15

Ala Leu Trp Ala Ala Pro Ala Thr Val Asn Val Glu Val Leu Gln Asp
            20                  25                  30

Lys Leu Asp His Pro Trp Ala Leu Ala Phe Leu Pro Asp Asn His Gly
        35                  40                  45

Met Leu Ile Thr Leu Arg Gly Gly Glu Leu Arg His Trp Gln Ala Gly
    50                  55                  60

Lys Gly Leu Ser Ala Pro Leu Ser Gly Val Pro Asp Val Trp Ala His
65                  70                  75                  80

Gly Gln Gly Gly Leu Leu Asp Val Val Leu Ala Pro Asp Phe Ala Gln
                85                  90                  95

Ser Arg Arg Ile Trp Leu Ser Tyr Ser Glu Val Gly Asp Asp Gly Lys
            100                 105                 110

Ala Gly Thr Ala Val Gly Tyr Gly Arg Leu Ser Asp Asp Leu Ser Lys
        115                 120                 125

Val Thr Asp Phe Arg Thr Val Phe Arg Gln Met Pro Lys Leu Ser Thr
    130                 135                 140

Gly Asn His Phe Gly Gly Arg Leu Val Phe Asp Gly Lys Gly Tyr Leu
145                 150                 155                 160

Phe Ile Ala Leu Gly Glu Asn Asn Gln Arg Pro Thr Ala Gln Asp Leu
                165                 170                 175

Asp Lys Leu Gln Gly Lys Leu Val Arg Leu Thr Asp Gln Gly Glu Ile
            180                 185                 190

Pro Asp Asp Asn Pro Phe Ile Lys Glu Ser Gly Val Arg Ala Glu Ile
        195                 200                 205

Trp Ser Tyr Gly Ile Arg Asn Pro Gln Gly Met Ala Met Asn Pro Trp
    210                 215                 220

Ser Asn Ala Leu Trp Leu Asn Glu His Gly Pro Arg Gly Gly Asp Glu
225                 230                 235                 240

Ile Asn Ile Pro Gln Lys Gly Lys Asn Tyr Gly Trp Pro Leu Ala Thr
                245                 250                 255

Trp Gly Ile Asn Tyr Ser Gly Phe Lys Ile Pro Glu Ala Lys Gly Glu
            260                 265                 270

Ile Val Ala Gly Thr Glu Gln Pro Val Phe Tyr Trp Lys Asp Ser Pro
        275                 280                 285

Ala Val Ser Gly Met Ala Phe Tyr Asn Ser Asp Lys Phe Pro Gln Trp
    290                 295                 300

Gln Gln Lys Leu Phe Ile Gly Ala Leu Lys Asp Lys Asp Val Ile Val
305                 310                 315                 320

Met Ser Val Asn Gly Asp Lys Val Thr Glu Asp Gly Arg Ile Leu Thr
                325                 330                 335

Asp Arg Gly Gln Arg Ile Arg Asp Val Arg Thr Gly Pro Asp Gly Tyr
            340                 345                 350

Leu Tyr Val Leu Thr Asp Glu Ser Ser Gly Glu Leu Leu Lys Val Ser
        355                 360                 365

Pro Arg Asn
    370

<210>  3
<211>  1077
<212>  DNA
<213>  Artificial

<220>
<223>  GDH 02; Sequence based on nucleotide sequence encoding aldose
       sugar dehydrogenase YliI (originates from Escherichia coli)

<400>  3
gcgccatatg gctcctgcaa cggtaaatgt cgaagtactg caagacaaac tcgaccatcc      60

ctgggcactg gcctttttac ccgataatca cggtatgtta atcactctgc gcggcggcga     120

gttgcgtcac tggcaagcag gaaaaggatt atctgcgccg ctttccggag ttccggacgt     180

ttgggcgcac gggcagggcg gcctgctgga cgtggtttta gcgcctgatt ttgctcagtc     240

tcgccgcatc tggttaagtt attccgaagt tggcgatgat ggcaaagccg gaactgctgt     300

gggttatggc cgcttaagtg atgatctctc aaaagtgacc gacttccgca ccgtcttccg     360

ccagatgcca aaactgtcta ccggcaacca ttttggcggg cggctggtat tcgacggtaa     420

agg ttatctt tttattgctc tgggcgaaaa caatcagcgc ccgacggcgc aggatctgga 480

taaattacag ggcaaactgg tgcgtctgac cgaccagggc gaaatcccgg atgataatcc 540

ttttataaag gaatccggtg tgcgcgccga gatctggtct tatggcattc gtaatccgca 600

aggaatggcg atgaatccgt ggagtaatgc actgtggctg aatgaacatg gcccgcgcgg 660

tggtgatgaa attaatatcc cgcaaaaagg caaaaactac ggctggccgc tggcaacctg 720

gggaatcaac tattcaggct ttaagatacc ggaagcgaaa ggggagatcg tcgccgggac 780

cgagcaacct gttttttact ggaaagattc gcccgctgtg agcggcatgg ccttctataa 840

cagcgataaa ttcccccagt ggcagcaaaa attatttatt ggcgcgctga aagataaaga 900

tgtcattgtg atgagcgtca acggcgacaa agtgacagaa gatggccgta ttttaacgga 960

cagagggcag cgaattcgtg atgttcgcac tggacccgac ggttatttat acgttctcac 1020

cgacgagtcc agtggggaat tacttaaagt tagcccacgc aattagcctg agcgcgc 1077

<210> 4
<211> 352
<212> PRT
<213> Escherichia coli

<400> 4

Met Ala Pro Ala Thr Val Asn Val Glu Val Leu Gln Asp Lys Leu Asp
1               5                   10                  15

His Pro Trp Ala Leu Ala Phe Leu Pro Asp Asn His Gly Met Leu Ile
            20                  25                  30

Thr Leu Arg Gly Gly Glu Leu Arg His Trp Gln Ala Gly Lys Gly Leu
        35                  40                  45

Ser Ala Pro Leu Ser Gly Val Pro Asp Val Trp Ala His Gly Gln Gly
    50                  55                  60

Gly Leu Leu Asp Val Val Leu Ala Pro Asp Phe Ala Gln Ser Arg Arg
65                  70                  75                  80

Ile Trp Leu Ser Tyr Ser Glu Val Gly Asp Asp Gly Lys Ala Gly Thr
                85                  90                  95

Ala Val Gly Tyr Gly Arg Leu Ser Asp Asp Leu Ser Lys Val Thr Asp
            100                 105                 110

Phe Arg Thr Val Phe Arg Gln Met Pro Lys Leu Ser Thr Gly Asn His
        115                 120                 125

Phe Gly Gly Arg Leu Val Phe Asp Gly Lys Gly Tyr Leu Phe Ile Ala
    130                 135                 140

Leu Gly Glu Asn Asn Gln Arg Pro Thr Ala Gln Asp Leu Asp Lys Leu
145                 150                 155                 160

Gln Gly Lys Leu Val Arg Leu Thr Asp Gln Gly Glu Ile Pro Asp Asp
                165                 170                 175

Asn Pro Phe Ile Lys Glu Ser Gly Val Arg Ala Glu Ile Trp Ser Tyr
            180                 185                 190

Gly Ile Arg Asn Pro Gln Gly Met Ala Met Asn Pro Trp Ser Asn Ala
        195                 200                 205

Leu Trp Leu Asn Glu His Gly Pro Arg Gly Gly Asp Glu Ile Asn Ile
    210                 215                 220

Pro Gln Lys Gly Lys Asn Tyr Gly Trp Pro Leu Ala Thr Trp Gly Ile
225                 230                 235                 240

Asn Tyr Ser Gly Phe Lys Ile Pro Glu Ala Lys Gly Glu Ile Val Ala
                245                 250                 255

Gly Thr Glu Gln Pro Val Phe Tyr Trp Lys Asp Ser Pro Ala Val Ser
            260                 265                 270

Gly Met Ala Phe Tyr Asn Ser Asp Lys Phe Pro Gln Trp Gln Gln Lys
        275                 280                 285

Leu Phe Ile Gly Ala Leu Lys Asp Lys Asp Val Ile Val Met Ser Val
    290                 295                 300

Asn Gly Asp Lys Val Thr Glu Asp Gly Arg Ile Leu Thr Asp Arg Gly
305                 310                 315                 320

Gln Arg Ile Arg Asp Val Arg Thr Gly Pro Asp Gly Tyr Leu Tyr Val
                325                 330                 335

Leu Thr Asp Glu Ser Ser Gly Glu Leu Leu Lys Val Ser Pro Arg Asn
            340                 345                 350

<210>  5
<211>  1450
<212>  DNA
<213>  Artificial

<220>

<223>  GDH 03; Nucleotide sequence based on sequence encoding gene gdhB
      (originates from Acinetobacter calcoaceticus)

<400>  5
atggacaaac acctgctggc gaaaatcgcg ctgctgtctg cggttcagct ggttaccctg      60

tctgcgttcg cggacgttcc gctgaccccg tctcagttcg cgaaagcgaa atctgaaaac     120

ttcgacaaaa aagttatcct gtctaacctg aacaaaccgc acgcgctgct gtggggtccg     180

gacaaccaga tctggctgac cgaacgtgcg accggtaaaa tcctgcgtgt taacccggaa     240

tctggttctg ttaaaaccgt tttccaggtt ccggaaatcg ttaacgacgc ggacggtcag     300

aacggtctgc tgggtttcgc gttccacccg gacttcaaaa acaacccgta catctacatc     360

tctggtacct tcaaaaaccc gaaatctacc gacaaagaac tgccgaacca gaccatcatc     420

cgtcgttaca cctacaacaa atctaccgac accctggaaa aaccggttga cctgctggcg     480

ggtctgccgt cttctaaaga ccaccagtct ggtcgtctgg ttatcggtcc ggaccagaaa     540

atctactaca ccatcggtga ccagggtcgt aaccagctgg cgtacctgtt cctgccgaac     600

caggcgcagc acaccccgac ccagcaggaa ctgaacggta aagactacca cacctacatg     660

ggtaaagttc tgcgtctgaa cctggacggt tctatcccga aagacaaccc gtctttcaac     720

ggtgttgttt ctcacatcta caccctgggt caccgtaacc cgcagggtct ggcgttcacc     780

ccgaacggta aactgctgca gtctgaacag ggtccgaact ctgacgacga aatcaacctg     840

atcgttaaag gtggtaacta cggttggccg aacgttgcgg gttacaaaga cgactctggt     900

tacgcgtacg cgaactactc tgcggcggcg aacaaatcta tcaaagacct ggcgcagaac     960

ggtgttaaag ttgcggcggg tgttccggtt accaaagaat ctgaatggac cggtaaaaac    1020

ttcgttccgc cgctgaaaac cctgtacacc gttcaggaca cctacaacta caacgacccg    1080

acctgcggtg aaatgaccta catctgctgg ccgaccgttg cgccgtcttc tgcgtacgtt    1140

tacaaaggtg gtaaaaaagc gatcaccggt tgggaaaaca ccctgctggt tccgtctctg    1200

aaacgtggtg ttatcttccg tatcaaactg gacccgacct actctaccac ctacgacgac    1260

gcggttccga tgttcaaatc taacaaccgt taccgtgacg ttatcgcgtc tccggacggt    1320

aacgttctgt acgttctgac cgacaccgcg ggtaacgttc agaaagacga cggttctgtt    1380

accaacaccc tggaaaaccc gggttctctg atcaaattca cctacaaagc gaaataagct    1440

cagcaagctt                                                           1450

<210>  6
<211>  478
<212>  PRT
<213>  Artificial

<220>
<223>  GDH 03;  Amino acid sequence of protein based on GdhB protein

(originates from Acinetobacter calcoaceticus)

<400> 6

Met Asp Lys His Leu Leu Ala Lys Ile Ala Leu Leu Ser Ala Val Gln
1               5                   10                  15

Leu Val Thr Leu Ser Ala Phe Ala Asp Val Pro Leu Thr Pro Ser Gln
            20                  25                  30

Phe Ala Lys Ala Lys Ser Glu Asn Phe Asp Lys Lys Val Ile Leu Ser
        35                  40                  45

Asn Leu Asn Lys Pro His Ala Leu Leu Trp Gly Pro Asp Asn Gln Ile
    50                  55                  60

Trp Leu Thr Glu Arg Ala Thr Gly Lys Ile Leu Arg Val Asn Pro Glu
65                  70                  75                  80

Ser Gly Ser Val Lys Thr Val Phe Gln Val Pro Glu Ile Val Asn Asp
                85                  90                  95

Ala Asp Gly Gln Asn Gly Leu Leu Gly Phe Ala Phe His Pro Asp Phe
            100                 105                 110

Lys Asn Asn Pro Tyr Ile Tyr Ile Ser Gly Thr Phe Lys Asn Pro Lys
        115                 120                 125

Ser Thr Asp Lys Glu Leu Pro Asn Gln Thr Ile Ile Arg Arg Tyr Thr
    130                 135                 140

Tyr Asn Lys Ser Thr Asp Thr Leu Glu Lys Pro Val Asp Leu Leu Ala
145                 150                 155                 160

Gly Leu Pro Ser Ser Lys Asp His Gln Ser Gly Arg Leu Val Ile Gly
                165                 170                 175

Pro Asp Gln Lys Ile Tyr Tyr Thr Ile Gly Asp Gln Gly Arg Asn Gln
            180                 185                 190

Leu Ala Tyr Leu Phe Leu Pro Asn Gln Ala Gln His Thr Pro Thr Gln
        195                 200                 205

Gln Glu Leu Asn Gly Lys Asp Tyr His Thr Tyr Met Gly Lys Val Leu
    210                 215                 220

Arg Leu Asn Leu Asp Gly Ser Ile Pro Lys Asp Asn Pro Ser Phe Asn
225                 230                 235                 240

Gly Val Val Ser His Ile Tyr Thr Leu Gly His Arg Asn Pro Gln Gly
                245                 250                 255

Leu Ala Phe Thr Pro Asn Gly Lys Leu Leu Gln Ser Glu Gln Gly Pro
            260                 265                 270

Asn Ser Asp Asp Glu Ile Asn Leu Ile Val Lys Gly Gly Asn Tyr Gly
        275                 280                 285

Trp Pro Asn Val Ala Gly Tyr Lys Asp Asp Ser Gly Tyr Ala Tyr Ala
    290                 295                 300

Asn Tyr Ser Ala Ala Ala Asn Lys Ser Ile Lys Asp Leu Ala Gln Asn
305                 310                 315                 320

Gly Val Lys Val Ala Ala Gly Val Pro Val Thr Lys Glu Ser Glu Trp
                325                 330                 335

Thr Gly Lys Asn Phe Val Pro Pro Leu Lys Thr Leu Tyr Thr Val Gln
            340                 345                 350

Asp Thr Tyr Asn Tyr Asn Asp Pro Thr Cys Gly Glu Met Thr Tyr Ile
        355                 360                 365

Cys Trp Pro Thr Val Ala Pro Ser Ser Ala Tyr Val Tyr Lys Gly Gly
    370                 375                 380

Lys Lys Ala Ile Thr Gly Trp Glu Asn Thr Leu Leu Val Pro Ser Leu
385                 390                 395                 400

Lys Arg Gly Val Ile Phe Arg Ile Lys Leu Asp Pro Thr Tyr Ser Thr
                405                 410                 415

Thr Tyr Asp Asp Ala Val Pro Met Phe Lys Ser Asn Asn Arg Tyr Arg
            420                 425                 430

Asp Val Ile Ala Ser Pro Asp Gly Asn Val Leu Tyr Val Leu Thr Asp
        435                 440                 445

Thr Ala Gly Asn Val Gln Lys Asp Asp Gly Ser Val Thr Asn Thr Leu
    450                 455                 460

Glu Asn Pro Gly Ser Leu Ile Lys Phe Thr Tyr Lys Ala Lys
465                 470                 475

<210>  7

<211>  1450
<212>  DNA
<213>  Artificial

<220>
<223>  GDH 04; Nucleotide sequence based on sequence encoding gene gdhB
       originating from Acinetobacter calcoaceticus

<400>  7
atggacaaac acctgctggc gaaaatcgcg ctgctgtctg cggttcagct ggttaccctg      60

tctgcgttcg cggacgttcc gctgaccccg tctcagttcg cgaaagcgaa atctgaaaac     120

ttcgacaaaa aagttatcct gtctaacctg aacaaaccgc acgcgctgct gtggggtccg     180

gacaaccaga tctggctgac cgaacgtgcg accggtaaaa tcctgcgtgt taacccggaa     240

tctggttctg ttaaaaccgt tttccaggtt ccggaaatcg ttaacgacgc ggacggtcag     300

aacggtctgc tgggtttcgc gttccacccg gacttcaaaa acaacccgta catctacatc     360

tctggtacct tcaaaaaccc gaaatctacc gacaaagaac tgccgaacca gaccatcatc     420

cgtcgttaca cctacaacaa atctaccgac accctggaaa aaccggttga cctgctggcg     480

ggtctgccgt cttctaaaga ccaccagtct ggtcgtctgg ttatcggtcc ggaccagaaa     540

atctactaca ccatcggtga ccagggtcgt aaccagctgg cgtacctgtt cctgccgaac     600

caggcgcagc acaccccgac ccagcaggaa ctgaacggta aagactacca cacctacatg     660

ggtaaagttc tgcgtctgaa cctggacggt aaaatcccga aagacaaccc gtctttcaac     720

ggtgttgttt ctcacatcta caccctgggt caccgtaacc cgcagggtct ggcgttcacc     780

ccgaacggta aactgctgca gtctgaacag ggtccgaact ctgacgacga aatcaacctg     840

atcgttaaag gtggtaacta cggttggccg aacgttgcgg gttacaaaga cgactctggt     900

tacgcgtacg cgaactactc tgcggcggcg aacaaatcta tcaaagacct ggcgcagaac     960

ggtgttaaag ttgcggcggg tgttccggtt accaaagaat ctgaatggac cggtaaaaac    1020

ttcgttccgc cgctgaaaac cctgtacacc gttcaggaca cctacaacta caacgacccg    1080

acctgcggtg aaatgaccta catctgctgg ccgaccgttg cgccgtcttc tgcgtacgtt    1140

tacaaaggtg gtaaaaaagc gatcaccggt tgggaaaaca ccctgctggt tccgtctctg    1200

aaacgtggtg ttatcttccg tatcaaactg gacccgacct actctaccac ctacgacgac    1260

gcggttccga tgttcaaatc taacaaccgt taccgtgacg ttatcgcgtc tccggacggt    1320

aacgttctgt acgttctgac cgacaccgcg ggtaacgttc agaaagacga cggttctgtt    1380

accaacaccc tggaaaaccc gggttctctg atcaaattca cctacaaagc gaaataagct    1440

cagcaagctt                                                           1450


<210>  8
<211>  478
<212>  PRT

<213>  Artificial

<220>
<223>  GDH 04; Amino acid sequence of protein based on GdhB protein
      (originates from Acinetobacter calcoaceticus)

<400>  8

Met Asp Lys His Leu Leu Ala Lys Ile Ala Leu Leu Ser Ala Val Gln
1               5                   10                  15

Leu Val Thr Leu Ser Ala Phe Ala Asp Val Pro Leu Thr Pro Ser Gln
            20                  25                  30

Phe Ala Lys Ala Lys Ser Glu Asn Phe Asp Lys Lys Val Ile Leu Ser
        35                  40                  45

Asn Leu Asn Lys Pro His Ala Leu Leu Trp Gly Pro Asp Asn Gln Ile
    50                  55                  60

Trp Leu Thr Glu Arg Ala Thr Gly Lys Ile Leu Arg Val Asn Pro Glu
65                  70                  75                  80

Ser Gly Ser Val Lys Thr Val Phe Gln Val Pro Glu Ile Val Asn Asp
                85                  90                  95

Ala Asp Gly Gln Asn Gly Leu Leu Gly Phe Ala Phe His Pro Asp Phe
            100                 105                 110

Lys Asn Asn Pro Tyr Ile Tyr Ile Ser Gly Thr Phe Lys Asn Pro Lys
        115                 120                 125

Ser Thr Asp Lys Glu Leu Pro Asn Gln Thr Ile Ile Arg Arg Tyr Thr
    130                 135                 140

Tyr Asn Lys Ser Thr Asp Thr Leu Glu Lys Pro Val Asp Leu Leu Ala
145                 150                 155                 160

Gly Leu Pro Ser Ser Lys Asp His Gln Ser Gly Arg Leu Val Ile Gly
                165                 170                 175

Pro Asp Gln Lys Ile Tyr Tyr Thr Ile Gly Asp Gln Gly Arg Asn Gln
            180                 185                 190

Leu Ala Tyr Leu Phe Leu Pro Asn Gln Ala Gln His Thr Pro Thr Gln
        195                 200                 205

Gln Glu Leu Asn Gly Lys Asp Tyr His Thr Tyr Met Gly Lys Val Leu
    210                 215                 220

Arg Leu Asn Leu Asp Gly Lys Ile Pro Lys Asp Asn Pro Ser Phe Asn
225                 230                 235                 240

Gly Val Val Ser His Ile Tyr Thr Leu Gly His Arg Asn Pro Gln Gly
                245                 250                 255

Leu Ala Phe Thr Pro Asn Gly Lys Leu Leu Gln Ser Glu Gln Gly Pro
            260                 265                 270

Asn Ser Asp Asp Glu Ile Asn Leu Ile Val Lys Gly Gly Asn Tyr Gly
        275                 280                 285

Trp Pro Asn Val Ala Gly Tyr Lys Asp Asp Ser Gly Tyr Ala Tyr Ala
    290                 295                 300

Asn Tyr Ser Ala Ala Ala Asn Lys Ser Ile Lys Asp Leu Ala Gln Asn
305                 310                 315                 320

Gly Val Lys Val Ala Ala Gly Val Pro Val Thr Lys Glu Ser Glu Trp
                325                 330                 335

Thr Gly Lys Asn Phe Val Pro Pro Leu Lys Thr Leu Tyr Thr Val Gln
            340                 345                 350

Asp Thr Tyr Asn Tyr Asn Asp Pro Thr Cys Gly Glu Met Thr Tyr Ile
        355                 360                 365

Cys Trp Pro Thr Val Ala Pro Ser Ser Ala Tyr Val Tyr Lys Gly Gly
    370                 375                 380

Lys Lys Ala Ile Thr Gly Trp Glu Asn Thr Leu Leu Val Pro Ser Leu
385                 390                 395                 400

Lys Arg Gly Val Ile Phe Arg Ile Lys Leu Asp Pro Thr Tyr Ser Thr
                405                 410                 415

Thr Tyr Asp Asp Ala Val Pro Met Phe Lys Ser Asn Asn Arg Tyr Arg
            420                 425                 430

Asp Val Ile Ala Ser Pro Asp Gly Asn Val Leu Tyr Val Leu Thr Asp
        435                 440                 445

Thr Ala Gly Asn Val Gln Lys Asp Asp Gly Ser Val Thr Asn Thr Leu
    450                 455                 460

Glu Asn Pro Gly Ser Leu Ile Lys Phe Thr Tyr Lys Ala Lys

465                 470                 475

<210>  9
<211>  799
<212>  DNA
<213>  Artificial

<220>
<223>  GDH 05; Nucleotide sequence based on gene deoC (originates from
       Escherichia coli)

<400>  9
ccggcatatg actgatctga aagcaagcag cctgcgtgca ctgaaattga tggacctgac      60

caccctgaat gacgacgaca ccgacgagaa agtgatcgcc ctgtgtcatc aggccaaaac     120

tccggtcggc aataccgccg ctatctgtat ctatcctcgc tttatcccga ttgctcgcaa     180

aactctgaaa gagcagggca ccccggaaat ccgtatcgct acggtaacca acttcccaca     240

cggtaacgac gacatcgaca tcgcgctggc agaaacccgt gcggcaatcg cctacggtgc     300

tgatgaagtt gacgttgtgt tcccgtaccg cgcgctgatg gcgggtaacg agcaggttgg     360

ttttgacctg gtgaaagcct gtaaagaggc ttgcgcggca gcgaatgtac tgctgaaagt     420

gatcatcgaa accggcgaac tgaaagacga agcgctgatc cgtaaagcgt ctgaaatctc     480

catcaaagcg ggtgcggact tcatcaaaac ctctaccggt aaagtggctg tgaacgcgac     540

gccggaaagc gcgcgcatca tgatggaagt gatccgtgat atgggcgtag aaaaaaccgt     600

tggtttcaaa ccggcgggcg gcgtgcgtac tgcggaagat gcgcagaaat atctcgccat     660

tgcagatgaa ctgttcggtg ctgactgggc agatgcgcgt cactaccgct ttggcgcttc     720

cagcctgctg gcaagcctgc tgaaagcgct gggtcacggc gacggtaaga gcgccagcag     780

ctactaatga gctgagcgg                                                  799

<210>  10
<211>  259
<212>  PRT
<213>  Escherichia coli

<400>  10

Met Thr Asp Leu Lys Ala Ser Ser Leu Arg Ala Leu Lys Leu Met Asp
1               5                   10                  15

Leu Thr Thr Leu Asn Asp Asp Asp Thr Asp Glu Lys Val Ile Ala Leu
            20                  25                  30

Cys His Gln Ala Lys Thr Pro Val Gly Asn Thr Ala Ala Ile Cys Ile
        35                  40                  45

Tyr Pro Arg Phe Ile Pro Ile Ala Arg Lys Thr Leu Lys Glu Gln Gly
    50                  55                  60

Thr Pro Glu Ile Arg Ile Ala Thr Val Thr Asn Phe Pro His Gly Asn
65                  70                  75                  80

Asp Asp Ile Asp Ile Ala Leu Ala Glu Thr Arg Ala Ala Ile Ala Tyr
                85                  90                  95

Gly Ala Asp Glu Val Asp Val Val Phe Pro Tyr Arg Ala Leu Met Ala
            100                 105                 110

Gly Asn Glu Gln Val Gly Phe Asp Leu Val Lys Ala Cys Lys Glu Ala
        115                 120                 125

Cys Ala Ala Ala Asn Val Leu Leu Lys Val Ile Ile Glu Thr Gly Glu
    130                 135                 140

Leu Lys Asp Glu Ala Leu Ile Arg Lys Ala Ser Glu Ile Ser Ile Lys
145                 150                 155                 160

Ala Gly Ala Asp Phe Ile Lys Thr Ser Thr Gly Lys Val Ala Val Asn
                165                 170                 175

Ala Thr Pro Glu Ser Ala Arg Ile Met Met Glu Val Ile Arg Asp Met
            180                 185                 190

Gly Val Glu Lys Thr Val Gly Phe Lys Pro Ala Gly Gly Val Arg Thr
        195                 200                 205

Ala Glu Asp Ala Gln Lys Tyr Leu Ala Ile Ala Asp Glu Leu Phe Gly
    210                 215                 220

Ala Asp Trp Ala Asp Ala Arg His Tyr Arg Phe Gly Ala Ser Ser Leu
225                 230                 235                 240

Leu Ala Ser Leu Leu Lys Ala Leu Gly His Gly Asp Gly Lys Ser Ala
                245                 250                 255

Ser Ser Tyr


<210>  11
<211>  3362
<212>  DNA
<213>  Artificial

<220>
<223>  Nucleotide sequence based on the pqqABCDE operon (originates from
       Gluconobacter oxydans)

<400> 11
cgcgggtacc tcgcatctgc cggttatcgg agaccaagga gaaaaatcat ggcctggaac 60
acaccgaaag ttaccgaaat cccgctgggc gcagaaatca actcgtatgt ctgcggcgag 120
aagaaataag ccgctttccc ggggacccgt ccttgaggaa taatggcacg gccgctcccc 180
catggagcgg ccgttttcgt tcatgggtgc tctgtggtgc cccagtcaga cggtttgtga 240
aaaaatgatt gatgtcatcg tgcttggcgc ggcggcaggg ggcggttttc cgcagtggaa 300
ctccgcagca cccggctgtg tggccgcccg cacgcgacag ggcgcgaaag cccggaccca 360
ggcctccctt gccgtcagtg ccgacggaaa gcgctggttc attctcaacg cctcgcccga 420
tctgcggcag cagatcatcg atacgccggc cctgcatcat cagggcagcc tgcgtggaac 480
gcccattcag ggcgtcgtcc tgacctgcgg cgagatcgac gccataaccg ggcttctgac 540
cctgcgtgag cgtgagcctt ttaccctgat gggcagcgac tcgacccttc agcagcttgc 600
ggacaatccg atcttcggtg cgctcgatcc ggaaatcgtc ccacgtgttc cgctcattct 660
cgatgaagcc acgtccctga tgaacaagga cgggattccg tccggtcttt tgctcacggc 720
cttcgccgtt ccgggcaagg cgccgcttta cgcggaagcc gcagggtcac gcccggacga 780
gacgctgggc ctttccatta cggatggatg caagacgatg ctcttcattc ccggctgtgc 840
gcagatcacg tcggaaatcg tggaacgggt agcggcagcc gatctcgtgt tctttgacgg 900
gacactgtgg cgggatgacg aaatgatccg cgccgggttg agcccgaaga gcggacagcg 960
gatgggacat gtgtccgtga atgatgccgg gggaccggtc gaatgtttca cgacatgcga 1020
aaaaccccgt aaagtgttga ttcatatcaa caactccaat ccaattctgt tcgaagacag 1080
ccccgaacgc aaagacgtcg aacgcgccgg atggacggtt gcggaagacg gcatgacttt 1140
cagactggac acaccatgac gctcctcaca cctgaccagc ttgaagcaca gcttcgccag 1200
atcggggccg agcggtatca caaccggcac ccgttccatc gcaagctgca tgacggcaag 1260
ctggacaagg cacaggttca ggcttgggcg ctgaaccgct attattatca ggcccgcatc 1320
ccggcgaagg atgcgacgct tctcgcacgt ctgccgacgg ccgaactgcg ccgcgaatgg 1380
cgtcgccgga tcgaggacca tgacggcacg gagcccggaa cgggcggtgt tgcgcgctgg 1440
ctgatgctga cggatggtct ggggctggac cgggattatg tggaaagcct cgatggtctg 1500
cttccagcca cgcgcttctc ggtcgatgcc tatgtgaact tcgtgcggga ccagtcgatt 1560
ctggcggcca ttgcgtcgtc gctgacggaa ctgttttcgc ccacgatcat cagcgagcgc 1620
gtctcgggga tgctgcggca ctacgacttt gtgtcggaaa agacgctggc ctatttcacg 1680
ccgcgcctga cgcaggcccc gcgggattcc gatttcgcgc tggcctatgt ccgcgaaaag 1740
gcccgcacgc cggagcagca gaaagaagtc ctgggagcgc tggagttcaa gtgctccgtg 1800
ctgtggacga tgctggatgc gctcgactac gcctatgtgg aaggccacat tccgccgggg 1860

gctttcgttc catgacggag gccccgcatg tcgtggcgga ggggacggtt ctctcctttg 1920

cccgggggca tcgtctccag cacgatcgtg tgcgggacgt gtggatcgtg caggcgcctg 1980

aaaaagcatt tgtagttgag ggcgccgcgc cgcatattct gcggctgctg gatgggaagc 2040

gcagcgtcgg cgagatcatc cagcagcttg caatcgagtt ttccgccccg cgtgaggtca 2100

ttgcgaaaga tgtcctcgcg cttctttctg aactgacaga aaagaacgtc ctgcacacat 2160

gacactccct tcgccgccga tgagccttct ggctgaactg acgcatcgat gcccgctttc 2220

ctgcccctac tgctccaatc cgcttgaact cgaacgcaag gcggcagaac tcgacacggc 2280

cacctggact gccgtactgg agcaggcggc cgagcttggg gtgctccagg ttcatttctc 2340

tggcggcgag cctatggcgc ggcctgatct ggtcgaactg gtctccgtcg cacggagact 2400

caacctgtat tccaacttga tcacgtccgg cgtgttgctg gacgaaccga aactggaagc 2460

tctcgacagg gcggggctgg atcacatcca gctctctttc caagacgtga cggaggcggg 2520

agccgagcgt atcggcggtc tcaagggagc gcaggcccgc aaggttgcgg cggcgcggct 2580

catccgcgcg tccggcattc cgatgacgct caattttgtg gtgcacaggg aaaatgtcgc 2640

ccgtatcccc gagatgttcg ccctggcgcg ggaactcgga gcggggcggg tggagatcgc 2700

gcatacccag tattatggct gggggctgaa aaaccgtgag gcgcttcttc ccagccggga 2760

tcagctggag gaatccacac gcgccgtgga agcggagcgc gctaagggtg gtttgtccgt 2820

tgattatgtg acgccggact atcatgcaga ccggcccaag ccctgcatgg ggggatgggg 2880

ccagcgtttc gtgaatgtca caccttcggg ccgggtcctg ccgtgtcatg cagccgaaat 2940

cattccggat gtcgcattcc cgaatgtgca ggatgtgacc ctgtccgaaa tctggaacat 3000

ctcaccgctg ttcaacatgt tccgcgggac ggactggatg ccggagccct gccgctcctg 3060

cgagcgcaag gagcgtgact ggggcgggtg tcgctgtcag gcgatggcgc tgacggggaa 3120

tgccgcgaat accgatcccg tatgcagtct ctccccctat cacgatcggg tggagcaggc 3180

cgtcgagaac aacatgcagc cagaaagcac gttgttctac aggcgttata cgtaatcgcc 3240

gtaaatattg actttgctaa gatggagaaa ggatgtgccg ttcaggacaa tcttgatata 3300

tcgagcaata atatctgttt ctatctgaag aggttctcca aactctccgc ccggatccgc 3360

gc 3362

<210> 12
<211> 26
<212> PRT
<213> Gluconobacter oxydans

<400> 12

Met Ala Trp Asn Thr Pro Lys Val Thr Glu Ile Pro Leu Gly Ala Glu
1               5                   10                  15

Ile Asn Ser Tyr Val Cys Gly Glu Lys Lys
            20                  25

<210>  13
<211>  302
<212>  PRT
<213>  Gluconobacter oxydans

<400>  13

Met Ile Asp Val Ile Val Leu Gly Ala Ala Ala Gly Gly Gly Phe Pro
1               5                   10                  15

Gln Trp Asn Ser Ala Ala Pro Gly Cys Val Ala Ala Arg Thr Arg Gln
            20                  25                  30

Gly Ala Lys Ala Arg Thr Gln Ala Ser Leu Ala Val Ser Ala Asp Gly
        35                  40                  45

Lys Arg Trp Phe Ile Leu Asn Ala Ser Pro Asp Leu Arg Gln Gln Ile
    50                  55                  60

Ile Asp Thr Pro His His Gln Gly Ser Leu Arg Gly Thr Pro Ile Gln
65                  70                  75                  80

Gly Val Val Leu Thr Cys Gly Glu Ile Asp Ala Ile Thr Gly Leu Leu
                85                  90                  95

Thr Leu Arg Glu Arg Glu Pro Phe Thr Leu Met Gly Ser Asp Ser Thr
            100                 105                 110

Leu Gln Gln Leu Ala Asp Asn Pro Ile Phe Gly Ala Leu Asp Pro Glu
        115                 120                 125

Ile Val Pro Arg Val Pro Leu Ile Leu Asp Glu Ala Thr Ser Leu Met
    130                 135                 140

Asn Lys Asp Gly Ile Pro Ser Gly Leu Leu Leu Thr Ala Phe Ala Val
145                 150                 155                 160

Pro Gly Lys Ala Pro Leu Tyr Ala Glu Ala Ala Gly Ser Arg Pro Asp
                165                 170                 175

Glu Thr Leu Gly Leu Ser Ile Thr Asp Gly Cys Lys Thr Met Leu Phe
            180                 185                 190

Ile Pro Gly Cys Ala Gln Ile Thr Ser Glu Ile Val Glu Arg Val Ala
        195                 200                 205

Ala Ala Asp Leu Val Phe Phe Asp Gly Thr Leu Trp Arg Asp Asp Glu
    210                 215                 220

Met Ile Arg Ala Gly Leu Ser Pro Lys Ser Gly Gln Arg Met Gly His
225                 230                 235                 240

Val Ser Val Asn Asp Ala Gly Gly Pro Val Glu Cys Phe Thr Thr Cys
                245                 250                 255

Glu Lys Pro Arg Lys Val Leu Ile His Ile Asn Asn Ser Asn Pro Ile
            260                 265                 270

Leu Phe Glu Asp Ser Pro Glu Arg Lys Asp Val Glu Arg Ala Gly Trp
        275                 280                 285

Thr Val Ala Glu Asp Gly Met Thr Phe Arg Leu Asp Thr Pro
    290                 295                 300

<210>  14
<211>  239
<212>  PRT
<213>  Gluconobacter oxydans

<400>  14

Met Thr Leu Leu Thr Pro Asp Gln Leu Glu Ala Gln Leu Arg Gln Ile
1               5                   10                  15

Gly Ala Glu Arg Tyr His Asn Arg His Pro Phe His Arg Lys Leu His
            20                  25                  30

Asp Gly Lys Leu Asp Lys Ala Gln Val Gln Ala Trp Ala Leu Asn Arg
        35                  40                  45

Tyr Tyr Tyr Gln Ala Arg Ile Pro Ala Lys Asp Ala Thr Leu Leu Ala
    50                  55                  60

Arg Leu Pro Thr Ala Glu Leu Arg Arg Glu Trp Arg Arg Arg Ile Glu
65                  70                  75                  80

Asp His Asp Gly Thr Glu Pro Gly Thr Gly Gly Val Ala Arg Trp Leu
                85                  90                  95

Met Leu Thr Asp Gly Leu Gly Leu Asp Arg Asp Tyr Val Glu Ser Leu
            100                 105                 110

Asp Gly Leu Leu Pro Ala Thr Arg Phe Ser Val Asp Ala Tyr Val Asn
        115                 120                 125

Phe Val Arg Asp Gln Ser Ile Leu Ala Ala Ile Ala Ser Ser Leu Thr
    130                 135                 140

Glu Leu Phe Ser Pro Thr Ile Ile Ser Glu Arg Val Ser Gly Met Leu
145                 150                 155                 160

Arg His Tyr Asp Phe Val Ser Glu Lys Thr Leu Ala Tyr Phe Thr Pro
                165                 170                 175

Arg Leu Thr Gln Ala Pro Arg Asp Ser Asp Phe Ala Leu Ala Tyr Val
            180                 185                 190

Arg Glu Lys Ala Arg Thr Pro Glu Gln Gln Lys Glu Val Leu Gly Ala
        195                 200                 205

Leu Glu Phe Lys Cys Ser Val Leu Trp Thr Met Leu Asp Ala Leu Asp
    210                 215                 220

Tyr Ala Tyr Val Glu Gly His Ile Pro Pro Gly Ala Phe Val Pro
225                 230                 235

<210>  15
<211>  96
<212>  PRT
<213>  Gluconobacter oxydans

<400>  15

Met Thr Glu Ala Pro His Val Val Ala Glu Gly Thr Val Leu Ser Phe
1               5                   10                  15

Ala Arg Gly His Arg Leu Gln His Asp Arg Val Arg Asp Val Trp Ile
            20                  25                  30

Val Gln Ala Pro Glu Lys Ala Phe Val Val Glu Gly Ala Ala Pro His
        35                  40                  45

Ile Leu Arg Leu Leu Asp Gly Lys Arg Ser Val Gly Glu Ile Ile Gln
    50                  55                  60

Gln Leu Ala Ile Glu Phe Ser Ala Pro Arg Glu Val Ile Ala Lys Asp
65                  70                  75                  80

Val Leu Ala Leu Leu Ser Glu Leu Thr Glu Lys Asn Val Leu His Thr
                85                  90                  95

<210>  16
<211>  358
<212>  PRT

<213>  Gluconobacter oxydans

<400>  16

Met Thr Leu Pro Ser Pro Pro Met Ser Leu Leu Ala Glu Leu Thr His
1               5                   10                  15

Arg Cys Pro Leu Ser Cys Pro Tyr Cys Ser Asn Pro Leu Glu Leu Glu
            20                  25                  30

Arg Lys Ala Ala Glu Leu Asp Thr Ala Thr Trp Thr Ala Val Leu Glu
        35                  40                  45

Gln Ala Ala Glu Leu Gly Val Leu Gln Val His Phe Ser Gly Gly Glu
    50                  55                  60

Pro Met Ala Arg Pro Asp Leu Val Glu Leu Val Ser Val Ala Arg Arg
65                  70                  75                  80

Leu Asn Leu Tyr Ser Asn Leu Ile Thr Ser Gly Val Leu Leu Asp Glu
                85                  90                  95

Pro Lys Leu Glu Ala Leu Asp Arg Ala Gly Leu Asp His Ile Gln Leu
            100                 105                 110

Ser Phe Gln Asp Val Thr Glu Ala Gly Ala Glu Arg Ile Gly Gly Leu
        115                 120                 125

Lys Gly Ala Gln Ala Arg Lys Val Ala Ala Ala Arg Leu Ile Arg Ala
    130                 135                 140

Ser Gly Ile Pro Met Thr Leu Asn Phe Val Val His Arg Glu Asn Val
145                 150                 155                 160

Ala Arg Ile Pro Glu Met Phe Ala Leu Ala Arg Glu Leu Gly Ala Gly
                165                 170                 175

Arg Val Glu Ile Ala His Thr Gln Tyr Tyr Gly Trp Gly Leu Lys Asn
            180                 185                 190

Arg Glu Ala Leu Leu Pro Ser Arg Asp Gln Leu Glu Glu Ser Thr Arg
        195                 200                 205

Ala Val Glu Ala Glu Arg Ala Lys Gly Gly Leu Ser Val Asp Tyr Val
    210                 215                 220

Thr Pro Asp Tyr His Ala Asp Arg Pro Lys Pro Cys Met Gly Gly Trp

225                 230                 235                 240

Gly Gln Arg Phe Val Asn Val Thr Pro Ser Gly Arg Val Leu Pro Cys
                245                 250                 255

His Ala Ala Glu Ile Ile Pro Asp Val Ala Phe Pro Asn Val Gln Asp
            260                 265                 270

Val Thr Leu Ser Glu Ile Trp Asn Ile Ser Pro Leu Phe Asn Met Phe
        275                 280                 285

Arg Gly Thr Asp Trp Met Pro Glu Pro Cys Arg Ser Cys Glu Arg Lys
    290                 295                 300

Glu Arg Asp Trp Gly Gly Cys Arg Cys Gln Ala Met Ala Leu Thr Gly
305                 310                 315                 320

Asn Ala Ala Asn Thr Asp Pro Val Cys Ser Leu Ser Pro Tyr His Asp
                325                 330                 335

Arg Val Glu Gln Ala Val Glu Asn Asn Met Gln Pro Glu Ser Thr Leu
            340                 345                 350

Phe Tyr Arg Arg Tyr Thr
        355

<210>  17
<211>  4279
<212>  DNA
<213>  Artificial

<220>
<223>  Nucleotide sequence based on the pqqABCDE operon (originates from
       Kluyvera intermedia)

<400>  17
gcgcggtacc aagcttcgct gccgcaaaac agcagatgtt ccagcagatg tgccaaaccc      60

ggccagcgat ccgcttcatc cagactgccg gcctgcacgc gcatcagcgc cgccgcctca     120

cgcgcgtcgg gctgatgata aagatggcag cgcaggccgt tggcgagcgt gagctggcgc     180

gcgtgcatca ggtgacgcgc tgcttaaaag atcagttcgg agttgctgcg attgcggaac     240

tgcagctgct ggattttgat gtcgctgcag ttggcttcac gtcgtgcttc gaggatcttg     300

ccgtgatgcg gcgatttgct gcacaccgga tcggtgttat cggcatcgcc ggtcagcata     360

aaggcctgac agcgacagcc gccaaagtct ttcgcttttt catcgcagga gcgacagggt     420

tccggcatcc agtcaaagcc gcgatagcgg ttaaaaccaa acgagttgta ccagatatcg     480

tccagcgtgc gctccagcac cgacgggaac gcaaccggca gctgacgcgc gctgtggcac     540

ggcaacgcgg tgcagtcggc gtgacgctga ggaagatcga tccccatccg cccatgcagg     600

gtttcggccg ctcttcgtag taatccggcg tcacgaacag caggattggt gagattgccg 660
ctggcgccca tgcgctgacg ataatcggcc accaccgctt cggcgttggc gatctgctcg 720
cgcgtcggca gcaatccttc acgattgagt tgcgcccagc catagaattg gcaagtcgcc 780
agctcgacat cgtcggcatc cagttcgata cagagatcga tgattttgtc gatctggtcg 840
atattgtggc gatgcagcac gaaattcagc accatcggat agccgtgcgc tttcaccgct 900
ttcgccatct ccagcttctg ctggaaggcc ttttttgacc cggccagcgc cgcgttcagc 960
gtttcatcgc tggcctggaa gctgatctgg atatgatcga gtccggcgtc ggcaaaggcg 1020
tcgagttttt tcgccgtcag gccgatgccg gaggtgatca ggttggtgta gaaaccgaga 1080
tcgcgcgcgg cgcgaatcaa ctccggcagg tctttacggg ttagcggttc gccgccggaa 1140
aaaccgagct gcacgctgcc catggcgcgc gcctgacgaa acacctcaat ccactgttcg 1200
gtggtcagct ctttcttctg ctgggaaaaa tccagcggat tggagcaata tggacactgc 1260
agcgggcagc gataggtcag ttctgccagc agccacagcg gtggcgtcac gcttttactc 1320
gggttcacga cactgtatcc atttttgttc aatggcggat tgcaggaact ctttgacgtc 1380
gtcgccgaca ccgcccgcgt ccgggaaacg cgcatccagc gtggcaacga tcgccgccac 1440
atcctgtttg ccatccacca gttcgaggat cgccacggcg gtctcgttca gtttggccat 1500
gccttctgga tagagcacca catggctatc ctgcgccgct tcccactgca tgcggtagcc 1560
gcgacggaaa gccggaatca cattatcttt catgttgtta taccagtctt gtgctgtgcc 1620
aggacacctg atcggtgacc gttatctttc atgttgttat accagtcttg tgctgtgcca 1680
ggacacctga tcggtgaccg tatgataagg cggacgttgc agcgcatacg ccatggtcat 1740
ggcatcgagc atggtccaga gaatatcgag tttaaactgc aggatctcca gcatgcggtt 1800
ctgctgttcg gcgcgggtaa acacctcaag cgccagcgcc agcccatgct ccacatcgcg 1860
gttcgcctgg ctcaaacggc tgcggaagta gaaatagcct tcctctttga tccacggata 1920
gtgctgcggc cagctgtcga gacgcgactg atgaatctgc ggcgcaaaca gctcggtgag 1980
cgagctacag gccgcttcct gccagttagc acggcgcgcg aagttcacgt aggcgtccac 2040

cgcaaaacgc acgcccggca gcaccagctt ctctgacagc aacacgtcac gttccagccc 2100
caccgcctca cccaactgca gccaggcttc aatcccgcct tcgtgaccgt tgctgccgtc 2160
gtgatcgagg atgcgctgca cccatttacg ccgcgtcgcc gcatccgggc aattcgccat 2220
gatcgccgca tctttcagcg gaatgttggt ctgataatag aagcggttcg ccacccagcc 2280
ctgaatctgc tcgcgcgtcg cctcgccatt gtgcatggca atgtgatacg ggtgatgtat 2340
gtggtagaac gcgcctttat cgcgcagcgc ctgctcaaac gcggcgggcg ataatgcttc 2400
agtgatgatc atgatgcaaa ccgcgcagcg cctgctcaaa cgcggcgggc gataatgctt 2460

cagtgatgat catgatgcaa actcctgcag atgaatcgcc atgccatccc aactcacttc 2520

aatgccctgc tgcgtcaaat aggcgcgctg cggggattgc tcattaagga tcggattggt 2580

gttgttaatg tgaatcaaaa tcttgcgttt ggccggcagc gcagccagca acgccattaa 2640

accttgttct tcggccagcg ctaagtggcc catcgctttg ccagtatttt tgccaacgcc 2700

ggtggcgagc agctcgtcgt cctgccacac cgtgccatca ataacaggca atcggctctt 2760

tgcagccacg gcatggatca ccgcatccgg ctcgccgagt cccggcgcgt agagcagcgt 2820

ttggccattg ccggtggttt catgaacagc gccacgttgt ggtccggcag ggcgatcgcg 2880

atacggcgaa tacggcggcg cgttgctgag aatcggaatc gcggtgaact gcaaactgtg 2940

gcagacatcg acgcggaacg gctccagcgg cgttaacgca tgatgctgca agccgccatt 3000

ccagtgctgc aacatggtga aaatcgggaa accgctggtg agatcgccat gcacttccgg 3060

cgtgcaccac acctgatgcg gacagccttc gcgcaggctc aacaagccag tggtgtgatc 3120

gatttggctg tcggtgagaa tgatgctgcc aatcgccgtg ccgcgcagca cacccttttt 3180

gatcagttct ggcgtgtggg caatttgctg gctgatgtcc ggcgaggcgt tgcacagcac 3240

ccaatcttca ccgttgtcgc tgacaatgat tgaagattgg gtgcgcgcct gcgcctgaat 3300

actgccgtta cgcaggccgc tgcaattggc gcagttacag ttccactgcg ggaagccgcc 3360

gcccgccgct gaaccgagga ctttaataaa catgaacgtt gaccaaagag agaaaacaaa 3420

atgcccgcac gaacggcggg cagaagtctt agcggttgga gatgtacaga gtcacttcca 3480

agcccagacg caggtcaata aacgcaggtt ttttccacat aatggtcttc ctcataaaac 3540

aaaaacgcag agaaatctct gctgaagagg attgcggctg tgacttagat cacatcgcaa 3600

tccgtacgcg ccgcaggatg ttgcgccaat gtagcgagca gatcaaccct gattcggtaa 3660

ggaatggtgc taatcctgcc aaatttgttg caacacgcgc tgccagttgc cccatgcgag 3720

ctgctcaagt gctgtttgat cgtagtcgaa cgaacgcaac aacgcataaa gatgcgggta 3780

atccgctcac gtctttcaag cgcctcaggc acgctgatgc catcaaaatc ggagcccagc 3840

gccacatgat cgcggcccat tatcgcgatc aggtgctcaa catgtttaac aatttcgatc 3900

aatgcggtgt cgctgtcgcg ttttccgtcg ctgcgcagaa aagcgttgcc aaaattcacg 3960

cccaccacgc cgccgctgtc gcggatggcg cgcagctgat cgtcggtgag attgcgcggc 4020

tgcgcgcaga gcgcgtgcgc attggagtgc gtcgccacca gcggcgcggt tgacaggcgc 4080

gcggtgtccc agaaggcttt ctcgttcatg tgcgacatat cgatcagcat gcgatggcga 4140

ttggcagcgg cgatcagctg ctcaccttgt tcggtcaagc ctggcccgct gtccggtgag 4200

ttggggaatg atccgctgac gccttcgcca aagcgattgg gcagattcca gaacggcccg 4260

atgctgcgtg gatccgcgc 4279

<210>  18
<211>  23
<212>  PRT
<213>  Kluyvera intermedia

<400>  18

Met Trp Lys Lys Pro Ala Phe Ile Asp Leu Arg Leu Gly Leu Glu Val
1               5                   10                  15

Thr Leu Tyr Ile Ser Asn Arg
            20

<210>  19
<211>  307
<212>  PRT
<213>  Kluyvera intermedia

<400>  19

Met Phe Ile Lys Val Leu Gly Ser Ala Ala Gly Gly Gly Phe Pro Gln
1               5                   10                  15

Trp Asn Cys Asn Cys Ala Asn Cys Ser Gly Leu Arg Asn Gly Ser Ile
            20                  25                  30

Gln Ala Gln Ala Arg Thr Gln Ser Ser Ile Ile Val Ser Asp Asn Gly
        35                  40                  45

Glu Asp Trp Val Leu Cys Asn Ala Ser Pro Asp Ile Ser Gln Gln Ile
    50                  55                  60

Ala His Thr Pro Glu Leu Ile Lys Lys Gly Val Leu Arg Gly Thr Ala
65                  70                  75                  80

Ile Gly Ser Ile Ile Leu Thr Asp Ser Gln Ile Asp His Thr Thr Gly
                85                  90                  95

Leu Leu Ser Leu Arg Glu Gly Cys Pro His Gln Val Trp Cys Thr Pro
            100                 105                 110

Glu Val His Gly Asp Leu Thr Ser Gly Phe Pro Ile Phe Thr Met Leu
        115                 120                 125

Gln His Trp Asn Gly Gly Leu Gln His His Ala Leu Thr Pro Leu Glu
    130                 135                 140

Pro Phe Arg Val Asp Val Cys His Ser Leu Gln Phe Thr Ala Ile Pro
145                 150                 155                 160

Ile Leu Ser Asn Ala Pro Pro Tyr Ser Pro Tyr Arg Asp Arg Pro Ala
                165                 170                 175

Gly Pro Gln Arg Gly Ala Val His Glu Thr Thr Gly Asn Gly Gln Thr
            180                 185                 190

Leu Leu Tyr Ala Pro Gly Leu Gly Glu Pro Asp Ala Val Ile His Ala
        195                 200                 205

Val Ala Ala Lys Ser Arg Leu Pro Val Ile Asp Gly Thr Val Trp Gln
    210                 215                 220

Asp Asp Glu Leu Leu Ala Thr Gly Val Gly Lys Asn Thr Gly Lys Ala
225                 230                 235                 240

Met Gly His Leu Ala Leu Ala Glu Glu Gln Gly Leu Met Ala Leu Leu
                245                 250                 255

Ala Ala Leu Pro Ala Lys Arg Lys Ile Leu Ile His Ile Asn Asn Thr
            260                 265                 270

Asn Pro Ile Leu Asn Glu Gln Ser Pro Gln Arg Ala Tyr Leu Thr Gln
        275                 280                 285

Gln Gly Ile Glu Val Ser Trp Asp Gly Met Ala Ile His Leu Gln Glu
    290                 295                 300

Phe Ala Ser
305

<210>  20
<211>  251
<212>  PRT
<213>  Kluyvera intermedia

<400>  20

Met Ile Ile Thr Glu Ala Leu Ser Pro Ala Ala Phe Glu Gln Ala Leu
1               5                   10                  15

Arg Asp Lys Gly Ala Phe Tyr His Ile His His Pro Tyr His Ile Ala
            20                  25                  30

Met His Asn Gly Glu Ala Thr Arg Glu Gln Ile Gln Gly Trp Val Ala
        35                  40                  45

Asn Arg Phe Tyr Tyr Gln Thr Asn Ile Pro Leu Lys Asp Ala Ala Ile
    50                  55                  60

Met Ala Asn Cys Pro Asp Ala Ala Thr Arg Arg Lys Trp Val Gln Arg
65                  70                  75                  80

Ile Leu Asp His Asp Gly Ser Asn Gly His Glu Gly Gly Ile Glu Ala
                85                  90                  95

Trp Leu Gln Leu Gly Glu Ala Val Gly Leu Glu Arg Asp Val Leu Leu
            100                 105                 110

Ser Glu Lys Leu Val Leu Pro Gly Val Arg Phe Ala Val Asp Ala Tyr
        115                 120                 125

Val Asn Phe Ala Arg Arg Ala Asn Trp Gln Glu Ala Ala Cys Ser Ser
    130                 135                 140

Leu Thr Glu Leu Phe Ala Pro Gln Ile His Gln Ser Arg Leu Asp Ser
145                 150                 155                 160

Trp Pro Gln His Tyr Pro Trp Ile Lys Glu Glu Gly Tyr Phe Tyr Phe
                165                 170                 175

Arg Ser Arg Leu Ser Gln Ala Asn Arg Asp Val Glu His Gly Leu Ala
            180                 185                 190

Leu Ala Leu Glu Val Phe Thr Arg Ala Glu Gln Gln Asn Arg Met Leu
        195                 200                 205

Glu Ile Leu Gln Phe Lys Leu Asp Ile Leu Trp Thr Met Leu Asp Ala
    210                 215                 220

Met Thr Met Ala Tyr Ala Leu Gln Arg Pro Pro Tyr His Thr Val Thr
225                 230                 235                 240

Asp Gln Val Ser Trp His Ser Thr Arg Leu Val
                245                 250

<210>  21
<211>  92
<212>  PRT
<213>  Kluyvera intermedia

<400>  21

Met Lys Asp Asn Val Ile Pro Ala Phe Arg Arg Gly Tyr Arg Met Gln
1               5                   10                  15

Trp Glu Ala Ala Gln Asp Ser His Val Val Leu Tyr Pro Glu Gly Met
            20                  25                  30

Ala Lys Leu Asn Glu Thr Ala Val Ala Ile Leu Glu Leu Val Asp Gly
      35                  40                  45

Lys Gln Asp Val Ala Ala Ile Val Ala Thr Leu Asp Ala Arg Phe Pro
    50                  55                  60

Asp Ala Gly Gly Val Gly Asp Asp Val Lys Glu Phe Leu Gln Ser Ala
65                  70                  75                  80

Ile Glu Gln Lys Trp Ile Gln Cys Arg Glu Pro Glu
                85                  90

<210>  22
<211>  374
<212>  PRT
<213>  Kluyvera intermedia

<400>  22

Val Asn Pro Ser Lys Ser Val Thr Pro Pro Leu Trp Leu Leu Ala Glu
1               5                   10                  15

Leu Thr Tyr Arg Cys Pro Leu Gln Cys Pro Tyr Cys Ser Asn Pro Leu
            20                  25                  30

Asp Phe Ser Gln Gln Lys Lys Glu Leu Thr Thr Glu Gln Trp Ile Glu
        35                  40                  45

Val Phe Arg Gln Ala Arg Ala Met Gly Ser Val Gln Leu Gly Phe Ser
    50                  55                  60

Gly Gly Glu Pro Leu Thr Arg Lys Asp Leu Pro Glu Leu Ile Arg Ala
65                  70                  75                  80

Ala Arg Asp Leu Gly Phe Tyr Thr Asn Leu Ile Thr Ser Gly Ile Gly
                85                  90                  95

Leu Thr Ala Lys Lys Leu Asp Ala Phe Ala Asp Ala Gly Leu Asp His
            100                 105                 110

Ile Gln Ile Ser Phe Gln Ala Ser Asp Glu Thr Leu Asn Ala Ala Leu
        115                 120                 125

Ala Gly Ser Lys Lys Ala Phe Gln Gln Lys Leu Glu Met Ala Lys Ala
    130                 135                 140

Val Lys Ala His Gly Tyr Pro Met Val Leu Asn Phe Val Leu His Arg
145                 150                 155                 160

His Asn Ile Asp Gln Ile Asp Lys Ile Ile Asp Leu Cys Ile Glu Leu
                165                 170                 175

Asp Ala Asp Asp Val Glu Leu Ala Thr Cys Gln Phe Tyr Gly Trp Ala
            180                 185                 190

Gln Leu Asn Arg Glu Gly Leu Leu Pro Thr Arg Glu Gln Ile Ala Asn
        195                 200                 205

Ala Glu Ala Val Val Ala Asp Tyr Arg Gln Arg Met Gly Ala Ser Gly
    210                 215                 220

Asn Leu Thr Asn Pro Ala Val Arg Asp Ala Gly Leu Leu Arg Arg Ala
225                 230                 235                 240

Ala Glu Thr Leu His Gly Arg Met Gly Ile Asp Leu Pro Gln Arg His
                245                 250                 255

Ala Asp Cys Thr Ala Leu Pro Cys His Ser Ala Arg Gln Leu Pro Val
            260                 265                 270

Ala Phe Pro Ser Val Leu Glu Arg Thr Leu Asp Asp Ile Trp Tyr Asn
        275                 280                 285

Ser Phe Gly Phe Asn Arg Tyr Arg Gly Phe Asp Trp Met Pro Glu Pro
    290                 295                 300

Cys Arg Ser Cys Asp Glu Lys Ala Lys Asp Phe Gly Gly Cys Arg Cys
305                 310                 315                 320

Gln Ala Phe Met Leu Thr Gly Asp Ala Asp Asn Thr Asp Pro Val Cys
                325                 330                 335

Ser Lys Ser Pro His His Gly Lys Ile Leu Glu Ala Arg Arg Glu Ala
            340                 345                 350

Asn Cys Ser Asp Ile Lys Ile Gln Gln Leu Gln Phe Arg Asn Arg Ser
        355                 360                 365

Asn Ser Glu Leu Ile Phe
    370

<210>  23
<211>  1116
<212>  DNA
<213>  Escherichia coli

<400>  23

atgcatcgac aatccttttt ccttgtgccc cttatttgtc tttcttccgc tctctgggcg 60

gctcctgcaa cggtaaatgt cgaagtactg caagacaaac tcgaccatcc ctgggcactg 120

gcctttttac ccgataatca cggtatgtta atcactctgc gcggcggcga gttgcgtcac 180

tggcaagcag gaaaaggatt atctgcgccg ctttccggag ttccggacgt ttgggcgcac 240

gggcagggcg gcctgctgga cgtggtttta gcgcctgatt ttgctcagtc tcgccgcatc 300

tggttaagtt attccgaagt tggcgatgat ggcaaagccg gaactgctgt gggttatggc 360

cgcttaagtg atgatctctc aaaagtgacc gacttccgca ccgtctttcg ccagatgcca 420

aaactgtcta ccggcaacca ttttggcggg cggctggtat tcgacggtaa aggttatctt 480

tttattgctc tgggcgaaaa caatcagcgc ccgacggcgc aggatctgga taaattacag 540

ggcaaactgg tgcgtctgac cgaccagggc gaaatcccgg atgataatcc ttttataaag 600

gaatccggtg cgcgcgccga gatctggtct tatggcattc gtaatccgca aggaatggcg 660

atgaatccgt ggagtaatgc actgtggctg aatgaacatg gcccgcgcgg tggtgatgaa 720

attaatatcc cgcaaaaagg caaaaactac ggctggccgc tggcaacctg gggaatcaac 780

tattcaggct ttaagatacc ggaagcgaaa ggggagatcg tcgccgggac cgagcaacct 840

gttttttact ggaaagattc gcccgctgtg agcggcatgg ccttctataa cagcgataaa 900

ttcccccagt ggcagcaaaa attatttatt ggcgcgctga aagataaaga tgtcattgtg 960

atgagcgtca acggcgacaa agtgacagaa gatggccgta ttttaacgga cagagggcag 1020

cgaattcgtg atgttcgcac tggacccgac ggttatttat acgttctcac cgacgagtcc 1080

agtggggaat tacttaaagt tagcccacgc aattag 1116

<210> 24
<211> 371
<212> PRT
<213> Escherichia coli

<400> 24

Met His Arg Gln Ser Phe Phe Leu Val Pro Leu Ile Cys Leu Ser Ser
1               5                   10                  15

Ala Leu Trp Ala Ala Pro Ala Thr Val Asn Val Glu Val Leu Gln Asp
            20                  25                  30

Lys Leu Asp His Pro Trp Ala Leu Ala Phe Leu Pro Asp Asn His Gly
        35                  40                  45

Met Leu Ile Thr Leu Arg Gly Gly Glu Leu Arg His Trp Gln Ala Gly
    50                  55                  60

Lys Gly Leu Ser Ala Pro Leu Ser Gly Val Pro Asp Val Trp Ala His

65                  70                  75                  80

Gly Gln Gly Gly Leu Leu Asp Val Val Leu Ala Pro Asp Phe Ala Gln
                85                  90                  95

Ser Arg Arg Ile Trp Leu Ser Tyr Ser Glu Val Gly Asp Asp Gly Lys
            100                 105                 110

Ala Gly Thr Ala Val Gly Tyr Gly Arg Leu Ser Asp Asp Leu Ser Lys
        115                 120                 125

Val Thr Asp Phe Arg Thr Val Phe Arg Gln Met Pro Lys Leu Ser Thr
    130                 135                 140

Gly Asn His Phe Gly Gly Arg Leu Val Phe Asp Gly Lys Gly Tyr Leu
145                 150                 155                 160

Phe Ile Ala Leu Gly Glu Asn Asn Gln Arg Pro Thr Ala Gln Asp Leu
                165                 170                 175

Asp Lys Leu Gln Gly Lys Leu Val Arg Leu Thr Asp Gln Gly Glu Ile
            180                 185                 190

Pro Asp Asp Asn Pro Phe Ile Lys Glu Ser Gly Ala Arg Ala Glu Ile
        195                 200                 205

Trp Ser Tyr Gly Ile Arg Asn Pro Gln Gly Met Ala Met Asn Pro Trp
    210                 215                 220

Ser Asn Ala Leu Trp Leu Asn Glu His Gly Pro Arg Gly Gly Asp Glu
225                 230                 235                 240

Ile Asn Ile Pro Gln Lys Gly Lys Asn Tyr Gly Trp Pro Leu Ala Thr
                245                 250                 255

Trp Gly Ile Asn Tyr Ser Gly Phe Lys Ile Pro Glu Ala Lys Gly Glu
            260                 265                 270

Ile Val Ala Gly Thr Glu Gln Pro Val Phe Tyr Trp Lys Asp Ser Pro
        275                 280                 285

Ala Val Ser Gly Met Ala Phe Tyr Asn Ser Asp Lys Phe Pro Gln Trp
    290                 295                 300

Gln Gln Lys Leu Phe Ile Gly Ala Leu Lys Asp Lys Asp Val Ile Val
305                 310                 315                 320

Met Ser Val Asn Gly Asp Lys Val Thr Glu Asp Gly Arg Ile Leu Thr
            325                 330                 335

Asp Arg Gly Gln Arg Ile Arg Asp Val Arg Thr Gly Pro Asp Gly Tyr
        340                 345                 350

Leu Tyr Val Leu Thr Asp Glu Ser Ser Gly Glu Leu Leu Lys Val Ser
    355                 360                 365

Pro Arg Asn
    370

<210>  25
<211>  2391
<212>  DNA
<213>  Escherichia coli

<400>  25
ttacttcaca tcatccggca gcgcataagc cacaatatag tcgcccatct tcgtaccaaa      60

tgaaccgtga ccgcctgcgg agatcaccac atactgctta ccattcactt cataggtcat     120

tggcgtagcc tgaccacccg ctggtaaacg accctgccac agtttttcac cgttgctcat     180

gttgtaagcg cgcaggtagt tatctgccgt agcggcgata aacagcacgt tacccgccgt     240

ggagattggc ccgcccagca tcggcatacc catattgaac ggcaccggaa ccggcatcgg     300

gaacggcata ctgtcctgcg gcgtaccaat acgtttcttc cacaccactt cattagtttt     360

cagatccagc gccgagatat aaccccatgc tggctgttta catggcagac caaatggtga     420

gaggaacggg ttgagcgtga caccatacgg tacaccgtac tgtggctgaa tgccggattc     480

cgtacccgtg cctttggcat ctttcggctg ctccatcggg ttgccaggac cacgcgggat     540

cagtttcgaa acaaacggca gtgccattgg gttggcaatc gccacttcac gatttggatc     600

aacggaaatc ccgccccatt cgaacatccc caggttaccc gggaagacca gcgtaccctg     660

ttcagatggc ggggtgaaaa tgccttcata gcgcatctgg tggaacatca cgcggcacac     720

cagttggtca aacatggtgg ctccccacat atccgcaccg ctcaaatctt tcgtcggacg     780

gaagctcagt tcagaaaacg gttgagttgg ggttacgtaa tcgcctttcg ctgcaccttg     840

gggaaccggt ttttccggtg ccggaaccac cagttcgcca ttacgacgat cgagcacaaa     900

aatgttgccg gttttcgccg gagcgtaaat aactggcact ttctgaccat taacggtgat     960

gtccgccagc gtcggctgtg ccggaagatc catgtcccac aggtcgtggt gaacggtctg    1020

gtagctccac gccagtttcc cggtagtggc attcagcgcc agaatcgagc tggcataacg    1080

ttcctgttcc ggtgtgcggt taccgcccca gatatccggc gtggtcacgc ccatcggcag    1140

atagaccaga tccagcttcg cgtcataggc cgctggtgcc caggagtttg gcgagttaaa    1200

ggtaaaggtg tgttcgtcag acgggattgc gttcggatct ttcgcgccgg gatcaaaagc    1260

ccacagcagc tccccggtgt tgacatcaaa accacggatc acgccagacg tttcgcgggt 1320

tgagaagtta tcggtaactg aaccggccat cacgatggtt ttatcggtga taatcggtgg 1380

cgaagtcggt tcatacagac ccggtttggt gtctggcata ttgctttgca gattgagcac 1440

gcctttattg gcgaaggttt cgcacagttt gccgttttca gcgttaatcg caatcagtcg 1500

accatcattg accggaagaa tgatacgacg cgggcaatcc gccatcactt ccggcgaagc 1560

ggtttctgct ttggcttcat gataagagac accacggcag gttacgtgct ggaaagactc 1620

gttggttttc agctcaggat cgtaatgcca tttctctttg ccgctggcgg catcaagcgc 1680

aaacaggcgc tggtgagcgg tacacaggta aagggtgtcg cccactttaa tcggcgtcac 1740

ttcattggtg atttcacccg gatcgttcgg ctgcttcaca tcgccagtac ggaacaccca 1800

ggcttctttc agattatgga cgttatcggc gttaatttgt ttcagcggcg aaaagcgttg 1860

accttcctga ttacgaccat aggcaggcca gtcctgatcg gctacggggg agatagcttc 1920

agcaggtgtg gcatcggcgc ttaaggtgcc gttgatctcc tgcggatcgt taaatccggc 1980

ccaggtcagg ataccaccgc taatcagcag tgcgaccacc agtgcggcaa ctgcgccgct 2040

ggcaggaatg accaggcgac gccagacaaa cggcaggatc agccagatgc cgaagaagac 2100

cagaatgtcg ctgcgcggag tcagcgccca gaagtcgaaa ccaacttccc agacgcccca 2160

aatcatggtg ccgagcagca gggctgcgta tagccaaagc gcggcgcgtt tactgcgcca 2220

cagcatccag gcgacgccga gcatcacaag gccagcgata gggtagtacc aggagccgcc 2280

aatcgcgacc agccagcctc cgccaatgag tagatacagc ccgcaaagcg ctgcaaaaag 2340

ggctgttagc gtgacgagta atcgtcgcga gcctgtattg ttaattgcca t 2391

<210> 26
<211> 796
<212> PRT
<213> Escherichia coli

<400> 26

Met Ala Ile Asn Asn Thr Gly Ser Arg Arg Leu Leu Val Thr Leu Thr
1               5                   10                  15

Ala Leu Phe Ala Ala Leu Cys Gly Leu Tyr Leu Leu Ile Gly Gly Gly
            20                  25                  30

Trp Leu Val Ala Ile Gly Gly Ser Trp Tyr Tyr Pro Ile Ala Gly Leu
        35                  40                  45

Val Met Leu Gly Val Ala Trp Met Leu Trp Arg Ser Lys Arg Ala Ala
    50                  55                  60

Leu Trp Leu Tyr Ala Ala Leu Leu Leu Gly Thr Met Ile Trp Gly Val
65                  70                  75                  80

Trp Glu Val Gly Phe Asp Phe Trp Ala Leu Thr Pro Arg Ser Asp Ile
                85                  90                  95

Leu Val Phe Phe Gly Ile Trp Leu Ile Leu Pro Phe Val Trp Arg Arg
            100                 105                 110

Leu Val Ile Pro Ala Ser Gly Ala Val Ala Ala Leu Val Val Ala Leu
        115                 120                 125

Leu Ile Ser Gly Gly Ile Leu Thr Trp Ala Gly Phe Asn Asp Pro Gln
    130                 135                 140

Glu Ile Asn Gly Thr Leu Ser Ala Asp Ala Thr Pro Ala Glu Ala Ile
145                 150                 155                 160

Ser Pro Val Ala Asp Gln Asp Trp Pro Ala Tyr Gly Arg Asn Gln Glu
                165                 170                 175

Gly Gln Arg Phe Ser Pro Leu Lys Gln Ile Asn Ala Asp Asn Val His
            180                 185                 190

Asn Leu Lys Glu Ala Trp Val Phe Arg Thr Gly Asp Val Lys Gln Pro
        195                 200                 205

Asn Asp Pro Gly Glu Ile Thr Asn Glu Val Thr Pro Ile Lys Val Gly
    210                 215                 220

Asp Thr Leu Tyr Leu Cys Thr Ala His Gln Arg Leu Phe Ala Leu Asp
225                 230                 235                 240

Ala Ala Ser Gly Lys Glu Lys Trp His Tyr Asp Pro Glu Leu Lys Thr
                245                 250                 255

Asn Glu Ser Phe Gln His Val Thr Cys Arg Gly Val Ser Tyr His Glu
            260                 265                 270

Ala Lys Ala Glu Thr Ala Ser Pro Glu Val Met Ala Asp Cys Pro Arg
        275                 280                 285

Arg Ile Ile Leu Pro Val Asn Asp Gly Arg Leu Ile Ala Ile Asn Ala
    290                 295                 300

Glu Asn Gly Lys Leu Cys Glu Thr Phe Ala Asn Lys Gly Val Leu Asn
305                 310                 315                 320

Leu Gln Ser Asn Met Pro Asp Thr Lys Pro Gly Leu Tyr Glu Pro Thr
                325                 330                 335

Ser Pro Pro Ile Ile Thr Asp Lys Thr Ile Val Met Ala Gly Ser Val
            340                 345                 350

Thr Asp Asn Phe Ser Thr Arg Glu Thr Ser Gly Val Ile Arg Gly Phe
        355                 360                 365

Asp Val Asn Thr Gly Glu Leu Leu Trp Ala Phe Asp Pro Gly Ala Lys
    370                 375                 380

Asp Pro Asn Ala Ile Pro Ser Asp Glu His Thr Phe Thr Phe Asn Ser
385                 390                 395                 400

Pro Asn Ser Trp Ala Pro Ala Ala Tyr Asp Ala Lys Leu Asp Leu Val
                405                 410                 415

Tyr Leu Pro Met Gly Val Thr Thr Pro Asp Ile Trp Gly Gly Asn Arg
            420                 425                 430

Thr Pro Glu Gln Glu Arg Tyr Ala Ser Ser Ile Leu Ala Leu Asn Ala
        435                 440                 445

Thr Thr Gly Lys Leu Ala Trp Ser Tyr Gln Thr Val His His Asp Leu
    450                 455                 460

Trp Asp Met Asp Leu Pro Ala Gln Pro Thr Leu Ala Asp Ile Thr Val
465                 470                 475                 480

Asn Gly Gln Lys Val Pro Val Ile Tyr Ala Pro Ala Lys Thr Gly Asn
                485                 490                 495

Ile Phe Val Leu Asp Arg Arg Asn Gly Glu Leu Val Val Pro Ala Pro
            500                 505                 510

Glu Lys Pro Val Pro Gln Gly Ala Ala Lys Gly Asp Tyr Val Thr Pro
        515                 520                 525

Thr Gln Pro Phe Ser Glu Leu Ser Phe Arg Pro Thr Lys Asp Leu Ser
    530                 535                 540

Gly Ala Asp Met Trp Gly Ala Thr Met Phe Asp Gln Leu Val Cys Arg
545                 550                 555                 560

Val Met Phe His Gln Met Arg Tyr Glu Gly Ile Phe Thr Pro Pro Ser

                565                 570                 575

Glu Gln Gly Thr Leu Val Phe Pro Gly Asn Leu Gly Met Phe Glu Trp
            580                 585                 590

Gly Gly Ile Ser Val Asp Pro Asn Arg Glu Val Ala Ile Ala Asn Pro
        595                 600                 605

Met Ala Leu Pro Phe Val Ser Lys Leu Ile Pro Arg Gly Pro Gly Asn
    610                 615                 620

Pro Met Glu Gln Pro Lys Asp Ala Lys Gly Thr Gly Thr Glu Ser Gly
625                 630                 635                 640

Ile Gln Pro Gln Tyr Gly Val Pro Tyr Gly Val Thr Leu Asn Pro Phe
                645                 650                 655

Leu Ser Pro Phe Gly Leu Pro Cys Lys Gln Pro Ala Trp Gly Tyr Ile
            660                 665                 670

Ser Ala Leu Asp Leu Lys Thr Asn Glu Val Val Trp Lys Lys Arg Ile
        675                 680                 685

Gly Thr Pro Gln Asp Ser Met Pro Phe Pro Met Pro Val Pro Val Pro
    690                 695                 700

Phe Asn Met Gly Met Pro Met Leu Gly Gly Pro Ile Ser Thr Ala Gly
705                 710                 715                 720

Asn Val Leu Phe Ile Ala Ala Thr Ala Asp Asn Tyr Leu Arg Ala Tyr
                725                 730                 735

Asn Met Ser Asn Gly Glu Lys Leu Trp Gln Gly Arg Leu Pro Ala Gly
            740                 745                 750

Gly Gln Ala Thr Pro Met Thr Tyr Glu Val Asn Gly Lys Gln Tyr Val
        755                 760                 765

Val Ile Ser Ala Gly Gly His Gly Ser Phe Gly Thr Lys Met Gly Asp
    770                 775                 780

Tyr Ile Val Ala Tyr Ala Leu Pro Asp Asp Val Lys
785                 790                 795

<210> 27
<211> 2427
<212> DNA

<213>  Gluconobacter oxydans

<400>  27
atgagcacaa tttcccggcc caggctctgg gccctgataa cggccgcggt attcgcgctt      60

tgcggcgcga tccttaccgt tggcggcgca tgggtcgctg ccatcggcgg ccctctttat     120

tatgtcatcc ttggcctggc acttctcgcc acggctttcc tctcattccg gcgcaatccg     180

gctgccctct atctgttcgc agtcgtcgtc ttcggaacgg tcatctggga actcaccgtt     240

gtcggtctcg acatctgggc cctgatcccg cgctcggaca tcgtcatcat cctcggcatc     300

tggctgctgc tgccgttcgt ctcgcgcgcc agatcggtgg cacgcggacg accgtcctgc     360

cgctcgccgg ccgttggcgt tgcggttctg gccctgttcg ccagcctctt caccgacccg     420

catgacatca gcggcgaact gccgacgcag atcgcaaacg cctcccccgc cgacccggac     480

aacgttccgg ccagcgagtg gcacgcttat ggtcgtacgc aggccggtga ccgctggtcc     540

ccgctgaacc agatcaatgc gacgaacgtc agcaacctca aggtcgcatg gcatatccac     600

accaaggata tgatgaactc caacgacccg ggcgaagcga cgaacgaagc gacgccgatc     660

gagttcaaca acacgcttta tatgtgctca ctgcatcaga agctgtttgc ggttgatggt     720

gccaccggca acgtcaagtg ggtctacgat ccgaagctcc agatcaaccc tggcttccag     780

catctgacct gccgtggcgt cagcttccac gaaacgccgg ccaatgccat ggattccgat     840

ggcaatcctg ctccgacgga ctgcgccaag gactccatcc tgccggtcaa tgatggccgt     900

ctggttgaag tcgatgccga cacgggcaag acctgctccg gcttcggcaa caatggcgag     960

atcgacctgc gcgttccgaa ccagccttac acgacgcctg gccagtacga gccgacgtcc    1020

ccgccggtca tcacagacaa gctgatcatc gccaacagcg ccatcaccga taacggttcg    1080

gtcaagcagg cttcgggcgc cacgcaggca ttcgacgtct acaccggcaa gcgcgtctgg    1140

gtgttcgatg cgtccaaccc ggatccgaac cagcttccgg atgagagcca ccctgtcttc    1200

cacccgaact cgccaaactc ctggatcgtg tcgtcctacg acgccaacct gaacctcgtg    1260

tacatcccga tgggcgtggg gactcccgac cagtggggcg gtgaccgcac gaaggattcc    1320

gagcgtttcg ctccgggtat cgttgcgctg aacgccgata cgggcaagct cgcctggttc    1380

taccagaccg ttcatcacga tctgtgggac atggagcttc cgtcccagcc gagcctcgtg    1440

gatgtgacac agaaggacgg cacgcttgtt ccggccatct acgctccgac caagaccggc    1500

gacattttcg tcctcgaccg tcgtaccggc aaggaaatcg tcccggctcc ggaaaccccg    1560

gttccccagg gtgctgctcc gggtgaccac accagcccga cccagccgat gtcgcagctg    1620

accctgcgtc cgaagaaccc gctgaacgac tccgatatct ggggcggcac gatcttcgac    1680

cagatgttct gcagcatcta tttccacacc ctccgctacg aaggcccctt cacgccgccg    1740

tcgctcaagg gctcgctcat cttcccgggt gatctgggaa tgttcgaatg gggtggtctg     1800

gccgtcgatc cgcagcgtca ggtggctttc gccaacccga tttccctgcc gttcgtctct    1860

cagcttgttc cccgcggacc gggcaacccg ctctggcctg aagaaaatgc caagggcacg    1920

ggtggtgaaa ccggcctgca gcacaactat ggcatcccgt atgccgtcaa cctgcatccg    1980

ttcctggatc cggtgctgct gccgttcggc atcaagatgc cgtgccgcac gccgccctgg    2040

ggctatgtcg ccggtattga cctgaagacc aacaaggtcg tctggcagca ccgcaacggc    2100

accctgcgtg actcgatgta tggcagctcc ctgccgatcc cgctgccgcc gatcaagatc    2160

ggtgtcccga gcctcggtgg cccgctctcc acggctggca atctcggctt cctgacggcg    2220

tccatggatt actacatccg tgcgtacaac ctgacgacgg gcaaggtgct gtggcaggac    2280

cgtctgccgg ctggtgctca ggcaacgccg atcacctatg ccatcaacgg caagcagtac    2340

atcgtgacct atgcaggcgg acacaactcg ttcccgaccc gcatgggcga cgacatcatc    2400

gcctacgccc tgcccgatca gaaatga                                        2427

<210>  28
<211>  808
<212>  PRT
<213>  Gluconobacter oxydans

<400>  28

Met Ser Thr Thr Ser Arg Pro Gly Leu Trp Ala Leu Ile Thr Ala Ala
1               5                   10                  15

Val Phe Ala Leu Cys Gly Ala Ile Leu Thr Val Gly Gly Ala Trp Val
            20                  25                  30

Ala Ala Ile Gly Gly Pro Leu Tyr Tyr Val Ile Leu Gly Leu Ala Leu
        35                  40                  45

Leu Ala Thr Ala Phe Leu Ser Phe Arg Arg Asn Pro Ala Ala Leu Tyr
    50                  55                  60

Leu Phe Ala Val Val Val Phe Gly Thr Val Ile Trp Glu Leu Thr Val
65                  70                  75                  80

Val Gly Leu Asp Ile Trp Ala Leu Ile Pro Arg Ser Asp Ile Val Ile
                85                  90                  95

Ile Leu Gly Ile Trp Leu Leu Leu Pro Phe Val Ser Arg Gln Ile Gly
            100                 105                 110

Gly Thr Arg Thr Thr Val Leu Pro Leu Ala Gly Ala Val Gly Val Ala
        115                 120                 125

Val Leu Ala Leu Phe Ala Ser Leu Phe Thr Asp Pro His Asp Ile Ser

130                 135                 140

Gly Asp Leu Pro Thr Gln Ile Ala Asn Ala Ser Pro Ala Asp Pro Asp
145                 150                 155                 160

Asn Val Pro Ala Ser Glu Trp His Ala Tyr Gly Arg Thr Gln Ala Gly
                165                 170                 175

Asp Arg Trp Ser Pro Leu Asn Gln Ile Asn Ala Ser Asn Val Ser Asn
            180                 185                 190

Leu Lys Val Ala Trp His Ile His Thr Lys Asp Met Met Asn Ser Asn
        195                 200                 205

Asp Pro Gly Glu Ala Thr Asn Glu Ala Thr Pro Ile Glu Phe Asn Asn
    210                 215                 220

Thr Leu Tyr Met Cys Ser Leu His Gln Lys Leu Phe Ala Val Asp Gly
225                 230                 235                 240

Ala Thr Gly Asn Val Lys Trp Val Tyr Asp Pro Lys Leu Gln Ile Asn
                245                 250                 255

Pro Gly Phe Gln His Leu Thr Cys Arg Gly Val Ser Phe His Glu Thr
            260                 265                 270

Pro Ala Asn Ala Thr Asp Ser Asp Gly Asn Pro Ala Pro Thr Asp Cys
        275                 280                 285

Ala Lys Arg Ile Ile Leu Pro Val Asn Asp Gly Arg Leu Val Glu Val
    290                 295                 300

Asp Ala Asp Thr Gly Lys Thr Cys Ser Gly Phe Gly Asn Asn Gly Glu
305                 310                 315                 320

Ile Asp Leu Arg Val Pro Asn Gln Pro Tyr Thr Thr Pro Gly Gln Tyr
                325                 330                 335

Glu Pro Thr Ser Pro Pro Val Ile Thr Asp Lys Leu Ile Ile Ala Asn
            340                 345                 350

Ser Ala Ile Thr Asp Asn Gly Ser Val Lys Gln Ala Ser Gly Ala Thr
        355                 360                 365

Gln Ala Phe Asp Val Tyr Thr Gly Lys Arg Val Trp Val Phe Asp Ala
    370                 375                 380

Ser Asn Pro Asp Pro Asn Gln Leu Pro Asp Asp Ser His Pro Val Phe
385                 390                 395                 400

His Pro Asn Ser Pro Asn Ser Trp Ile Val Ser Ser Tyr Asp Arg Asn
                405                 410                 415

Leu Asn Leu Val Tyr Ile Pro Met Gly Val Gly Thr Pro Asp Gln Trp
            420                 425                 430

Gly Gly Asp Arg Thr Lys Asp Ser Glu Arg Phe Ala Pro Gly Ile Val
        435                 440                 445

Ala Leu Asn Ala Asp Thr Gly Lys Leu Ala Trp Phe Tyr Gln Thr Val
    450                 455                 460

His His Asp Leu Trp Asp Met Asp Val Pro Ser Gln Pro Ser Leu Val
465                 470                 475                 480

Asp Val Thr Gln Lys Asp Gly Thr Leu Val Pro Ala Ile Tyr Ala Pro
                485                 490                 495

Thr Lys Thr Gly Asp Ile Phe Val Leu Asp Arg Arg Thr Gly Lys Glu
            500                 505                 510

Ile Val Pro Ala Pro Glu Thr Pro Val Pro Gln Gly Ala Ala Pro Gly
        515                 520                 525

Asp His Thr Ser Pro Thr Gln Pro Met Ser Gln Leu Thr Leu Arg Pro
    530                 535                 540

Lys Asn Pro Leu Asn Asp Ser Asp Ile Trp Gly Gly Thr Ile Phe Asp
545                 550                 555                 560

Gln Met Phe Cys Ser Ile Tyr Phe His Thr Leu Arg Tyr Glu Gly Pro
                565                 570                 575

Phe Thr Pro Pro Ser Leu Lys Gly Ser Leu Ile Phe Pro Gly Asp Leu
            580                 585                 590

Gly Met Phe Glu Trp Gly Gly Leu Ala Val Asp Pro Gln Arg Gln Val
        595                 600                 605

Ala Phe Ala Asn Pro Ile Ser Leu Pro Phe Val Ser Gln Leu Val Pro
    610                 615                 620

Arg Gly Pro Gly Asn Pro Leu Trp Pro Glu Lys Asp Ala Lys Gly Thr
625                 630                 635                 640

Gly Gly Glu Thr Gly Leu Gln His Asn Tyr Gly Ile Pro Tyr Ala Val
                645                 650                 655

Asn Leu His Pro Phe Leu Asp Pro Val Leu Leu Pro Phe Gly Ile Lys
            660                 665                 670

Met Pro Cys Arg Thr Pro Pro Trp Gly Tyr Val Ala Gly Ile Asp Leu
        675                 680                 685

Lys Thr Asn Lys Val Val Trp Gln His Arg Asn Gly Thr Leu Arg Asp
    690                 695                 700

Ser Met Tyr Gly Ser Ser Leu Pro Ile Pro Leu Pro Pro Ile Lys Ile
705                 710                 715                 720

Gly Val Pro Ser Leu Gly Gly Pro Leu Ser Thr Ala Gly Asn Leu Gly
                725                 730                 735

Phe Leu Thr Ala Ser Met Asp Tyr Tyr Ile Arg Ala Tyr Asn Leu Thr
            740                 745                 750

Thr Gly Lys Val Leu Trp Gln Asp Arg Leu Pro Ala Gly Ala Gln Ala
        755                 760                 765

Thr Pro Ile Thr Tyr Ala Ile Asn Gly Lys Gln Tyr Ile Val Thr Tyr
    770                 775                 780

Ala Gly Gly His Asn Ser Phe Pro Thr Arg Met Gly Asp Asp Ile Ile
785                 790                 795                 800

Ala Tyr Ala Leu Pro Asp Gln Lys
                805

<210>  29
<211>  2406
<212>  DNA
<213>  Acinetobacter calcoaceticus

<400>  29
atgaatcaac ctacttcaag atcaggttta acgactttta ccgtaattat tattgggtta      60

ctggcgttat tcctgttaat tggaggtatt tggctcgcta cactaggcgg ttcaatttac     120

tacattatag ctggagtatt actcctcatt gttgcatggc aactctacaa gcgtgcttct     180

actgctttgt ggttttatgc tgcattaatg ctaggtacca ttatctggag tgtctgggaa     240

gttggaacag acttttgggc gcttgcacca cgtttagata ttttaggtat tcttggttta     300

tggttattgg ttccggctgt aactcgtgga atcaacaacc ttggatcaag taaagttgcc     360

ttatcttcaa ctttagcaat tgcaatcgtg ttgatggttt attctatctt caatgatccg 420
caagaaatta atggtgaaat caaaacacct caaccagaaa cagctcaagc cgtgcctggt 480
gttgctgaaa gtgattggcc agcttatggt cgtactcaag caggcgtgcg ttattctcca 540
ttgaaacaga tcaatgatca aaacgtaaaa gacttgaaag ttgcttggac tttacgtact 600
ggcgatctta agacagataa cgactctggc gaaacgacta atcaggttac accgattaaa 660
atcggtaata acatgtttat ctgtacagct caccagcagt taattgctat tgatcctgct 720
acaggtaaag aaaaatggcg ttttgatccg aaacttaaaa cggataaatc gttccagcat 780
ctaacttgtc gtggtgtgat gtactacgat gcaaacaata caactgagtt tgcaacgagt 840
cttcaaagca agaaatctag ctctacacaa tgtccacgta aggtatttgt accagtcaac 900
gatggccgtt tagtggctgt aaatgctgac actggtaaag catgtactga ctttggtcaa 960
aatggtcaag tgaacttaca agagttcatg ccatatgctt atccaggcgg ttataacccg 1020
acatctcctg gtatcgtgac tggttcaact gttgttattg ctggttctgt aacagataac 1080
tactcaaata aagagccatc tggtgtgatt cgtggatacg acgttaacac tggtaaactt 1140
ctttgggtat ttgacactgg cgcagcggat ccaaatgcaa tgccgggcga aggtactact 1200
tttgttcaca actcaccaaa tgcgtgggca cctttagcat acgatgccaa acttgacatc 1260
gtttatgtac caacaggtgt aggtacacca gacatctggg gtggtgaccg taccgagctg 1320
aaagagcgtt atgcaaactc aatgttagcg attaatgctt ctactggtaa attagtgtgg 1380
aacttccaga caacccatca cgatttatgg gatatggatg taccatcaca accatcttta 1440
gctgatatca aaaacaaagc tggccaaact gttcctgcaa tctatgtatt gacgaaaaca 1500
ggtaatgcct ttgtgcttga tcgccgtaat ggtcaaccga ttgttcctgt aactgagaaa 1560
ccagttccac aaacagttaa acgtggacca caaactaaag gtgagttcta ttcaaaaact 1620
cagccattct ctgacttgaa cttggcgcca caagataaat tgactgataa agacatgtgg 1680
ggtgccacta tgcttgatca gctcatgtgt cgtgtatctt tcaaacgtct aaattacgat 1740
ggtatttata cgccaccatc tgaaaacggt actttagttt tccctggtaa cttaggtgta 1800
tttgaatggg gcggtatgtc agttaaccct gatcgtcagg ttgctgtaat gaacccgatt 1860
ggtctgccat tcgtcagtcg tttaattcct gctgatccaa accgtgcaca aactgcaaaa 1920
ggtgcgggaa ctgagcaggg cgtacaaccg atgtacggcg taccatatgg tgttgaaatt 1980
agcgcattct tatctccgct tggtttacct tgtaaacaac cggcttgggg ctatgtagct 2040
ggcgttgatt tgaaaactca tgaagtggta tggaaaaaac gtattggtac aatccgtgac 2100
agtttaccga acttgttcca gttgccagct gtgaaaattg gtgtgcctgg tttaggtggt 2160
tcaatttcta ctgccggtaa tgtcatgttt gttggtgcaa ctcaagataa ctacttacgt 2220

gcgtttaacg ttactaacgg taagaaactt tgggaagcgc gtttaccagc aggtggacaa 2280

gcaacaccaa tgacttatga aatcaatggt aagcaatatg ttgtaatcat ggctggtggt 2340

catggttcat ttggtacgaa aatgggcgac tatttagtgg cttatgcctt accagataac 2400

aaataa 2406

<210> 30
<211> 801
<212> PRT
<213> Acinetobacter calcoaceticus

<400> 30

Met Asn Gln Pro Thr Ser Arg Ser Gly Leu Thr Thr Phe Thr Val Ile
1               5                   10                  15

Ile Ile Gly Leu Leu Ala Leu Phe Leu Leu Ile Gly Gly Ile Trp Leu
            20                  25                  30

Ala Thr Leu Gly Gly Ser Ile Tyr Tyr Ile Ile Ala Gly Val Leu Leu
        35                  40                  45

Leu Ile Val Ala Trp Gln Leu Tyr Lys Arg Ala Ser Thr Ala Leu Trp
    50                  55                  60

Phe Tyr Ala Ala Leu Met Leu Gly Thr Ile Ile Trp Ser Val Trp Glu
65                  70                  75                  80

Val Gly Thr Asp Phe Trp Ala Leu Ala Pro Arg Leu Asp Ile Leu Gly
                85                  90                  95

Ile Leu Gly Leu Trp Leu Leu Val Pro Ala Val Thr Arg Gly Ile Asn
            100                 105                 110

Asn Leu Gly Ser Ser Lys Val Ala Leu Ser Ser Thr Leu Ala Ile Ala
        115                 120                 125

Ile Val Leu Met Val Tyr Ser Ile Phe Asn Asp Pro Gln Glu Ile Asn
    130                 135                 140

Gly Glu Ile Lys Thr Pro Gln Pro Glu Thr Ala Gln Ala Val Pro Gly
145                 150                 155                 160

Val Ala Glu Ser Asp Trp Pro Ala Tyr Gly Arg Thr Gln Ala Gly Val
                165                 170                 175

Arg Tyr Ser Pro Leu Lys Gln Ile Asn Asp Gln Asn Val Lys Asp Leu
            180                 185                 190

Lys Val Ala Trp Thr Leu Arg Thr Gly Asp Leu Lys Thr Asp Asn Asp
      195                 200                 205

Ser Gly Glu Thr Thr Asn Gln Val Thr Pro Ile Lys Ile Gly Asn Asn
    210                 215                 220

Met Phe Ile Cys Thr Ala His Gln Gln Leu Ile Ala Ile Asp Pro Ala
225                 230                 235                 240

Thr Gly Lys Glu Lys Trp Arg Phe Asp Pro Lys Leu Lys Thr Asp Lys
                245                 250                 255

Ser Phe Gln His Leu Thr Cys Arg Gly Val Met Tyr Tyr Asp Ala Asn
            260                 265                 270

Asn Thr Thr Glu Phe Ala Thr Ser Leu Gln Ser Lys Lys Ser Ser Ser
      275                 280                 285

Thr Gln Cys Pro Arg Lys Val Phe Val Pro Val Asn Asp Gly Arg Leu
    290                 295                 300

Val Ala Val Asn Ala Asp Thr Gly Lys Ala Cys Thr Asp Phe Gly Gln
305                 310                 315                 320

Asn Gly Gln Val Asn Leu Gln Glu Phe Met Pro Tyr Ala Tyr Pro Gly
                325                 330                 335

Gly Tyr Asn Pro Thr Ser Pro Gly Ile Val Thr Gly Ser Thr Val Val
            340                 345                 350

Ile Ala Gly Ser Val Thr Asp Asn Tyr Ser Asn Lys Glu Pro Ser Gly
      355                 360                 365

Val Ile Arg Gly Tyr Asp Val Asn Thr Gly Lys Leu Leu Trp Val Phe
    370                 375                 380

Asp Thr Gly Ala Ala Asp Pro Asn Ala Met Pro Gly Glu Gly Thr Thr
385                 390                 395                 400

Phe Val His Asn Ser Pro Asn Ala Trp Ala Pro Leu Ala Tyr Asp Ala
                405                 410                 415

Lys Leu Asp Ile Val Tyr Val Pro Thr Gly Val Gly Thr Pro Asp Ile
            420                 425                 430

Trp Gly Gly Asp Arg Thr Glu Leu Lys Glu Arg Tyr Ala Asn Ser Met

435                 440                 445

Leu Ala Ile Asn Ala Ser Thr Gly Lys Leu Val Trp Asn Phe Gln Thr
    450                 455                 460

Thr His His Asp Leu Trp Asp Met Asp Val Pro Ser Gln Pro Ser Leu
465                 470                 475                 480

Ala Asp Ile Lys Asn Lys Ala Gly Gln Thr Val Pro Ala Ile Tyr Val
                485                 490                 495

Leu Thr Lys Thr Gly Asn Ala Phe Val Leu Asp Arg Arg Asn Gly Gln
            500                 505                 510

Pro Ile Val Pro Val Thr Glu Lys Pro Val Pro Gln Thr Val Lys Arg
        515                 520                 525

Gly Pro Gln Thr Lys Gly Glu Phe Tyr Ser Lys Thr Gln Pro Phe Ser
    530                 535                 540

Asp Leu Asn Leu Ala Pro Gln Asp Lys Leu Thr Asp Lys Asp Met Trp
545                 550                 555                 560

Gly Ala Thr Met Leu Asp Gln Leu Met Cys Arg Val Ser Phe Lys Arg
                565                 570                 575

Leu Asn Tyr Asp Gly Ile Tyr Thr Pro Pro Ser Glu Asn Gly Thr Leu
            580                 585                 590

Val Phe Pro Gly Asn Leu Gly Val Phe Glu Trp Gly Gly Met Ser Val
        595                 600                 605

Asn Pro Asp Arg Gln Val Ala Val Met Asn Pro Ile Gly Leu Pro Phe
    610                 615                 620

Val Ser Arg Leu Ile Pro Ala Asp Pro Asn Arg Ala Gln Thr Ala Lys
625                 630                 635                 640

Gly Ala Gly Thr Glu Gln Gly Val Gln Pro Met Tyr Gly Val Pro Tyr
                645                 650                 655

Gly Val Glu Ile Ser Ala Phe Leu Ser Pro Leu Gly Leu Pro Cys Lys
            660                 665                 670

Gln Pro Ala Trp Gly Tyr Val Ala Gly Val Asp Leu Lys Thr His Glu
        675                 680                 685

Val Val Trp Lys Lys Arg Ile Gly Thr Ile Arg Asp Ser Leu Pro Asn
    690                 695                 700

Leu Phe Gln Leu Pro Ala Val Lys Ile Gly Val Pro Gly Leu Gly Gly
705                 710                 715                 720

Ser Ile Ser Thr Ala Gly Asn Val Met Phe Val Gly Ala Thr Gln Asp
                725                 730                 735

Asn Tyr Leu Arg Ala Phe Asn Val Thr Asn Gly Lys Lys Leu Trp Glu
            740                 745                 750

Ala Arg Leu Pro Ala Gly Gly Gln Ala Thr Pro Met Thr Tyr Glu Ile
        755                 760                 765

Asn Gly Lys Gln Tyr Val Val Ile Met Ala Gly Gly His Gly Ser Phe
    770                 775                 780

Gly Thr Lys Met Gly Asp Tyr Leu Val Ala Tyr Ala Leu Pro Asp Asn
785                 790                 795                 800

Lys

<210>  31
<211>  1437
<212>  DNA
<213>  Acinetobacter calcoaceticus

<400>  31
atgaataaac atttattggc taaaattgct ttattaagcg ctgttcagct agttacactc      60

tcagcatttg ctgatgttcc tctaactcca tctcaatttg ctaaagcgaa atcagagaac     120

tttgacaaga aagttattct atctaatcta aataagccgc atgctttgtt atggggacca     180

gataatcaaa tttggttaac tgagcgagca acaggtaaga ttctaagagt taatccagag     240

tcgggtagtg taaaaacagt ttttcaggta ccagagattg tcaatgatgc tgatgggcag     300

aatggtttat taggttttgc cttccatcct gattttaaaa ataatcctta tatctatatt     360

tcaggtacat ttaaaaatcc gaaatctaca gataaagaat taccgaacca aacgattatt     420

cgtcgttata cctataataa atcaacagat acgctcgaga agccagtcga tttattagca     480

ggattacctt catcaaaaga ccatcagtca ggtcgtcttg tcattgggcc agatcaaaag     540

atttattata cgattggtga ccaagggcgt aaccagcttg cttatttgtt cttgccaaat     600

caagcacaac atacgccaac tcaacaagaa ctgaatggta aagactatca cacctatatg     660

ggtaaagtac tacgcttaaa tcttgatgga agtattccaa aggataatcc aagttttaac     720

ggggtggtta gccatattta tacacttgga catcgtaatc cgcagggctt agcattcact 780

ccaaatggta aattattgca gtctgaacaa ggcccaaact ctgacgatga aattaacctc 840

attgtcaaag gtggcaatta tggttggccg aatgtagcag gttataaaga tgatagtggc 900

tatgcttatg caaattattc agcagcagcc aataagtcaa ttaaggattt agctcaaaat 960

ggagtaaaag tagccgcagg ggtccctgtg acgaaagaat ctgaatggac tggtaaaaac 1020

tttgtcccac cattaaaaac tttatatacc gttcaagata cctacaacta taacgatcca 1080

acttgtggag agatgaccta catttgctgg ccaacagttg caccgtcatc tgcctatgtc 1140

tataagggcg gtaaaaaagc aattactggt tgggaaaata cattattggt tccatcttta 1200

aaacgtggtg tcattttccg tattaagtta gatccaactt atagcactac ttatgatgac 1260

gctgtaccga tgtttaagag caacaaccgt tatcgtgatg tgattgcaag tccagatggg 1320

aatgtcttat atgtattaac tgatactgcc ggaaatgtcc aaaaagatga tggctcagta 1380

acaaatacat tagaaaaccc aggatctctc attaagttca cctataaggc taagtaa 1437

<210> 32
<211> 478
<212> PRT
<213> Acinetobacter calcoaceticus

<400> 32

Met Asn Lys His Leu Leu Ala Lys Ile Ala Leu Leu Ser Ala Val Gln
1               5                   10                  15

Leu Val Thr Leu Ser Ala Phe Ala Asp Val Pro Leu Thr Pro Ser Gln
            20                  25                  30

Phe Ala Lys Ala Lys Ser Glu Asn Phe Asp Lys Lys Val Ile Leu Ser
        35                  40                  45

Asn Leu Asn Lys Pro His Ala Leu Leu Trp Gly Pro Asp Asn Gln Ile
    50                  55                  60

Trp Leu Thr Glu Arg Ala Thr Gly Lys Ile Leu Arg Val Asn Pro Glu
65                  70                  75                  80

Ser Gly Ser Val Lys Thr Val Phe Gln Val Pro Glu Ile Val Asn Asp
                85                  90                  95

Ala Asp Gly Gln Asn Gly Leu Leu Gly Phe Ala Phe His Pro Asp Phe
            100                 105                 110

Lys Asn Asn Pro Tyr Ile Tyr Ile Ser Gly Thr Phe Lys Asn Pro Lys
        115                 120                 125

Ser Thr Asp Lys Glu Leu Pro Asn Gln Thr Ile Ile Arg Arg Tyr Thr
    130                 135                 140

Tyr Asn Lys Ser Thr Asp Thr Leu Glu Lys Pro Val Asp Leu Leu Ala
145                 150                 155                 160

Gly Leu Pro Ser Ser Lys Asp His Gln Ser Gly Arg Leu Val Ile Gly
                165                 170                 175

Pro Asp Gln Lys Ile Tyr Tyr Thr Ile Gly Asp Gln Gly Arg Asn Gln
            180                 185                 190

Leu Ala Tyr Leu Phe Leu Pro Asn Gln Ala Gln His Thr Pro Thr Gln
        195                 200                 205

Gln Glu Leu Asn Gly Lys Asp Tyr His Thr Tyr Met Gly Lys Val Leu
    210                 215                 220

Arg Leu Asn Leu Asp Gly Ser Ile Pro Lys Asp Asn Pro Ser Phe Asn
225                 230                 235                 240

Gly Val Val Ser His Ile Tyr Thr Leu Gly His Arg Asn Pro Gln Gly
                245                 250                 255

Leu Ala Phe Thr Pro Asn Gly Lys Leu Leu Gln Ser Glu Gln Gly Pro
            260                 265                 270

Asn Ser Asp Asp Glu Ile Asn Leu Ile Val Lys Gly Gly Asn Tyr Gly
        275                 280                 285

Trp Pro Asn Val Ala Gly Tyr Lys Asp Asp Ser Gly Tyr Ala Tyr Ala
    290                 295                 300

Asn Tyr Ser Ala Ala Ala Asn Lys Ser Ile Lys Asp Leu Ala Gln Asn
305                 310                 315                 320

Gly Val Lys Val Ala Ala Gly Val Pro Val Thr Lys Glu Ser Glu Trp
                325                 330                 335

Thr Gly Lys Asn Phe Val Pro Pro Leu Lys Thr Leu Tyr Thr Val Gln
            340                 345                 350

Asp Thr Tyr Asn Tyr Asn Asp Pro Thr Cys Gly Glu Met Thr Tyr Ile
        355                 360                 365

Cys Trp Pro Thr Val Ala Pro Ser Ser Ala Tyr Val Tyr Lys Gly Gly

370                 375                 380

Lys Lys Ala Ile Thr Gly Trp Glu Asn Thr Leu Leu Val Pro Ser Leu
385                 390                 395                 400

Lys Arg Gly Val Ile Phe Arg Ile Lys Leu Asp Pro Thr Tyr Ser Thr
                405                 410                 415

Thr Tyr Asp Asp Ala Val Pro Met Phe Lys Ser Asn Asn Arg Tyr Arg
            420                 425                 430

Asp Val Ile Ala Ser Pro Asp Gly Asn Val Leu Tyr Val Leu Thr Asp
        435                 440                 445

Thr Ala Gly Asn Val Gln Lys Asp Asp Gly Ser Val Thr Asn Thr Leu
    450                 455                 460

Glu Asn Pro Gly Ser Leu Ile Lys Phe Thr Tyr Lys Ala Lys
465                 470                 475

<210>  33
<211>  1059
<212>  DNA
<213>  Vibrio parahaemolyticus

<400>  33
ttggctcttg gcgcgattgg agcagtgctc tcaacttccg tatacgcttg gcaagccgag      60

cttgttgcgt ctggcctcaa ggtaccatgg gggatgagct ctattgacga caaccaattg     120

cttgtcacac aacgccacgg cgaaataggt gtggttaacc ttgatgatgg cttctatcgt     180

actgtcgcta caccggcaca ggtgagtgct gttggtcagg gaggcttgct tgatgttgcc     240

aagtctcctt tcgaagacaa tacgttctat tttacctatt caaagcaagt ggaagatgca     300

tacgaaacgg cattagcttc cgctcaatat caacaaggta agctgattgg atggaaagag     360

cttctcgtga ctcagtctgg atctgatacg ggcaggcatt ttggtagccg aatcaccttt     420

gacgaacgct acgtgtatat gtctgtcggc gatagaggcg ttcgcgacaa cgggcaaaat     480

cagagaacac acgctggctc gatcctgcgc ctattaccaa acgggctagc cccagaagac     540

aacccgttta ccaaagtaaa ctcagcgtta aacgagatat ggagctatgg tcaccgcaat     600

cctcaaggac tcttttacga ccgcagcact caacagctat ggtctatcga gcatggtccg     660

cgcggcggag atgaaatcaa cttgattgtc aaaggagaaa actatggctg gcctgttacc     720

tcccacggca aagagtattg ggggccaatc tcagttggtg aagccaaaac cctgccgggt     780

attcaaacac cgaagaaagt gtatgtgcct tctatcgcac ctagctctct cattctttac     840

agaggggata agtacccatc cctaaatggc aaactgatat ctggcgcatt gaagctcacc     900

cacctcaatg tcgtcacact cgatgagcaa gcaaatattg ttgctgaaga gcggctacta     960

gaatccttag gagagcgaat acgagatctg gaagtaacac cagtaggaga gattctgttt    1020

agcactgaca gtgggaatat ctaccgtctc gtggagtaa                           1059

<210>  34
<211>  352
<212>  PRT
<213>  Vibrio parahaemolyticus

<400>  34

Met Ala Leu Gly Ala Ile Gly Ala Val Leu Ser Thr Ser Val Tyr Ala
1               5                   10                  15

Trp Gln Ala Glu Leu Val Ala Ser Gly Leu Lys Val Pro Trp Gly Met
            20                  25                  30

Ser Ser Ile Asp Asp Asn Gln Leu Leu Val Thr Gln Arg His Gly Glu
        35                  40                  45

Ile Gly Val Val Asn Leu Asp Asp Gly Phe Tyr Arg Thr Val Ala Thr
    50                  55                  60

Pro Ala Gln Val Ser Ala Val Gly Gln Gly Gly Leu Leu Asp Val Ala
65                  70                  75                  80

Lys Ser Pro Phe Glu Asp Asn Thr Phe Tyr Phe Thr Tyr Ser Lys Gln
                85                  90                  95

Val Glu Asp Ala Tyr Glu Thr Ala Leu Ala Ser Ala Gln Tyr Gln Gln
            100                 105                 110

Gly Lys Leu Ile Gly Trp Lys Glu Leu Leu Val Thr Gln Ser Gly Ser
        115                 120                 125

Asp Thr Gly Arg His Phe Gly Ser Arg Ile Thr Phe Asp Glu Arg Tyr
    130                 135                 140

Val Tyr Met Ser Val Gly Asp Arg Gly Val Arg Asp Asn Gly Gln Asn
145                 150                 155                 160

Gln Arg Thr His Ala Gly Ser Ile Leu Arg Leu Leu Pro Asn Gly Leu
                165                 170                 175

Ala Pro Glu Asp Asn Pro Phe Thr Lys Val Asn Ser Ala Leu Asn Glu
            180                 185                 190

Ile Trp Ser Tyr Gly His Arg Asn Pro Gln Gly Leu Phe Tyr Asp Arg

195                 200                 205

Ser Thr Gln Gln Leu Trp Ser Ile Glu His Gly Pro Arg Gly Gly Asp
    210                 215                 220

Glu Ile Asn Leu Ile Val Lys Gly Glu Asn Tyr Gly Trp Pro Val Thr
225                 230                 235                 240

Ser His Gly Lys Glu Tyr Trp Gly Pro Ile Ser Val Gly Glu Ala Lys
                245                 250                 255

Thr Leu Pro Gly Ile Gln Thr Pro Lys Lys Val Tyr Val Pro Ser Ile
            260                 265                 270

Ala Pro Ser Ser Leu Ile Leu Tyr Arg Gly Asp Lys Tyr Pro Ser Leu
        275                 280                 285

Asn Gly Lys Leu Ile Ser Gly Ala Leu Lys Leu Thr His Leu Asn Val
    290                 295                 300

Val Thr Leu Asp Glu Gln Ala Asn Ile Val Ala Glu Glu Arg Leu Leu
305                 310                 315                 320

Glu Ser Leu Gly Glu Arg Ile Arg Asp Leu Glu Val Thr Pro Val Gly
                325                 330                 335

Glu Ile Leu Phe Ser Thr Asp Ser Gly Asn Ile Tyr Arg Leu Val Glu
            340                 345                 350

<210>  35
<211>  2339
<212>  DNA
<213>  Rhizobium leguminosarum

<400>  35
tcacggcaac gtataggcga tcacataatc gccgggcgtg gtgccgaccg agccgtggcc      60

gccggcgacc atgacgacat attgcttgtt gtcatcggtc atataggtca tcggcgtcgc     120

ctggccgccg gcgggaagcc gtccctgcca gagctcccgg ccactcgtca cgtcataggc     180

gcgcaggtag ttgtcgaccg cggcccccag gaaggcgacg ccgcccttgg tcagcatcgg     240

cccgccgatg ccgggcacgc ccaccttgaa gggcaggggc aatggcgtca tgtcgtggac     300

ggtgccgttc ttgtgcatat aggcgatctt gccggtgcgc aggtcgacgc cggcgacata     360

accccatggc ggcgcctggc aggggatttg gagcagcccg aggaagggac ccatgaagac     420

gccgtagggc gcgccgtcat tgcggttgag cccctgttcg ctgcccttct cgtcctggcc     480

tctcggagga atgtcggcgg ccggcaccag gcgcgaggta aaggcgagat aggtcggcat     540

gccgaacatg atctgccgct ccggatccac cgccaccgag ccccagttga aggtgccgaa 600

attgccggga tagacgatcg tccctttcag cgaaggcggc gtaaagcggc cctcatagtg 660

gtagcggtgg aagtcgatgc ggcaggccat ctgatcgaac agcgatacgc cccacatgtc 720

tctttcctgg agcggctttg gcgagaaggt gaggtccgag atcggctgcg tcggcgaggt 780

atgatcgccc gagaccgcgc cgcctggcgc tggtatttcc ttgatcggga tgatcggctc 840

gccgctgcgc cggtcgagca catagatatc gccctgtttg gtcgggccga ccaaggccgg 900

aaccacggtg ccgtcttgct tcgtcaggtc gatcagcgcc ggctgggccg gcacgtccat 960

gtcccagaga tcgtgatgca ctgtctggcg cacccagcgc aactggccgg tggcgatatc 1020

gagcgcgacg atagaggagg agaatttctc gacattgtcg ctgcggccga tgccgatctg 1080

gtcgggtact tggttgccga gcgggatgta gaccatgccg agcgcctcgt cgacgctgaa 1140

gaccgaccaa ctgttcggcg agttggtcgt ataggtctgg ccctcggtga tcggcgtcgt 1200

cacatcggga ttgccggaat cccaattcca gaccagggcg ccggtattga tgtcgaaggc 1260

gcggatgacg ccggattgct cctcagtcga ataattgtcg ttcaccgccc cgccgacgat 1320

gattttgccc gccaccgcga ccggcggcga agtggaataa tagtatccgg ccgggttgaa 1380

ccgcatgccg gtttccaaat gcagcacgcc ctggtcggca aagctggtgc agaccttccc 1440

gtccgccgca tcgagcgcga tcagccgggc gtctgaggtc ggcaggtaga cgcgctcggc 1500

gcagggctgg ccggcggcaa cggttggatc ggcataatag gtgacgccgc ggcaggtctg 1560

atgctgccgg tcggggttca tgcccgagtt ggcgtcgtat ttccatttct ccttgccggt 1620

cttggcgtcg agcgcgatcg cccagttatg cggcgtgcag agatagagcg tgtccttcac 1680

cttcagcggc gtcacctgat aggtcgtctc gccgacatcg tccggccgct tgacgtcgcc 1740

ggtctggtat cgccacgctt ccttgagggt ggagacgttt tcggcggtga tctggtcgag 1800

tggcgaatag cgctggccga agggcgtgcg gccgtattga tgccattcgc cgtccgggac 1860

gctgccgccg aaggcgggat tggcggcgac cgtatccttg ggcagctcgc cggcgagatc 1920

atgggggtca gtcgtcatcg aatagagggc gacaaggatg gcgagaatga caggcgcggc 1980

gagcggccag gggtttgcgg cataggtgat gcccgttggg ctgcgcaggc caagcggccg 2040

gcggatccat ggcgtcagca gccagagccc gagcaggatg atcatgccgc cgcggggccc 2100

aagctgccac cagtcgaagc cgacctccca aatcgcccag gcgagtgctg cgacgacgag 2160

caccgcatga cccagagcgc caccgccttg cgcatcagca gcagcccggc ggtaatcagg 2220

aacatcagcc cggcgaacag atagaagacg ctgccgccga gcgtgacgag ccagagcccg 2280

ccgccgctga gagtgagccc gacgatgatg aagaagatgg aggtgacgat gatcgccat 2339

<210> 36

<211>  779
<212>  PRT
<213>  Rhizobium leguminosarum

<400>  36

Met Ala Ile Ile Val Thr Ser Ile Phe Phe Ile Ile Val Gly Leu Thr
1               5                   10                  15

Leu Ser Gly Gly Gly Leu Trp Leu Val Thr Leu Gly Gly Ser Val Phe
            20                  25                  30

Tyr Leu Phe Ala Gly Leu Met Phe Leu Ile Thr Ala Gly Leu Leu Leu
        35                  40                  45

Met Arg Lys Ala Val Ala Leu Trp Val Tyr Ala Val Leu Val Val Ala
    50                  55                  60

Ala Leu Ala Trp Ala Ile Trp Glu Val Gly Phe Asp Trp Trp Gln Leu
65                  70                  75                  80

Gly Pro Arg Gly Gly Met Ile Ile Leu Leu Gly Leu Trp Leu Leu Thr
                85                  90                  95

Pro Trp Ile Arg Arg Pro Leu Gly Leu Arg Ser Pro Thr Gly Ile Thr
            100                 105                 110

Tyr Ala Ala Asn Pro Trp Pro Leu Ala Ala Pro Val Ile Leu Ala Ile
        115                 120                 125

Leu Val Ala Leu Tyr Ser Met Thr Thr Asp Pro His Asp Leu Ala Gly
    130                 135                 140

Glu Leu Pro Lys Asp Thr Val Ala Ala Asn Pro Ala Phe Gly Gly Ser
145                 150                 155                 160

Val Pro Asp Gly Glu Trp His Gln Tyr Gly Arg Thr Pro Phe Gly Gln
                165                 170                 175

Arg Tyr Ser Pro Leu Asp Gln Ile Thr Ala Glu Asn Val Ser Thr Leu
            180                 185                 190

Lys Glu Ala Trp Arg Tyr Gln Thr Gly Asp Val Lys Arg Pro Asp Asp
        195                 200                 205

Val Gly Glu Thr Thr Tyr Gln Val Thr Pro Leu Lys Val Lys Asp Thr
    210                 215                 220

Leu Tyr Leu Cys Thr Pro His Asn Trp Ala Ile Ala Leu Asp Ala Lys

225                 230                 235                 240

Thr Gly Lys Glu Lys Trp Lys Tyr Asp Ala Asn Ser Gly Met Asn Pro
                245                 250                 255

Asp Arg Gln His Gln Thr Cys Arg Gly Val Thr Tyr Tyr Ala Asp Pro
            260                 265                 270

Thr Val Ala Ala Gly Gln Pro Cys Ala Glu Arg Val Tyr Leu Pro Thr
        275                 280                 285

Ser Asp Ala Arg Leu Ile Ala Leu Asp Ala Ala Asp Gly Lys Val Cys
    290                 295                 300

Thr Ser Phe Ala Asp Gln Gly Val Leu His Leu Glu Thr Gly Met Arg
305                 310                 315                 320

Phe Asn Pro Ala Gly Tyr Tyr Tyr Ser Thr Ser Pro Pro Val Ala Val
                325                 330                 335

Ala Gly Lys Ile Ile Val Gly Gly Ala Val Asn Asp Asn Tyr Ser Thr
            340                 345                 350

Glu Glu Gln Ser Gly Val Ile Arg Ala Phe Asp Ile Asn Thr Gly Ala
        355                 360                 365

Leu Val Trp Asn Trp Asp Ser Gly Asn Pro Asp Val Thr Thr Pro Ile
    370                 375                 380

Thr Glu Gly Gln Thr Tyr Thr Thr Asn Ser Pro Asn Ser Trp Ser Val
385                 390                 395                 400

Phe Ser Val Asp Glu Ala Leu Gly Met Val Tyr Ile Pro Leu Gly Asn
                405                 410                 415

Gln Val Pro Asp Gln Ile Gly Ile Gly Arg Ser Asp Asn Val Glu Lys
            420                 425                 430

Phe Ser Ser Ser Ile Val Ala Leu Asp Ile Ala Thr Gly Gln Leu Arg
        435                 440                 445

Trp Val Arg Gln Thr Val His His Asp Leu Trp Asp Met Asp Val Pro
    450                 455                 460

Ala Gln Pro Ala Leu Ile Asp Leu Thr Lys Gln Asp Gly Thr Val Val
465                 470                 475                 480

Pro Ala Leu Val Gly Pro Thr Lys Gln Gly Asp Ile Tyr Val Leu Asp
                485                 490                 495

Arg Arg Ser Gly Glu Pro Ile Ile Pro Ile Lys Glu Ile Pro Ala Pro
            500                 505                 510

Gly Gly Ala Val Ser Gly Asp His Thr Ser Pro Thr Gln Pro Ile Ser
        515                 520                 525

Asp Leu Thr Phe Ser Pro Lys Pro Leu Gln Glu Arg Asp Met Trp Gly
    530                 535                 540

Val Ser Leu Phe Asp Gln Met Ala Cys Arg Ile Asp Phe His Arg Tyr
545                 550                 555                 560

His Tyr Glu Gly Arg Phe Thr Pro Pro Ser Leu Lys Gly Thr Ile Val
                565                 570                 575

Tyr Pro Gly Asn Phe Gly Thr Phe Asn Trp Gly Ser Val Ala Val Asp
            580                 585                 590

Pro Glu Arg Gln Ile Met Phe Gly Met Pro Thr Tyr Leu Ala Phe Thr
        595                 600                 605

Ser Arg Leu Val Pro Ala Ala Asp Ile Pro Pro Arg Gly Gln Asp Glu
    610                 615                 620

Lys Gly Ser Glu Gln Gly Leu Asn Arg Asn Asp Gly Ala Pro Tyr Gly
625                 630                 635                 640

Val Phe Met Gly Pro Phe Leu Gly Leu Leu Gln Ile Pro Cys Gln Ala
                645                 650                 655

Pro Pro Trp Gly Tyr Val Ala Gly Val Asp Leu Arg Thr Gly Lys Ile
            660                 665                 670

Ala Tyr Met His Lys Asn Gly Thr Val His Asp Met Thr Pro Leu Pro
        675                 680                 685

Leu Pro Phe Lys Val Gly Val Pro Gly Ile Gly Gly Pro Met Leu Thr
    690                 695                 700

Lys Gly Gly Val Ala Phe Leu Gly Ala Ala Val Asp Asn Tyr Leu Arg
705                 710                 715                 720

Ala Tyr Asp Val Thr Ser Gly Arg Glu Leu Trp Gln Gly Arg Leu Pro
                725                 730                 735

Ala Gly Gly Gln Ala Thr Pro Met Thr Tyr Met Thr Asp Asp Asn Lys
            740                 745                 750

Gln Tyr Val Val Met Val Ala Gly Gly His Gly Ser Val Gly Thr Thr
        755                 760                 765

Pro Gly Asp Tyr Val Ile Ala Tyr Thr Leu Pro
    770                 775

<210>  37
<211>  2244
<212>  DNA
<213>  Salmonella enterica

<400>  37

ttatttcgcg tcgtcaggca aagcataggc cacgatatag tcgcccatct tcgtaccaaa      60

cgaaccgtgg cctccggcag aaacgaccac gtactgctta ccgtttacct cataggtcat     120

cggcgttgcc tggcctccgg cgggtaaacg tccctgccac agtttttccc cgttgctcat     180

gttataagcg cgcaggtagt tgtcggcggt cgcggcaatg aacagcacat taccggcggt     240

tgagattggc ccgcccagca ttggcatccc catgttgaac ggcaccggaa ccggcatcgg     300

gaacggcata ctgtcgcgcg gcgtccctat acgtttcttc cacacgattt cattggtctt     360

cagatccaac gcggaaatat agccccaggc cggttgttta cacggcaggc caaacggcga     420

aaggaacgga ttcagcgtga cgccgaatgg tacgccgtac tgtggctgaa tacccgcttc     480

tgtaccagta cctttcgcgt ctttcggcgg ctccatcgga ttacccgggc cgcgtgggat     540

cagcttcgag acaaacggca gcgccatcgg gttagcgata gcgacctgac ggtctggatc     600

gacggaaata ccgccccatt caaacatccc caggttgcca gggaatacca atgtcccctg     660

ctcggacggc ggggtgaaga taccttcata gcgcaactgg tggaacatga cgcggcacac     720

cagttggtcg aacatggtcg cgccccacat atccgcgccg gagagatctt ttttcgggcg     780

gaaggttaaa tcagagaacg gctgagtttt ggcgacgtaa tcgccttttg ccgcgccctg     840

cgggaccggt ttttccgggg cgggcacgac cagctcgcca ttacgtctgt ccagcacaaa     900

gatattaccg gttttcgctg gcgcatagat aaccggaacg gtagtaccgt caacggtaat     960

atccgccagc gtcggctgtg ccggcaggtc catatcccac agatcgtgat ggacggtctg    1020

ataactccag gccagtttcc cggtggtggc attcagcgcc aggatggagc tggcataacg    1080

ttcctgctcc ggcgtgcggt taccgcccca gatatccggc gtggtcacgc ccatcggcag    1140

atagaccaga tccagtttcg catcataggc ggcaggcgcc caggagttcg gcgagttaaa    1200

ggtaaaagcg tgctcatcgg ctggaatcgc gttcggatct ttcgcccccg gatcgaaggc    1260

ccacatgagt ttaccgctat taacatcaaa accacggata acgccggacg tttcgcgggt    1320

agagaagtta tccgtgaccg aaccggcaat gacaattgtt ttatcggtga taatcggcgg 1380

cgacgtcggt tcatacagtc ccggcgtggt atccggcata ttggtttgca gattcaggac 1440

gcctttgttg gcgaaggttt cacacagctt gccggtttcg gcattcaccg cgaaaaggcg 1500

accgtcgttg accgggagga taatacggcg cggacagtcg gcgatgacct ccggcgaggc 1560

ggtatccgct ttggcctcat gataagagac gccgcggcag gtcacatgct ggaaagacga 1620

atcggtcttc aactgaggat caaaatgcca cttctcttta ccgctggcgg cgtccagcgc 1680

gaacagacgc tggtgagctg tacacagata aagcgtatcg ccgactttaa tcggggtcac 1740

ttcgttggtg atttcgcccg gatcgttggg ttgtttcaga tcgccggtgc ggaagaccca 1800

cgcttctttc agttggtgaa cgttatccgc agtgatctgt ttcagcggcg aatagcgttg 1860

gccttcctga ttacgtccat aggccggcca gtcttcgtcg gcgatcgacg agcttgttgc 1920

ggcgggcgtg gcgtcggcgc gaagcgtacc gttgatctcc tgcgggtcgt tgaagccagc 1980

ccaggtcaga atgccgccgc taatcagcag cgccacgacc agcgccgcca ctgcgccgct 2040

ggaaggaacc accagccgat gccagacaaa cggcaatatt agccagatgc cgaaaaacac 2100

cagaatatcg ctacgcggcg tgagcgccca gaagtcgaac ccgacttccc acacgcccca 2160

aatcattgtc gccagcagca gggcggcata cagccacagt gcggcacgtt tactgcgcca 2220

tagcaatccg gccacaacca gcat 2244

<210> 38
<211> 747
<212> PRT
<213> Salmonella enterica

<400> 38

Met Leu Val Val Ala Gly Leu Leu Trp Arg Ser Lys Arg Ala Ala Leu
1               5                   10                  15

Trp Leu Tyr Ala Ala Leu Leu Leu Ala Thr Met Ile Trp Gly Val Trp
            20                  25                  30

Glu Val Gly Phe Asp Phe Trp Ala Leu Thr Pro Arg Ser Asp Ile Leu
        35                  40                  45

Val Phe Phe Gly Ile Trp Leu Ile Leu Pro Phe Val Trp His Arg Leu
    50                  55                  60

Val Val Pro Ser Ser Gly Ala Val Ala Ala Leu Val Val Ala Leu Leu
65                  70                  75                  80

Ile Ser Gly Gly Ile Leu Thr Trp Ala Gly Phe Asn Asp Pro Gln Glu
                85                  90                  95

Ile Asn Gly Thr Leu Arg Ala Asp Ala Thr Pro Ala Ala Thr Ser Ser
            100                 105                 110

Ser Ile Ala Asp Glu Asp Trp Pro Ala Tyr Gly Arg Asn Gln Glu Gly
        115                 120                 125

Gln Arg Tyr Ser Pro Leu Lys Gln Ile Thr Ala Asp Asn Val His Gln
    130                 135                 140

Leu Lys Glu Ala Trp Val Phe Arg Thr Gly Asp Leu Lys Gln Pro Asn
145                 150                 155                 160

Asp Pro Gly Glu Ile Thr Asn Glu Val Thr Pro Ile Lys Val Gly Asp
                165                 170                 175

Thr Leu Tyr Leu Cys Thr Ala His Gln Arg Leu Phe Ala Leu Asp Ala
            180                 185                 190

Ala Ser Gly Lys Glu Lys Trp His Phe Asp Pro Gln Leu Lys Thr Asp
        195                 200                 205

Ser Ser Phe Gln His Val Thr Cys Arg Gly Val Ser Tyr His Glu Ala
    210                 215                 220

Lys Ala Asp Thr Ala Ser Pro Glu Val Ile Ala Asp Cys Pro Arg Arg
225                 230                 235                 240

Ile Ile Leu Pro Val Asn Asp Gly Arg Leu Phe Ala Val Asn Ala Glu
                245                 250                 255

Thr Gly Lys Leu Cys Glu Thr Phe Ala Asn Lys Gly Val Leu Asn Leu
            260                 265                 270

Gln Thr Asn Met Pro Asp Thr Thr Pro Gly Leu Tyr Glu Pro Thr Ser
        275                 280                 285

Pro Pro Ile Ile Thr Asp Lys Thr Ile Val Ile Ala Gly Ser Val Thr
    290                 295                 300

Asp Asn Phe Ser Thr Arg Glu Thr Ser Gly Val Ile Arg Gly Phe Asp
305                 310                 315                 320

Val Asn Ser Gly Lys Leu Met Trp Ala Phe Asp Pro Gly Ala Lys Asp
                325                 330                 335

Pro Asn Ala Ile Pro Ala Asp Glu His Ala Phe Thr Phe Asn Ser Pro

340                 345                 350

Asn Ser Trp Ala Pro Ala Ala Tyr Asp Ala Lys Leu Asp Leu Val Tyr
        355                 360                 365

Leu Pro Met Gly Val Thr Thr Pro Asp Ile Trp Gly Gly Asn Arg Thr
    370                 375                 380

Pro Glu Gln Glu Arg Tyr Ala Ser Ser Ile Leu Ala Leu Asn Ala Thr
385                 390                 395                 400

Thr Gly Lys Leu Ala Trp Ser Tyr Gln Thr Val His His Asp Leu Trp
                405                 410                 415

Asp Met Asp Leu Pro Ala Gln Pro Thr Leu Ala Asp Ile Thr Val Asp
            420                 425                 430

Gly Thr Thr Val Pro Val Ile Tyr Ala Pro Ala Lys Thr Gly Asn Ile
        435                 440                 445

Phe Val Leu Asp Arg Arg Asn Gly Glu Leu Val Val Pro Ala Pro Glu
    450                 455                 460

Lys Pro Val Pro Gln Gly Ala Ala Lys Gly Asp Tyr Val Ala Lys Thr
465                 470                 475                 480

Gln Pro Phe Ser Asp Leu Thr Phe Arg Pro Lys Lys Asp Leu Ser Gly
                485                 490                 495

Ala Asp Met Trp Gly Ala Thr Met Phe Asp Gln Leu Val Cys Arg Val
            500                 505                 510

Met Phe His Gln Leu Arg Tyr Glu Gly Ile Phe Thr Pro Pro Ser Glu
        515                 520                 525

Gln Gly Thr Leu Val Phe Pro Gly Asn Leu Gly Met Phe Glu Trp Gly
    530                 535                 540

Gly Ile Ser Val Asp Pro Asp Arg Gln Val Ala Ile Ala Asn Pro Met
545                 550                 555                 560

Ala Leu Pro Phe Val Ser Lys Leu Ile Pro Arg Gly Pro Gly Asn Pro
                565                 570                 575

Met Glu Pro Pro Lys Asp Ala Lys Gly Thr Gly Thr Glu Ala Gly Ile
            580                 585                 590

Gln Pro Gln Tyr Gly Val Pro Phe Gly Val Thr Leu Asn Pro Phe Leu
        595                 600                 605

Ser Pro Phe Gly Leu Pro Cys Lys Gln Pro Ala Trp Gly Tyr Ile Ser
    610                 615                 620

Ala Leu Asp Leu Lys Thr Asn Glu Ile Val Trp Lys Lys Arg Ile Gly
625                 630                 635                 640

Thr Pro Arg Asp Ser Met Pro Phe Pro Met Pro Val Pro Val Pro Phe
                645                 650                 655

Asn Met Gly Met Pro Met Leu Gly Gly Pro Ile Ser Thr Ala Gly Asn
            660                 665                 670

Val Leu Phe Ile Ala Ala Thr Ala Asp Asn Tyr Leu Arg Ala Tyr Asn
        675                 680                 685

Met Ser Asn Gly Glu Lys Leu Trp Gln Gly Arg Leu Pro Ala Gly Gly
    690                 695                 700

Gln Ala Thr Pro Met Thr Tyr Glu Val Asn Gly Lys Gln Tyr Val Val
705                 710                 715                 720

Val Ser Ala Gly Gly His Gly Ser Phe Gly Thr Lys Met Gly Asp Tyr
                725                 730                 735

Ile Val Ala Tyr Ala Leu Pro Asp Asp Ala Lys
            740                 745

<210>  39
<211>  2046
<212>  DNA
<213>  Solibacter usitatus

<400>  39
atgatccgat cgctagcgtt cgccctcgcc tccgttcgac tgcttgccgc ctcggattgg      60

ccggggtacg gtggcggccc cgcggggatt cgtcactcgc ccttgaagca gatcaatcgc     120

tccaacgtgt cgcacctcaa ggtcgcgtgg acgtacgaca ccaaggatgg gccgggcgat     180

ccacagacgc agccgatcgt ggtcgatggc gttctctatg gcgtaactcc gacccacaaa     240

gccatcgcgc taaacggcgc caccggaaag ctgctgtgga ccttcgactc cggcattcgc     300

ggacgtggtc ccaatcgctc ggtagtctgg tggtcgccgc gaacggagcg gcggctcttc     360

gtcgcggtgc agagctttat ctacgcgctc gacccagcca ccgggaagcc catggcggat     420

ttcggcgagc agggccgcat cgatctccgc cagggcctgg ggcgcgatgc cgccaagcag     480

tcctacgtcc tgaccagccc gggaattatt tatcgcgatc tgttgatcgt gggcggccgc 540

ctgccggaag cgctgccggc gccgcccggc gacatccgcg cctatgatgt ccgcaccggc 600

aaacagcgct ggaccttcca cacgattccg catccgggcg agcccggcta cgagacctgg 660

ccctccgatg cctggaccta ctccggcggc gccaacaact gggccggcat ggcgctcgac 720

gagaagcgtg gcatcgtcta cgtccccacc ggctcagccg ccagcgattt ttatggcgcc 780

gaccgcctgg gcgacaacct ctacgccaac tgcctgctgg cgctgaacgc cgccaccggc 840

gagcgcgtct ggcacttcca ggcggtgcat catgacatct gggatcgtga ctttccctcc 900

ccgcccgcgc tggtcaccgt ccggcacgaa ggccgcacgg tcgatgccgt tgtgcagacc 960

tccaagcagg gctgggtcta cctgttcaat cgcgccaccg gcaagccgtt gttccccatc 1020

gagagccggc cctatcccgt cagcgagctg ccgggcgaac gtgctgccgc cgaacagtcg 1080

ctcccgacca ggccggccgc ctttgcgcgc caggctctga ctgaagacgt gctcacccgc 1140

cgcaccgatg caacccacgc gtgggcgctt gccgagttcc acaagctgcg cagcgccgga 1200

cagttcctgc ccttcggggc agggaatgag accgtcattt ttccaggctt cgatggcggc 1260

gccgagtggg gcggtccggc cttcgatccc tccacgggat tgctgtacgt caacgccaat 1320

gaaatggcct ggagggcggg cctcaaggaa tcccagccgg aaaactcagc ccggcaactg 1380

tatgtggcgc agtgcggcac ctgccatggc gacgacctca agggctcgcc tccgcagttc 1440

ccggcgttga acgaccttgc cggaaagcgc tcggcccagg aaatcggcgc aatcattcgc 1500

gagggcggcg ggcgcatgcc tgctttcccc cggcttcgtc ccgacgacat caccgcgctc 1560

agcgattatc tgctccgcgg ccgcagcaag gagctggaga gcaccgcctc actggatcgc 1620

gaaccgcgat ttcgttttac cggctatcac aagttcgtcg atcccgatgg ctatccggca 1680

atcgcgccgc cgtggggcac gctcaacgcc atcaacctca ataccggcga atacgcgtgg 1740

cgcatcccgc tcggcgaata tcccgacctc gcggccaaaa acacgggatc ggaaaactac 1800

ggcggcccca tcgtgaccgc cggcggcctc gtgtttatcg cggccaccaa ctatgaccgc 1860

aagttccgag ccttcgacaa ggccaccggc gaactgctct ggcagactac gctcccgatg 1920

gccggcaacg ccacacccat cacctatgaa atcgccggcc gccagtacgt cgtgatctac 1980

gcgaccggcg gcaagtccgg caagtggggt ccttcgggcg gaatctacat ggcgttctca 2040

ctgtaa 2046

<210> 40
<211> 681
<212> PRT
<213> Solibacter usitatus

<400> 40

Met Ile Arg Ser Leu Ala Phe Ala Leu Ala Ser Val Arg Leu Leu Ala

1               5                   10                  15

Ala Ser Asp Trp Pro Gly Tyr Gly Gly Gly Pro Ala Gly Ile Arg His
            20                  25                  30

Ser Pro Leu Lys Gln Ile Asn Arg Ser Asn Val Ser His Leu Lys Val
        35                  40                  45

Ala Trp Thr Tyr Asp Thr Lys Asp Gly Pro Gly Asp Pro Gln Thr Gln
    50                  55                  60

Pro Ile Val Val Asp Gly Val Leu Tyr Gly Val Thr Pro Thr His Lys
65                  70                  75                  80

Ala Ile Ala Leu Asn Gly Ala Thr Gly Lys Leu Leu Trp Thr Phe Asp
                85                  90                  95

Ser Gly Ile Arg Gly Arg Gly Pro Asn Arg Ser Val Val Trp Trp Ser
            100                 105                 110

Pro Arg Thr Glu Arg Arg Leu Phe Val Ala Val Gln Ser Phe Ile Tyr
        115                 120                 125

Ala Leu Asp Pro Ala Thr Gly Lys Pro Met Ala Asp Phe Gly Glu Gln
    130                 135                 140

Gly Arg Ile Asp Leu Arg Gln Gly Leu Gly Arg Asp Ala Ala Lys Gln
145                 150                 155                 160

Ser Tyr Val Leu Thr Ser Pro Gly Ile Ile Tyr Arg Asp Leu Leu Ile
                165                 170                 175

Val Gly Gly Arg Leu Pro Glu Ala Leu Pro Ala Pro Pro Gly Asp Ile
            180                 185                 190

Arg Ala Tyr Asp Val Arg Thr Gly Lys Gln Arg Trp Thr Phe His Thr
        195                 200                 205

Ile Pro His Pro Gly Glu Pro Gly Tyr Glu Thr Trp Pro Ser Asp Ala
    210                 215                 220

Trp Thr Tyr Ser Gly Gly Ala Asn Asn Trp Ala Gly Met Ala Leu Asp
225                 230                 235                 240

Glu Lys Arg Gly Ile Val Tyr Val Pro Thr Gly Ser Ala Ala Ser Asp
                245                 250                 255

Phe Tyr Gly Ala Asp Arg Leu Gly Asp Asn Leu Tyr Ala Asn Cys Leu
            260                 265                 270

Leu Ala Leu Asn Ala Ala Thr Gly Glu Arg Val Trp His Phe Gln Ala
        275                 280                 285

Val His His Asp Ile Trp Asp Arg Asp Phe Pro Ser Pro Pro Ala Leu
    290                 295                 300

Val Thr Val Arg His Glu Gly Arg Thr Val Asp Ala Val Val Gln Thr
305                 310                 315                 320

Ser Lys Gln Gly Trp Val Tyr Leu Phe Asn Arg Ala Thr Gly Lys Pro
                325                 330                 335

Leu Phe Pro Ile Glu Ser Arg Pro Tyr Pro Val Ser Glu Leu Pro Gly
            340                 345                 350

Glu Arg Ala Ala Ala Glu Gln Ser Leu Pro Thr Arg Pro Ala Ala Phe
        355                 360                 365

Ala Arg Gln Ala Leu Thr Glu Asp Val Leu Thr Arg Arg Thr Asp Ala
    370                 375                 380

Thr His Ala Trp Ala Leu Ala Glu Phe His Lys Leu Arg Ser Ala Gly
385                 390                 395                 400

Gln Phe Leu Pro Phe Gly Ala Gly Asn Glu Thr Val Ile Phe Pro Gly
                405                 410                 415

Phe Asp Gly Gly Ala Glu Trp Gly Gly Pro Ala Phe Asp Pro Ser Thr
            420                 425                 430

Gly Leu Leu Tyr Val Asn Ala Asn Glu Met Ala Trp Arg Ala Gly Leu
        435                 440                 445

Lys Glu Ser Gln Pro Glu Asn Ser Ala Arg Gln Leu Tyr Val Ala Gln
    450                 455                 460

Cys Gly Thr Cys His Gly Asp Asp Leu Lys Gly Ser Pro Pro Gln Phe
465                 470                 475                 480

Pro Ala Leu Asn Asp Leu Ala Gly Lys Arg Ser Ala Gln Glu Ile Gly
                485                 490                 495

Ala Ile Ile Arg Glu Gly Gly Gly Arg Met Pro Ala Phe Pro Arg Leu
            500                 505                 510

Arg Pro Asp Asp Ile Thr Ala Leu Ser Asp Tyr Leu Leu Arg Gly Arg
        515                 520                 525

Ser Lys Glu Leu Glu Ser Thr Ala Ser Leu Asp Arg Glu Pro Arg Phe
    530                 535                 540

Arg Phe Thr Gly Tyr His Lys Phe Val Asp Pro Asp Gly Tyr Pro Ala
545                 550                 555                 560

Ile Ala Pro Pro Trp Gly Thr Leu Asn Ala Ile Asn Leu Asn Thr Gly
                565                 570                 575

Glu Tyr Ala Trp Arg Ile Pro Leu Gly Glu Tyr Pro Asp Leu Ala Ala
            580                 585                 590

Lys Asn Thr Gly Ser Glu Asn Tyr Gly Gly Pro Ile Val Thr Ala Gly
        595                 600                 605

Gly Leu Val Phe Ile Ala Ala Thr Asn Tyr Asp Arg Lys Phe Arg Ala
    610                 615                 620

Phe Asp Lys Ala Thr Gly Glu Leu Leu Trp Gln Thr Thr Leu Pro Met
625                 630                 635                 640

Ala Gly Asn Ala Thr Pro Ile Thr Tyr Glu Ile Ala Gly Arg Gln Tyr
                645                 650                 655

Val Val Ile Tyr Ala Thr Gly Gly Lys Ser Gly Lys Trp Gly Pro Ser
            660                 665                 670

Gly Gly Ile Tyr Met Ala Phe Ser Leu
        675                 680

<210> 41
<211> 2169
<212> DNA
<213> Klebsiella pneumoniae

<400> 41
atggcaactg gcaacgcgcc gcgcggattc ccccggatcc tgcagtggct tctcgccgga 60

ctgatgctca tcatcggtct ggctgtgggt attcttgggg caaaactggc cctcgtcggc 120

ggtacactgt acttcgcgct gatgggcgtg gtcatggtca tcgcggcggt gttgattttc 180

cgcaaccgcc gcggcggtat tctgttatac gccgtggcgt ttatcgcctc ggtgatctgg 240

gcgattagtg acgctggctg gaactactgg ccgctcttct cgcgcctgtt tgcgctcggc 300

gtactggcct tcctggccgc cctggtctgg cctttcctcg ccagcccacc ggcgaaaaaa 360

ggcccggcct atggcgtcgc cgccgttctc gccgtggcgc tggcggtgag ctttggctgg 420
atgttcaaat ccgcaccgct ggtcagcgcg actgaagcgg taccagtcaa gcccgtcgcg 480
ccaggcgaac agcagaaaaa ctgggcgcac tggggtaaca ccacccacgg cgaccgtttc 540
gccgccctcg accaaatcaa caaacaaaac gtcaaccagc tgcaggttgc ctgggtagcg 600
catactggcg atatcccaca gagcaacggc tcgggcgcag aagatcagaa caccccgctg 660
cagattggcg atactctcta tgtctgtacc ccttacagta aagtgctggc gctggatgtg 720
gacagcggaa aagagaaatg gcgctatgac tccaaatcct cttcacccaa ctggcagcgc 780
tgccgcggct tagggtatta cgaagatagc caggcgcaaa ccgcgctagc gtcaggcacg 840
cagccggccg cctgttcccg tcgtctgttc ctgccgacca tcgacgcccg cctgatcgcc 900
attgatgccg ataccggcaa actgtgcgaa aacttcggcg atggcggtat tgtcgacctc 960
agcgtcggca tgggtgaagt gaaagcgggt tactatcagc agacctccac cccgctggtg 1020
gcgggcaatg tggtggtggt gggcggccgc gtcgcggata actactccac tggcgaaccg 1080
ccgggcgtgg tccgcgcgtt tgatgtccac accggcaaac tggcatgggc gtgggatccg 1140
ggcaatccag cgctgaccgg tgttccgccg gaaggccaga cctacacgcg cggtacgcca 1200
aacgtctggt cggcgatgtc ctacgacgcg aagctgaatc tgatctatct gcccaccggc 1260
aacgccacgc cagatttctt tggcggcgaa cgtaccgcgc tggacgataa atacagctcc 1320
tctatcgtgg ctgtggatgc caccaccggc caggtgcgct ggcacttcca gaccacacac 1380
cacgatcggt gggactttga cctgccgtct cagccgctgc tgtacgatct gcctgacggc 1440
aagggcggca ccaccccggt gctggtgcag accagcaagc agggcatgat ctttatgctc 1500
aaccgcgaga ccggcgagcc ggtggccaaa gtggaagagc gtccggtgcc ggcgggcaat 1560
gtcaaaggtg aacgctactc gccgacgcaa ccttactcgg tagggatgcc gatgatcggc 1620
aaccagacgt tgaccgagtc cgatatgtgg ggagcgacac cgatcgacct gctgctgtgc 1680
cgtattcagt tcaaagagat gcgccatcag ggcgtcttta ccccgccagg tgaagaccgt 1740
tccctgcagt tcccgggctc gctcggcggc atgaactggg gcagtgtttc gctggatcca 1800
aacaacagcc tgatgtttgt caacgatatg cgtctgggcc tggccaacta tatggtgccg 1860
cgcgcgaagg tggcgaaaga cgccagcggg atcgagatgg gcatcgtgcc gatggagggc 1920
acgccgtttg gcgcgatacg cgaacgtttc ctgtcgccgc tgggcattcc gtgccagaag 1980
ccgccgttcg gcaccatgtc cgcggtagat ctgaaaaccg ggaaactggt gtggcaggtg 2040
ccggttggca ccgtagaaga caccgggccg ctgggcatac gtatgcacat gccaattcca 2100
atcggcatgc cgacccttgg cgcatcgcta gcaacgcagt ccggactgct gttcttcgcc 2160
ggcacctag 2169

<210>  42
<211>  722
<212>  PRT
<213>  Klebsiella pneumoniae

<400>  42
   Met Ala Thr Gly Asn Ala Pro Arg Gly Phe Pro Arg Ile Leu Gln Trp
   1               5                   10                  15
   Leu Leu Ala Gly Leu Met Leu Ile Ile Gly Leu Ala Val Gly Ile Leu
               20                  25                  30
   Gly Ala Lys Leu Ala Leu Val Gly Gly Thr Leu Tyr Phe Ala Leu Met
           35                  40                  45
   Gly Val Val Met Val Ile Ala Ala Val Leu Ile Phe Arg Asn Arg Arg
       50                  55                  60
   Gly Gly Ile Leu Leu Tyr Ala Val Ala Phe Ile Ala Ser Val Ile Trp
   65                  70                  75                  80
   Ala Ile Ser Asp Ala Gly Trp Asn Tyr Trp Pro Leu Phe Ser Arg Leu
                   85                  90                  95
   Phe Ala Leu Gly Val Leu Ala Phe Leu Ala Ala Leu Val Trp Pro Phe
               100                 105                 110
   Leu Ala Ser Pro Pro Ala Lys Lys Gly Pro Ala Tyr Gly Val Ala Ala
           115                 120                 125
   Val Leu Ala Val Ala Leu Ala Val Ser Phe Gly Trp Met Phe Lys Ser
       130                 135                 140
   Ala Pro Leu Val Ser Ala Thr Glu Ala Val Pro Val Lys Pro Val Ala
   145                 150                 155                 160
   Pro Gly Glu Gln Gln Lys Asn Trp Ala His Trp Gly Asn Thr Thr His
                   165                 170                 175
   Gly Asp Arg Phe Ala Ala Leu Asp Gln Ile Asn Lys Gln Asn Val Asn
               180                 185                 190
   Gln Leu Gln Val Ala Trp Val Ala His Thr Gly Asp Ile Pro Gln Ser
           195                 200                 205
   Asn Gly Ser Gly Ala Glu Asp Gln Asn Thr Pro Leu Gln Ile Gly Asp
       210                 215                 220
   Thr Leu Tyr Val Cys Thr Pro Tyr Ser Lys Val Leu Ala Leu Asp Val
   225                 230                 235                 240
   Asp Ser Gly Lys Glu Lys Trp Arg Tyr Asp Ser Lys Ser Ser Ser Pro
                   245                 250                 255
   Asn Trp Gln Arg Cys Arg Gly Leu Gly Tyr Tyr Glu Asp Ser Gln Ala
               260                 265                 270
   Gln Thr Ala Leu Ala Ser Gly Thr Gln Pro Ala Ala Cys Ser Arg Arg
           275                 280                 285
   Leu Phe Leu Pro Thr Ile Asp Ala Arg Leu Ile Ala Ile Asp Ala Asp
       290                 295                 300
   Thr Gly Lys Leu Cys Glu Asn Phe Gly Asp Gly Gly Ile Val Asp Leu
   305                 310                 315                 320
   Ser Val Gly Met Gly Glu Val Lys Ala Gly Tyr Tyr Gln Gln Thr Ser
                   325                 330                 335
   Thr Pro Leu Val Ala Gly Asn Val Val Val Val Gly Gly Arg Val Ala
               340                 345                 350
   Asp Asn Tyr Ser Thr Gly Glu Pro Pro Gly Val Val Arg Ala Phe Asp
           355                 360                 365
   Val His Thr Gly Lys Leu Ala Trp Ala Trp Asp Pro Gly Asn Pro Ala
       370                 375                 380
   Leu Thr Gly Val Pro Pro Glu Gly Gln Thr Tyr Thr Arg Gly Thr Pro
   385                 390                 395                 400
   Asn Val Trp Ser Ala Met Ser Tyr Asp Ala Lys Leu Asn Leu Ile Tyr
                   405                 410                 415
   Leu Pro Thr Gly Asn Ala Thr Pro Asp Phe Phe Gly Gly Glu Arg Thr
               420                 425                 430
   Ala Leu Asp Asp Lys Tyr Ser Ser Ser Ile Val Ala Val Asp Ala Thr
           435                 440                 445
   Thr Gly Gln Val Arg Trp His Phe Gln Thr Thr His His Asp Arg Trp
       450                 455                 460
   Asp Phe Asp Leu Pro Ser Gln Pro Leu Leu Tyr Asp Leu Pro Asp Gly
   465                 470                 475                 480
   Lys Gly Gly Thr Thr Pro Val Leu Val Gln Thr Ser Lys Gln Gly Met
                   485                 490                 495
   Ile Phe Met Leu Asn Arg Glu Thr Gly Glu Pro Val Ala Lys Val Glu
               500                 505                 510
   Glu Arg Pro Val Pro Ala Gly Asn Val Lys Gly Glu Arg Tyr Ser Pro
           515                 520                 525
   Thr Gln Pro Tyr Ser Val Gly Met Pro Met Ile Gly Asn Gln Thr Leu
       530                 535                 540
   Thr Glu Ser Asp Met Trp Gly Ala Thr Pro Ile Asp Leu Leu Leu Cys
   545                 550                 555                 560
   Arg Ile Gln Phe Lys Glu Met Arg His Gln Gly Val Phe Thr Pro Pro
                   565                 570                 575
   Gly Glu Asp Arg Ser Leu Gln Phe Pro Gly Ser Leu Gly Gly Met Asn
               580                 585                 590
   Trp Gly Ser Val Ser Leu Asp Pro Asn Asn Ser Leu Met Phe Val Asn
           595                 600                 605
   Asp Met Arg Leu Gly Leu Ala Asn Tyr Met Val Pro Arg Ala Lys Val
       610                 615                 620
   Ala Lys Asp Ala Ser Gly Ile Glu Met Gly Ile Val Pro Met Glu Gly
   625                 630                 635                 640
   Thr Pro Phe Gly Ala Ile Arg Glu Arg Phe Leu Ser Pro Leu Gly Ile
                   645                 650                 655
   Pro Cys Gln Lys Pro Pro Phe Gly Thr Met Ser Ala Val Asp Leu Lys
               660                 665                 670
   Thr Gly Lys Leu Val Trp Gln Val Pro Val Gly Thr Val Glu Asp Thr
           675                 680                 685
   Gly Pro Leu Gly Ile Arg Met His Met Pro Ile Pro Ile Gly Met Pro
       690                 695                 700
   Thr Leu Gly Ala Ser Leu Ala Thr Gln Ser Gly Leu Leu Phe Phe Ala
   705                 710                 715                 720
   Gly Thr

<210>  43
<211>  2346
<212>  DNA
<213>  Rhodobacter sphaeroides

<400>  43
ttgaccgcta ccctgaccgc catcgtcctt gccctcgccg ggctgggcct cctcatcccg      60

ggcgcctggc tggccgttct gggcgggagc tggttctacc tcgtggccgg agccctcatg     120

ctgctgacgg catggcaggt gttccgccgc tcctcctccg ccgaatggat ctatggcggc     180

ctcatcctcg gcgcgctcgt ctggtccgtc tgggaagtgg gcttcgactg gtgggaactc     240

attccccgcg gcggcatcat cgcgcttctg ggcatctggc tctacctgcc cttcatgcgg     300

cggcagctgc tgaccgagga cggccgcccc gcgcccggcc tgcccttggc cctgcccctt     360

ctggcggcca tcggggccgc catctggtca tggaccggag acgaagcggg acatgcaggc     420

tcgctgccga cggaagtggt cagcgccgag cccgatctcg gcccctccgc gcctcccggc     480

gagtggcacc agtacggccg cacgcagtac gggcagcgct acagcccgct cgaacagatc     540

aacatccaga atgtcgccga gctcgagcag gtctggcagt atcagaccgg cgatgtgaag     600

ctgccgcagg acgtgaccga gaccacctat caggtcacgc cgctgaaggt cgccgaccgt     660

ctctacatct gcaccccgca cgatctggcc atcgcgctcg atgcggcgac cggcaaggaa     720

gcctggcgct tcgacgcgcg ctccgggctc gagtccgacc ggcagcacca gacctgccgc     780

ggcgtgacct actggcggga tccggcacgc gccgagggag agctctgcgc cgagcgggtc     840

tatctgccga cggccgatgc gcggctgatc gcgctcgacg caaagtccgg cgcggtctgc     900

accttcttcg ccgacgaggg tacgctccat ctcgagaacg ggatgcccta caccccggca     960

ggcttctact attccacctc cccgcccgtg gcggtcggag gccggatcat catcggcggc    1020

gcggtcaacg acaatttctc ggtctattcg cagtcgggcg tgatccgggc cttcgatgcc    1080

aacaccgggg cgctcctgtg gaactgggac agcgccaacc ccgacaagac cgcccccatc    1140

gactggatga acggcgagac ctataccgca aattcgccca actcctggtc ggtcttctcg    1200

gtcgatgagg aacgcggcct cgtctacatc ccgctcggca accaggtccc cgaccagctg    1260

ggcttcaacc gctcgcccgc cgtcgaggaa cattcctcct cggtcgtggc gctcgacgtg    1320

gcgacgggtc agaaggcctg ggtgttccag accgtgcacc atgacctgtg ggacatggac    1380

gtgcccgccc agcccgttct gatcgatctc gacatcgacg gtcagacggt gcccgcgctc    1440

gtgcagccga cgaagcaggg cgacatctat gtgctcaacc gcgagacggg cgagccgatc    1500

ctgcccgtca ccgaagagcc cgccccgcag gagggcgctc tgcgcgaaga gacccctgcc    1560

ccgacgcagc ccacctcggc gctgagcttc aaacccgagg cgctgcgcga gaaggacatg    1620

tggggcgtca cgctctacga ccagctcgcc tgccggatcc agtatcaccg gttgaactac 1680

gagggccgct acacgccccc gtcgctgaac ggcaccatcg tctatccggg caatttcggc 1740

accttcaact ggggctccgt cgccgtcgat cccgagcggc aggtgatgtt cggcatgccc 1800

acctacctgc ccttcacgag ccagctggtt ccggccgagg acatcccgcc gcccggcgca 1860

gatcagaagg ccagcgaaca gggcctgaac cgcaacgagg gcgcgcccta cggcgtcatc 1920

atggggccct tcctcgggcc gctcggcgtg ccctgctcgg cgccgccgtg gggcttcgtc 1980

gcgggggcgg acctccgcac cggagagatc gcctacatgc accgcaacgg caccgtgcgc 2040

gacatgacgc ccctgccgct gcccttcaag gtgggcgtgc cggggatcgg cgggccgatc 2100

gtcacgcgcg gcggcgtggc cttcctcgga gcggcggtcg acgactacct gcgcgcctac 2160

gatgtgacca cgggcgatca gctctggcag gcgcggctgc ccgcgggcgg ccagtcgacg 2220

ccgatgacct acgagcagga cggccggcag ttcgtggtga tcgtggcggg cgggcacggc 2280

tcggtcggca cgaaaccggg cgactatgtg atcgcctacg ccctgcccga cggctcggag 2340

ggttga 2346

<210> 44
<211> 781
<212> PRT
<213> Rhodobacter sphaeroides

<400> 44

Met Thr Ala Thr Leu Thr Ala Ile Val Leu Ala Leu Ala Gly Leu Gly
1               5                   10                  15

Leu Leu Ile Pro Gly Ala Trp Leu Ala Val Leu Gly Gly Ser Trp Phe
            20                  25                  30

Tyr Leu Val Ala Gly Ala Leu Met Leu Leu Thr Ala Trp Gln Val Phe
        35                  40                  45

Arg Arg Ser Ser Ser Ala Glu Trp Ile Tyr Gly Gly Leu Ile Leu Gly
    50                  55                  60

Ala Leu Val Trp Ser Val Trp Glu Val Gly Phe Asp Trp Trp Glu Leu
65                  70                  75                  80

Ile Pro Arg Gly Gly Ile Ile Ala Leu Leu Gly Ile Trp Leu Tyr Leu
                85                  90                  95

Pro Phe Met Arg Arg Gln Leu Leu Thr Glu Asp Gly Arg Pro Ala Pro
            100                 105                 110

Gly Leu Pro Leu Ala Leu Pro Leu Leu Ala Ala Ile Gly Ala Ala Ile

115                 120                 125

Trp Ser Trp Thr Gly Asp Glu Ala Gly His Ala Gly Ser Leu Pro Thr
    130                 135                 140

Glu Val Val Ser Ala Glu Pro Asp Leu Gly Pro Ser Ala Pro Pro Gly
145                 150                 155                 160

Glu Trp His Gln Tyr Gly Arg Thr Gln Tyr Gly Gln Arg Tyr Ser Pro
                165                 170                 175

Leu Glu Gln Ile Asn Ile Gln Asn Val Ala Glu Leu Glu Gln Val Trp
            180                 185                 190

Gln Tyr Gln Thr Gly Asp Val Lys Leu Pro Gln Asp Val Thr Glu Thr
        195                 200                 205

Thr Tyr Gln Val Thr Pro Leu Lys Val Ala Asp Arg Leu Tyr Ile Cys
    210                 215                 220

Thr Pro His Asp Leu Ala Ile Ala Leu Asp Ala Ala Thr Gly Lys Glu
225                 230                 235                 240

Ala Trp Arg Phe Asp Ala Arg Ser Gly Leu Glu Ser Asp Arg Gln His
                245                 250                 255

Gln Thr Cys Arg Gly Val Thr Tyr Trp Arg Asp Pro Ala Arg Ala Glu
            260                 265                 270

Gly Glu Leu Cys Ala Glu Arg Val Tyr Leu Pro Thr Ala Asp Ala Arg
        275                 280                 285

Leu Ile Ala Leu Asp Ala Lys Ser Gly Ala Val Cys Thr Phe Phe Ala
    290                 295                 300

Asp Glu Gly Thr Leu His Leu Glu Asn Gly Met Pro Tyr Thr Pro Ala
305                 310                 315                 320

Gly Phe Tyr Tyr Ser Thr Ser Pro Pro Val Ala Val Gly Gly Arg Ile
                325                 330                 335

Ile Ile Gly Gly Ala Val Asn Asp Asn Phe Ser Val Tyr Ser Gln Ser
            340                 345                 350

Gly Val Ile Arg Ala Phe Asp Ala Asn Thr Gly Ala Leu Leu Trp Asn
        355                 360                 365

Trp Asp Ser Ala Asn Pro Asp Lys Thr Ala Pro Ile Asp Trp Met Asn
    370                 375                 380

Gly Glu Thr Tyr Thr Ala Asn Ser Pro Asn Ser Trp Ser Val Phe Ser
385                 390                 395                 400

Val Asp Glu Glu Arg Gly Leu Val Tyr Ile Pro Leu Gly Asn Gln Val
                405                 410                 415

Pro Asp Gln Leu Gly Phe Asn Arg Ser Pro Ala Val Glu Glu His Ser
            420                 425                 430

Ser Ser Val Val Ala Leu Asp Val Ala Thr Gly Gln Lys Ala Trp Val
        435                 440                 445

Phe Gln Thr Val His His Asp Leu Trp Asp Met Asp Val Pro Ala Gln
    450                 455                 460

Pro Val Leu Ile Asp Leu Asp Ile Asp Gly Gln Thr Val Pro Ala Leu
465                 470                 475                 480

Val Gln Pro Thr Lys Gln Gly Asp Ile Tyr Val Leu Asn Arg Glu Thr
                485                 490                 495

Gly Glu Pro Ile Leu Pro Val Thr Glu Glu Pro Ala Pro Gln Glu Gly
            500                 505                 510

Ala Leu Arg Glu Glu Thr Pro Ala Pro Thr Gln Pro Thr Ser Ala Leu
        515                 520                 525

Ser Phe Lys Pro Glu Ala Leu Arg Glu Lys Asp Met Trp Gly Val Thr
    530                 535                 540

Leu Tyr Asp Gln Leu Ala Cys Arg Ile Gln Tyr His Arg Leu Asn Tyr
545                 550                 555                 560

Glu Gly Arg Tyr Thr Pro Pro Ser Leu Asn Gly Thr Ile Val Tyr Pro
                565                 570                 575

Gly Asn Phe Gly Thr Phe Asn Trp Gly Ser Val Ala Val Asp Pro Glu
            580                 585                 590

Arg Gln Val Met Phe Gly Met Pro Thr Tyr Leu Pro Phe Thr Ser Gln
        595                 600                 605

Leu Val Pro Ala Glu Asp Ile Pro Pro Pro Gly Ala Asp Gln Lys Ala
    610                 615                 620

Ser Glu Gln Gly Leu Asn Arg Asn Glu Gly Ala Pro Tyr Gly Val Ile
625                 630                 635                 640

Met Gly Pro Phe Leu Gly Pro Leu Gly Val Pro Cys Ser Ala Pro Pro
                645                 650                 655

Trp Gly Phe Val Ala Gly Ala Asp Leu Arg Thr Gly Glu Ile Ala Tyr
            660                 665                 670

Met His Arg Asn Gly Thr Val Arg Asp Met Thr Pro Leu Pro Leu Pro
        675                 680                 685

Phe Lys Val Gly Val Pro Gly Ile Gly Gly Pro Ile Val Thr Arg Gly
    690                 695                 700

Gly Val Ala Phe Leu Gly Ala Ala Val Asp Asp Tyr Leu Arg Ala Tyr
705                 710                 715                 720

Asp Val Thr Thr Gly Asp Gln Leu Trp Gln Ala Arg Leu Pro Ala Gly
                725                 730                 735

Gly Gln Ser Thr Pro Met Thr Tyr Glu Gln Asp Gly Arg Gln Phe Val
            740                 745                 750

Val Ile Val Ala Gly Gly His Gly Ser Val Gly Thr Lys Pro Gly Asp
        755                 760                 765

Tyr Val Ile Ala Tyr Ala Leu Pro Asp Gly Ser Glu Gly
    770                 775                 780

<210>  45
<211>  2391
<212>  DNA
<213>  Enterobacter sp.

<400>  45
atggctgaaa caaaaactca acagtcgcgt ctactggtaa ctttaacagc agcgttcgca      60

gcgttttgcg cactctatct gttgatcggt ggcgtctggc tggtcgcatt aggcggctcc     120

tggtattacc cgattgcggg tctggttatg gttggcgtaa ccgttctgct cttaaaaggt     180

aaacgatccg cactgtggct ctacgctgca ctgcttcttg tcacaatgtt ttggggagtc     240

tgggaagttg gcttcgactt ctgggcgctg acaccgcgca gcgacatcct ggtcttcttt     300

gggatctggc tgatcctgcc gttcgtctgg cgtagcctga gggttccttc cagcggtgca     360

gttgcggcgc tggtggtttc cctgctgatt accggcggca tgctaacctg ggctgggttt     420

aacgatccgc aggaagtgaa aggcacgctg agcgcagatt ccacacccgc tgcggcgatc     480

tctgacgttg cagacggtga ctggccggct tatggtcgca accaggaagg ccaacgttat 540
tctccgctga agcaaatcaa cgcggataac gttaaaaacc tgaaggaagc ctgggtgttc 600
cgcaccggcg accttaagca gccaaacgac ccgggcgaat tgaccaacga agtgacgcca 660
atcaaagtcg gcaacatgct ttatctgtgt actgcacacc agcgtctgtt tgcgcttgat 720
gcggcgaccg gtaaagagaa atggcacttc gacccgcagt tgaattctaa tccgtcgttc 780
cagcacatta cctgccgtgg cgtgtcctat cacgaagccc gcgcggataa tgccagcccg 840
gaagtggttg ctgattgccc gcgtcgcatc atgctgccgg tcaacgatgg tcgactgttc 900
gccatcaacg ccgaaaccgg caaactgtgc gaaacctttg gcaacaaagg cattctgaat 960
ctgcagacca atatgccgga taccacgccg ggtctgtacg aaccgacttc cccgccgatt 1020
atcaccgata aaacaattgt gattgccggt tcggtcacgg ataacttctc gactcgcgaa 1080
acgtccggcg ttatccgtgg tttcgacgta aacacgggca aactgctgtg ggccttcgat 1140
ccgggcgcga aagatcctaa cgcgattccg tcggatgaac acaccttcac cttcaactcg 1200
ccaaactcat gggcgcctgc ggcgtatgac gcgaagctag acctggtgta cctgccaatg 1260
ggtgtgacca cgccagatat ctggggcgga aaccgcacgc cagagcaaga gcgttacgcc 1320
agcgcgattg tggcgctgaa tgcgacgacc gggaaactgg cctggagcta tcagaccgta 1380
caccacgacc tgtgggatat ggatatgcca tcccagccga cgctggcgga tatcaccgtt 1440
aacggtaaaa ccgttccagt gatttatgcc ccggcgaaaa ccggcaacat ctttgtactc 1500
gaccgtacca acggcaaact ggtggttccg gcaccggaaa aaccggttcc gcaaggtgca 1560
gccaaaggcg actatgtctc taaaacccag cctttctcgg acctgagctt ccgtccaaag 1620
aaagacctga ccggtgccga tatgtggggc gccaccatgt tcgaccagct ggtgtgccgc 1680
gtgatgttcc atcagctgcg ctatgaaggc atcttcacgc cgccgtctga gcagggcacg 1740
ctggtcttcc cgggtaatct ggggatgttc gaatggggcg gtatctcggt tgatcctaac 1800
cgtcaggtgg cgattgcaaa cccgatggcg ttgccgttcg tctctaagct tatccctcgc 1860
ggcccgggta acccgatgga gcagccgaaa gatgcaaaag gtagcggtac agaagcgggc 1920
attcagccgc agtacggcgt accgtttggc gtgacgctga accccttcct gtcgccgttt 1980
ggcctgccgt gtaaacagcc tgcatggggt tatatttctg cgctggatct gaaaaccaat 2040
caggttgtgt ggaaaaaacg tattggtacg ccacaggaca gcatgccgtt cccgatgccg 2100
attcccgtgc cgttcaatat ggggatgcca atgctgggtg gtccgatttc caccgccggt 2160
aacgtcctgt tcatcgcggc gaccgcagat aactacctgc gcgcgtacaa catgagcaac 2220
ggtgaaaagc tgtggcaagg ccgtctgcct gcgggtgggc aagcgacgcc gatgacctat 2280
gaagtggatg gcaagcagta tgttgttatc tctgcgggcg gtcacggttc gtttggtacg 2340
aagatggcgg actatattgt cgcgtatgct ctgcctgacg atgtgaaata a 2391

<210>  46
<211>  796
<212>  PRT
<213>  Enterobacter sp.

<400>  46

Met Ala Glu Thr Lys Thr Gln Gln Ser Arg Leu Leu Val Thr Leu Thr
1               5                   10                  15

Ala Ala Phe Ala Ala Phe Cys Ala Leu Tyr Leu Leu Ile Gly Gly Val
            20                  25                  30

Trp Leu Val Ala Leu Gly Gly Ser Trp Tyr Tyr Pro Ile Ala Gly Leu
        35                  40                  45

Val Met Val Gly Val Thr Val Leu Leu Leu Lys Gly Lys Arg Ser Ala
    50                  55                  60

Leu Trp Leu Tyr Ala Ala Leu Leu Leu Val Thr Met Phe Trp Gly Val
65                  70                  75                  80

Trp Glu Val Gly Phe Asp Phe Trp Ala Leu Thr Pro Arg Ser Asp Ile
                85                  90                  95

Leu Val Phe Phe Gly Ile Trp Leu Ile Leu Pro Phe Val Trp Arg Ser
            100                 105                 110

Leu Arg Val Pro Ser Ser Gly Ala Val Ala Ala Leu Val Val Ser Leu
        115                 120                 125

Leu Ile Thr Gly Gly Met Leu Thr Trp Ala Gly Phe Asn Asp Pro Gln
    130                 135                 140

Glu Val Lys Gly Thr Leu Ser Ala Asp Ser Thr Pro Ala Ala Ala Ile
145                 150                 155                 160

Ser Asp Val Ala Asp Gly Asp Trp Pro Ala Tyr Gly Arg Asn Gln Glu
                165                 170                 175

Gly Gln Arg Tyr Ser Pro Leu Lys Gln Ile Asn Ala Asp Asn Val Lys
            180                 185                 190

Asn Leu Lys Glu Ala Trp Val Phe Arg Thr Gly Asp Leu Lys Gln Pro
        195                 200                 205

Asn Asp Pro Gly Glu Leu Thr Asn Glu Val Thr Pro Ile Lys Val Gly
    210                 215                 220

Asn Met Leu Tyr Leu Cys Thr Ala His Gln Arg Leu Phe Ala Leu Asp
225                 230                 235                 240

Ala Ala Thr Gly Lys Glu Lys Trp His Phe Asp Pro Gln Leu Asn Ser
                245                 250                 255

Asn Pro Ser Phe Gln His Ile Thr Cys Arg Gly Val Ser Tyr His Glu
            260                 265                 270

Ala Arg Ala Asp Asn Ala Ser Pro Glu Val Val Ala Asp Cys Pro Arg
        275                 280                 285

Arg Ile Met Leu Pro Val Asn Asp Gly Arg Leu Phe Ala Ile Asn Ala
    290                 295                 300

Glu Thr Gly Lys Leu Cys Glu Thr Phe Gly Asn Lys Gly Ile Leu Asn
305                 310                 315                 320

Leu Gln Thr Asn Met Pro Asp Thr Thr Pro Gly Leu Tyr Glu Pro Thr
                325                 330                 335

Ser Pro Pro Ile Ile Thr Asp Lys Thr Ile Val Ile Ala Gly Ser Val
            340                 345                 350

Thr Asp Asn Phe Ser Thr Arg Glu Thr Ser Gly Val Ile Arg Gly Phe
        355                 360                 365

Asp Val Asn Thr Gly Lys Leu Leu Trp Ala Phe Asp Pro Gly Ala Lys
    370                 375                 380

Asp Pro Asn Ala Ile Pro Ser Asp Glu His Thr Phe Thr Phe Asn Ser
385                 390                 395                 400

Pro Asn Ser Trp Ala Pro Ala Ala Tyr Asp Ala Lys Leu Asp Leu Val
                405                 410                 415

Tyr Leu Pro Met Gly Val Thr Thr Pro Asp Ile Trp Gly Gly Asn Arg
            420                 425                 430

Thr Pro Glu Gln Glu Arg Tyr Ala Ser Ala Ile Val Ala Leu Asn Ala
        435                 440                 445

Thr Thr Gly Lys Leu Ala Trp Ser Tyr Gln Thr Val His His Asp Leu
    450                 455                 460

Trp Asp Met Asp Met Pro Ser Gln Pro Thr Leu Ala Asp Ile Thr Val
465                 470                 475                 480

Asn Gly Lys Thr Val Pro Val Ile Tyr Ala Pro Ala Lys Thr Gly Asn
                485                 490                 495

Ile Phe Val Leu Asp Arg Thr Asn Gly Lys Leu Val Val Pro Ala Pro
            500                 505                 510

Glu Lys Pro Val Pro Gln Gly Ala Ala Lys Gly Asp Tyr Val Ser Lys
        515                 520                 525

Thr Gln Pro Phe Ser Asp Leu Ser Phe Arg Pro Lys Lys Asp Leu Thr
    530                 535                 540

Gly Ala Asp Met Trp Gly Ala Thr Met Phe Asp Gln Leu Val Cys Arg
545                 550                 555                 560

Val Met Phe His Gln Leu Arg Tyr Glu Gly Ile Phe Thr Pro Pro Ser
                565                 570                 575

Glu Gln Gly Thr Leu Val Phe Pro Gly Asn Leu Gly Met Phe Glu Trp
            580                 585                 590

Gly Gly Ile Ser Val Asp Pro Asn Arg Gln Val Ala Ile Ala Asn Pro
        595                 600                 605

Met Ala Leu Pro Phe Val Ser Lys Leu Ile Pro Arg Gly Pro Gly Asn
    610                 615                 620

Pro Met Glu Gln Pro Lys Asp Ala Lys Gly Ser Gly Thr Glu Ala Gly
625                 630                 635                 640

Ile Gln Pro Gln Tyr Gly Val Pro Phe Gly Val Thr Leu Asn Pro Phe
                645                 650                 655

Leu Ser Pro Phe Gly Leu Pro Cys Lys Gln Pro Ala Trp Gly Tyr Ile
            660                 665                 670

Ser Ala Leu Asp Leu Lys Thr Asn Gln Val Val Trp Lys Lys Arg Ile
        675                 680                 685

Gly Thr Pro Gln Asp Ser Met Pro Phe Pro Met Pro Ile Pro Val Pro
    690                 695                 700

Phe Asn Met Gly Met Pro Met Leu Gly Gly Pro Ile Ser Thr Ala Gly
705                 710                 715                 720

Asn Val Leu Phe Ile Ala Ala Thr Ala Asp Asn Tyr Leu Arg Ala Tyr
                725                 730                 735

Asn Met Ser Asn Gly Glu Lys Leu Trp Gln Gly Arg Leu Pro Ala Gly
            740                 745                 750

Gly Gln Ala Thr Pro Met Thr Tyr Glu Val Asp Gly Lys Gln Tyr Val
        755                 760                 765

Val Ile Ser Ala Gly Gly His Gly Ser Phe Gly Thr Lys Met Gly Asp
    770                 775                 780

Tyr Ile Val Ala Tyr Ala Leu Pro Asp Asp Val Lys
785                 790                 795

<210>  47
<211>  1431
<212>  DNA
<213>  Bacillus cereus

<400>  47
ttgacaaaag ttaaagttag tttacgaccc attgttcata atataaattt accaaccgta      60

ttgaaaacaa caatacttcc aggcgaatcg accgaaagac tatttatcgc aacccaatta     120

ggagagattt tttacatagg gaatggagtt ataaagatat ttttagatat tcgtcaccta     180

attattaaat taggtacatt tgaagaaggt gtttctagta gcggttatga tgaacgcgga     240

ttgcttggac ttgcgttcca tccacaattt tatcaaaatg ggttatttta tcttcattat     300

tcagtagctg gaactcaagg gccaggtgca ttttctgagc aatttaaacc gaatccttgt     360

gaccctaaaa cgctaaattt aaagtgggtt aatagaaata cgcaatatga tcacattgat     420

acagtagaag aatggacttt acaatctaat ggtcaagctc aaaagcgacg aacattactg     480

aatgtaagaa gaccattttt taatcataac ggagtcaata gtttaaactt ttcacctgag     540

actggaaaac ttgtttttac aaatggagat ggcggatcag gttatgatcc atttaattta     600

agccaagatg atttagaaat agcaggtaaa ataattgaaa tcgatgtaag taaaaataca     660

tttataaata accctcctgt agttacacgc ttcgatgaac ttcctttatc tatacaagaa     720

acacttacag taattgcaaa aggagtacgt aatataaccg gcatttcatt tcaaaggttt     780

tataatcaat atatcaaata cgccggaaat gtcggacaag atattgtaga gtctattttt     840

tcatttgttc aatataaacc tataccggtt acagaacttg ttcaaatgca ttttatgagg     900

ttaactccca atcaagatgg ggttatcaat tttgggtggc gaggatggga aggggattta     960

cctacttctt ttatacgaca ttgttctgag aatcagactt tggatgagag aacaatggtt    1020

tattatgatg aaacaataca aacttcagtc aagcgtattc tgcctctact tagttatttt 1080

cataaagatt ctagaacaga taagtttgga ggaacttcac ttacaggagt tcagccatat 1140

atgggaaatg caattccaaa tttaacaggt agcgtagtgt ttactgacct tgctaagaaa 1200

gaagattctc gaccgccagt taaaggtgtt ttagcctata ctagagcagg tacagacggt 1260

aaacatgctg actttcatgc tattgaaacc aattatgatt ttggtacgca agcagcttat 1320

tatatgagtt taggaacaaa tttgaatcaa actaaattat atttaggagt ttacagttct 1380

atgaaagtaa ctgattttaa taaaggtacc atttttgaaa ttattccctg a 1431

<210> 48
<211> 476
<212> PRT
<213> Bacillus cereus

<400> 48

Met Thr Lys Val Lys Val Ser Leu Arg Pro Ile Val His Asn Ile Asn
1               5                   10                  15

Leu Pro Thr Val Leu Lys Thr Thr Ile Leu Pro Gly Glu Ser Thr Glu
            20                  25                  30

Arg Leu Phe Ile Ala Thr Gln Leu Gly Glu Ile Phe Tyr Ile Gly Asn
        35                  40                  45

Gly Val Ile Lys Ile Phe Leu Asp Ile Arg His Leu Ile Ile Lys Leu
    50                  55                  60

Gly Thr Phe Glu Glu Gly Val Ser Ser Ser Gly Tyr Asp Glu Arg Gly
65                  70                  75                  80

Leu Leu Gly Leu Ala Phe His Pro Gln Phe Tyr Gln Asn Gly Leu Phe
                85                  90                  95

Tyr Leu His Tyr Ser Val Ala Gly Thr Gln Gly Pro Gly Ala Phe Ser
            100                 105                 110

Glu Gln Phe Lys Pro Asn Pro Cys Asp Pro Lys Thr Leu Asn Leu Lys
        115                 120                 125

Trp Val Asn Arg Asn Thr Gln Tyr Asp His Ile Asp Thr Val Glu Glu
    130                 135                 140

Trp Thr Leu Gln Ser Asn Gly Gln Ala Gln Lys Arg Arg Thr Leu Leu
145                 150                 155                 160

Asn Val Arg Arg Pro Phe Phe Asn His Asn Gly Val Asn Ser Leu Asn

                    165                 170                 175

Phe Ser Pro Glu Thr Gly Lys Leu Val Phe Thr Asn Gly Asp Gly Gly
            180                 185                 190

Ser Gly Tyr Asp Pro Phe Asn Leu Ser Gln Asp Asp Leu Glu Ile Ala
        195                 200                 205

Gly Lys Ile Ile Glu Ile Asp Val Ser Lys Asn Thr Phe Ile Asn Asn
    210                 215                 220

Pro Pro Val Val Thr Arg Phe Asp Glu Leu Pro Leu Ser Ile Gln Glu
225                 230                 235                 240

Thr Leu Thr Val Ile Ala Lys Gly Val Arg Asn Ile Thr Gly Ile Ser
                245                 250                 255

Phe Gln Arg Phe Tyr Asn Gln Tyr Ile Lys Tyr Ala Gly Asn Val Gly
            260                 265                 270

Gln Asp Ile Val Glu Ser Ile Phe Ser Phe Val Gln Tyr Lys Pro Ile
        275                 280                 285

Pro Val Thr Glu Leu Val Gln Met His Phe Met Arg Leu Thr Pro Asn
    290                 295                 300

Gln Asp Gly Val Ile Asn Phe Gly Trp Arg Gly Trp Glu Gly Asp Leu
305                 310                 315                 320

Pro Thr Ser Phe Ile Arg His Cys Ser Glu Asn Gln Thr Leu Asp Glu
                325                 330                 335

Arg Thr Met Val Tyr Tyr Asp Glu Thr Ile Gln Thr Ser Val Lys Arg
            340                 345                 350

Ile Leu Pro Leu Leu Ser Tyr Phe His Lys Asp Ser Arg Thr Asp Lys
        355                 360                 365

Phe Gly Gly Thr Ser Leu Thr Gly Val Gln Pro Tyr Met Gly Asn Ala
    370                 375                 380

Ile Pro Asn Leu Thr Gly Ser Val Val Phe Thr Asp Leu Ala Lys Lys
385                 390                 395                 400

Glu Asp Ser Arg Pro Pro Val Lys Gly Val Leu Ala Tyr Thr Arg Ala
                405                 410                 415

Gly Thr Asp Gly Lys His Ala Asp Phe His Ala Ile Glu Thr Asn Tyr
            420                 425                 430

Asp Phe Gly Thr Gln Ala Ala Tyr Tyr Met Ser Leu Gly Thr Asn Leu
        435                 440                 445

Asn Gln Thr Lys Leu Tyr Leu Gly Val Tyr Ser Ser Met Lys Val Thr
    450                 455                 460

Asp Phe Asn Lys Gly Thr Ile Phe Glu Ile Ile Pro
465                 470                 475

<210> 49
<211> 1176
<212> DNA
<213> Micromonospora sp.

<400> 49
gtgagcccgc gtcttgcgta cccccgtgtc agccgcctcc ggacggcgct ggcggcgtcc      60

tgcgcggcgt tgctcctggg cgccgccggc tgctccctcg gcgagcccga gccggacccg     120

gccggcgcac cgcccaacct gcccacaccg tccggcaccg cgagtcccgg cggggccggt     180

cagcaggttg tcgccaccgt gctggccaag gggctggagg tgccgtgggg catcgcgttc      240

ctgcccgacg gcggggcgct ggtcaccgag cgggacaagg gacggatcct ccaggtcggc     300

ccggagtccg gcccggacgg gctggccgtc cggccggtcc agaccgtgcc ggacgtggcc     360

gccggcggcg agggcggcct gctgggcatc gcggtctccc ccgcgtacgc caaggaccgc     420

acggtcttcg tctactacac ggccgaggac gacaaccgga tcgcgaagat gcagctcggg     480

caggcgccga agccgatcct gaccggcatc ccgaagtccg gcacgcacaa cggcggcggg     540

ctcggcttcg gcccggacgg ccacctctac gcgagcaccg gcgacgccgg gcgcaccgag     600

aacgcccagg acgccaagag cctcggcggc aagatcctcc ggatcacgcc ggacggcaag     660

cctgcgccgg gcaaccccac cgccggatcg ccggtctggt cgacgggcca ccgcaacgtc     720

cagggcttcg cctggacgcc ggacaagaag atgtacgcgg tggagttcgg ccagaacacc     780

tgggacgaga tcaaccagat caacaagggc gggaactacg gctggccccg ggtcgagggg     840

cgcggcgacg acaagcgcta cgtgaacccg atcacacagt ggcccaccgg tgacgcctcc     900

tgttccggcc tggcggcggc ggaccgtctg ctcgtcgccg cctgcctgcg cggccagcga     960

ctctggctgg tggagctggc cggcaacggc acagtcctcg gtcagccgcg cgacctgctc    1020

aacggccggt acggtcggct acgggccgtc gcggcggcgc cggacggctc gctctgggtg    1080

agcacgtcga accgggacgg gcggggtacg cccaccgccg aggacgaccg cctcctgcgg    1140

ctcgtcttcg ccgacggggg cgccggccgg agctga                              1176

<210>  50
<211>  391
<212>  PRT
<213>  Micromonospora sp.

<400>  50

Met Ser Pro Arg Leu Ala Tyr Pro Arg Val Ser Arg Leu Arg Thr Ala
1               5                   10                  15

Leu Ala Ala Ser Cys Ala Ala Leu Leu Leu Gly Ala Ala Gly Cys Ser
            20                  25                  30

Leu Gly Glu Pro Glu Pro Asp Pro Ala Gly Ala Pro Pro Asn Leu Pro
        35                  40                  45

Thr Pro Ser Gly Thr Ala Ser Pro Gly Gly Ala Gly Gln Gln Val Val
    50                  55                  60

Ala Thr Val Leu Ala Lys Gly Leu Glu Val Pro Trp Gly Ile Ala Phe
65                  70                  75                  80

Leu Pro Asp Gly Gly Ala Leu Val Thr Glu Arg Asp Lys Gly Arg Ile
                85                  90                  95

Leu Gln Val Gly Pro Glu Ser Gly Pro Asp Gly Leu Ala Val Arg Pro
            100                 105                 110

Val Gln Thr Val Pro Asp Val Ala Ala Gly Gly Glu Gly Gly Leu Leu
        115                 120                 125

Gly Ile Ala Val Ser Pro Ala Tyr Ala Lys Asp Arg Thr Val Phe Val
    130                 135                 140

Tyr Tyr Thr Ala Glu Asp Asp Asn Arg Ile Ala Lys Met Gln Leu Gly
145                 150                 155                 160

Gln Ala Pro Lys Pro Ile Leu Thr Gly Ile Pro Lys Ser Gly Thr His
                165                 170                 175

Asn Gly Gly Gly Leu Gly Phe Gly Pro Asp Gly His Leu Tyr Ala Ser
            180                 185                 190

Thr Gly Asp Ala Gly Arg Thr Glu Asn Ala Gln Asp Ala Lys Ser Leu
        195                 200                 205

Gly Gly Lys Ile Leu Arg Ile Thr Pro Asp Gly Lys Pro Ala Pro Gly
    210                 215                 220

Asn Pro Thr Ala Gly Ser Pro Val Trp Ser Thr Gly His Arg Asn Val
225                 230                 235                 240

Gln Gly Phe Ala Trp Thr Pro Asp Lys Lys Met Tyr Ala Val Glu Phe
                245                 250                 255

Gly Gln Asn Thr Trp Asp Glu Ile Asn Gln Ile Asn Lys Gly Gly Asn
            260                 265                 270

Tyr Gly Trp Pro Arg Val Glu Gly Arg Gly Asp Asp Lys Arg Tyr Val
        275                 280                 285

Asn Pro Ile Thr Gln Trp Pro Thr Gly Asp Ala Ser Cys Ser Gly Leu
    290                 295                 300

Ala Ala Ala Asp Arg Leu Leu Val Ala Ala Cys Leu Arg Gly Gln Arg
305                 310                 315                 320

Leu Trp Leu Val Glu Leu Ala Gly Asn Gly Thr Val Leu Gly Gln Pro
                325                 330                 335

Arg Asp Leu Leu Asn Gly Arg Tyr Gly Arg Leu Arg Ala Val Ala Ala
            340                 345                 350

Ala Pro Asp Gly Ser Leu Trp Val Ser Thr Ser Asn Arg Asp Gly Arg
        355                 360                 365

Gly Thr Pro Thr Ala Glu Asp Asp Arg Leu Leu Arg Leu Val Phe Ala
    370                 375                 380

Asp Gly Gly Ala Gly Arg Ser
385                 390

<210>  51
<211>  2475
<212>  DNA
<213>  Xanthomonas campestris

<400>  51
atgactgatc aatcctctaa acgcgggctc ggcacttggc tgttggtggc ctatgccgtg      60

gtgctcgccg tgcttggtgc ggcgctcgcc tatgaaggcg ggcgcctggt ggccgttggt     120

ggctcctggt actacgtgct ggccggtatc gccgtgctgg tggccggtgt gctgctggca     180

ctgggcaaac gcgctggcct gtggctgttt ggcgctacgt tggcggccac catcgtgtgg     240

gcgctgtggg aagtgggcct ggatggttgg ggcctgatcc cacgtctggc ctggatctcg     300

gtgctcggcc tggtgctgtt gccgttctgg ggcgtggcgc ggcggcgcat gcagccgttg     360

tccgggctcg gctatgccgt ggtcaccggc gtgctgccgg tgctgggcgc agcgctgatc     420

ctgtggccgc tgctggtgcc gcgcaatgtg gaactggccg atgcatccaa gcagccagcc 480
gatgcggcaa cgccgttcag ccgtggcagc gtgcccagcc cggacgggaa cgtggcggcc 540
aaccacgatg ccagtaactg gaccgcctac gccggttcca acctgtccaa ccattacacc 600
cccggcgcgc agatcacgcc ggagaacgtc aagggcctca aggtggcctg ggaattccat 660
accggcgatc tcaagccgaa ggattccaag ctgggctatg cgttccagaa caccccgctc 720
aaggtcggcg acctgctcta catctgcacc ccgacccaga aggtgatcgc ggtggaggcg 780
gccaacggca aggagcgctg gcgcttcgac ccgcagacca atccgaaggc gatggccggc 840
gtggccgcca ccacctgccg tggtgtgtcg tactaccagg cgccggaagg cactgccgag 900
tgcccgacgc ggatcttctg gccgatggtc gatggccgcc tgggtgcgct ggatgcgcag 960
accggcaagc tctgcgccag cttcggtaac aacggctatg tggacctcaa tgccggcacc 1020
ggcaacacca agccgggctt cgtcggcccg acctcgccgc cggtggtcat gcgcggtgtg 1080
gtgatccagc ccaccgggca ggtgcgcgac ggtcaggaac gcgatgcgcc gtccggcgtg 1140
gtgcgcggct tcgacgcgct caccggccag ctgcgctggg cctgggatct gggcaacccg 1200
gccatcaccg ccgaaccgcc ggccggccag acctataccc gctccacccc gaacgtgtgg 1260
tcgctgatgg ccgccgatga cgagctgggg ctggtgtatc tgcccaccgg caacgctgcc 1320
ggcgacttct tcggcaaggg ccgcaccccg caggaagagg aatacaccgc ctcgctggtg 1380
gccgtggatg cggcaaccgg caaggagcgc tggcacttcc gtaccgtcaa tcacgatctg 1440
tgggactacg acatcggccc gcagccgaac ctggttgatt ggccggttgc cggcggtggc 1500
acccgccccg ccgtgatcca ggccaccaag tccggccagg tgttcgtgct cgaccgggcc 1560
accggccagc cgatcatgcc ggtcaagcag attgcggtac cgcagggcac cgatcatggc 1620
gactggaccg catccaccca gccggtgtcg ccgggcatgc ccaacaccgt gggtgcgccc 1680
agccgtgact acgagaccat cgttgaatct gacgcgtggg gcatgacccc gttcgaccag 1740
ctcgcttgcc gcatcgagtt caagaagctg cggtacgaag gcatgttcac cccgccaagc 1800
ctgcagggct cgctgtcgtt caccggcaac catggcggca tcaactgggg cggcgtgtcg 1860
gtcgatctgc agcgcggcat catggtgatg aacagcaatc gcctgcccta taccgagcac 1920
gtctacccgc gcacggtgat gaacgagctg ggcgtggtgt cggtgttcaa cggcagcagc 1980
aagaccaagg gctacatggc gcaggaaggc ctggcctatg gcgcgcgcaa ggagccgtgg 2040
atgtcgccgc tcaacacgcc gtgcgtggca ccgccgtggg gctatatctc cggcgtggat 2100
ctgcgcacgc agcaggtgat ctggcgccgt ccgctgggca ccggttacga ccagggccca 2160
atgggcatcc cgtccaagac caagttcgag atcggcaccc cgaacaacag cggctcgctg 2220
gccactgccg gcggcgtcac cttcatcggt gccagcctgg acaacttcat ccgcggcttt 2280

gacacccgca ccggcaagca ggtctgggag acccgcgtgc ctgcgggccc gcaggccgcg 2340

ccgctgagct acaccatcga tggcaagcag tacatcgttg ccgcggtggg tggccatgac 2400

cgcatggaaa ccaagtccgg cgacagcgtc attgcctggg cactgcccga cgacgcggcc 2460

gccagcgcca aataa 2475

<210> 52
<211> 824
<212> PRT
<213> Xanthomonas campestris

<400> 52

Met Thr Asp Gln Ser Ser Lys Arg Gly Leu Gly Thr Trp Leu Leu Val
1               5                   10                  15

Ala Tyr Ala Val Val Leu Ala Val Leu Gly Ala Ala Leu Ala Tyr Glu
            20                  25                  30

Gly Gly Arg Leu Val Ala Val Gly Gly Ser Trp Tyr Tyr Val Leu Ala
        35                  40                  45

Gly Ile Ala Val Leu Val Ala Gly Val Leu Leu Ala Leu Gly Lys Arg
    50                  55                  60

Ala Gly Leu Trp Leu Phe Gly Ala Thr Leu Ala Ala Thr Ile Val Trp
65                  70                  75                  80

Ala Leu Trp Glu Val Gly Leu Asp Gly Trp Gly Leu Ile Pro Arg Leu
                85                  90                  95

Ala Trp Ile Ser Val Leu Gly Leu Val Leu Leu Pro Phe Trp Gly Val
            100                 105                 110

Ala Arg Arg Arg Met Gln Pro Leu Ser Gly Leu Gly Tyr Ala Val Val
        115                 120                 125

Thr Gly Val Leu Pro Val Leu Gly Ala Ala Leu Ile Leu Trp Pro Leu
    130                 135                 140

Leu Val Pro Arg Asn Val Glu Leu Ala Asp Ala Ser Lys Gln Pro Ala
145                 150                 155                 160

Asp Ala Ala Thr Pro Phe Ser Arg Gly Ser Val Pro Ser Pro Asp Gly
                165                 170                 175

Asn Val Ala Ala Asn His Asp Ala Ser Asn Trp Thr Ala Tyr Ala Gly
            180                 185                 190

Ser Asn Leu Ser Asn His Tyr Thr Pro Gly Ala Gln Ile Thr Pro Glu
        195                 200                 205

Asn Val Lys Gly Leu Lys Val Ala Trp Glu Phe His Thr Gly Asp Leu
    210                 215                 220

Lys Pro Lys Asp Ser Lys Leu Gly Tyr Ala Phe Gln Asn Thr Pro Leu
225                 230                 235                 240

Lys Val Gly Asp Leu Leu Tyr Ile Cys Thr Pro Thr Gln Lys Val Ile
                245                 250                 255

Ala Val Glu Ala Ala Asn Gly Lys Glu Arg Trp Arg Phe Asp Pro Gln
            260                 265                 270

Thr Asn Pro Lys Ala Met Ala Gly Val Ala Ala Thr Thr Cys Arg Gly
        275                 280                 285

Val Ser Tyr Tyr Gln Ala Pro Glu Gly Thr Ala Glu Cys Pro Thr Arg
    290                 295                 300

Ile Phe Trp Pro Met Val Asp Gly Arg Leu Gly Ala Leu Asp Ala Gln
305                 310                 315                 320

Thr Gly Lys Leu Cys Ala Ser Phe Gly Asn Asn Gly Tyr Val Asp Leu
                325                 330                 335

Asn Ala Gly Thr Gly Asn Thr Lys Pro Gly Phe Val Gly Pro Thr Ser
            340                 345                 350

Pro Pro Val Val Met Arg Gly Val Val Ile Gln Pro Thr Gly Gln Val
        355                 360                 365

Arg Asp Gly Gln Glu Arg Asp Ala Pro Ser Gly Val Val Arg Gly Phe
    370                 375                 380

Asp Ala Leu Thr Gly Gln Leu Arg Trp Ala Trp Asp Leu Gly Asn Pro
385                 390                 395                 400

Ala Ile Thr Ala Glu Pro Pro Ala Gly Gln Thr Tyr Thr Arg Ser Thr
                405                 410                 415

Pro Asn Val Trp Ser Leu Met Ala Ala Asp Asp Glu Leu Gly Leu Val
            420                 425                 430

Tyr Leu Pro Thr Gly Asn Ala Ala Gly Asp Phe Phe Gly Lys Gly Arg
        435                 440                 445

Thr Pro Gln Glu Glu Glu Tyr Thr Ala Ser Leu Val Ala Val Asp Ala
    450                 455                 460

Ala Thr Gly Lys Glu Arg Trp His Phe Arg Thr Val Asn His Asp Leu
465                 470                 475                 480

Trp Asp Tyr Asp Ile Gly Pro Gln Pro Asn Leu Val Asp Trp Pro Val
                485                 490                 495

Ala Gly Gly Gly Thr Arg Pro Ala Val Ile Gln Ala Thr Lys Ser Gly
            500                 505                 510

Gln Val Phe Val Leu Asp Arg Ala Thr Gly Gln Pro Ile Met Pro Val
        515                 520                 525

Lys Gln Ile Ala Val Pro Gln Gly Thr Asp His Gly Asp Trp Thr Ala
    530                 535                 540

Ser Thr Gln Pro Val Ser Pro Gly Met Pro Asn Thr Val Gly Ala Pro
545                 550                 555                 560

Ser Arg Asp Tyr Glu Thr Ile Val Glu Ser Asp Ala Trp Gly Met Thr
                565                 570                 575

Pro Phe Asp Gln Leu Ala Cys Arg Ile Glu Phe Lys Lys Leu Arg Tyr
            580                 585                 590

Glu Gly Met Phe Thr Pro Pro Ser Leu Gln Gly Ser Leu Ser Phe Thr
        595                 600                 605

Gly Asn His Gly Gly Ile Asn Trp Gly Gly Val Ser Val Asp Leu Gln
    610                 615                 620

Arg Gly Ile Met Val Met Asn Ser Asn Arg Leu Pro Tyr Thr Glu His
625                 630                 635                 640

Val Tyr Pro Arg Thr Val Met Asn Glu Leu Gly Val Val Ser Val Phe
                645                 650                 655

Asn Gly Ser Ser Lys Thr Lys Gly Tyr Met Ala Gln Glu Gly Leu Ala
            660                 665                 670

Tyr Gly Ala Arg Lys Glu Pro Trp Met Ser Pro Leu Asn Thr Pro Cys
        675                 680                 685

Val Ala Pro Pro Trp Gly Tyr Ile Ser Gly Val Asp Leu Arg Thr Gln

690                 695                 700

Gln Val Ile Trp Arg Arg Pro Leu Gly Thr Gly Tyr Asp Gln Gly Pro
705                 710                 715                 720

Met Gly Ile Pro Ser Lys Thr Lys Phe Glu Ile Gly Thr Pro Asn Asn
                725                 730                 735

Ser Gly Ser Leu Ala Thr Ala Gly Gly Val Thr Phe Ile Gly Ala Ser
            740                 745                 750

Leu Asp Asn Phe Ile Arg Gly Phe Asp Thr Arg Thr Gly Lys Gln Val
        755                 760                 765

Trp Glu Thr Arg Val Pro Ala Gly Pro Gln Ala Ala Pro Leu Ser Tyr
    770                 775                 780

Thr Ile Asp Gly Lys Gln Tyr Ile Val Ala Ala Val Gly Gly His Asp
785                 790                 795                 800

Arg Met Glu Thr Lys Ser Gly Asp Ser Val Ile Ala Trp Ala Leu Pro
                805                 810                 815

Asp Asp Ala Ala Ala Ser Ala Lys
            820

<210>  53
<211>  2412
<212>  DNA
<213>  Pseudomonas fluorescens

<400>  53
atgagcactg aaggtgcttc gagtcgaagc cgtcttctgc cgaacctgct cggcattctg      60

cttctgctga tgggcctggc catgctggcc gggggaatca agctgagtac gctcggcggc     120

tcgctgtatt acctgctggc cggtatcggc atcacgctga ccggcattct gatgctgatg     180

cgccgtcgcg cagcattggg cctgtacgcc atcgtgctgt tcgccagcac tgtctgggcg     240

ctgtgggaag ttggtctgga ctggtggcaa ctggtgccgc gtctggcgct gtggtttgtc     300

ctcggtttcg tgatgctgct gccgtggttc cgtcgtccgc tgctgctcgc aggccctgcg     360

ccgatgggca ccggcggttt gaccgtggct gtgattctgg ccggcgtcac tgccctggcc     420

agcctgttca cccacccggg cgaaaccttc ggcgaactgg gtcgcgacac cgccgacacc     480

accagcaccg ccccgcaaat gccggacggc gactggcagg cctacggccg caccgaattc     540

ggtgaccgct actcgccact gaaacagatc accccggcca acgtcggcaa gctgcaagaa     600

gcctggcgca tccagaccgg cgacctgccg actgccgacg acccggtcga gctgaccaac     660

gaaaacaccc cgctgaaagc caacggcatg ctttacgcct gcactgcgca cagcaaagtg 720

ctggcactgg acccggacac cggcaaggaa ctatggcgct tcgatccgca gatcaaaagc 780

ccggaaggct tcaagggctt cgcccacatg acctgccgtg gcgtgtcgta ctacgacgaa 840

gccgcgtacg ccaagtctga aaacgcggcg tccaccgtga tctccgaggc cggcaaagcc 900

gtcgcccagg cctgcccgcg tcgcctgtac ctgcctaccg ccgatgcccg cctgatcgca 960

ctgaacgccg acaccggcaa gatctgcgaa ggcttcggca ccaacggtgt ggttgacctg 1020

acccagggca tcggcccgtt caccgctggc ggttactact ccacctctcc tgccgcgatc 1080

acccgtgatc tggtgatcat gggcggtcac gtcaccgaca acgaatcgac caacgagccg 1140

tccggcgtga tccgcgccta cgacgtgcgc gacggccatc tggtctggaa ctgggacagc 1200

aacaacccgg acgccaccga acccttggcc ccgggccaga cctacagccg caactcggcc 1260

aacatgtggt cgctggccag cgtcgatgaa aaactcggca tggtctatct gccattgggc 1320

aaccagacgc ctgaccagtg ggcggcgcat cgcacccccg gtgccgagaa attcagcgcc 1380

ggcctggtcg cgctggacct ggcgaccggc aaggtgcgct ggaactacca gttcacccac 1440

cacgacctgt gggacatgga cgtcggcagc cagccaaccc tgctggacat gaaaaccgcc 1500

gacggcatca agcctgcgct gatcgccccg accaagcagg gcagcctgta cgtcctcgac 1560

cgtcgtgacg gcacgccgat catcccgatc cgcgagatcc cggtgccgca aggcgccgtg 1620

aaaggcgacc acaccgcccc gacccaggcc cgttcggacc tgaacctgct ggccccggaa 1680

ctgaccgaaa aagccatgtg gggcgccagc ccgttcgacc agatgttgtg ccgcatccag 1740

ttcaaggaac tgcgctacga aggccaatac acgcctccgt cggaacaggg cagcctgatc 1800

tacccgggta acgtcggcgt gttcaactgg ggcggtgttt ctgtcgaccc ggttcgccag 1860

atgctgttca ccagcccgaa ctacatggct ttcgtctcca agatgatccc acgtgccgac 1920

gtacccgccg acagcaagcg cgaaagcgaa acttccggca tccagaaaaa caccggtgcg 1980

ccgtatgccg tgaccatgca cccgttcatg tcgccactgg gcgtaccttg ccaggccccg 2040

gcctggggct acgtcgccgg tatcgacctg accaccggca aagtcgtctg gaaacgcaaa 2100

aacggtacca gccgcgacag ctcgccaatc ccgatcggct tcaccctggg cgtgccaagc 2160

atgggcggct cgatcgtcac tgccggcggc gtcggcttcc tcagcggcac gctggaccag 2220

tacctgcgcg cctacgacgt gaacaccggt aaagaactgt ggaaatcgcg tctgccggcc 2280

ggcggccagg cgaccccaat gacctacacc ggcaaggacg gcaagcaata cgtcctgctc 2340

gtggtgggtg gtcacggctc gctgggcacc aagatgggtg actatgtgat tgcctacaaa 2400

ctgccggaat aa 2412

<210> 54
<211> 803

<212>  PRT
<213>  Pseudomonas fluorescens

<400>  54
   Met Ser Thr Glu Gly Ala Ser Ser Arg Ser Arg Leu Leu Pro Asn Leu
   1               5                   10                  15
   Leu Gly Ile Leu Leu Leu Leu Met Gly Leu Ala Met Leu Ala Gly Gly
               20                  25                  30
   Ile Lys Leu Ser Thr Leu Gly Gly Ser Leu Tyr Tyr Leu Leu Ala Gly
           35                  40                  45
   Ile Gly Ile Thr Leu Thr Gly Ile Leu Met Leu Met Arg Arg Arg Ala
       50                  55                  60
   Ala Leu Gly Leu Tyr Ala Ile Val Leu Phe Ala Ser Thr Val Trp Ala
   65                  70                  75                  80
   Leu Trp Glu Val Gly Leu Asp Trp Trp Gln Leu Val Pro Arg Leu Ala
                   85                  90                  95
   Leu Trp Phe Val Leu Gly Phe Val Met Leu Leu Pro Trp Phe Arg Arg
               100                 105                 110
   Pro Leu Leu Leu Ala Gly Pro Ala Pro Met Gly Thr Gly Gly Leu Thr
           115                 120                 125
   Val Ala Val Ile Leu Ala Gly Val Thr Ala Leu Ala Ser Leu Phe Thr
       130                 135                 140
   His Pro Gly Glu Thr Phe Gly Glu Leu Gly Arg Asp Thr Ala Asp Thr
   145                 150                 155                 160
   Thr Ser Thr Ala Pro Gln Met Pro Asp Gly Asp Trp Gln Ala Tyr Gly
                   165                 170                 175
   Arg Thr Glu Phe Gly Asp Arg Tyr Ser Pro Leu Lys Gln Ile Thr Pro
               180                 185                 190
   Ala Asn Val Gly Lys Leu Gln Glu Ala Trp Arg Ile Gln Thr Gly Asp
           195                 200                 205
   Leu Pro Thr Ala Asp Asp Pro Val Glu Leu Thr Asn Glu Asn Thr Pro
       210                 215                 220
   Leu Lys Ala Asn Gly Met Leu Tyr Ala Cys Thr Ala His Ser Lys Val
   225                 230                 235                 240
   Leu Ala Leu Asp Pro Asp Thr Gly Lys Glu Leu Trp Arg Phe Asp Pro
                   245                 250                 255
   Gln Ile Lys Ser Pro Glu Gly Phe Lys Gly Phe Ala His Met Thr Cys
               260                 265                 270
   Arg Gly Val Ser Tyr Tyr Asp Glu Ala Ala Tyr Ala Lys Ser Glu Asn
           275                 280                 285
   Ala Ala Ser Thr Val Ile Ser Glu Ala Gly Lys Ala Val Ala Gln Ala
       290                 295                 300
   Cys Pro Arg Arg Leu Tyr Leu Pro Thr Ala Asp Ala Arg Leu Ile Ala
   305                 310                 315                 320
   Leu Asn Ala Asp Thr Gly Lys Ile Cys Glu Gly Phe Gly Thr Asn Gly
                   325                 330                 335
   Val Val Asp Leu Thr Gln Gly Ile Gly Pro Phe Thr Ala Gly Gly Tyr
               340                 345                 350
   Tyr Ser Thr Ser Pro Ala Ala Ile Thr Arg Asp Leu Val Ile Met Gly
           355                 360                 365
   Gly His Val Thr Asp Asn Glu Ser Thr Asn Glu Pro Ser Gly Val Ile
       370                 375                 380
   Arg Ala Tyr Asp Val Arg Asp Gly His Leu Val Trp Asn Trp Asp Ser
   385                 390                 395                 400
   Asn Asn Pro Asp Ala Thr Glu Pro Leu Ala Pro Gly Gln Thr Tyr Ser
                   405                 410                 415
   Arg Asn Ser Ala Asn Met Trp Ser Leu Ala Ser Val Asp Glu Lys Leu
               420                 425                 430
   Gly Met Val Tyr Leu Pro Leu Gly Asn Gln Thr Pro Asp Gln Trp Gly
           435                 440                 445
   Gly Asp Arg Thr Pro Gly Ala Glu Lys Phe Ser Ala Gly Leu Val Ala
       450                 455                 460
   Leu Asp Leu Ala Thr Gly Lys Val Arg Trp Asn Tyr Gln Phe Thr His
   465                 470                 475                 480
   His Asp Leu Trp Asp Met Asp Val Gly Ser Gln Pro Thr Leu Leu Asp
   485 490 495
   Met Lys Thr Ala Asp Gly Ile Lys Pro Ala Leu Ile Ala Pro Thr Lys
   500 505 510
   Gln Gly Ser Leu Tyr Val Leu Asp Arg Arg Asp Gly Thr Pro Ile Ile
   515 520 525
   Pro Ile Arg Glu Ile Pro Val Pro Gln Gly Ala Val Lys Gly Asp His
   530 535 540
   Thr Ala Pro Thr Gln Ala Arg Ser Asp Leu Asn Leu Leu Ala Pro Glu
   545 550 555 560
   Leu Thr Glu Lys Ala Met Trp Gly Ala Ser Pro Phe Asp Gln Met Leu
   565 570 575
   Cys Arg Ile Gln Phe Lys Glu Leu Arg Tyr Glu Gly Gln Tyr Thr Pro
   580 585 590
   Pro Ser Glu Gln Gly Ser Leu Ile Tyr Pro Gly Asn Val Gly Val Phe
   595 600 605
   Asn Trp Gly Gly Val Ser Val Asp Pro Val Arg Gln Met Leu Phe Thr
   610 615 620
   Ser Pro Asn Tyr Met Ala Phe Val Ser Lys Met Ile Pro Arg Ala Asp
   625 630 635 640
   Val Pro Ala Asp Ser Lys Arg Glu Ser Glu Thr Ser Gly Ile Gln Lys
   645 650 655
   Asn Thr Gly Ala Pro Tyr Ala Val Thr Met His Pro Phe Met Ser Pro
   660 665 670
   Leu Gly Val Pro Cys Gln Ala Pro Ala Trp Gly Tyr Val Ala Gly Ile
   675 680 685
   Asp Leu Thr Thr Gly Lys Val Val Trp Lys Arg Lys Asn Gly Thr Ser
   690 695 700
   Arg Asp Ser Ser Pro Ile Pro Ile Gly Phe Thr Leu Gly Val Pro Ser
   705 710 715 720
   Met Gly Gly Ser Ile Val Thr Ala Gly Gly Val Gly Phe Leu Ser Gly
   725 730 735
   Thr Leu Asp Gln Tyr Leu Arg Ala Tyr Asp Val Asn Thr Gly Lys Glu
   740 745 750
   Leu Trp Lys Ser Arg Leu Pro Ala Gly Gly Gln Ala Thr Pro Met Thr
   755 760 765
   Tyr Thr Gly Lys Asp Gly Lys Gln Tyr Val Leu Leu Val Val Gly Gly
   770 775 780
   His Gly Ser Leu Gly Thr Lys Met Gly Asp Tyr Val Ile Ala Tyr Lys
   785 790 795 800
   Leu Pro Glu

<210>  55
<211>  3525
<212>  DNA
<213>  Rhodopirellula baltica

<400>  55
atgaggtctc ttatcagcac tatgtcgtcg ccaattgctt tcccctctcg ctcgttttcc      60

cgttctttgc gtcggtcgcg gcgtccgtct tccttcattc gctccgcgat cgggctcggc     120

ctcatcgtcg ctgcgatgcc ggtctgtgcc gttctcagtg gtaccgattc gatcggctcg     180

gtgagtgccg acgagccagc tgccgcggcg gtgacgaata ccaaacccga agcgctggaa     240

ccggaaatcg ccgaggcgtc cgaagaagcg gcccaggcga tggcgggatt caagatccct     300

gaaggctggg aaatcaaact tttcgcggcc gagccccaag tcgccaacat cgtcgcgttt     360

ggcgtggatt cgaagggccg agtctacgtt tgcgaaagct accggcagaa tcgcggcgtg     420

acggacaacc gaggtcacga cgacgaatgg ttgctggctg atttgtcggc cgagacagtt     480

caagaccgaa ttgattacca caaacggttg ctcggtgaag cggcgatcac ttacgcacaa     540

catgacgatc ggatccgccg gttaacggac accgacggcg atggtgtcgc cgatgagtcc     600

accgtcgtgg ccgatggctt caacggtttg gaagagggga ccggcgcggg agttttgatc     660

aacggttcgg atatctatta cacctgcatt cccaaactgt ggaaattgac cgacgcggat     720

gacgacggcg tcgcggaaga ttcgcaagtg ttgtcggatg ggtatggcgt ccgagtcgct     780

ttccgtggtc acgacatgca cgggttgatc cgaggctatg acgggcgttt gtacttctcg     840

atcggagacc gtggttacca cgtgacgact cccgaaggaa agctgttgtc caacccagcc     900

gtcggcgctg tgttccgttg cgaaatggac ggcagccagt tggaagttta ttgcaacggt     960

ttgcggaacc cgcaggagtt ggccttcaac gacatcggcg actggttcac cgtcgacaac    1020

aactccgaca gtggcgacaa agctcgtttg gttcatctgt tggaaggtgg cgacacgggt    1080

tggcggatgc actatcagta cttgcctgat cgcggacctt tcaatcgttt gaagatttgg 1140

gagccgcacc acaacgaaca accggctcac cttgttccgc cgatcatcaa cttcaccgac 1200
ggaccatcgg gactcgcctt ttatccgggc accggattcg gggaactact cgacaatcaa 1260
tttctgattt gtgatttccg tggcggccca tccaatagcg gcatccggtc gttctcggtg 1320
gatcccgatg gggcctttta caaaatgggc gccgatgacc agccgatttg gaacattctc 1380
gcgaccgacg ttgcgttcac tccttcgggt gagcttttgg tcagtgactg ggtcgatggt 1440
tgggacggac ttggcaaagg gcgcttgtac aagctgagtg acccggcaca gcaagacacc 1500
gatgttgtga cggaagtcaa ggaactgctc agtggtgatt tcacctccgc atcggtggag 1560
gatctgaccg agcaacttcg ccacatcgat cgtcgagtgc gtttgaatgc tcagtgggaa 1620
ctcgccagcc gcggtgaaac gaaacccctg atcgacatcg cgtcagcgac tgacttggat 1680
gctcgtttgc gtttacacgg catttgggga tgcgagcacg cggtgcgttt ggatggttcg 1740
aagcaagcgg atgtgttggc cgccaatcgc ggttggttga ccgattccga cccggtcatt 1800
cgtgccgccg cctgtgcttt ggccggcgat cggaacgatg ccgaatcagt cgcgaagatc 1860
agcgagttgt tggctgacga atcgcctcgc gtgaagtact tcgccgcgat gtcattgtcc 1920
gaattgttga cttcgcaaac tggcaacgct tctgtcaatc agcaagcgat cggcagtgtc 1980
ttgcagattt tggcgaagaa cgacaacaac gatcctgccc tccgacacgc gtgcacgttt 2040
ttcctgcgac gtgttgcatc ggaagacttg ttggcgggat tggcgacgca cagcagtgtt 2100
cccgttcgcc gagccgccat cgccgctctt cgtggtcaag gcagcgagaa ggtgacggca 2160
tttttgtccg acgccagttc actggtcttg gcggaagccg cgatggcaat tcacgaccga 2220
ccaattccgg ctggcgtgga cgagttggcg aagctgattt cggtcgcgga tttgccgctg 2280
aattcggagg cgttgttgcg tcgcgtgttg acggccaatt accgaattgg cacggctgac 2340
tccgctgcgg ctctagcgtc cttcgctgcg tcgggtgaaa agccgacgtg ggctcgaatc 2400
gaagcgatcg acatgttggc caattgggcc actccggaac cgcgtgaccg agtcacgaac 2460
gaatatcgac ctttggaaag tcgtcctgaa atcgtcgctc gcgaagcgtt ggccaagcac 2520
atcgaagtct tgatgatcac cgaccaaacg gttcgcgaaa aagcgatcga tgttggatcc 2580
aaacttggga tcaagaaaat cgctccgcag ttgattgcac gcaccaatga cgctcaatcg 2640
cgacccgcat cacgagcgtc ggcattgacg gcgttggctc gtttgcaacc cgctcaagcg 2700
gtccagatcg ctcgggcgat cgatatcgag caaccgactc agttggtcac cgccgcactc 2760
tccgttctgg gcaagcacga tggccccgcg tccgttgatc gcttcattgc cgcgacggcg 2820
accgagagcc aactcgttcg aggattggct tgggacttgt tggccaacgt ggagtcgaaa 2880
gaggccctcg agcacatcat caaaggtgtg aacgcttacc tcgaaaatga tcttccggcc 2940

gatgttcagt tgaacctggt cgaagccgcc gcgaaacgtt tgcctgaaga ctggaacgcg 3000

aagatcgccg ctcatcgaga gcaattggcg acgacggaac cattggcgaa gtggatggat 3060

tcgttgcacg gtggtgatcc cgacaaggga gcgaagctgt tctttggcaa gaccgaactt 3120

tcgtgtgtgc gttgccacaa agtggatcgc gcgggtggtg aagttggtcc ggtcctgaca 3180

acgattggca aaacgcgaga ccgtcgcacg ttgctggaag cgatcgctct gccggatgca 3240

aagatcgcag aaggcttcga gacggctgta atcgcggacg aagatggaca ggtcttcacc 3300

gggattgtgg gagcggagac cgacgatgtg attgaactga tcgccgccga cgggtcacgg 3360

tctcggatcg agaaggacta catcatcgct cggaagaaag gcaagtcatc gatgccggct 3420

ggattgactg agcagatgac cgcccgcgaa ttgcgtgatt tggttgccta tttggcgagc 3480

cttcaagtcg acccacgggc cggcgaggaa gaaggccacg aatga 3525

<210> 56
<211> 1174
<212> PRT
<213> Rhodopirellula baltica

<400> 56

Met Arg Ser Leu Ile Ser Thr Met Ser Ser Pro Ile Ala Phe Pro Ser
1               5                   10                  15

Arg Ser Phe Ser Arg Ser Leu Arg Arg Ser Arg Arg Pro Ser Ser Phe
            20                  25                  30

Ile Arg Ser Ala Ile Gly Leu Gly Leu Ile Val Ala Ala Met Pro Val
        35                  40                  45

Cys Ala Val Leu Ser Gly Thr Asp Ser Ile Gly Ser Val Ser Ala Asp
    50                  55                  60

Glu Pro Ala Ala Ala Ala Val Thr Asn Thr Lys Pro Glu Ala Leu Glu
65                  70                  75                  80

Pro Glu Ile Ala Glu Ala Ser Glu Glu Ala Ala Gln Ala Met Ala Gly
                85                  90                  95

Phe Lys Ile Pro Glu Gly Trp Glu Ile Lys Leu Phe Ala Ala Glu Pro
            100                 105                 110

Gln Val Ala Asn Ile Val Ala Phe Gly Val Asp Ser Lys Gly Arg Val
        115                 120                 125

Tyr Val Cys Glu Ser Tyr Arg Gln Asn Arg Gly Val Thr Asp Asn Arg

130                 135                 140

Gly His Asp Asp Glu Trp Leu Leu Ala Asp Leu Ser Ala Glu Thr Val
145                 150                 155                 160

Gln Asp Arg Ile Asp Tyr His Lys Arg Leu Leu Gly Glu Ala Ala Ile
                165                 170                 175

Thr Tyr Ala Gln His Asp Asp Arg Ile Arg Arg Leu Thr Asp Thr Asp
            180                 185                 190

Gly Asp Gly Val Ala Asp Glu Ser Thr Val Val Ala Asp Gly Phe Asn
        195                 200                 205

Gly Leu Glu Glu Gly Thr Gly Ala Gly Val Leu Ile Asn Gly Ser Asp
    210                 215                 220

Ile Tyr Tyr Thr Cys Ile Pro Lys Leu Trp Lys Leu Thr Asp Ala Asp
225                 230                 235                 240

Asp Asp Gly Val Ala Glu Asp Ser Gln Val Leu Ser Asp Gly Tyr Gly
                245                 250                 255

Val Arg Val Ala Phe Arg Gly His Asp Met His Gly Leu Ile Arg Gly
            260                 265                 270

Tyr Asp Gly Arg Leu Tyr Phe Ser Ile Gly Asp Arg Gly Tyr His Val
        275                 280                 285

Thr Thr Pro Glu Gly Lys Leu Leu Ser Asn Pro Ala Val Gly Ala Val
    290                 295                 300

Phe Arg Cys Glu Met Asp Gly Ser Gln Leu Glu Val Tyr Cys Asn Gly
305                 310                 315                 320

Leu Arg Asn Pro Gln Glu Leu Ala Phe Asn Asp Ile Gly Asp Trp Phe
                325                 330                 335

Thr Val Asp Asn Asn Ser Asp Ser Gly Asp Lys Ala Arg Leu Val His
            340                 345                 350

Leu Leu Glu Gly Gly Asp Thr Gly Trp Arg Met His Tyr Gln Tyr Leu
        355                 360                 365

Pro Asp Arg Gly Pro Phe Asn Arg Leu Lys Ile Trp Glu Pro His His
    370                 375                 380

Asn Glu Gln Pro Ala His Leu Val Pro Pro Ile Ile Asn Phe Thr Asp
385                 390                 395                 400

Gly Pro Ser Gly Leu Ala Phe Tyr Pro Gly Thr Gly Phe Gly Glu Leu
                405                 410                 415

Leu Asp Asn Gln Phe Leu Ile Cys Asp Phe Arg Gly Gly Pro Ser Asn
            420                 425                 430

Ser Gly Ile Arg Ser Phe Ser Val Asp Pro Asp Gly Ala Phe Tyr Lys
        435                 440                 445

Met Gly Ala Asp Asp Gln Pro Ile Trp Asn Ile Leu Ala Thr Asp Val
    450                 455                 460

Ala Phe Thr Pro Ser Gly Glu Leu Leu Val Ser Asp Trp Val Asp Gly
465                 470                 475                 480

Trp Asp Gly Leu Gly Lys Gly Arg Leu Tyr Lys Leu Ser Asp Pro Ala
                485                 490                 495

Gln Gln Asp Thr Asp Val Val Thr Glu Val Lys Glu Leu Leu Ser Gly
            500                 505                 510

Asp Phe Thr Ser Ala Ser Val Glu Asp Leu Thr Glu Gln Leu Arg His
        515                 520                 525

Ile Asp Arg Arg Val Arg Leu Asn Ala Gln Trp Glu Leu Ala Ser Arg

    530                 535                 540

Gly Glu Thr Lys Pro Leu Ile Asp Ile Ala Ser Ala Thr Asp Leu Asp
545                 550                 555                 560

Ala Arg Leu Arg Leu His Gly Ile Trp Gly Cys Glu His Ala Val Arg
                565                 570                 575

Leu Asp Gly Ser Lys Gln Ala Asp Val Leu Ala Ala Asn Arg Gly Trp
            580                 585                 590

Leu Thr Asp Ser Asp Pro Val Ile Arg Ala Ala Ala Cys Ala Leu Ala
        595                 600                 605

Gly Asp Arg Asn Asp Ala Glu Ser Val Ala Lys Ile Ser Glu Leu Leu
    610                 615                 620

Ala Asp Glu Ser Pro Arg Val Lys Tyr Phe Ala Ala Met Ser Leu Ser
625                 630                 635                 640

Glu Leu Leu Thr Ser Gln Thr Gly Asn Ala Ser Val Asn Gln Gln Ala
                645                 650                 655

Ile Gly Ser Val Leu Gln Ile Leu Ala Lys Asn Asp Asn Asn Asp Pro
            660                 665                 670

Ala Leu Arg His Ala Cys Thr Phe Phe Leu Arg Arg Val Ala Ser Glu
        675                 680                 685

Asp Leu Leu Ala Gly Leu Ala Thr His Ser Ser Val Pro Val Arg Arg
    690                 695                 700

Ala Ala Ile Ala Ala Leu Arg Gly Gln Gly Ser Glu Lys Val Thr Ala
705                 710                 715                 720

Phe Leu Ser Asp Ala Ser Ser Leu Val Leu Ala Glu Ala Ala Met Ala
                725                 730                 735

Ile His Asp Arg Pro Ile Pro Ala Gly Val Asp Glu Leu Ala Lys Leu
            740                 745                 750

Ile Ser Val Ala Asp Leu Pro Leu Asn Ser Glu Ala Leu Leu Arg Arg
        755                 760                 765

Val Leu Thr Ala Asn Tyr Arg Ile Gly Thr Ala Asp Ser Ala Ala Ala
    770                 775                 780

Leu Ala Ser Phe Ala Ala Ser Gly Glu Lys Pro Thr Trp Ala Arg Ile
785                 790                 795                 800

Glu Ala Ile Asp Met Leu Ala Asn Trp Ala Thr Pro Glu Pro Arg Asp
                805                 810                 815

Arg Val Thr Asn Glu Tyr Arg Pro Leu Glu Ser Arg Pro Glu Ile Val
            820                 825                 830

Ala Arg Glu Ala Leu Ala Lys His Ile Glu Val Leu Met Ile Thr Asp
        835                 840                 845

Gln Thr Val Arg Glu Lys Ala Ile Asp Val Gly Ser Lys Leu Gly Ile
    850                 855                 860

Lys Lys Ile Ala Pro Gln Leu Ile Ala Arg Thr Asn Asp Ala Gln Ser
865                 870                 875                 880

Arg Pro Ala Ser Arg Ala Ser Ala Leu Thr Ala Leu Ala Arg Leu Gln

885                 890                 895

Pro Ala Gln Ala Val Gln Ile Ala Arg Ala Ile Asp Ile Glu Gln Pro
            900                 905                 910

Thr Gln Leu Val Thr Ala Ala Leu Ser Val Leu Gly Lys His Asp Gly
        915                 920                 925

Pro Ala Ser Val Asp Arg Phe Ile Ala Ala Thr Ala Thr Glu Ser Gln
    930                 935                 940

Leu Val Arg Gly Leu Ala Trp Asp Leu Leu Ala Asn Val Glu Ser Lys
945                 950                 955                 960

Glu Ala Leu Glu His Ile Ile Lys Gly Val Asn Ala Tyr Leu Glu Asn
                965                 970                 975

Asp Leu Pro Ala Asp Val Gln Leu Asn Leu Val Glu Ala Ala Ala Lys
            980                 985                 990

Arg Leu Pro Glu Asp Trp Asn Ala  Lys Ile Ala Ala His  Arg Glu Gln
        995                 1000                 1005

Leu Ala  Thr Thr Glu Pro Leu  Ala Lys Trp Met Asp  Ser Leu His
    1010                 1015                 1020

Gly Gly  Asp Pro Asp Lys Gly  Ala Lys Leu Phe Phe  Gly Lys Thr
    1025                 1030                 1035

Glu Leu  Ser Cys Val Arg Cys  His Lys Val Asp Arg  Ala Gly Gly
    1040                 1045                 1050

Glu Val  Gly Pro Val Leu Thr  Thr Ile Gly Lys Thr  Arg Asp Arg
    1055                 1060                 1065

Arg Thr  Leu Leu Glu Ala Ile  Ala Leu Pro Asp Ala  Lys Ile Ala
    1070                 1075                 1080

Glu Gly  Phe Glu Thr Ala Val  Ile Ala Asp Glu Asp  Gly Gln Val
    1085                 1090                 1095

Phe Thr  Gly Ile Val Gly Ala  Glu Thr Asp Asp Val  Ile Glu Leu
    1100                 1105                 1110

Ile Ala  Ala Asp Gly Ser Arg  Ser Arg Ile Glu Lys  Asp Tyr Ile
    1115                 1120                 1125

Ile Ala Arg Lys Lys Gly Lys Ser Ser Met Pro Ala Gly Leu Thr
    1130                1135                1140

Glu Gln Met Thr Ala Arg Glu Leu Arg Asp Leu Val Ala Tyr Leu
    1145                1150                1155

Ala Ser Leu Gln Val Asp Pro Arg Ala Gly Glu Glu Glu Gly His
    1160                1165                1170

Glu

<210> 57
<211> 2382
<212> DNA
<213> Erwinia tasmaniensis

<400> 57
atgcatagta aagcatcaag aatactgatc ctgctaacgg ttatattcgc cgcgctaagc      60

ggcttatatc tgttaatcgg cggtatctgg ttggctaaac ttggcggttc tctctactac     120

atcatcgctg gtgtggttct gttggtcacc gcattcctgc tgcaccgccg tcgcggttca     180

gcgctgctgc tgtacgcgct gttcctgctg ggtacgaccg tctggtcgct gtgggaagtg     240

ggctctgact tctgggcgct gactccgcgt ctggatatca ccttcttcct cggactgtgg     300

ttggtattgc catttatctg gcgtgagctg aacggcacag ggtcattctc ccgcgtggcc     360

ctttctgccg tgctggtctt tgtcgtcgcg gtcctcgcct attcaatatt taacgatccg     420

caggaaatca acgggtcact gaatgctgaa caggcggaag cggtgccggc tgaagatggc     480

gtcgcaccgg gcgactggcc ggcctatggc cgtacccagg gcgggacgcg ctactctcct     540

ctgaagcaga taaatgataa aaacgtgggt gaactgcagg aagcctggac tttccagacc     600

ggtgacctga aatccccaag cgatccgggt gagatcacca atgaggccac gccgatcaaa     660

atcggcaatg cgctctacct gtgcaccgcg catcagcagc tgttcgcgct ggatgcggcc     720

accggtaaga agaagtggat gttcgatccg aagctgaagc cgaatccaac cttccagcac     780

gtcacctgtc gcggcgtttc ttattatcag accccgcagg cggctgcgac gccggccgga     840

accgaaccgg ctctctgctc acgtcgtatt ctgctgccgg tcaatgacgg cagcatgtat     900

gcgctggacg cagaaaccgg cgcgctgtgc gaacagtttg gtgacaaagg cagactgaac     960

ctgcagagca acatgccgta tgcaaaagtg ggttcttacg agccgacttc accgccgatc    1020

gtcacggcca ccagcattgt aatggcgggc gcggttaccg ataactactc cactaaacag    1080

ccgtctggcg tggtgcgtgg ttttgacgtg aacaccggca aactgctgtg ggcattcgac    1140

agcggagcga aagatccgaa tctgctgccg catgacgatc agaagtatac gccgaactca    1200

cctaactcgt gggcaccggc agcctatgat gacaagctgg acctggtcta tctgccgatg 1260

ggtgtgtcaa cgccggatat ctggggcggt catcgtaccc cggaaatgga gcgtttcgcc 1320

aacggcgtgc tggcgctgaa tgccaccacc ggtaaactgg catggttcta tcagaccgtg 1380

catcacgacc tgtgggatat ggacgtacct gcacagccga ctctggccga tatcgacgac 1440

aaagacggac acaaggtgcc ggtgatttat atcccgacca aaaccggcga tatctttgtg 1500

ctgaaccgca ccaacggcca gcccgtggtg ccagcgccgg aaatggtggt gccgggtggc 1560

cctgctaagg gcgatcgtct ttcgccgacc cagccatact ctgaactgag cttccgtcca 1620

aaagcccatc tggcgggtaa agacatgtgg ggtgcaacca tctacgatca gctggtctgc 1680

cgcgttatgt tccaccagct gcgctatgaa ggtccgttca ctccgccatc cgagcagggt 1740

acattggtat tcccaggcaa cctcggtatg tttgagtggg gcggtatcgc ggtggatggc 1800

gatcgtcagg ttgccatcac taacccaatg gcgctgccgt tcgtttcccg cctgatccca 1860

cgcgggccgg gcaatcccat tgagccggat gaaaatgata agggcggcac cggtagcgag 1920

aaaggtattc agccgcagta cggtttgccg tacggcgtga cgctgaaccc attcctctct 1980

ccgatcggct taccttgtaa gcagccttca tggggctata tttctgcggt tgatctgaag 2040

accaacgaaa tcgtgtggaa aaagcgtatt ggcaccgtgc gcgacagctc accgcttccg 2100

cttccgttta aaatgggcat gccaatgctg ggtgggccag ttgccaccgc gggcaacctg 2160

ttctttattg cggcaacggc agataactac ctgcgtgcgt ttaacgtgac taacggcaaa 2220

cagctgtggg aagcgcgcct gcccgcgggt ggtcaggcaa cgccaatgac gtatgaagtt 2280

aacggcaagc agtacgtgct gatttttgcg ggtggacatg gctcgtttgg caccaagctg 2340

ggcgactatg tgaaggccta tgcactgccg gacagcaagt aa 2382

<210> 58
<211> 793
<212> PRT
<213> Erwinia tasmaniensis

<400> 58

Met His Ser Lys Ala Ser Arg Ile Leu Ile Leu Leu Thr Val Ile Phe
1               5                   10                  15

Ala Ala Leu Ser Gly Leu Tyr Leu Leu Ile Gly Gly Ile Trp Leu Ala
            20                  25                  30

Lys Leu Gly Gly Ser Leu Tyr Tyr Ile Ile Ala Gly Val Val Leu Leu
        35                  40                  45

Val Thr Ala Phe Leu Leu His Arg Arg Arg Gly Ser Ala Leu Leu Leu
    50                  55                  60

Tyr Ala Leu Phe Leu Leu Gly Thr Thr Val Trp Ser Leu Trp Glu Val
65                  70                  75                  80

Gly Ser Asp Phe Trp Ala Leu Thr Pro Arg Leu Asp Ile Thr Phe Phe
                85                  90                  95

Leu Gly Leu Trp Leu Val Leu Pro Phe Ile Trp Arg Glu Leu Asn Gly
            100                 105                 110

Thr Gly Ser Phe Ser Arg Val Ala Leu Ser Ala Val Leu Val Phe Val
        115                 120                 125

Val Ala Val Leu Ala Tyr Ser Ile Phe Asn Asp Pro Gln Glu Ile Asn
    130                 135                 140

Gly Ser Leu Asn Ala Glu Gln Ala Glu Ala Val Pro Ala Glu Asp Gly
145                 150                 155                 160

Val Ala Pro Gly Asp Trp Pro Ala Tyr Gly Arg Thr Gln Gly Gly Thr
                165                 170                 175

Arg Tyr Ser Pro Leu Lys Gln Ile Asn Asp Lys Asn Val Gly Glu Leu
            180                 185                 190

Gln Glu Ala Trp Thr Phe Gln Thr Gly Asp Leu Lys Ser Pro Ser Asp
        195                 200                 205

Pro Gly Glu Ile Thr Asn Glu Ala Thr Pro Ile Lys Ile Gly Asn Ala
    210                 215                 220

Leu Tyr Leu Cys Thr Ala His Gln Gln Leu Phe Ala Leu Asp Ala Ala
225                 230                 235                 240

Thr Gly Lys Lys Lys Trp Met Phe Asp Pro Lys Leu Lys Pro Asn Pro
                245                 250                 255

Thr Phe Gln His Val Thr Cys Arg Gly Val Ser Tyr Tyr Gln Thr Pro
            260                 265                 270

Gln Ala Ala Ala Thr Pro Ala Gly Thr Glu Pro Ala Leu Cys Ser Arg
        275                 280                 285

Arg Ile Leu Leu Pro Val Asn Asp Gly Ser Met Tyr Ala Leu Asp Ala
    290                 295                 300

Glu Thr Gly Ala Leu Cys Glu Gln Phe Gly Asp Lys Gly Arg Leu Asn
305                 310                 315                 320

Leu Gln Ser Asn Met Pro Tyr Ala Lys Val Gly Ser Tyr Glu Pro Thr
                325                 330                 335

Ser Pro Pro Ile Val Thr Ala Thr Ser Ile Val Met Ala Gly Ala Val
            340                 345                 350

Thr Asp Asn Tyr Ser Thr Lys Gln Pro Ser Gly Val Val Arg Gly Phe
        355                 360                 365

Asp Val Asn Thr Gly Lys Leu Leu Trp Ala Phe Asp Ser Gly Ala Lys
    370                 375                 380

Asp Pro Asn Leu Leu Pro His Asp Asp Gln Lys Tyr Thr Pro Asn Ser
385                 390                 395                 400

Pro Asn Ser Trp Ala Pro Ala Ala Tyr Asp Asp Lys Leu Asp Leu Val
                405                 410                 415

Tyr Leu Pro Met Gly Val Ser Thr Pro Asp Ile Trp Gly Gly His Arg
            420                 425                 430

Thr Pro Glu Met Glu Arg Phe Ala Asn Gly Val Leu Ala Leu Asn Ala
        435                 440                 445

Thr Thr Gly Lys Leu Ala Trp Phe Tyr Gln Thr Val His His Asp Leu
    450                 455                 460

Trp Asp Met Asp Val Pro Ala Gln Pro Thr Leu Ala Asp Ile Asp Asp
465                 470                 475                 480

Lys Asp Gly His Lys Val Pro Val Ile Tyr Ile Pro Thr Lys Thr Gly
                485                 490                 495

Asp Ile Phe Val Leu Asn Arg Thr Asn Gly Gln Pro Val Val Pro Ala
            500                 505                 510

Pro Glu Met Val Val Pro Gly Gly Pro Ala Lys Gly Asp Arg Leu Ser
        515                 520                 525

Pro Thr Gln Pro Tyr Ser Glu Leu Ser Phe Arg Pro Lys Ala His Leu
    530                 535                 540

Ala Gly Lys Asp Met Trp Gly Ala Thr Ile Tyr Asp Gln Leu Val Cys
545                 550                 555                 560

Arg Val Met Phe His Gln Leu Arg Tyr Glu Gly Pro Phe Thr Pro Pro

                565                 570                 575

Ser Glu Gln Gly Thr Leu Val Phe Pro Gly Asn Leu Gly Met Phe Glu
            580                 585                 590

Trp Gly Gly Ile Ala Val Asp Gly Asp Arg Gln Val Ala Ile Thr Asn
        595                 600                 605

Pro Met Ala Leu Pro Phe Val Ser Arg Leu Ile Pro Arg Gly Pro Gly
    610                 615                 620

Asn Pro Ile Glu Pro Asp Glu Asn Asp Lys Gly Gly Thr Gly Ser Glu
625                 630                 635                 640

Lys Gly Ile Gln Pro Gln Tyr Gly Leu Pro Tyr Gly Val Thr Leu Asn
                645                 650                 655

Pro Phe Leu Ser Pro Ile Gly Leu Pro Cys Lys Gln Pro Ser Trp Gly
            660                 665                 670

Tyr Ile Ser Ala Val Asp Leu Lys Thr Asn Glu Ile Val Trp Lys Lys
        675                 680                 685

Arg Ile Gly Thr Val Arg Asp Ser Ser Pro Leu Pro Leu Pro Phe Lys
    690                 695                 700

Met Gly Met Pro Met Leu Gly Gly Pro Val Ala Thr Ala Gly Asn Leu
705                 710                 715                 720

Phe Phe Ile Ala Ala Thr Ala Asp Asn Tyr Leu Arg Ala Phe Asn Val
                725                 730                 735

Thr Asn Gly Lys Gln Leu Trp Glu Ala Arg Leu Pro Ala Gly Gly Gln
            740                 745                 750

Ala Thr Pro Met Thr Tyr Glu Val Asn Gly Lys Gln Tyr Val Leu Ile
        755                 760                 765

Phe Ala Gly Gly His Gly Ser Phe Gly Thr Lys Leu Gly Asp Tyr Val
    770                 775                 780

Lys Ala Tyr Ala Leu Pro Asp Ser Lys
785                 790

<210> 59
<211> 2400
<212> DNA
<213> Burkholderia xenovorans

<400> 59
atgcacagtt ccgcctcaat tcgcgtagcg catttgctta gcgttctgtt tgcctcattg 60
tcgggggtgt acctgctggt cggcgggatc tggctagcgg cgaccggcgg atcgctgtac 120
tacgttttcg ccggtatcgt catgctcgtt acggcggttc tgatttatcg gcgttcccca 180
ctagcactcg gcctctacgc attactgctg ttgtgcacga tcgtgtggtc gctgtgggag 240
gtcggcacgg atttctgggc tcttgccccg cgtctggacg tactggtcgt cttcggcatc 300
tggctgctgt tgccgtatgt ctatcgcgcg ttcgagccgt ccgcaaaaac gcatggtctc 360
gcgctcgggg ccacgttgat cgtgagcgtc gcagtgctgg tttttgcagt cttcaacgac 420
ccacaggaaa tcaacggcac gctggcgagc gcgcctgccc aggcggcggc gacgcccgcc 480
ggcgagcgcg tcgccgaggg cgactggccc gcctacggcc gtacccagca cggtacgcgc 540
tactctccgc tcaagcagat caacgcggat aacgtcaagg atctgaaggt cgcgtggata 600
ttccggaccg gcgacatgaa gcgctccacc gacccaggtg aaatcacgaa cgaagttacc 660
ccgatcaagg tgggcgacat gctgtatctg tgctcgccgc accagatact gttcgcgctt 720
gacgctgcca ccggccagga aaaatggcgc ttcgatccca agctgaagac cgatcccagt 780
tttcagcatg tcacgtgccg tggggtgtcg taccatgatt cgaccgcttc gggcagtaac 840
aacgacagcc cggaccaggc cccggctacg gcgcctgcaa tctgcgcgcg ccgcgtctac 900
ctgcctgtca acaacgggca tctgtacgcg ctcgacgcgc agaccggcca gctatgcccg 960
gacttcgcca atcacggcga cctcgacctg caagtgggcc agcccgtgac gaccgcgggt 1020
caatatgaac ccacttcgcc gccggtcatc accgggaagg tgatcgtaat cgcaggctca 1080
gtcgaggata actattcgac gcgggagccc tcgggagtga tccggggttt cgatgtcaac 1140
accggcaaac tgttgtgggc gttcgatccg ggcgcgaaga acccggaagc ggtgctcggc 1200
gcaggccagc attattcggt caactcgccg aactcctggg caccggctgt ttatgacgca 1260
aagctcgatc ttgtctatct gccgatgggc gtaagcacgc ccgacatctg gggcggctac 1320
cgcacgccgg agcaggaacg ctttgcgagc gggctgctcg cgctcaacgc gacgaccggc 1380
aagctcgcct ggttttatca gacggtccac cacgacctgt gggacatgga cctgccggcg 1440
cagccgacgc tcgccgatat cacggaccgg tcgggcaacc tcgtgccggc ggtctacgcc 1500
ccggcaaaga cgggcaacat tttcgttctt gaccgccgca caggcacgcc gatcgttcct 1560
gctccggaga agccggttcc gcaaggcgcg gcgcaaggcg atcacgtgtc gcccacccag 1620
ccgttttcgg cgctgacctt ccggccggac aggaaactga ccggcgcgga catgtggggc 1680
gcaacgatgt ttgaccagct cgtgtgccga gtgatgttcc agcgcctgaa ctatgacggc 1740
acgtttaccc ctccgtcggt gaagggcacc ctggtttttc cgggtaacct cgggatgttc 1800
gagtggggcg gcatagcagt ggataccgat cgccagatcg cgattgcgaa cccgatcgca 1860

ctgccgttcg tgtccaggtt gatgccgcgt gggccgggca acccgatcga accggccgcc 1920

ggcggcacgg gcggcagcgg taccgaatcc ggtatccagc cgcagtacgg cgtgcccttc 1980

ggtgtgacgt tgaatgcctt catgtcgccg ctcggcctgc cgtgcaagca gccggcgtgg 2040

ggctacatat ccgcggtcga tctgaagacc aaccagattg tctggaaaaa gcgcattggc 2100

accgtgcgtg acagttcacc gctgccgctg ccgttcaaga tggggatgcc gatgctcggc 2160

ggcccgatga caacggccgg cgacgtgttc ttcatcgggg ccaccgccga caactacatc 2220

cgcgcgttcg acaccaacac cggcaagcag ttgtggcaag cacgtttgcc cgcgggtggc 2280

caggcgacgc caatgaccta cgaggcgaaa gggaagcagt acgtggtcat cgcggccggc 2340

ggccacggct cgttcggcac caggttgggc gactacgtga tggcttatgc gctgccttga 2400

<210> 60
<211> 799
<212> PRT
<213> Burkholderia xenovorans

<400> 60

Met His Ser Ser Ala Ser Ile Arg Val Ala His Leu Leu Ser Val Leu
1               5                   10                  15

Phe Ala Ser Leu Ser Gly Val Tyr Leu Leu Val Gly Gly Ile Trp Leu
            20                  25                  30

Ala Ala Thr Gly Gly Ser Leu Tyr Tyr Val Phe Ala Gly Ile Val Met
        35                  40                  45

Leu Val Thr Ala Val Leu Ile Tyr Arg Arg Ser Pro Leu Ala Leu Gly
    50                  55                  60

Leu Tyr Ala Leu Leu Leu Leu Cys Thr Ile Val Trp Ser Leu Trp Glu
65                  70                  75                  80

Val Gly Thr Asp Phe Trp Ala Leu Ala Pro Arg Leu Asp Val Leu Val
                85                  90                  95

Val Phe Gly Ile Trp Leu Leu Leu Pro Tyr Val Tyr Arg Ala Phe Glu
            100                 105                 110

Pro Ser Ala Lys Thr His Gly Leu Ala Leu Gly Ala Thr Leu Ile Val
        115                 120                 125

Ser Val Ala Val Leu Val Phe Ala Val Phe Asn Asp Pro Gln Glu Ile
    130                 135                 140

Asn Gly Thr Leu Ala Ser Ala Pro Ala Gln Ala Ala Ala Thr Pro Ala
145                 150                 155                 160

Gly Glu Arg Val Ala Glu Gly Asp Trp Pro Ala Tyr Gly Arg Thr Gln
                165                 170                 175

His Gly Thr Arg Tyr Ser Pro Leu Lys Gln Ile Asn Ala Asp Asn Val
            180                 185                 190

Lys Asp Leu Lys Val Ala Trp Ile Phe Arg Thr Gly Asp Met Lys Arg
        195                 200                 205

Ser Thr Asp Pro Gly Glu Ile Thr Asn Glu Val Thr Pro Ile Lys Val
    210                 215                 220

Gly Asp Met Leu Tyr Leu Cys Ser Pro His Gln Ile Leu Phe Ala Leu
225                 230                 235                 240

Asp Ala Ala Thr Gly Gln Glu Lys Trp Arg Phe Asp Pro Lys Leu Lys
                245                 250                 255

Thr Asp Pro Ser Phe Gln His Val Thr Cys Arg Gly Val Ser Tyr His
            260                 265                 270

Asp Ser Thr Ala Ser Gly Ser Asn Asn Asp Ser Pro Asp Gln Ala Pro
        275                 280                 285

Ala Thr Ala Pro Ala Ile Cys Ala Arg Arg Val Tyr Leu Pro Val Asn
    290                 295                 300

Asn Gly His Leu Tyr Ala Leu Asp Ala Gln Thr Gly Gln Leu Cys Pro
305                 310                 315                 320

Asp Phe Ala Asn His Gly Asp Leu Asp Leu Gln Val Gly Gln Pro Val
                325                 330                 335

Thr Thr Ala Gly Gln Tyr Glu Pro Thr Ser Pro Pro Val Ile Thr Gly
            340                 345                 350

Lys Val Ile Val Ile Ala Gly Ser Val Glu Asp Asn Tyr Ser Thr Arg
        355                 360                 365

Glu Pro Ser Gly Val Ile Arg Gly Phe Asp Val Asn Thr Gly Lys Leu
    370                 375                 380

Leu Trp Ala Phe Asp Pro Gly Ala Lys Asn Pro Glu Ala Val Leu Gly
385                 390                 395                 400

Ala Gly Gln His Tyr Ser Val Asn Ser Pro Asn Ser Trp Ala Pro Ala
                405                 410                 415

Val Tyr Asp Ala Lys Leu Asp Leu Val Tyr Leu Pro Met Gly Val Ser
            420                 425                 430

Thr Pro Asp Ile Trp Gly Gly Tyr Arg Thr Pro Glu Gln Glu Arg Phe
        435                 440                 445

Ala Ser Gly Leu Leu Ala Leu Asn Ala Thr Thr Gly Lys Leu Ala Trp
    450                 455                 460

Phe Tyr Gln Thr Val His His Asp Leu Trp Asp Met Asp Leu Pro Ala
465                 470                 475                 480

Gln Pro Thr Leu Ala Asp Ile Thr Asp Arg Ser Gly Asn Leu Val Pro
                485                 490                 495

Ala Val Tyr Ala Pro Ala Lys Thr Gly Asn Ile Phe Val Leu Asp Arg
            500                 505                 510

Arg Thr Gly Thr Pro Ile Val Pro Ala Pro Glu Lys Pro Val Pro Gln
        515                 520                 525

Gly Ala Ala Gln Gly Asp His Val Ser Pro Thr Gln Pro Phe Ser Ala
    530                 535                 540

Leu Thr Phe Arg Pro Asp Arg Lys Leu Thr Gly Ala Asp Met Trp Gly
545                 550                 555                 560

Ala Thr Met Phe Asp Gln Leu Val Cys Arg Val Met Phe Gln Arg Leu
                565                 570                 575

Asn Tyr Asp Gly Thr Phe Thr Pro Pro Ser Val Lys Gly Thr Leu Val
            580                 585                 590

Phe Pro Gly Asn Leu Gly Met Phe Glu Trp Gly Gly Ile Ala Val Asp
        595                 600                 605

Thr Asp Arg Gln Ile Ala Ile Ala Asn Pro Ile Ala Leu Pro Phe Val
    610                 615                 620

Ser Arg Leu Met Pro Arg Gly Pro Gly Asn Pro Ile Glu Pro Ala Ala
625                 630                 635                 640

Gly Gly Thr Gly Gly Ser Gly Thr Glu Ser Gly Ile Gln Pro Gln Tyr
                645                 650                 655

Gly Val Pro Phe Gly Val Thr Leu Asn Ala Phe Met Ser Pro Leu Gly
            660                 665                 670

Leu Pro Cys Lys Gln Pro Ala Trp Gly Tyr Ile Ser Ala Val Asp Leu
        675                 680                 685

Lys Thr Asn Gln Ile Val Trp Lys Lys Arg Ile Gly Thr Val Arg Asp
    690                 695                 700

Ser Ser Pro Leu Pro Leu Pro Phe Lys Met Gly Met Pro Met Leu Gly
705                 710                 715                 720

Gly Pro Met Thr Thr Ala Gly Asp Val Phe Phe Ile Gly Ala Thr Ala
                725                 730                 735

Asp Asn Tyr Ile Arg Ala Phe Asp Thr Asn Thr Gly Lys Gln Leu Trp
            740                 745                 750

Gln Ala Arg Leu Pro Ala Gly Gly Gln Ala Thr Pro Met Thr Tyr Glu
        755                 760                 765

Ala Lys Gly Lys Gln Tyr Val Val Ile Ala Ala Gly Gly His Gly Ser
    770                 775                 780

Phe Gly Thr Arg Leu Gly Asp Tyr Val Met Ala Tyr Ala Leu Pro
785                 790                 795

<210>  61
<211>  2361
<212>  DNA
<213>  Pantoea citrea

<400>  61
atgtctcggg tctctactat acttcatggg ttaacacgga tattttcgct attctgtgcc      60

gcatggctgc tgattggcgg cgtgtggcta ttatctgtcg gagggagtgc ttactacctt     120

atctgcggta tagccatggc gatattttct atattgttat ggaagcgcag aagcagtgca     180

ttttacctct attcgcttat cctgattctg tcagccctct gggcatggcg ggaggcgggg     240

actgacttct ggaacctggt tccacgtctg gatatctgga tactgtttgg tatctggttg     300

atattgccgt tcagttaccg gcgttttaac actgccggta aaaaaccatt gctggcaatg     360

gtgattggcc tcgggattaa tgccctgctg ctgctgggcg cgtcactgca cgacccacag     420

gaaatcaacg gtgtgcttaa tgtcagtgat aagccgccag ccgaatctgc ggcgtctgca     480

gcagactggc cggcctatgg acgtactcag gaaggggtcc gttattctcc gctgacacaa     540

atcaacgata aaaacgttca acaattacag gttgcctggc agttccatac cggtgaccat 600

aaaacagcga atgacccggg tgagatcact aatgaagtga caccgctgaa agtcggtaat 660

atgctgtacc tctgtacacc gcatcagata ctgattgccc tggatgctgc cagtggcaga 720

gagaaatggc gttttgatcc gcagctcaaa tctgatccga cattccagca tattacctgc 780

cggggtgttt cctatcatga aatcaaatct gttcagggcg actcatcagc gccggccgcc 840

tgttcacggc gtattttcct gccggttgat gacggtcgtc tgttcgcggt tgatgcctta 900

accggacaac gttgcagtaa ttttgctaat aatggtgaac tgaacctgca acacctgcag 960

ccgaacgctt atccgggagg gtatgagcca acctcaccgc ccatcattac tgataaagtg 1020

gtgatcattg ccggttctgt cactgataac ttgtctaccc gtgaaccatc aggggtcatc 1080

cgcggtttcg atatcgacag cgggaaactc ttatgggtat ttgatccggg agcaaaagac 1140

cctaatgccg tgcctgctga cgggcagaca tttgtggcga actcaccaaa ctcctgggcg 1200

ccggcggctt atgacgcgca gagggatatt atttatctgc ctatgggggt ttcgaccccg 1260

gatatctggg gcggtgccgt aacgcgttgc aggaacgttt tgccagcggt ttactggctc 1320

tgcatgcctc aactggcaag ctggcatggt tttaccagac ggtacaccat gcaatcattc 1380

tgggacatgg atttaccctc acagccaacc ctggcagata ttaccgatga gcagggtaag 1440

accgtgccgg tggtctatgt gccggccaaa acgggtaata tttttgttct gaaccgtgac 1500

accggaaaac ccgtcgttcc tgcccctgaa acaccggtac cgcagggacc ggcgaaaggc 1560

gaccatctgt ctccaaccca gcctttttct gagctgactt tccgtcctaa gaataagctg 1620

cagggtaggg atatgtgggg cgcaaccatg tttgaccagc tgatgtgccg ggtgatgttc 1680

cataaactgc gctatgaagg gccgtttacc ccgccgtctg agcagggtac cctggttttc 1740

ccaggtgatt tcgggatgtt tgaatggggc ggtatctctg ttaacaccga tcagcagttt 1800

gcgattgcta atccgatggc gatgcctttt atttctaaac tgatcccacg cggaccaggc 1860

aatcccatag aacccggggc tgatggtgcc gccggttccg gttcagagtc cggggtacag 1920

catatgtatg gtgtgcctta cggagttgaa ctgaatccgt tcctgtcacc gttgggctta 1980

ccttgtctgc aaccttcatg gggctttgtc tcagcgatca atttacgtaa tcaccagatc 2040

atctggaaaa aacggattgg tactgttcgt gacagtgcgc cggtacctct accctttaaa 2100

atgggtgttc caatgttggg cggtccggtg acaacggcag gtaatatctt ctttgttgct 2160

ggcacactgg acaactacct gcgggcgtac agtgtccgtg acggcaaact gctgtggcag 2220

gctcgtctgc cggcaggtgg acaagcgaca ccaatgacgt atgaggttga tggtaagcaa 2280

tatgtcgtga ttatggccgg cggacatggt tcttttggga cccggctggg cgattcactg 2340

atcgcttata aactaccgta a 2361

<210> 62
<211> 786
<212> PRT
<213> Pantoea citrea

<400> 62

Met Ser Arg Val Ser Thr Ile Leu His Gly Leu Thr Arg Ile Phe Ser
1               5                   10                  15

Leu Phe Cys Ala Ala Trp Leu Leu Ile Gly Gly Val Trp Leu Leu Ser
            20                  25                  30

Val Gly Gly Ser Ala Tyr Tyr Leu Ile Cys Gly Ile Ala Met Ala Ile
        35                  40                  45

Phe Ser Ile Leu Leu Trp Lys Arg Arg Ser Ser Ala Phe Tyr Leu Tyr
    50                  55                  60

Ser Leu Ile Leu Ile Leu Ser Ala Leu Trp Ala Trp Arg Glu Ala Gly
65                  70                  75                  80

Thr Asp Phe Trp Asn Leu Val Pro Arg Leu Asp Ile Trp Ile Leu Phe
                85                  90                  95

Gly Ile Trp Leu Ile Leu Pro Phe Ser Tyr Arg Arg Phe Asn Thr Ala
            100                 105                 110

Gly Lys Lys Pro Leu Leu Ala Met Val Ile Gly Leu Gly Ile Asn Ala
        115                 120                 125

Leu Leu Leu Leu Gly Ala Ser Leu His Asp Pro Gln Glu Ile Asn Gly
    130                 135                 140

Val Leu Asn Val Ser Asp Lys Pro Pro Ala Glu Ser Ala Ala Ser Ala
145                 150                 155                 160

Ala Asp Trp Pro Ala Tyr Gly Arg Thr Gln Glu Gly Val Arg Tyr Ser
                165                 170                 175

Pro Leu Thr Gln Ile Asn Asp Lys Asn Val Gln Gln Leu Gln Val Ala
            180                 185                 190

Trp Gln Phe His Thr Gly Asp His Lys Thr Ala Asn Asp Pro Gly Glu
        195                 200                 205

Ile Thr Asn Glu Val Thr Pro Leu Lys Val Gly Asn Met Leu Tyr Leu
    210                 215                 220

Cys Thr Pro His Gln Ile Leu Ile Ala Leu Asp Ala Ala Ser Gly Arg
225                 230                 235                 240

Glu Lys Trp Arg Phe Asp Pro Gln Leu Lys Ser Asp Pro Thr Phe Gln
                245                 250                 255

His Ile Thr Cys Arg Gly Val Ser Tyr His Glu Ile Lys Ser Val Gln
            260                 265                 270

Gly Asp Ser Ser Ala Pro Ala Ala Cys Ser Arg Arg Ile Phe Leu Pro
        275                 280                 285

Val Asp Asp Gly Arg Leu Phe Ala Val Asp Ala Leu Thr Gly Gln Arg
    290                 295                 300

Cys Ser Asn Phe Ala Asn Asn Gly Glu Leu Asn Leu Gln His Leu Gln
305                 310                 315                 320

Pro Asn Ala Tyr Pro Gly Gly Tyr Glu Pro Thr Ser Pro Pro Ile Ile
                325                 330                 335

Thr Asp Lys Val Val Ile Ile Ala Gly Ser Val Thr Asp Asn Leu Ser
            340                 345                 350

Thr Arg Glu Pro Ser Gly Val Ile Arg Gly Phe Asp Ile Asp Ser Gly
        355                 360                 365

Lys Leu Leu Trp Val Phe Asp Pro Gly Ala Lys Asp Pro Asn Ala Val
    370                 375                 380

Pro Ala Asp Gly Gln Thr Phe Val Ala Asn Ser Pro Asn Ser Trp Ala
385                 390                 395                 400

Pro Ala Ala Tyr Asp Ala Gln Arg Asp Ile Ile Tyr Leu Pro Met Gly
                405                 410                 415

Val Ser Thr Pro Asp Ile Trp Gly Gly Ala Val Thr Arg Cys Arg Asn
            420                 425                 430

Val Leu Pro Ala Val Tyr Trp Leu Cys Met Pro Gln Leu Ala Ser Trp
        435                 440                 445

His Gly Phe Thr Arg Arg Tyr Thr Met Gln Ser Phe Trp Asp Met Asp
    450                 455                 460

Leu Pro Ser Gln Pro Thr Leu Ala Asp Ile Thr Asp Glu Gln Gly Lys

465                 470                 475                 480

Thr Val Pro Val Val Tyr Val Pro Ala Lys Thr Gly Asn Ile Phe Val
                485                 490                 495

Leu Asn Arg Asp Thr Gly Lys Pro Val Val Pro Ala Pro Glu Thr Pro
            500                 505                 510

Val Pro Gln Gly Pro Ala Lys Gly Asp His Leu Ser Pro Thr Gln Pro
        515                 520                 525

Phe Ser Glu Leu Thr Phe Arg Pro Lys Asn Lys Leu Gln Gly Arg Asp
    530                 535                 540

Met Trp Gly Ala Thr Met Phe Asp Gln Leu Met Cys Arg Val Met Phe
545                 550                 555                 560

His Lys Leu Arg Tyr Glu Gly Pro Phe Thr Pro Pro Ser Glu Gln Gly
                565                 570                 575

Thr Leu Val Phe Pro Gly Asp Phe Gly Met Phe Glu Trp Gly Gly Ile
            580                 585                 590

Ser Val Asn Thr Asp Gln Gln Phe Ala Ile Ala Asn Pro Met Ala Met
        595                 600                 605

Pro Phe Ile Ser Lys Leu Ile Pro Arg Gly Pro Gly Asn Pro Ile Glu
    610                 615                 620

Pro Gly Ala Asp Gly Ala Ala Gly Ser Gly Ser Glu Ser Gly Val Gln
625                 630                 635                 640

His Met Tyr Gly Val Pro Tyr Gly Val Glu Leu Asn Pro Phe Leu Ser
                645                 650                 655

Pro Leu Gly Leu Pro Cys Leu Gln Pro Ser Trp Gly Phe Val Ser Ala
            660                 665                 670

Ile Asn Leu Arg Asn His Gln Ile Ile Trp Lys Lys Arg Ile Gly Thr
        675                 680                 685

Val Arg Asp Ser Ala Pro Val Pro Leu Pro Phe Lys Met Gly Val Pro
    690                 695                 700

Met Leu Gly Gly Pro Val Thr Thr Ala Gly Asn Ile Phe Phe Val Ala
705                 710                 715                 720

Gly Thr Leu Asp Asn Tyr Leu Arg Ala Tyr Ser Val Arg Asp Gly Lys
                725                 730                 735

Leu Leu Trp Gln Ala Arg Leu Pro Ala Gly Gly Gln Ala Thr Pro Met
            740                 745                 750

Thr Tyr Glu Val Asp Gly Lys Gln Tyr Val Val Ile Met Ala Gly Gly
        755                 760                 765

His Gly Ser Phe Gly Thr Arg Leu Gly Asp Ser Leu Ile Ala Tyr Lys
    770                 775                 780

Leu Pro
785

<210>  63
<211>  5498
<212>  DNA
<213>  Klebsiella pneumoniae

<400>  63
catgtggaag aaacctgctt ttatcgattt acgtctcggt ctggaagtga cgctgtacat      60

ttctaaccgt taatcgcccc gcccgccgtt cgcgcgggca ccttcattca ttacccggtc     120

cgtcttcatg ttcattaaag tcctcggctc cgccgccggc ggcggtttcc cgcaatggaa     180

ctgcaactgc gccaactgtc agggtctgcg caacggcacc attcaggcca gtgcccgcac     240

ccagtcgtcg atcatcgtca gcgataacgg caaagagtgg gtgctgtgca atgcctcgcc     300

ggatatcagc cagcagattg cccatacccc cgagttaaat aaacccggcg tactgcgcgg     360

gacgtctatc ggcggcatta ttctcaccga cagccagatc gaccacacca ccgggttgct     420

gagcctgcgc gaaggctgcc cgcaccaggt gtggtgcacg ccggaggttc atgaggatct     480

ctccaccggc ttcccggtgt ttaccatgct gcgacactgg aacggcggcc tggtgcatca     540

tcccatcgcg ccgcagcagc cttttaccgt tgacgcctgc cctgatttgc agtttaccgc     600

cgtgcctatc gccagcaacg cgccgcccta ttcgccgtat cgcgaccggc cgctgccagg     660

ccataacgtg gcgctgttta tcgaataccg ccgcaacggg cagacgctgt tctatgcccc     720

ggggctgggt gagccggatg aagcccttct gccgtggctg caaaaagcgg actgtctgct     780

gatcgatggc accgtctggc aggatgacga gctgcaggcc gccggcgtcg ggcgcaatac     840

cggccgtgat atgggacacc tggcgctcag cgatgagcac gggatgatgg ccctgctggc     900

ctctctgccg gcaaaacgca aaattctcat tcatattaat aacaccaacc cgatccttaa     960

cgagctgtct ccccagcgcc aggcgctaaa acaacagggg attgaagtga gctgggacgg    1020

gatggcaatc acccttcagg ataccgcatg ctgatcaccg acacgctgtc gccgcaggcc    1080

tttgaagagg ctctgcgggc taaaggcgcc ttctaccata ttcaccaccc ttaccacatc    1140

gccatgcata acggcgaagc gacccgcgag caaattcagg gttgggtggc gaaccggttt 1200
tattaccaga ccaccattcc gctgaaagac gcggcgatta tggctaactg cccggatgcg 1260
cagacccggc gcaaatgggt gcagcggatc ctcgaccacg acggtagcca cggcgaagat 1320
ggcgggattg aagcctggct gcggctgggg gaagcagtcg gtttgagccg cgacgacctg 1380
ctcagcgagc gtcacgtgct gcccggcgtg cgcttcgcgg tggatgccta tcttaatttc 1440
gctcgtcgcg cctgctggca ggaggcggcc tgcagctccc tgaccgagct gttcgcccca 1500
cagatccatc agtcgcgcct cgacagctgg ccgcagcact atccgtggat caaagaggaa 1560

ggctattttt acttccgcag tcgtctgagc caggctaacc gcgacgttga gcatggtctg 1620
gcgctggcga agacctactg tgacagcgct gaaaaacaga accggatgct ggagatcctg 1680
cagtttaagc tcgacatcct gtggtcgatg ctcgatgcca tgaccatggc ctacgctctg 1740
caacgcccgc cctatcacac ggtcaccgac aaggcggcct ggcacacgac ccgactggtg 1800
taatcatgca aaaaacgtcc atcgttgcct ttcgtcgcgg ctaccgactg cagtgggaag 1860
ccgcccagga gagccatgtg atcctctatc cggagggaat ggctaaactc aatgagaccg 1920
ccgcggcgat cctcgagctg gtcgatggcc ggcgcgacgt cgcggcgatt atcgccatgc 1980
ttaacgaacg tttcccggaa gccggcggcg tcgatgacga cgtcgtcgag ttcctgcaga 2040
tcgcctgtca acagaagtgg atcacctgcc gtgagccaga ataaacccgc cgtcaatccg 2100
ccgctgtggc tgctggcgga gctgacctac cgctgcccgc tgcagtgtcc ctactgttcc 2160
aatccgctgg acttcgcccg gcaggaaaag gagctgacca ccgaacaatg gatcgaggtc 2220
tttcgccagg cgcgagcgat gggcagcgta cagctgggct tttccggcgg cgagccgctg 2280
acccgtaaag atctgccgga gctgatccgc gccgcgcgcg acctcgggtt ctataccaac 2340
ctgatcacct cgggaattgg gctaaccgag agcaaactcg acgccttcag cgaggccgga 2400
ctggaccata tccagattag cttccaggcc agcgatgagg tgctcaacgc cgctcttgcc 2460
ggcaataaaa aagccttcca gcagaagctg gcgatggcca gagcggtgaa agcgcgcgac 2520
tacccgatgg tgctgaactt cgtcctccac cggcataaca tcgaccagct cgataaaatt 2580
atcgagctgt gcattgagct ggaagccgat gacgtcgagc tcgccacctg ccagttttac 2640
ggctgggcgt ttcttaatcg cgaggggtta ctgccgaccc gggaacagat cgcccgcgcc 2700
gagcaggtgg tcgccgatta ccggcagaaa atggccgcca gcggtaacct caccaacctg 2760
ctattcgtca ccccggacta ttacgaggaa cgcccgaaag gctgtatggg cggctgggga 2820
tcgattttcc tcagcgtcac tccggaaggc actgcgttgc cgtgccacag cgcgcgccag 2880
ctgccggtgg cgttcccgtc ggtgctggag cagagtctgg aatcgatctg gtatgactcg 2940
ttcggcttca accgttatcg cgggtatgac tggatgccgg agccgtgccg ctcctgtgat 3000

gaaaaagaga aagacttcgg cggctgccgc tgtcaggcct ttatgctgac cggcagcgcc 3060
gataacgccg acccggtgtg cagcaaatcc ccacatcatc acaaaatcct tgaggcccgg 3120
cgcgaagcgg cctgcagcga catcaaagtc agccagctgc agttccgcaa ccgtacccgc 3180
tcgcagctta tctacaaaac ccgggaactg taatgacgct ggcgacccgc actgtcactc 3240
tgccgggcgg cctgcaggct accctggttc atcagccgca ggccgatcgc gcggcggccc 3300
tggtgcgggt tgccgccggc agccaccatg aaccgtcgtg cttccccggt ctggcgcacc 3360
tgctggaaca cctgctgttt tacggcggtg agcgctaccg caatgatgaa cggctgatga 3420
gctgggtgca gcgccaggca gggaatgtga atgcctccac cctgtcccgc cacagcgcgt 3480
tctttttcga ggtcgccgcc gaggatctgg ctgacggcgt cgcgcgcctg caggagatgc 3540
tgcaggcgcc gctgctgctc agggacgata ttcaacgcga agtcgcggtt atcgacgccg 3600
aaaacggcct gatccaacag catgagttgt cgcgacggga agccgccgtg cgtcacgccg 3660
ccatcgcgcc cgcggcgttt cgccgctttc aggtcggcga cgccgggtcg ctgggggagg 3720
atttcctcgc gctacaggcg gccttacgtg actttcaccg cagccactac gtcgcccgcc 3780
ggatgcaact ctggctgcag gggccgcagt cgctggaggt gctcggcgaa ctggcgaccc 3840
gtttcgccac tgggcttgcc ccgggcgagg caccgccgcc agcgccgccg ctcagtctgg 3900
gcgagccccc tcaactgcag ttggccgtct ccagccagcc cgcgctgtgg cgctgcccgc 3960
tgatcgcctt aagtgacaat gtcacgttac tgcgcgagtt tttgctggat gaagcccccg 4020
gtagcctgat ggccggcctg cgccagcgcg ggatggccga ggacgtggcg ctgaactggc 4080
tgtatcagga tcagcacttc ggctggctgg cgctgatttt cgccagcgac cggccggaac 4140
aggtcgaccg gcagataacc cactggttgc aggcgctaca gcagacgacg cctgagcagc 4200
agcaacacta ctatcagctg tcccggcgcc gttttcaggc gctgtcgccc ctcgatcagc 4260
tgcgccagcg ggcattcggc tttgcccccg gggcgccgcc caccgggttc gccgattttt 4320
gcgccgccct gctggccgcc cccacggtca gcctggcctg ccagacgcag ccccccggag 4380
caacggtagc cacccagggc tttagcctgc cgctcagccg ctggtcgcca cgtccggtct 4440
ctgacccggc gctggcattc gctttttatc cgcaggccgc tggcgagctc gtggccgaaa 4500
gcccggcgga agccgcgcca ctgcgtcacc tcccgtcacc gggagagccg ccgacgctcc 4560
tgctgcgacc gcccttctac tgctcgccca cgccggccgg ggggctggcg cgcggggaac 4620
agctgcgtcc attacttgcc gccctgcgcc atgccggggg acacggcgag tggcatctgt 4680
tcgacggcag ctggcagctg atcctgcagt tgcctgcgtc cggccaatgg ccggaggcga 4740
ttctgcaggc catcgtgcgg cagctcgcgc tcccggtcgc cccgctgccc ccaccgccgg 4800
agagtattgc gatccgtcat ctcatggccc agctccccga acggctgggt acgtcagcgc 4860

accaggaagg ttggctggcg gccctgattg gcggcagcgc ggaagatgcg cagtgggtag 4920

cgcgtcagct gagccggctt accgtcccgg ttaatccgcc gatgcccgct ccggccacct 4980

gccgcggcgg cgtcgagcgg ctggcttatc cccggggcga cacggcgcta ctggtctttc 5040

ttccgctgcc ggaaggcgct tcattggcgg ccctgcgggt gctggcgcag ttctgcgagc 5100

cgccgttttt ccagcgcctg cgggtggagc agcagatagg ctatgtggtg agctgccgct 5160

atcagcgcgt tgccgatcgc gacggactgc tgatggcgct ccagtccccg gatcgccgcc 5220

ccggggagct actccgctgc tgtaaaacct ttctgcgcca gctggccccc gtggatgagg 5280

cgaccttcag gttgttacag cagcggctgg ccgctcaggc ccgcgcccgg gtagagccgc 5340

aggtgcgcgc cctggacgcg ctgcgccagg agtataactt gccgggggtg acgccgcagg 5400

cggctgacgc gctgcgcgtt gaagaggtgg tcgccctgtg gcatgagatg acccgccggc 5460

gtcgtcgctg gcgggtgctg ttcacgacag ggagttaa 5498

<210> 64
<211> 3473
<212> DNA
<213> Methylobacterium extorguens

<400> 64
atgaagtggg ctgcccccat cgtttccgag atctgcgtcg gcatggaagt cacgagctac 60

gagtcggccg agatcgacac cttcaactaa ggtgatttga gccgggttgg ggttgcaggc 120

atcagcgggt tttcaccatg catgtcgtaa tcctgggctc ggctgcgggc ggcggcgttc 180

ctcaatggaa ctgccgctgc tccatctgct ccctggcctg ggcgggcgat tcccgcgtca 240

ggccgcgcac gcagtcgagc atcgcagtct ctcctgacgg ggaacgctgg ctcctgctga 300

acgcctctcc cgatatccgt cagcagatcc aggccaatcc gcagatgcat ccgcgcgagg 360

gcctgcgcca ctcgccgatc cacgcggtgc tgctgacgaa cggcgacgtc gatcacgttg 420

cgggcctgct gaccctgcgc gagggccagc ccttcacgct ctacgcgaca cccggcatcc 480

tggcctccgt ctccgacaac cgcgtcttcg acgtgatggc cgccgacgtg gtgaagcggc 540

agacgatcgc cctcaacgag accttcgagc cggtgcccgg cctctcggtg acgctgttct 600

ccgtccccgg caaggtgccg ctctggctgg aagacgcctc gatggagatc ggggcggaga 660

ccgaaaccac ggtcggcacg atgatcgagg ccgggggcaa gcgcctcgcc tacatccccg 720

gctgcgcccg ggtgacggag gatctcaaag cccgcatcgc cggcgcagac gcgctcctgt 780

tcgacggcac ggtgctggag gacgacgaca tgatccgcgc cggtgtcggc accaagaccg 840

gctggcgcat gggccatatc cagatgaacg gcgagaccgg ctcgatcgcg tctctcgccg 900

atatcgagat cggccgacgg gtcttcgttc acatcaacaa caccaatccg gtcctgatcg 960

aggattcgta cgagcgcgcg agcgtcgagg cgcgcggctg gaccgtcgcc catgacggcc 1020

tgaccctcga tctctgatca ggctgatgtc ttgggaagag cccggtctgg aaatttagtg 1080

ccggactgaa tatgttttgc acgatccaat cgtgcggcag cggccctgcc ccgatcggta 1140

ccgggcccca tttaaaaata aatccaggaa acgcgactcg aagctcgggg gaaaccgaac 1200

gccatgaccg cccaattccc gccgcccgtc ccggacaccg agcaacgcct gctgagccac 1260

gaggagcttg aggcggcgct ccgcgatatc ggtgcacggc gctaccacaa cctccacccg 1320

ttccaccggc tgctgcacga cggcaagctg tcgaaggatc aggtccgggc ctgggcgctc 1380

aaccgctact attatcaggc gatgattccg gtgaaggatg cagcgctgct ggctcgcctg 1440

ccggatgcgc agcttcgccg aatctggcgc cagcgcatcg tcgatcacga cggcgaccat 1500

gagggcgacg gcggcatcga gcgttggctc aagcttgccg aaggcgtcgg cttcacccgc 1560

gactacgtgc tctcgaccaa gggcatcctg tcggcgaccc gcttctcggt cgatgcctat 1620

gtccacttcg tctccgagcg cagcctgctc gaagccatcg cctcctcgct gaccgagatg 1680

ttctcgccga cgatcatctc cgagcgcgtc gccgggatgc tgaagaacta cgacttcatc 1740

accaaggaca cgctggccta tttcgacaag cgcctgaccc aggccccgcg cgacgccgat 1800

ttcgccctcg actacgtcaa gcggcacgcc accacgcctg agatgcagcg ggcggcgata 1860

gatgcgttga cgttcaagtg caacgtgctc tggacgcaac tcgatgcgct ctacttcgcc 1920

tatgtcgccc ccggcatggt gccgccggat gcttggcagc cgggcgaggg ccttgttgcc 1980

gagacgaact ccgccgagga cagccccgcc gctgcggcca gccccgccgc gacgacagct 2040

gaacccacgg ccttctcggg cagtgacgtg ccgcgcctgc cccgcggcgt gcgcctgcgc 2100

ttcgacgagg tccgcaacaa gcacgtgctg ctcgcccccg agcgcacctt cgacctcgac 2160

gacaacgccg tcgcggtcct caagctcgtc gatggccgga acacggtttc gcagatcgcc 2220

cagattctgg gtcagaccta cgacgccgac ccggccatca tcgaagccga catcctcccg 2280

atgctggccg gcctcgcgca aaaaagggtt ctggagcgat gaatgcaccg acacccgccc 2340

cctcccccgt ggacgtcatt ccggcgccgg tgggtctgct cgccgagctg acgcaccgct 2400

gcccgctgcg ctgcccatac tgctcgaacc cgctggagct cgaccggcgc tcggccgagc 2460

tggacacgca gacgtggctg cgggtgctga cggaggcggc ggggctcggt gtgctgcacg 2520

tccacctgtc gggcggtgaa ccgaccgccc gccccgacat cgtcgagatc acggccaaat 2580

gcgccgaact cggcctgtac tcgaacctga tcacctccgg cgtcggcggt gccttagcga 2640

agctcgacgc gctctacgac gtcggcctcg accacgtgca gctctccgtc caaggggtgg 2700

acgcggccaa cgcggaaaag atcggcggcc ttaagaacgc gcagccgcag aagatgcaat 2760

tcgctgcccg ggtcaccgaa ctcggcctgc cgctgacgct gaactcggtg atccaccgcg 2820

gcaacatcca cgaggtgccg ggcttcatcg acctcgcggt caagctcggc gccaagcggc 2880

tggaggtggc ccatacccag tattacggct gggcctatgt gaaccgcgcc gcgctgatgc 2940

cggataagag ccaggtcgac gagtcgatcc gcatcgtcga ggccgcgcgc gagcgcctca 3000

agggtcagct cgtcatcgac ctcgtggttc cggactacta cgccaagtac ccgaaggcct 3060

gcgccggcgg ctggggccgc aagctgatga acgtgacgcc gcagggcaag gtgctgccct 3120

gccacgccgc agaaaccatc cccggcctcg aattctggta cgtcaccgac cacgcgctcg 3180

gcgagatctg gacgaagtcc ccggcctttg ccgcctatcg cggcacgtcc tggatgaagg 3240

agccctgccg ctcctgcgac cggcgcgaga aggattgggg cgggtgccgc tgccaggcgc 3300

tggcgctcac gggcgacgcg gccaacaccg atccggcctg ctccctttcg ccgctgcacg 3360

cgaaaatgcg ggatcttgcc aaggaagagg ctgccgagac cccgcccgat tatatatacc 3420

gcagcatcgg gacgaatgtg caaaacccgt tgagcgaaaa ggcacccctt tga 3473

<210> 65
<211> 2738
<212> DNA
<213> Methylobacterium extorguens

<400> 65
atgcacctct accggaaggc catcgcgccc gtgggaccgc agcgaggcct gaatcacgcc 60

ggccgtgtgg acgccgcccc gttcgggcgc tccgaggcgg gcggcccgga ggtctccgcc 120

ttcgtgctcg acaacgggct cgacgtggtg gtggtgcccg atcaccgggc gccggttgcc 180

acgcacatgg tctggtaccg caacggctcg gccgatgatc cgatcggcca gtccggtatc 240

gcccacttcc tcgaacacct gatgttcaag ggcaccgagc ggcacccggc cggcgccttc 300

tcgaaagcgg tctcgtcgct cggcggccag gagaacgcct tcaccagcta cgattacacc 360

gcctatttcc agcgcgtcgc ccgcgaccac ctctcgacga tgatggcctt cgaggccgac 420

cggatgagcg gcctcgtgct cgacgacgcc gtggtggcgc ccgagcgcga cgtggtgctg 480

gaggagcgcc ggatgcgggt cgagaccgat ccgtcggcgc agctctccga ggcgatgtcc 540

gcctcgctgt tcgtgcacca tccctacggc atcccgatca tcggctggat gcacgagatc 600

gaggagctga accgcaccca cgccatcgac tattacaagc gcttctacac ccctgagaac 660

gcgatcctcg tggtggccgg cgacgtgacg ccggacgagg tgcggcgtct ggccgaggat 720

acctacggcc gggtgacgcc gcagggcgcg cggccgctgc gcactcgccc gcgcgagccg 780

gagccgcggg cgatgcgtcg gatcgcggtg gccgacccga aggtcgagca gccgaccctg 840

cagcgcctct acctcacccc ctcctgcatg accgcccgcg acggcgaggg ctacgccctc 900

gaactgctcg ccgaggtcgt cggcggcggc tcgacctcgt tcctctaccg caagctggtg 960

ctggagatgg gcgtcgcggt gaatgccggc gcttggtaca tgggctcggc gatggatgac 1020

acgcgctttg ccgtctacgc cgtgccggcc gagggcgtga ccctggaggc cctcgaagag 1080

catatcgatc gcgtgctgcg ccgcgtcccc gaggcgctcg gtgcggaggc gatcgagcgg 1140

gccaagatcc ggctcatggc cgagacggtc tattcctccg attcgcagag ctcgctcgcg 1200

cgcatctacg gctcggcgct cgccatcggc gagaccgtcg aggaggtgcg ccgctggccg 1260

gtcgagatcg aagcggtcac ccatgaccgg ctcgtcgcgg tcgccgcccg ctatctcgtg 1320

cccgcccgct cggtgaccgg ctacctgacc aaggcgcgcg acccggacgt ggcgatcgcc 1380

tgagacggcc ctgattcttc gcgccgccct cgatggcggc gccgtttgcg aaaagacgca 1440

ttaaggaact tccgatgaat ctcgccgaga ccggcacccg caccgcgacg tcgccgaccc 1500

aggcgttgcc gctcgcggcc gcccccggca tcgaggcttg gcacgtcgcc tcgccggtgg 1560

tgccgatgat cgcgctctcc ttcaccttcg agggcggcgc ggcgcaggat gcggagggca 1620

aggcgggcac cgcgcagatg atggcgcggc tgctcgacga gggcgcgggc gatctcgact 1680

cggatgcctt ccaggaggcg ctcgcggccc gcgcgatcga gctgagcttc cacaccggcc 1740

ccgattccat cggcggctcg ctcaagacgc tgctcacgca tgccgacgag gcgatccgtc 1800

tgctggccct gtcgctggcc gagccgcgct tcgatcaacc ctcgatcgag cgcgtgcggg 1860

cgcagatgat cgccagcctg cgctaccagc agaacgatcc cggcgtgctg gcctcccgcc 1920

gctacttccg cgaggccttc ccggggcatg cctacggccg ctcctcgtcg ggcaccatcg 1980

agaccctgtc ggcgatcacc cgagacgacc tcgtcgccct gcaccgggcc gtgatcggtc 2040

gcggcagcct caaggtcgcg gctgtgggcg ccttcgacga ggcgacgatc accggcatga 2100

tcgcccgcgc cttcggcgct ctgcccgagg ccggcccgct caaagccatt ccgccgaccg 2160

cgatcaacga actcggccgc cgcatcgtcg tcgatctcga cgtgccgcaa tcggtgatcc 2220

gcttcggcat gccgggcgtg gcgtggcgcg accccgactt catcccggcc tatgtgctga 2280

accacatcct gggcggcggc gccttcacct cgcgcctgtt ccaggaagtg cgcgagaagc 2340

gcggcctggc ctactcggtc ggcacctctc tgacctcgca ccgcgccgtc gccatgacct 2400

ggggctacac cgccaccaag aacgagcgcg tcgtcgaggc cctcgacgtg atcggtgacg 2460

agatccagcg cctcatcacc gacggcccct ccgacgagga gttgcagaag gccaaggact 2520

atctcaccgg ctcctacgcg ctcggcttcg acacctcgac caagatcgcc aaccaattgg 2580

tgcagatcgc cttcgagggg ctcggcatgg actacatcgc ccgccgcaac gacctcgtgg 2640

cgagcgtgac ccaggccgac atccgccggg ccggcgcccg cacgctcggc gacggcaaga 2700

tgctggtcgt cgccgcgggg cggcccacgg ggctgtag 2738

<210> 66
<211> 3194
<212> DNA
<213> Pseudomonas aeruginosa

<400> 66
atgtggacca agcccagctt caccgacctg cgtctcggtt tcgaagtgac cctctacttc 60

gccaaccgct gacccatccg gccccggtca tccggggcct ccctccagcc cgcggggcag 120

acccatgcac atccgtattc tcggttcggc cgccggcggc ggctttcccc agtggaactg 180

caactgccgc aactgtcgcg gggtccgcga cggcagcgta gcggcccagc cacgcacgca 240

atcctccatc gccctgtcgg acgacggcga gcgctggatc ctctgcaacg cctcgcccga 300

catccgtgtc cagatcgccg ccttcccggc cctgcagccg gcgcgtcggc cgcgcgatac 360

ggcgatcggc gcgatcgtcc tgctcgacag ccagatcgac cacaccaccg gcctgctcag 420

cctgcgcgaa ggctgcccgc acgaagtctg gtgcacgcag atggtccacc aggacctcag 480

cgaaggcttt ccgctgttcc gcatgctttc ccactggaac ggcggcctgc gccaccggcc 540

gatcgccctc gatggcgagc ctttcgccat cccggcctgt ccgcgcctgc gcttcaccgc 600

gatccccctg cgcagcagcg cgccaccgta ttccccgcat cgcggcgacc cgcatccggg 660

cgacaacatc ggcctgttcg tcgaggacct cgacagcgcc ggcacactgt tctacgcgcc 720

gggcctcggc gaggtggacg aggcgctgct cgaatggatg cgccgcgccg actgcctgct 780

ggtggacggc acgctctggc gcgacgacga gatgctggcc tgcgaggtcg gcgacaagct 840

cggccggcag atgggccacc tggcgcagag cgggccgggc ggcatgctcg aggtactggc 900

gaaggtgccg gccgcgcgca aggtgctcat ccatatcaac aacaccaatc ccatcctcga 960

caccgcttcg gccgagcgcg ccgaactgga tgccagtggc atcgaagtgg cctgggatgg 1020

catgcacatc cagctgtagg gggaccgaca tgagccgtgc cgccatggac cgcgccgagt 1080

tcgaacgggc gctgcgcgac aaggggcgct actaccatat ccaccatccg ttccatgtcg 1140

cgatgtacga gggccgtgcc agtcgcgaac agatccaggg ctgggtggcg aaccgcttct 1200

actaccagct caacatcccg ctgaaggacg cggcgatcct ggccaactgt cccgaccgcg 1260

aggtccgccg cgagtgggtc cagcgcatcc tcgaccacga cggcgccccc ggcgaggcgg 1320

gaggcatcga ggcctggctg cgcctggcgg aggcggtggg cctggagcgc gagcaggtgc 1380

tgtccgaaga acgggtgctg cccggcgtgc gcttcgccgt cgacgcctat gtcaacttcg 1440

cccgtcgcgc cagctggcag gaggcggcga gcagttcgct gaccgaactc ttcgccccgc 1500

agatccacca gtcgcggctg gacagctggc cgcgccacta tccgtggatc gaggcggccg 1560

gctacgagta tttccgcagc cgcctggccc aggcccggcg tgacgtcgag cacggcctgc 1620

ggatcaccct ggagcactat cggacccgcg aggcacagga acgcatgctg gagatcctgc 1680

aattcaagct ggacgtgctg tggagcatgc tcgacgcgat gagcatggcc tacgagctgg 1740

aacgtccgcc gtaccatacg gtgacccgtg agcgggtctg gcaccggggg ctggcgccat 1800

gagcctgcca tcgctcgaca gcgtgccggt cctgcgccgg ggcttccgct tccagttcga 1860

accggcccag gactgccatg tgctgctcta tcccgaaggg atggtcaagc tcaacgacag 1920

cgccggggag atcctcaagc tggtcgatgg ccgccgcgac gtggcggcca tcgtcgcggc 1980

gctgcgcgaa cgttttcccg aagttcccgg catagacgaa gacatcctga cgttcctcga 2040

ggtggcccat gcgcaattct ggatcgagct gcagtgaaag cgtggggccg ccgctctggc 2100

tgctggccga gctgacctac cgctgcccgt tgcagtgccc gtactgctcg aacccgctgg 2160

aattcgcccg cgagggcgcc gagttgggca ccgcggaatg gatcgaggtg ttccgccagg 2220

ctcgcgagct gggcgccgcc cagctcggtt tctccggcgg cgagccgctg ctgcgccagg 2280

acctcgccga actgatcgag gcggggcgcg gcctggcttt ctacaccaac ctgatcacct 2340

ccggcatcgg cctcgacgag gcacgcctgg cgcgcttcgc cgaggccggg ctggaccacg 2400

tgcagatcag cttccaggcc gccgacgaag aggtgaacaa cctgctcgcc ggctcgcgca 2460

aggccttcgc ccagaaactg gcgatggccc gcgcggtgaa agcccatggc tacccgatgg 2520

tgctcaactt cgtcacccac cggcacaaca tcgacaacat cgagcggatc atccagctgt 2580

gcatcgagct ggaggccgac tacgtggaac tggccacctg ccagttctac ggttgggccg 2640

cgctgaaccg cgccgggttg ctgccgaccc gcgcccaact ggagcgcgcc gagcggatca 2700

ccgccgaata ccgccagcgg ctggccgccg aaggcaatcc gtgcaagctg atcttcgtca 2760

cccccgacta ctacgaggaa cggccgaagg cctgcatggg cggctgggcc agcgtgttcc 2820

tcgacattac cccggacggc accgcgctgc cgtgccacag cgcgcggcaa ctgccgctga 2880

agttccccaa cgtgcgcgag cacagcctgc gccacatctg gtacgagtcg ttcggcttca 2940

accgctatcg cggcgacgcc tggatgcccg agccgtgccg ctcctgcgag gaaaaggagc 3000

gtgaccacgg cggctgccgc tgccaggcgt tcctccttac cggcgacgcc gacgccaccg 3060

acccggtctg cgccaagtcg gcccgccacg acctgatcct cgacgcccgg cgccaggccg 3120

aggaggcgcc gctgggcctg gacgcgctga cctggcgcaa tcagcgcgcc tcgcgcctga 3180

tctgcaaggc ctga 3194

<210> 67
<211> 2292
<212> DNA
<213> Pseudomonas aeruginosa

<400> 67
gtgctgccca atggcctgcg cctgcacctg gcgcatgacc cggcggcatc gcgcgccgct 60

gcctggctgc gggtggcggc gggcagccac gacgagccga ccgcgcatcc cggcctggca 120

cacttcctcg agcacctgct gttcctcggc ggcgcggcgt ttcctggcga cgagcgtctc 180

atgccgtggt tgcaggtgcg cggcggccag gtcaacgcca gcacccgggg caggagcacc 240

gactatttct tcgaggtggc ggcggagcac cttggcgccg gtctggcacg cctgttcgac 300

atgctcgtgc gaccgctgct ggatatcgac gcgcaacggc gcgagcgcga agtgctggaa 360
gccgagtacc tggcgcgcgc ggccgacgag cagaccctga tcgatgcggc gctggccctc 420
ggcctgcccg ccgggcatcc cctgcggcgt ttcgttgccg ggcgccgcga cagcctggcg 480
ctggagaccg atgcgttcca gcgggcgctg cgcgaattcc atgccgccca ctatcacgcc 540
ggcaattgcc agctatggct gcaagggccg caggcactgg acgagttgga gaggctggcg 600
cggcgtgcct gcgccgacct gccgggtcgc tcgccaggcg cgagtccgtc gccgccgccg 660
ttgtcaccct tcgccggcgc ggcgctagcg ctgcgcctgc cgggtccgcc gcgtctggtg 720
ctgggctttg ccatcgacgc tttgcggggg gctgacgaac agaccctgct ggcattcgcc 780
gaattgcttg gcgatcgctc gccgggcggg ttgctggcgg cactcggcga acagggcctg 840
ggcgaatcgg tggcgctgcg ggtggtccat cgggacgcgc ggcaggcgct cctggcgctg 900
accttcgaac tgttcgacgg cagcgcggcg gcggcgctgg aggagacctt tttcgactgg 960
ctgcgcgccc tgcgcgacga tgccgcgagc ctgctggcgg cgcgccggcc gttgctggcg 1020
gagcccactg cgccgctgga gcgactgcgc cagcgcgtgc ttggcctgcc agcggagatt 1080
cgcccggcct gcctggatgc gctgcgcgcc gaacgctgcc tgcggttgca cctggacagc 1140
gaactcgacg gcgccgaagc gcgctggtcg gcgggcttcc gcctgagcgt ggcgcccgtc 1200
gccgcagcgc cgccgccgct ggcagcgcag cggcatgcct ggcgcttcga actgccgccg 1260
ccgccgagca ccgccgccga gggcgcgctg ttcctgcgct ggcgctttcc cggcatatcc 1320
gcacggtcac gcttcctggc gctgcgccag gcgttgcgtc cgctgtgcgg ccaggcgcgc 1380
ctgcgggggg tggagatggg cctggaagcg ctcggcgaag actggacgct gagcctgctc 1440
ggagcacgcg accgcctcga ggccgccgtg cgaccggccc tggcccggct cctcgcggcg 1500
ccggccgact ggcgcgcaag cggcgagcgc ctgtcgttcg ccgagcggcg ccgcagcgcc 1560
actggcctgc cgatccggca actgctggac gccttgcccg gcgtactcgg cgagccgctg 1620
gcggaggtcg acgacgactg gcggcggacg cgctgggacg gcctggtcat gcaggccgcg 1680
atgcctgatc cacgatgggt gcccgggcag gctaccggca agcgccttga gcctctgcca 1740
gcgatgcccg gacggcatcg ccgcgagctg gccgtggacg gtgagtcggc gttgctgctg 1800
ttctgtccgc tgcccgcgcc ggaggtgccg atggaggcgg cctggcgcct gctggcgcgc 1860
ctgcacgagc cggccttcca gcggcgcctg cgcgacgagt tgcaactggg ctacgcgctg 1920
ttctgcggct tccgtgaggt cggcgcacgg cgcggcctgc tgttcgccgc gcaatctccg 1980
cgtgcctgcc cggaacgcct gctggagcac acggagatct tcctccagcg ttccgtcgag 2040
gcgctcgcgc aactgccggc gcaacgcctg gccggcctgc gcgatgccct cgccgacgac 2100
ctgcgccggg cgccggggac ttttgccgag cgggcgcgac acgcctgggc ggagcatctc 2160

ggcggtggcc agggccgctc ccggctgctc gccgaggctg ctcgcggcct gggcgtcgac 2220

gatctgcttg ccgcccaggc cgcgctgctg gaggcgcacg gcggctggtg ggtgctttcc 2280

agccgacgct ga 2292

<210> 68
<211> 3187
<212> DNA
<213> Gluconobacter oxydans

<400> 68
atggcctgga acacaccgaa agttaccgaa atcccgctgg gcgcagaaat caactcgtat 60

gtctgcggcg agaagaaata agccgctttc ccggggaccc gtccttgagg aataatggca 120

cggccgctcc cccatggagc ggccgttttc gttcatgggt gctctgtggt gccccagtca 180

gacggtttgt gaaaaaatga ttgatgtcat cgtgcttggc gcggcggcag gggcggtttt 240

tccgcagtgg aactccgcag cacccggctg tgtggccgcc cgcacgcgac agggcgcgaa 300

agcccggacc caggcctccc ttgccgtcag tgccgacgga aagcgctggt tcattctcaa 360

cgcctcgccc gatctgcggc agcagatcat cgatacgccg gccctgcatc atcagggcag 420

cctgcgtgga acgcccattc agggcgtcgt cctgacctgc ggcgagatcg acgccataac 480

cgggcttctg accctgcgtg agcgtgagcc ttttaccctg atgggcagcg actcgaccct 540

tcagcagctt gcggacaatc cgatcttcgg tgcgctcgat ccggaaatcg tcccacgtgt 600

tccgctcatt ctcgatgaag ccacgtccct gatgaacaag gacgggattc cgtccggtct 660

tttgctcacg gccttcgccg ttccgggcaa ggcgccgctt tacgcggaag ccgcagggtc 720

acgcccggac gagacgctgg gcctttccat tacggatgga tgcaagacga tgctcttcat 780

tcccggctgt gcgcagatca cgtcggaaat cgtggaacgg gtagcggcag ccgatctcgt 840

gttctttgac gggacactgt ggcgggatga cgaaatgatc cgcgccgggt tgagcccgaa 900

gagcggacag cggatgggac atgtgtccgt gaatgatgcc gggggaccgg tcgaatgttt 960

cacgacatgc gaaaaacccc gtaaagtgtt gattcatatc aacaactcca atccaattct 1020

gttcgaagac agccccgaac gcaaagacgt cgaacgcgcc ggatggacgg ttgcggaaga 1080

cggcatgact ttcagactgg acacaccatg acgctcctca cacctgacca gcttgaagca 1140

cagcttcgcc agatcggggc cgagcggtat cacaaccggc acccgttcca tcgcaagctg 1200

catgacggca agctggacaa ggcacaggtt caggcttggg cgctgaaccg ctattattat 1260

caggcccgca tcccggcgaa ggatgcgacg cttctcgcac gtctgccgac ggccgaactg 1320

cgccgcgaat ggcgtcgccg gatcgaggac catgacggca cggagcccgg aacgggcggt 1380

gttgcgcgct ggctgatgct gacggatggt ctggggctgg accgggatta tgtggaaagc 1440

ctcgatggtc tgcttccagc cacgcgcttc tcggtcgatg cctatgtgaa cttcgtgcgg 1500

gaccagtcga ttctggcggc cattgcgtcg tcgctgacgg aactgttttc gcccacgatc 1560

atcagcgagc gcgtctcggg gatgctgcgg cactacgact ttgtgtcgga aaagacgctg 1620

gcctatttca cgccgcgcct gacgcaggcc ccgcgggatt ccgatttcgc gctggcctat 1680

gtccgcgaaa aggcccgcac gccggagcag cagaaagaag tcctgggagc gctggagttc 1740

aagtgctccg tgctgtggac gatgctggat gcgctcgact acgcctatgt ggaaggccac 1800

attccgccgg gggctttcgt tccatgacgg aggccccgca tgtcgtggcg gaggggacgg 1860

ttctctcctt tgcccggggg catcgtctcc agcacgatcg tgtgcgggac gtgtggatcg 1920

tgcaggcgcc tgaaaaagca tttgtagttg agggcgccgc gccgcatatt ctgcggctgc 1980

tggatgggaa gcgcagcgtc ggcgagatca tccagcagct tgcaatcgag ttttccgccc 2040

cgcgtgaggt cattgcgaaa gatgtcctcg cgcttctttc tgaactgaca gaaaagaacg 2100

tcctgcacac atgacactcc cttcgccgcc gatgagcctt ctggctgaac tgacgcatcg 2160

atgcccgctt tcctgcccct actgctccaa tccgcttgaa ctcgaacgca aggcggcaga 2220

actcgacacg gccacctgga ctgccgtact ggagcaggcg gccgagcttg ggtgctccca 2280

ggttcatttc tctggcggcg agcctatggc gcggcctgat ctggtcgaac tggtctccgt 2340

cgcacggaga ctcaacctgt attccaactt gatcacgtcc ggcgtgttgc tggacgaacc 2400

gaaactggaa gctctcgaca gggcggggct ggatcacatc cagctctctt tccaagacgt 2460

gacggaggcg ggagccgagc gtatcggcgg tctcaaggga gcgcaggccc gcaaggttgc 2520

ggcggcgcgg ctcatccgcg cgtccggcat tccgatgacg ctcaattttg tggtgcacag 2580

ggaaaatgtc gcccgtatcc ccgagatgtt cgccctggcg cgggaactcg gagcggggcg 2640

ggtggagatc gcgcataccc agtattatgg ctgggggctg aaaaaccgtg aggcgcttct 2700

tcccagccgg gatcagctgg aggaatccac acgcgccgtg gaagcggagc gcgctaaggg 2760

tggtttgtcc gttgattatg tgacgccgga ctatcatgca gaccggccca agccctgcat 2820

ggggggatgg ggccagcgtt tcgtgaatgt cacaccttcg ggccgggtcc tgccgtgtca 2880

tgcagccgaa atcattccgg atgtcgcatt cccgaatgtg caggatgtga ccctgtccga 2940

aatctggaac atctcaccgc tgttcaacat gttccgcggg acggactgga tgccggagcc 3000

ctgccgctcc tgcgagcgca aggagcgtga ctggggcggg tgtcgctgtc aggcgatggc 3060

gctgacgggg aatgccgcga ataccgatcc cgtatgcagt ctctccccct atcacgatcg 3120

ggtggagcag gccgtcgaga acaacatgca gccagaaagc acgttgttct acaggcgtta 3180

tacgtaa 3187

<210> 69
<211> 5474
<212> DNA
<213> Kluyvera intermedia

<400>  69
atgtggaaaa aacctgcgtt tattgacctg cgtctgggct tggaagtgac tctgtacatc      60

tccaaccgct aagacttctg cccgccgttc gtgcgggcat tttgttttct ctctttggtc     120

aacgttcatg tttattaaag tcctcggttc agcggcgggc ggcggcttcc cgcagtggaa     180

ctgtaactgc gccaattgca gcggcctgcg taacggcagt attcaggcgc aggcgcgcac     240

ccaatcttca atcattgtca gcgacaacgg tgaagattgg gtgctgtgca acgcctcgcc     300

ggacatcagc cagcaaattg cccacacgcc agaactgatc aaaaagggtg tgctgcgcgg     360

cacggcgatt ggcagcatca ttctcaccga cagccaaatc gatcacacca ctggcttgtt     420

gagcctgcgc gaaggctgtc cgcatcaggt gtggtgcacg ccggaagtgc atggcgatct     480

caccagcggt ttcccgattt tcaccatgtt gcagcactgg aatggcggct tgcagcatca     540

tgcgttaacg ccgctggagc cgttccgcgt cgatgtctgc cacagtttgc agttcaccgc     600

gattccgatt ctcagcaacg cgccgccgta ttcgccgtat cgcgatcgcc ctgccggacc     660

acaacgtggc gctgttcatg aaaccaccgg caatggccaa acgctgctct acgcgccggg     720

actcggcgag ccggatgcgg tgatccatgc cgtggctgca aagagccgat tgcctgttat     780

tgatggcacg gtgtggcagg acgacgagct gctcgccacc ggcgttggca aaaatactgg     840

caaagcgatg ggccacttag cgctggccga agaacaaggt ttaatggcgt tgctggctgc     900

gctgccggcc aaacgcaaga ttttgattca cattaacaac accaatccga tccttaatga     960

gcaatccccg cagcgcgcct atttgacgca gcagggcatt gaagtgagtt gggatggcat    1020

ggcgattcat ctgcaggagt ttgcatcatg atcatcactg aagcattatc gcccgccgcg    1080

tttgagcagg cgctgcgcgg tttgcatcat gatcatcact gaagcattat cgcccgccgc    1140

gtttgagcag gcgctgcgcg ataaaggcgc gttctaccac atacatcacc cgtatcacat    1200

tgccatgcac aatggcgagg cgacgcgcga gcagattcag ggctgggtgg cgaaccgctt    1260

ctattatcag accaacattc cgctgaaaga tgcggcgatc atggcgaatt gcccggatgc    1320

ggcgacgcgg cgtaaatggg tgcagcgcat cctcgatcac gacggcagca acggtcacga    1380

aggcgggatt gaagcctggc tgcagttggg tgaggcggtg gggctggaac gtgacgtgtt    1440

gctgtcagag aagctggtgc tgccgggcgt gcgttttgcg gtggacgcct acgtgaactt    1500

cgcgcgccgt gctaactggc aggaagcggc ctgtagctcg ctcaccgagc tgtttgcgcc    1560

gcagattcat cagtcgcgtc tcgacagctg gccgcagcac tatccgtgga tcaaagagga    1620

aggctatttc tacttccgca gccgtttgag ccaggcgaac cgcgatgtgg agcatgggct    1680

ggcgctggcg cttgaggtgt ttacccgcgc cgaacagcag aaccgcatgc tggagatcct    1740

gcagtttaaa ctcgatattc tctggaccat gctcgatgcc atgaccatgg cgtatgcgct    1800

gcaacgtccg ccttatcata cggtcaccga tcaggtgtcc tggcacagca caagactggt 1860

ataacaacat gaaagataac ggtcaccgat caggtgtcct ggcacagcac aagactggta 1920

taacaacatg aaagataatg tgattccggc tttccgtcgc ggctaccgca tgcagtggga 1980

agcggcgcag gatagccatg tggtgctcta tccagaaggc atggccaaac tgaacgagac 2040

cgccgtggcg atcctcgaac tggtggatgg caaacaggat gtggcggcga tcgttgccac 2100

gctggatgcg cgtttcccgg acgcgggcgg tgtcggcgac gacgtcaaag agttcctgca 2160

atccgccatt gaacaaaaat ggatacagtg tcgtgaaccc gagtaaaagc gtgacgccac 2220

cgctgtggct gctggcagaa ctgacctatc gctgcccgct gcagtgtcca tattgctcca 2280

atccgctgga tttttcccag cagaagaaag agctgaccac cgaacagtgg attgaggtgt 2340

ttcgtcaggc gcgcgccatg ggcagcgtgc agctcggttt ttccggcggc gaaccgctaa 2400

cccgtaaaga cctgccggag ttgattcgcg ccgcgcgcga tctcggtttc tacaccaacc 2460

tgatcacctc cggcatcggc ctgacggcga aaaaactcga cgcctttgcc gacgccggac 2520

tcgatcatat ccagatcagc ttccaggcca gcgatgaaac gctgaacgcg gcgctggccg 2580

ggtcaaaaaa ggccttccag cagaagctgg agatggcgaa agcggtgaaa gcgcacggct 2640

atccgatggt gctgaatttc gtgctgcatc gccacaatat cgaccagatc gacaaaatca 2700

tcgatctctg tatcgaactg gatgccgacg atgtcgagct ggcgacttgc caattctatg 2760

gctgggcgca actcaatcgt gaaggattgc tgccgacgcg cgagcagatc gccaacgccg 2820

aagcggtggt ggccgattat cgtcagcgca tgggcgccag cggcaatctc accaatcctg 2880

ctgttcgtga cgccggatta ctacgaagag cggccgaaac cctgcatggg cggatgggga 2940

tcgatcttcc tcagcgtcac gccgactgca ccgcgttgcc gtgccacagc gcgcgtcagc 3000

tgccggttgc gttcccgtcg gtgctggagc gcacgctgga cgatatctgg tacaactcgt 3060

ttggttttaa ccgctatcgc ggctttgact ggatgccgga accctgtcgc tcctgcgatg 3120

aaaaagcgaa agactttggc ggctgtcgct gtcaggcctt tatgctgacc ggcgatgccg 3180

ataacaccga tccggtgtgc agcaaatcgc cgcatcacgg caagatcctc gaagcacgac 3240

gtgaagccaa ctgcagcgac atcaaaatcc agcagctgca gttccgcaat cgcagcaact 3300

ccgaactgat cttttaagca gcgcgtcacc tgatgcacgc gcgccagctc acgctcgcca 3360

acggcctgcg ctgccatctt tatcatcagc ccgacgcgcg tgaggcggcg gcgctgatgc 3420

gcgtgcaggc cggcagtctg gatgaagcgg atcgctggcc gggtttggca catctgctgg 3480

aacatctgct gttttgcggc agcgaagctt ttcacggcga cgatcgcctg atgccttggc 3540

tgcagcagca gggcggccag gtgaacgcca ccacgcagtt gagccgcagc gcctatttct 3600

ttcagctccc agctgcggcg ttgtcagcag gggtgctgcg gctgtgcaat atgttggcgt 3660

caccgttgct gacggcgcag gccattcagc aggaaacggc ggtgattgag gctgagtatc 3720

aactgctgca aaaccatgcc gacacgctga gtgaagctgc cgtgctggat aagtggcagg 3780

gacgttttca gcgcttccgc gtcggcagtc ggcaggcctt tggtgacaat gtcacggagt 3840

tgcagtccgc attacgcgac ttccatagcc gcctgtattg cgctgaaaac atggaacttt 3900

ggctacaagg cccgcaatca ctggatgagc tcgaacagct ggcggcacgc ttcggcggca 3960

gtttgctggc gggtggagaa tgcgctgcgg ctgttgaagc tacgctgttg cgaggcgatc 4020

gtttactgct atcaggcggt gaagaaaatt tctggctgac gatgttggtc gcgggcgatg 4080

agcagactgt gcgtgacaat gtcaccttat taaaagcatt ctggcaggac gaagcgccct 4140

acagcttgct ggcgcagctg cgcgttgagg cgttatgcga aacctttgac gcgcattggt 4200

tatggcaaga tgagcaacaa gcgttactgg cgctgcgctt tagcgccagg tgcatttccc 4260

cggcgcaggc gcaacagatt gagcaacgcg tttggcagca tctggctgcg ttagctgaat 4320

gtacggcgct tcagctgcgg cactacgcac agctcgcgca gcaggatttc gcgaccttga 4380

ccttgtcgcc gctggaacag ttgcgcggcc gggcgttggg cttcgcgccg ggttgcgcgc 4440

tgccagacaa ctttacggat tttgccgcag cgttgccaga ttgtccgcgc acgcgtctgc 4500

tcacgcagca acagattgac ggcgcaccgc atgcacacgc agggcttcac cttgcagctg 4560

gtcgagtggc tgccgccgtc gttggctgcg accaaacccg cagcgtttca cttctatcct 4620

gcttcagccg ctgttccgct ccctcgtctc ccacccgttg cgcagccgtt gccgctgatt 4680

gcaccggtaa aacaggtaga aacgctgctg ctgcgtccgg cgttttatca caccttgagg 4740

aggaagacgc gcagtcgcgg cagcgccagt tacgcccgtt gctggcggaa ttgcgccacg 4800

cgggtggcaa cggcagctgg caacagacgc atgccagctg gcagctactg ctcaatttgc 4860

ctgcatccgc cgatcgcgcg ttgtttagcg tgcatcaggc attacaggcg ctgaacacgc 4920

cgatgctggc gcaacccgca gcgaatactc cttcgattgt gattcgtcaa ttgctcgccg 4980

cgttgcccac tcgcttgatc aagccattac ccgcgccaca atggctggcg gcatggtgcg 5040

gcaccaatac cgcattaagc cagcgcgtgg cgcatctgct cagcgatttt acgccggatc 5100

tggcgcgcga cacgccgccg gcacgactgc aacacggcat cgtgcccatc gcctgcgacg 5160

ggcgcgatca ggcgctgctg ctatttatgc cgttaccgca ggccgacgat gccagcctgg 5220

ccgctttacg cgtgctggcg ctgatgctgg aaacgcgctt tttccagcgg ttgcgcgtgg 5280

atcagcaaat tggctatgtg gtgtcggcgc gctatcagcg ggtggcggat gtggatggct 5340

tgatgctggc gctgcaatca ccggatatct cctggcgcgc gctgctgggt cattgcaaac 5400

gctttatgcg ttgagatggt agccgagatc gcggcaatct cgccgcaaaa gctggcggca 5460

tggcaggcaa gctt 5474

<210>  70
<211>  5641
<212>  DNA
<213>  Erwinia amylovora

<400>  70
atgcagtgga ctaaaccaac gtttatcgat atgcgtctgg ggctggaagt cacgttatat      60

atatctaatc gttaacccct cgccttatca caatgtgaat aacgctggct aaaacggtga     120

tgcctgtggt taagcccagg cattttttca ttccctttcc cggttaaccc atgaaaatca     180

aagttctcgg atctgcggcc ggcggcggtt ttccgcaatg gaactgctat tgccctaatt     240

gccagggcgt gcgtaacggt agcatccgcg ccacggcccg cacccagtct tccatcgcgg     300

tcagcgataa cggcagcgac tgggtgcttt gcaacgcctc gcccgatatc tgtcatcaga     360

ttgctgccaa ccccgaactt catccacagg gtaagctgcg cggttcggga attggctcca     420

tcattcttac cgacagtcag atcgatcact gcaccggctt gctgaatctg cgagaaggtt     480

gcccacatca tgtgtggtgc acagcagagg tgcacgcgga tctgaccagt ggttttccga     540

tcttcaccat gctgcaacac tggaacggtg gactggtgca tcatgctatt cagctcgcgc     600

agcctttttc cgtcgcggtg tgtccggcgt tgcggtttac tgctattccc atcctcagca     660

acgccccacc ttactcaccg tatcgcgggc gtccgcttcc cggacacaat atcgcgctga     720

tgatcgaaaa taccgccagc ggcagcaaac tgctgtatgc ccccggtttg ggcgaaccag     780

atgcgcagct gttggatttg atgtcgcagg ccgactgcct gctggttgac ggtacgctgt     840

ggcaggacaa cgagctggca aataccggcg ttgggcgcaa caccggccgc gatatgggcc     900

acctggcgct ggatgaagat cgcgggctga tggcgctact gggcgatctg cccgcaccgc     960

gcaagatcct gatccatatt aataatacta atccgatcct tgatgaatcc tccgccgagc    1020

gtctggcgtt gagcgcacgc ggcatcgagg tcagctacga cggcatgagc atagaactat    1080

gacccaaccg gcattgatga ctccacaaca gtttgaacag gcgctgcgcg ctcgcggcgc    1140

ttactaccat attcatcacc cgtaccatat cgctatgcat aacggcgagg ctacgcgtga    1200

gcagatccag ggctgggtgg cgaaccgcta ttattaccag acccgtattc ctttgaaaga    1260

cgccgcgatt atggcgaact gccgggatgc gttaacccgg cgtaaatggg tacagcgtat    1320

tctcgaccat gatggccagg gcgacagcga gggcggtatc gaagcctggc tgcggcttgg    1380

tgaagccgtt ggcctggatc gtgatgtgct gcagtcagag cagcgggtgc tgcccggcgt    1440

gcgctttgcc gttgatgcct acgtttcctt tgcccgccgt gccgtctggc aggaagccgc    1500

ctgtagctca ctgactgaac tgtatgcccc tgaaatccat cagtcgcgtc tcaacagctg    1560

gccgcagcac tacccgtgga tcgaggaaga gggctacggc tatttccgtg gtcgcctggg    1620

ccaggcccgg cgcgatgttg aacatggttt acagctggcg cttgagtact gcaacagcgt    1680

tgaaaaacag cagcgtatgc tggagatcct gcaatttaag ctggatattt tgtggagcat    1740

gctggatgcc atgaccatgg cgtatacact taaccgtgcg ccttatcaca ccgtgaccga 1800

tcaacccgtc tggcataaag gaaatctgct gtgaaatgtg accaacgcat tcccatattc 1860

cgccgtggat atcgtctgca atgggaagag atgcagaatt gccatgtcat cctctatccc 1920

gaagggatgg ctaaactcaa cgacagcgca acgatgatcc tcgaactggt ggatggtcaa 1980

cgctcgctgg ctgatatcgc ccgtacgctg aatgtgcggt tcccggacgc gggtggcgtt 2040

gatgatgacg tcaccgattt ctttgccgcc gcgcgtgaac agaagtggat aattttccgt 2100

gaacccagct gaatcaccga tcaaaccgcc gctctggcta ctggcggagc tgacctaccg 2160

ctgcccgtta caatgcccgt attgctccaa cccgcttgac tttgcacggc aggaaaaaga 2220

gctgacgact gcccagtgga tcgacgtctt taagcaggcg cgggcgatgg gcgcggtaca 2280

gattggcttt tcgggtggcg aaccgctggt gcgtaacgat ctgcccgagc tgatccgcag 2340

cgcgcgcgat ctcggtttct ataccaacct gattacctcc gggatcggcc tgacgcagaa 2400

gaagatcgac gccttcgccg aagcgggact ggatcatatt cagatcagct tccaggccag 2460

cgatgaaacc ctgaatgcgg cgctggccgg atcgacaaag gcatttcaac aaaagctgga 2520

gatggcgcgt gcggttaaag cgcacggcta tccaatggtg ctgaattttg tgctgcatcg 2580

ccacaatatc gaccagcttg accgcattat cgagctgtgc atcgaactgg aagccgatga 2640

tgtcgagctg gcaacctgcc agttttacgg ctgggcgcag ctcaaccgcg agggactgct 2700

gcctacccgc gaccagctgg cacgcgccga ggcggtggtg caccactacc gcgagaagat 2760

ggccgccagc ggtaatctgg ccaatctgct gttcgtcacc ccggactatt atgaagagcg 2820

gcctaagggc tgcatgggcg gctggggcgc gatcttcctc agcgtcacgc cggaaggcat 2880

ggcgctgccc tgtcacagcg cccgccagct accgatacct ttcccgtcgg tgctggagca 2940

cagcctgcag gatatctggt ttaactcgtt tggctttaac cgttatcgcg gctttgactg 3000

gatgccggag ccatgtcgat cctgtgatga aaaagagaaa gacttcggcg gctgccgctg 3060

ccaggcgttt atgctgaccg gcaacgctga taacgccgac ccggtatgca gcaagtcaga 3120

acatcacggc accatccttg ccgcgcgtga acaggccaac tgcagccaca ttcaggtgaa 3180

ccagctacgg ttccgtaatc gcgcaaactc ccagcgggtt aacgcccagc tgatcttcaa 3240

gggctgacgg atgcagccac agcgacgacg gctcgacaac ggtttgcgcg tggtgttgat 3300

tagcgatgcg caggcagtac aggctgcagc gcttttccag gtggataccg gtagccatta 3360

tgaaccggac agctggccgg ggctggctca tctgttggaa catttgctgt ttgccggcag 3420

ttgcgcttat gcggatgacg agcggctgat ggcctggtta ccggccaggg gagggcggct 3480

gaatgccacc acccagggca gcagcacggc cttttttttc gagtgcgatg ccgggctgct 3540

ggctccgggg ctggctcgtt tgagcgatat gctgctggct ccgctgttgg cagaaaatgc 3600

catccgccag gaagtggcta ccattgacgc cgagtgccgc ctgctggccg ggcagcagga 3660
tacgttgtgc aatgccgcgc agagcatggc atttgccgca catccgtggc agcgttttca 3720
tatcggtaac gccgcgagtt ttaccgggga ctggccggca ctgcggctgg cgttacagca 3780
gtttcatcaa cgctattatc acgcggcgaa tatcacgctg tggttgcagg gaccgcagtc 3840
gcccgaagcg ttatggcagc tggcccgaca gtacggcagt gcgttttctg ctgttggtgt 3900
gccaccgcca gcgcttccgt tactgcatta ttcatcgcag cctgatatgg ctttgcaact 3960
ggcgggatcg ccgcgtttgc gtctctcttt cctgctggac aggccgcgca gtaacgagct 4020
gactttgctg cgccagctct tactggatga ggccgcaggt ggcctgatgg caacgctgcg 4080
tgcgcaccat ctctgcgatg gcgcgcggct gctggtgcct tatcacagcg cgatgcaaac 4140
actggtcagc gttgaactgg cgctgattga tgagcagcag gccgcagagg tggaaggttg 4200
ggtgcatcac tggctgcaac gactgacggc actgacggcg cggcagcgcc agcactatat 4260
gcggctcgct gactttcagt ttgccaggct ttcgccgatg gatcagttac gcgaacacgc 4320
cgttggtttt gctccgccgc aagtacagca agacgactgg ccagggttct gtgcgcggct 4380
gtcagtagcg cgtttagccc gcttgtggat cggcgcgcag tcgctggcgc agcagtgcag 4440
cgttcagggc ttcaggctgc gctgcgctgc gcaaacgcgt gcgccggtaa ccgcgttaat 4500
ccccgctgaa gcgctgactt ttttctgcga aagccagcct gacgctcagg cagcattacc 4560
cgccgggcag gtcgcgctga accatcagcg ggcgggtaag ggccgtgcgg cgctgttgct 4620
aagcccgctg gatgaacttc atgcgccctg ggagtcatcc ctgcaatcgc gcctgagagc 4680
gctggcagcc gactgtgccc ataagggcgg cgatctgagc gtcagctgcc agcagggtca 4740
ttggctgatc cagctgtgcg gcagcccggc gttaatggtg cgcacgctgg caacgctgat 4800
ccgccagctg tgtgagatat cccctgtgat gatcgcgctg ggcgagcggc aataccaacg 4860
ccagcagcag gcgcagcgtg aggggattgc ggtacgcgcc ctgatcgatg cactgcccgc 4920
gctgttgcgt tcatcggttt accaaccggc agggcggcgg ctgccgcgcc tggcgtggca 4980
ggcggtcctc gatggcgggg acgatgcgct ccgccagtcg ttgtcgcagc tgcttagcgc 5040
atttcccggc accatcaatc cgccgggccg gatgcagcca gagccgttag caccgcagcc 5100
tgaatatcag gtggcgacca tcagccacga tgcgaccttg ttacgcttct gcccgctggt 5160
ggaaaacagc acacagtgcc tggcggcctg gcagctgctg gcgctgatct atcaacctgc 5220
atttttccaa cgtctgcgcg tggagcagaa tattggttat gtggtcagct gccgttttta 5280
ccaggcggcg gggcggtcgg ggctgctgtt tgccctgcaa tctccccatc tgagcaccgg 5340
cgagctttcg gcgcatatcg accgtttctt gccaggaatg gacgatgaac tggccgctct 5400
cagcatggaa acagtgcgtg aaaagggggc ggcattgctg gcgcaacaaa ggctggcagc 5460
gtctgatttc cagcaggagt gccggcagcg ctggctggct gcgcaacagt cggtacctca 5520

gcccgatgag tacgttatcc aggggctgac cccggaacgc ctgtcagact accatcagcg 5580

cctgctctcc gaccgccata acgcctggac gctggttggc atccctgcaa atcgcttctg 5640

a 5641

<210> 71
<211> 25
<212> DNA
<213> Artificial

<220>
<223> Primer sequence

<400> 71
gcgccatatg catcgacaat ccttt 25

<210> 72
<211> 35
<212> DNA
<213> Artificial

<220>
<223> Primer sequence

<400> 72
gcgcgctcag gctaattgcg tgggctaact ttaag 35

<210> 73
<211> 28
<212> DNA
<213> Artificial

<220>
<223> Primer sequence

<400> 73
gcgccatatg gctcctgcaa cggtaaat 28

<210> 74
<211> 30
<212> DNA
<213> Artificial

<220>
<223> Primer sequence

<400> 74
ccggcatatg actgatctga aagcaagcag 30

<210> 75
<211> 31
<212> DNA
<213> Artificial

<220>
<223> Primer sequence

<400>  75
ccgctcagct cattagtagc tgctggcgct c                                    31


<210>  76
<211>  39
<212>  DNA
<213>  Artificial

<220>
<223>  Primer sequence

<400>  76
gcaggctgag cttaacttta agaaggagat atacatatg                            39


<210>  77
<211>  35
<212>  DNA
<213>  Artificial

<220>
<223>  Primer sequence

<400>  77
gcgcgctcag cctaattgcg tgggctaact ttaag                                35


<210>  78
<211>  37
<212>  DNA
<213>  Artificial

<220>
<223>  Primer sequence

<400>  78
gcgcggtacc gcacatgtcg cggatgttca ggtgttc                              37


<210>  79
<211>  37
<212>  DNA
<213>  Artificial

<220>
<223>  Primer sequence

<400>  79
gcgcggatcc gggcggagag tttggagaac ctcttca                              37


<210>  80
<211>  30
<212>  DNA
<213>  Artificial

<220>
<223>  Primer sequence

<400>  80
gcgcggatcc acgcagcatc gggccgttct                                      30

<210> 81
<211> 31
<212> DNA
<213> Artificial

<220>
<223> Primer sequence

<400> 81
gcgcggtacc aagcttcgct gccgcaaaac a 31


<210> 82
<211> 42
<212> DNA
<213> Artificial

<220>
<223> Linker sequence

<400> 82
gcgcaagcat gcggatccgg taccaagctt gcatgcacac ta 42

## Description

### Field of the Invention

The present invention relates in general to the field of chemical technology and in particular to a process for preparing an active pharmaceutical ingredient (API) or intermediates thereof by using an enzyme. Particularly, the present invention relates to a preparation of HMG-CoA reductase inhibitors, known also as statins, wherein a certain enzyme is provided for catalyzing a particular step in the synthesis. In certain embodiment, this invention relates to a process for preparing an intermediate by providing said enzyme. The invention further relates to an expression system effectively translating said enzyme. In addition, the invention relates to a specific use of such enzyme for preparing API or intermediate thereof, and in particular for preparing statin or interemediate thereof.

### Background of the Invention

Synthetic routes are routinely performed by carrying out chemical reactions in vitro. The chemistry can become complex and can require expensive reagents, multiple long steps, possibly with low yields because of low stereoseledivity. In specific cases, it is possible to employ a microorganism or its part that is capable of using a starting material as a substrate in its biochemical pathways that convert the starting material to a desired compound. With the aid of microorganisms or their parts, like for example enzymes, optionally together with synthetic process steps, it may be possible to put together complete synthesis pathways for preparing an API or an intermediate thereof, however, it is a challenging task.

One example of complex synthesis route is the synthesis of inhibitors of the enzyme 3-hydroxy-3-methylglutaryl-coenzyme A reductase (HMG CoA reductase), which were found to be an excellent tool to reduce lipids and cholesterol blood levels in order to reduce mortality due to serious cardiovascular events in atherosclerosis triggered pathological processes and diseases. Among HMG CoA reductase inhibitors, a natural lovastatin is known, which has been superseded by semisynthetic pravastatin, simvastatin and later by completely synthetic atorvastatin, cerivastatin, rosuvastatin, fluvastatin, pitavastatin, bervastatin and dalvastatin. The clinically effective HMG CoA reductase inhibitors are also known as statins (characterised by INN name ending -statin).

All statins share a characteristic side chain consisting of respectively a heptenoic or heptanoic acid moiety (free acid, salt or lactone) connected to a statin backbone (Scheme 1). Biological activity of statins is linked to this structure and its stereochemistry. Normally, multiple chemical steps are required to prepare the heptenoic or heptanoic acid moiety. The construction of this side chain still represents a challenge for a chemist.

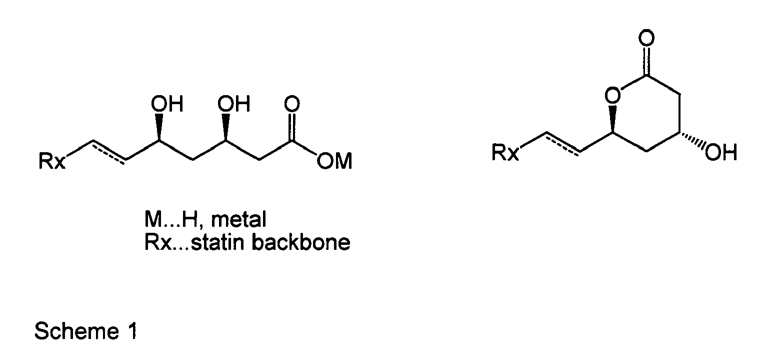

A first attempt to prepare a side chain and thus statin via lactol was disclosed in US 7414119. WO 2006/134482 disclosed the application of aldolases (in particular 2-deoxyribose-5-phosphate aldolase; DERA; EC class EC 4.1.2.4.) for the synthesis of lactols, which enabled direct coupling to obtain atorvastatin.

For further use and eventually yielding statin compounds lactol must be oxidised. In J. Am. Chem. Soc. 116 (1994), p. 8422-8423 and WO 2008/119810 the purified lactol was oxidised to lactone by Br₂/BaCO₃. WO 2006/134482 discloses a use of a catalytic dehydrogenation (e.g. Pt/C, Pd/C) for the same purpose.

It is an object of the present invention to provide a new process that generally allows oxidation or dehydrogenation in preparing API or intermediate thereof in reduced number of process steps, in shorter time, high yield and in an improved, more economic and simplified fashion. Specific aspects of the present invention can be applied for preparing statins or their intermediates.

### Summary of the invention

To solve the aforementioned object, the present invention provides a process according to claim 1. Preferred embodiments are set forth in the subclaims. The present invention further prodives a reaction system according to claim 9, a process for preparing a pharmaceutical composition according to claim 13, expression systems according to claims 14 and 15 and uses according to claims 18 to 21.

Surprisingly it has been found that oxidation from certain lactol to lactone moieties typical for statin type compound synthesis is possible using particular enzymes. It is noted that oxidizing lactols with chemical reagents requires lactols to be first isolated, which is extremely burdensome and consumes large amounts of organic solvents. In addition, 4-hydroxy-lactols have to be first protected with the hydroxyl protection group, which adds to a number of reaction steps and to final impurity profile of an API. On the other hand, the oxidation or dehydrogenation of the compound of formula (II) performed with the enzyme catalyst as defined above according to the present invention is extremely favourable over the prior art chemical oxidation with Br₂/BaCO₃ or the chemical catalytic dehydrogenation (e.g. Pt/C, Pd/C). Chemical oxidants are not specific and thus need previous purification of lactol, otherwise too much reagent is consumed in oxidation of the side products or reagents, or even solvents. The purification of lactol is demanding and requires substantial amounts of a solvent for extraction, which is linked to the fact that lactol is normally hydrophilic and is hardly extracted to the organic solvent, such as for example ethylacetate. Also to note is that solvents used in the extraction need to be evaporated, which is not desired when working on the industrial scale. All aforementioned pitfalls of the chemical oxidation are solved by the process of the present invention. Circumventing the purification and evaporation steps by using the enzyme capable of catalyzing oxidation or dehydrogenation shortens significantly the process of preparing the compound of formula (I).

Although lactols such as compound (II) are non-natural type compounds, they have surprisingly been found to work as effective substrates for the enzymes particularly chosen and disclosed herein. Based on this finding, use of the enzyme capable of catalyzing oxidation or dehydrogenation provides a valuable tool for generally preparing a synthetic API or synthetic intermediate thereof. Generally, the substrate within the use of the present invention for preparing a non-natural, synthetic API or intermediate thereof will therefore be different from naturally occurring ones of the enzyme capable of catalyzing oxidation or dehydrogenation, thereby excluding for example natural substrates selected from ethanol, methanol, acetaldehyde, acetic acid, naturally occurring sugars or amino acids, or sugar acids derived from sugars, including monosaccharides having a carboxylic group such as gluconic acid.

Aspects, advantageous features and preferred embodiments of the present invention summarized in the following items, respectively alone or in combination, contribute to solving the object of the invention.
(1) A process for preparing a compound of formula (I) 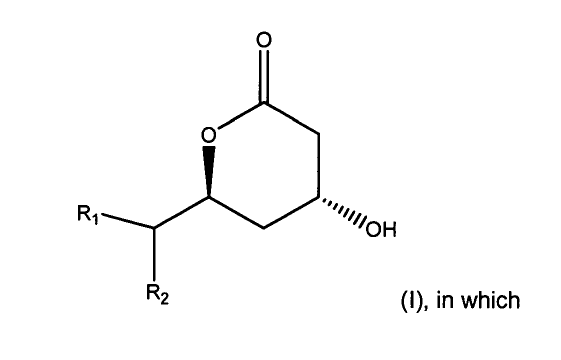 R1 independently from R2 denotes H, X, N₃, CN, NO₂, OH, (CH₂)ₙ-CH₃, O-(CH₂)ₙ-CH₃, S-(CH₂)ₙ-CH₃, NR³R⁴, OCO(CH₂)ₙCH₃, NR₃CO(CH₂)ₙCH₃, CH2-R5, optionally substituted mono- or bicyclic aryl, heterocyclic or alicyclic group; and
   R2 independently from R1 denotes H, (CH₂)ₘ-CH₃, or aryl;
   or both of R1 and R2 denote either X, OH or O((CH₂)ₙCH₃);
   or R¹ and R² together denote =O, =CH-R5, or together form a ring -(CH₂)ₚ-, -(CH₂)ᵣ(1,2-arylene)-(CH₂)ₛ-, wherein
   any one of CH₂ or CH₃ groups denoted above may optionally be further substituted by X, N₃, CN, NO₂, OH, (CH₂)ₙ-CH₃, aryl, O-(CH₂)ₙ-CH₃, OCO(CH₂)ₙCH₃, NR³R⁴, NR³CO(CH₂)ₙCH₃; or
   each CH₂ linking carbon atoms can be replaced by O, S or NR³; wherein
   R³ and R⁴ independently from each other, or together, denote H, (CH₂)ₘ-CH₃, or together form a ring -(CH₂)ₚ-, -(CH₂)ᵣ-(1,2-arylene)-(CH₂)ₛ-, -(CO)ᵣ-(1,2-arylene)-(CO)ₛ-;
   R5 denotes optionally substituted mono- or bicyclic aryl, heterocyclic or alicyclic group,
   X denotes F, Cl, Br or I;
   n represents an integer from 0 to 10;
   m represents an integer from 0 to 3;
   p represents an integer from 2 to 6;
   and at least one from r and s is 1;
   or a pharmaceutically acceptable salt, ester, or stereoisomer thereof,
   the process comprising bringing in contact a compound of formula (II), 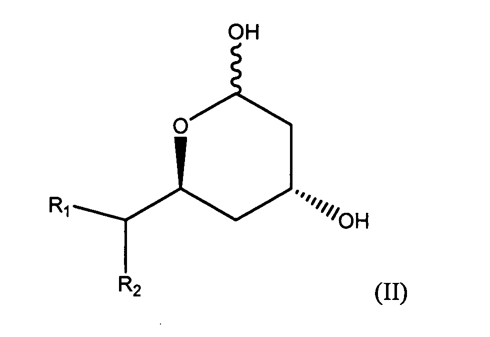 wherein R1 and R2 are defined as above, with an enzyme capable of catalyzing oxidation or dehydrogenation, and optionally salifying, esterifying or stereoselectively resolving the product.
(2) The process according to item 1, wherein
   R5 denotes a moiety selected from the formula (III), (IV), (V), (VI), (VII), (VIII) and (IX); 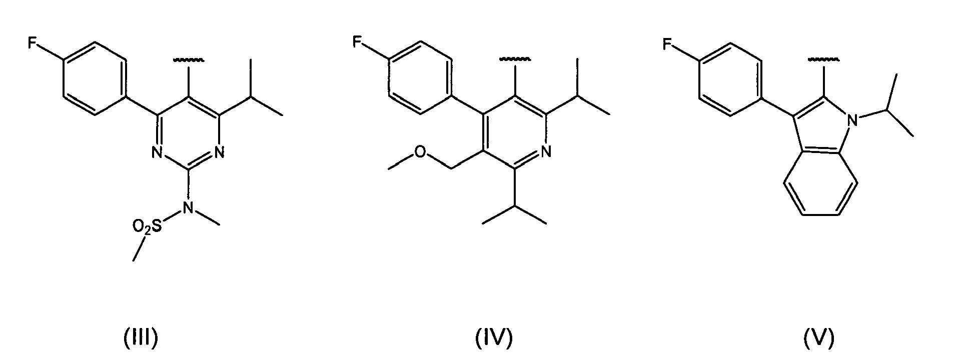 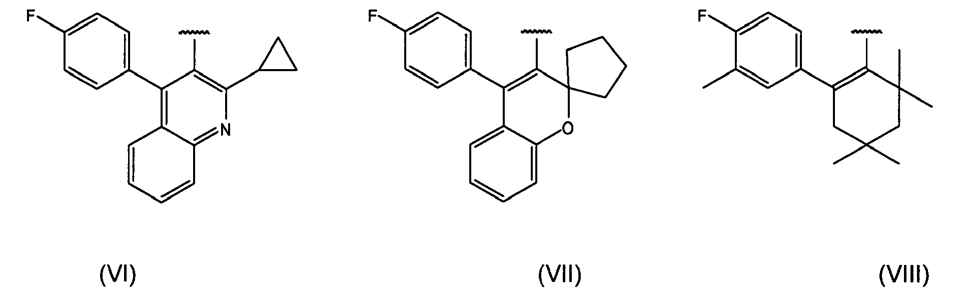 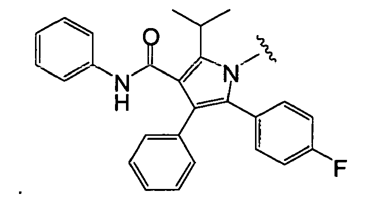 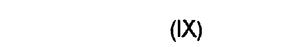
(3) The process for preparing a compound of formula (I) according to items 1 or 2, in which
   R1 independently from R2 denotes H, X, N₃, CN, NO₂, OH, (CH₂)ₙ-CH₃, O-(CH₂)ₙ-CH₃, S-(CH₂)ₙ-CH₃, NR³R⁴, OCO(CH₂),CH₃, or NR³CO(CH₂)ᵣCH₃, optionally substituted mono- or bicyclic aryl, heterocyclic or alicyclic group; and

   R2 independently from R1 denotes H, (CH₂)ₘ-CH₃, or aryl;
   or both of R1 and R2 denote X, OH or O(CH₂)ₙCH₃;
   or R¹ and R² together denote =O, or together form a ring -(CH₂)ₚ-, -(CH₂)ᵣ-(1,2-arylene)-(CH₂)ₛ-, wherein
   any one of CH₂ or CH₃ groups denoted above may optionally be further substituted by X, N₃, CN, NO₂, OH, (CH₂)ₙ-CH₃, aryl, O-(CH₂)ₙ-CH₃, OCO(CH₂)ₙCH₃, NR³R⁴, NR³CO(CH₂)ₙCH₃; or
   each CH₂ linking carbon atoms can be replaced by O, S or NR³; wherein
   R³ and R⁴ independently from each other, or together, denote H, (CH₂)ₘ-CH₃, or together form a ring -(CH₂)ₚ-, -(CH₂)ᵣ-(1,2-arylene)-(CH₂)ₛ-, -(CO)ᵣ-(1,2-arylene)-(CO)ₛ-;
   X denotes F, Cl, Br or I;
   n represents an integer from 0 to 10;
   m represents an integer from 0 to 3;
   p represents an integer from 2 to 6;
   and at least one from r and s is 1.
(4) The process for preparing a compound of formula (I) according to any one of the previous items, in which
   R1 independently from R2 denotes H, X, N₃, CN, NO₂, OH, (CH₂)ₙ-CH₃, O-(CH₂)ₙ-CH₃, S-(CH₂)ₙ-CH₃, NR³R⁴, OCO(CH₂)ₙCH_{3,} or NR³CO(CH₂)ₙCH₃; and
   R2 independently from R1 denotes H or (CH₂)ₘ-CH₃;
   or both of R1 and R2 denote X, OH or O(CH₂)ₙCH₃;
   or R¹ and R² together denote =O, -(CH₂)ₚ-, -(CH₂)ᵣ-(1,2-arylene)-(CH₂)ₛ-, wherein
   R³ and R⁴ independently from each other, or together, denote H, (CH₂)ₘ-CH₃, or together form a ring -(CH₂)ₚ-, -(CH₂)ᵣ-(1,2-arylene)-(CH₂)ₛ-, -(CO)ᵣ-(1,2-arylene)-(CO)ₛ-;
   X denotes F, Cl, Br or I;
   n represents an integer from 0 to 10;
   m represents an integer from 0 to 3;
   p represents an integer from 2 to 6;
   and at least one from r and s is 1.
(5) The process according to any one of the previous items, wherein the enzyme capable of catalyzing oxidation or dehydrogenation is an oxidase or a dehydrogenase.
(6) The process according to any one of the previous items, wherein the enzyme capable of catalyzing oxidation or dehydrogenation is capable of catalyzing aldose dehydrogenation.
(7) The process according to any one of the previous items, wherein the enzyme capable of catalyzing oxidation or dehydrogenation is an aldose dehydrogenase.
(8) The process according to any one of the previous items, wherein the enzyme capable of catalyzing oxidation or dehydrogenation is specific for oxidation of lactol hydroxy group.
(9) The process according to any one of the previous items, wherein the enzyme capable of catalyzing oxidation or dehydrogenation is aldose 1-dehydrogenase (EC 1.1.5.2).
(10) The process according to any one of the previous items, wherein the enzyme capable of catalyzing oxidation or dehydrogenation is selected from a group of pyrroloquinoline quinine (PQQ) dependent dehydrogenases.
(11) The process according to any one of the previous items, wherein the enzyme is water-soluble.
(12) The process according to any one of the previous items, wherein the enzyme is selected from the group consisting of dehydrogenases encoded by dehydrogenase-encoding genes comprised within, or constituted by, any one of nucleotide sequences of SEQ ID NOS. 01, 03, 05, 07, 09, 23, 25, 27, 29, 31, 33, 35, 37, 39, 41, 43, 45, 47, 49, 51, 53, 55, 57, 59 and 61; or dehydrogenases defined by any one of amino acid sequences comprised within, or constituted by, SEQ ID NOS. 02, 04, 06, 08, 10, 24, 26, 28, 30, 32, 34, 36, 38, 40, 42, 44, 46, 48, 50, 52, 54, 56, 58, 60 and 62; or any dehydrogenase having a nucleotide sequence identity or an amino acid sequence identity respectively of at least 50 % to said sequences, preferably 70 % to, more preferably 90 % to said sequences, provided that the resulting sequence variants maintain dehydrogenase activity.
(13) The process according to any one of the previous items, wherein the enzyme is Ylil aldose dehydrogenase.
(14) The process according to any one of the previous items, wherein 2-deoxyribose-5-phosphate aldolase (DERA, EC 4.1.2.4) enzyme is used for preparing the compound of formula (II).
(15) The process according to item 14, wherein 2-deoxyribose-5-phosphate aldolase (DERA, EC 4.1.2.4) enzyme is used for a synthetic step preceding, or alternatively simultaneously at least in an overlapping time period with, bringing in contact the compound of formula (II) with the enzyme capable of catalyzing oxidation or dehydrogenation.
(16) The process according to any one of the previous items, wherein the compound of formula (II) is brought in contact with the enzyme capable of catalyzing oxidation or dehydrogenation without prior isolation or purification of the compound of formula (II).
(17) The process according to any one of the previous items, wherein the enzyme capable of catalyzing oxidation or dehydrogenation, optionally also DERA enzyme independently, are comprised within living whole cell, inactivated whole cell, homogenized whole cell, or cell free extract; or are purified, immobilized and/or are in the form of an extracellularly expressed protein, preferably are within living whole cell, inactivated whole cell or homogenized whole cell, more preferably are within living whole cell or inactivated whole cell, particularly are comprised within living whole cell.
(18) The process according to any one of items 14 to 17, wherein the compound of formula (I) is prepared at least in part simultaneously with the preparation of the compound of formula (II).
   In the preferred embodiments as defined in items 13 to 17, the arrangement of having the compound (II) prepared by using 2-deoxyribose-5-phosphate aldolase (DERA) enzyme and allowing the product to be used, preferably to be simultaneously used at least in an overlapping time period, with a subsequent oxidation reaction by the enzyme capable of catalyzing oxidation or dehydrogenation is especially advantageous in terms of process efficiency and reduced time required for the process. When the processes proceed at least partially simultaneously, the prepared compound of formula (II) can get immediately consumed as a substrate in a subsequent oxidation reaction, which shifts the steady state equilibrium of the first reaction in a direction of the product.
(19) The process according to any one of the previous items, wherein electron acceptor is added to the enzyme capable of catalyzing oxidation or dehydrogenation, preferably oxygen is added.
   When according to this further preferred embodiment oxygen is added to the enzyme capable of catalyzing oxidation or dehydrogenation, or the process is run in the presence of oxygen, like for example under aerated conditions, preferably when oxygen is bubbled to the dehydrogenation or oxidation reaction catalysed by the dehydrogenation or oxidation enzyme, the reaction becomes irreversible, which secures the obtained product and further enhances shifting of the steady state equilibrium of the first reaction, when the compounds of formula (II) and (I) are prepared simultaneously. The presence of oxygen, particularly the presence of dissolved oxygen above 5%, wherein 100% dissolved oxygen is understood as saturated solution of oxygen at given process conditions, is again a favourable process parameter that increases yield and reduces reaction times. In addition, allowing oxygen to be present might in a specific case promote proliferation of a microorganism used.
(20) A reaction system, or a one-pot process for preparing a compound of formula (I) 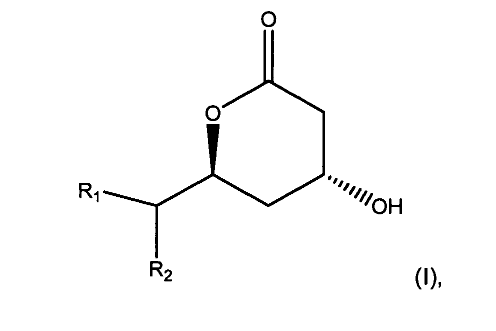 in which R1 and R2 are as defined in any one of items 1 to 4, or a pharmaceutically acceptable salt, ester or stereoisomer thereof,
   the reaction sytem being capable of or arranged for, or the one-pot process comprising reacting a compound of formula (X), 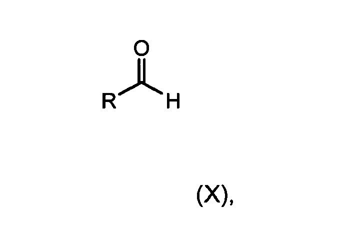 in which R denotes R1-CH-R2 moiety of formula (I), R1 and R2 being as defined in any one of tems 1 to 4,
   with acetaldehyde in the presence of 2-deoxyribose-5-phosphate aldolase (DERA) enzyme and an enzyme capable of catalyzing oxidation or dehydrogenation, and optionally salifying, esterifying or stereoselectively resolving the product.
   Here, the enzyme capable of catalyzing oxidation or dehydrogenation may preferably be represented by an enzyme as defined in any one of items 5 to 12.
   The term "reaction sytem" means a technical system, for example an *in vitro*-system, a reactor or a cultivation vessel or a fermentor.
   The terms "capable of" or "arranged for" means that the reaction system is suitable, or conditions and configurations are set that the defined reaction can take place.
(21) The system or process according to item 20, wherein a compound of formula (II) as defined in item 1 is generated as intermediate, which is not isolated.
(22) The system or process according to any one of items 20 or 21, wherein the enzyme capable of catalyzing oxidation or dehydrogenation, optionally also DERA enzyme independently, are comprised within living whole cell, inactivated whole cell, homogenized whole cell, or cell free extract; or are purified, immobilized and/or are in the form of an extracellularly expressed protein, preferably are within living whole cell, inactivated whole cell or homogenized whole cell, more preferably are within living whole cell or inactivated whole cell, particularly are comprised within living whole cell.
(23) The system or process according to any of the items 20 to 22, wherein both said enzymes, i.e. DERA and the enzyme capable of catalyzing oxidation or dehydrogenation, are expressed by a same cell.
(24) The process according to item 17, or the system or process according to items 22 or 23, wherein the cell is a bacteria, yeast, insect cell or a mammalian cell, preferably is bacteria or yeast, more preferably is bacteria.
(25) The system or process according to item 24, wherein the bacteria is selected from the group of genera consisting of *Escherichia, Corynebacterium, Pseudomonas, Streptomyces, Rhodococcus, Bacillus, Lactobacillus, Klebsiella, Enteorobacter, Acinetobacter, Rhizobioum. Methylobacterium, Kluyvera, Gluconobacter, Erwinia, Rahnella and Deinococcus.* In a more particular sense the microorganisms may be selected from *Klebsiella pneumoniae, Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans, Corynebacterium glutamicum, Escherichia coli, Bacillus licheniformis,* and *Lactobacillus lactis,* most preferably from *Escherichia coli, Gluconobacter oxydans, Acinetobacter calcoaceticus and Kluyvera intermedium,* particularly is *Escherichia coli.*
(26) The system or process according to item 24, wherein the yeast is selected from the group of genera consisting of Saccharomyces, Pichia, Shizosaccharomyces and Candida, preferably Saccharomyces.
(27) The system or process according to item 24, wherein mammalian cell is Chinese hamster ovary cell or a hepatic cell, preferably is Chinese hamster ovary cell.
(28) The process according to any one of the previous items, further comprising subjecting said compound (I) to conditions sufficient to prepare a statin, or a pharmaceutically acceptable salt thereof, preferably lovastatin, pravastatin, simvastatin, atorvastatin, cerivastatin, rosuvastatin, fluvastatin, pitavastatin, bervastatin, or dalvastatin, or a pharmaceutically acceptable salt thereof, more preferably atorvastatin, rosuvastatin or pitavastatin, or a pharmaceutically acceptable salt thereof, particularly rosuvastatin, or a pharmaceutically acceptable salt thereof.
(29) A process for preparing a statin or a pharmaceutically acceptable salt, ester or stereoisomer thereof, comprising steps of:
   (i) bringing in contact the compound of formula (II), 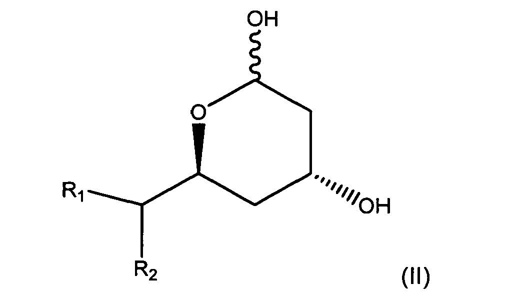 wherein R1 and R2 are defined as in any one of items 1 to 4, with an enzyme capable of catalyzing oxidation or dehydrogenation, to prepare a compound of formula (I) as defined in in any one of items 1 to 4; and
   (ii) subjecting said compound (I) to conditions sufficient to prepare a statin;
   (iii) optionally salifying, esterifying or stereoselectively resolving the product.
(30) The process according to previous item, wherein the compound of formula (II) is prepared by 2-deoxyribose-5-phosphate aldolase (DERA, EC 4.1.2.4) enzyme.
(31) The process according to previous item, wherein the compounds of formula (II) and (I) are prepared at least in part simultaneously.
(32) The process according to any one of items 29 to 31, wherein the statin is lovastatin, pravastatin, simvastatin, atorvastatin, cerivastatin, rosuvastatin, fluvastatin, pitavastatin, bervastatin, or dalvastatin, more preferably atorvastatin, rosuvastatin or pitavastatin, particularly rosuvastatin.
(33) The process according to any one of the items 29 to 32, wherein further special conditions as defined in any one of items 5 to 19, or 20 to 27, either alone or in combination, are further met.
(34) The process according to any one of items 29 to 33, further comprising formulating said statin, or a pharmaceutically acceptable salt thereof, preferably lovastatin, pravastatin, simvastatin, atorvastatin, cerivastatin, rosuvastatin, fluvastatin, pitavastatin, bervastatin, or dalvastatin, or a pharmaceutically acceptable salt thereof, more preferably atorvastatin, rosuvastatin or pitavastatin, or a pharmaceutically acceptable salt thereof, particularly rosuvastatin, or a pharmaceutically acceptable salt thereof, in a pharmaceutical formulation.
(35) An expression system comprsing one or more cell types, the respective cell type(s) being genetically engineered to express, in the totality of cell type(s), both 2-deoxyribose-5-phosphate aldolase (DERA) enzyme and an enzyme capable of catalyzing oxidation or dehydrogenation.
(36) An expression system capable of translating 2-deoxyribose-5-phosphate aldolase (DERA) enzyme and an enzyme capable of catalyzing oxidation or dehydrogenation, and overexpressing both of the genes needed for said translation.
(37) The expression system according to item 35 or 36, wherein the enzyme capable of catalyzing oxidation or dehydrogenation is an oxidase or a dehydrogenase.
(38) The expression system according to any one of the previous two items, wherein the enzyme capable of catalyzing oxidation or dehydrogenation is capable of catalyzing aldose dehydrogenation.
(39) The expression system according to any one of items 35 to 38, wherein the enzyme capable of catalyzing oxidation or dehydrogenation is an aldose dehydrogenase.
(40) The expression system according to any one of items 35 to 38, wherein the enzyme capable of catalyzing oxidation or dehydrogenation is selected from a broad spectrum sugar dehydrogenases.
(41) The expression system according to any one of items 35 to 40, wherein the enzyme capable of catalyzing oxidation or dehydrogenation is specific for oxidation at position C1.
(42) The expression system according to any one of items 35 to 41, wherein the enzyme capable of catalyzing oxidation or dehydrogenation is aldose 1-dehydrogenase (EC 1.1.5.2).
(43) The expression system according to any one of items 35 to 42, further capable of expressing a cluster of genes for providing pyrroloquinoline quinine (PQQ).
(44) The expression system according to any one of items 35 to 43, wherein said expression system is a bacteria, yeast, insect cell or a mammalian cell, preferably is bacteria or yeast, more preferably is bacteria.
(45) The expression system according to item 44, wherein the bacteria is selected from the group of genus consisting of of *Escherichia, Corynebacterium, Pseudomonas, Streptomyces, Rhodococcus, Bacillus, Lactobacillus, Klebsiella, Enteorobacter, Acinetobacter, Rhizobioum. Methylobacterium, Kluyvera, Gluconobacter, Erwinia, Rahnella and Deinococcus.* In a more particular sense the microorganisms may be selected from *Klebsiella pneumoniae, Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans, Corynebacterium glutamicum, Escherichia coli, Bacillus licheniformis,* and *Lactobacillus lactis,* most preferably from *Escherichia coli, Gluconobacter oxydans, Acinetobacter calcoaceticus and Kluyvera intermedium,* particularly is *Escherichia coli.*
(46) The expression system according to item 44, wherein the yeast is selected from the group of genus consisting of Saccharomyces, Pichia, Shizosaccharomyces and Candida, preferably Saccharomyces.
(47) The expression system according to item 44, wherein mammalian cell is Chinese hamster ovary cell or a hepatic cell, preferably is Chinese hamster ovary cell.
(48) The expression system according to any one of previous items, which is arranged to express the enzyme capable of catalyzing oxidation or dehydrogenation, DERA (deoxyribose 5-phosphate aldolase), PQQ dependant dehydrogenase and PQQ biosynthetic pathway genes simultaneously.
(49) The expression system according to any one of the previous items, wherein the enzyme capable of catalyzing oxidation or dehydrogenation is selected from the group consisting of dehydrogenases encoded by dehydrogenase-encoding genes comprised within, or constituted by, any one of nucleotide sequences of SEQ ID NOS. 01, 03, 05, 07, 09, 23, 25, 27, 29, 31, 33, 35, 37, 39, 41, 43, 45, 47, 49, 51, 53, 55, 57, 59 and 61; or dehydrogenases defined by any one of amino acid sequences comprised within, or constituted by, SEQ ID NOS. 02, 04, 06, 08, 10, 24, 26, 28, 30, 32, 34, 36, 38, 40, 42, 44, 46, 48, 50, 52, 54, 56, 58, 60 and 62;
   or any dehydrogenase having nucleotide sequence identity or an amino acid sequence identity respectively of at least 50 % to said sequences, preferably 70 % to, more preferably 90 % to said sequences, provided that the resulting sequence variants maintain dehydrogenase activity.
(50) The expression system according to any one of the previous items, wherein the PQQ is provided by expression of a PQQ-synthesis encoding gene comprised within, or constituted by, any one of the nucleotide sequences of SEQ ID NOS. 11, 17, 63, 64, 65, 66, 67, 68, 69, 70; or by expression of a PQQ-synthesis gene encoding any one of the aminoacid sequence SEQ ID NOS. 12, 13, 14, 15, 16, 18, 19, 20, 21 and 22; or by expression of a PQQ-synthesis encoding gene having a nucleotide sequence identity or an amino acid sequence identity respectively of at least 50 % to said sequences, preferably 70 % to, more preferably 90 % to said sequences, provided that the resulting sequence variants maintain activity to produce PQQ.
(51) The expression system according to the previous item, providing for PQQ gene cluster.
(52) Use of an enzyme capable of catalyzing oxidation or dehydrogenation for preparing a synthetic API or intermediate thereof.
(53) The use according to item 52, wherein the synthetic API or intermediate thereof comprises a lactone structural moiety.
   According to this embodiment, the enzyme capable of catalyzing oxidation or dehydrogenation, more specifically an enzyme as defined in any one of items 5 to 12, can act upon a precursor compound comprising the corresponding lactol structural moiety.
(54) Use of an enzyme capable of catalyzing oxidation or dehydrogenation according to item 52 or 53 simultaneously with, or subsequentl to, a DERA enzyme.
(55) Use according to any one of items 52 to 54, wherein conversion of ethanol, monosaccharide or acetaldehyde by the enzyme capable of catalyzing oxidation or dehydrogenation is excluded.
(56) Use of an aldose dehydrogenase enzyme for preparing a compound of formula (I), wherein the formula (I) is as defined in any one of item 1 to 4.
(57) Use of the aldose dehydrogenase enzyme according to item 56, wherein said enzyme is contacted with a compound of formula (II) as defined in item 1.
(58) Use according to any one of items 52 to 57, wherein any one of the conditions of items 2 to 51, either alone or in combination, is further met.
(59) Use of an aldose dehydrogenase enzyme for preparing statin or a pharmaceutically acceptable salt thereof, preferably lovastatin, pravastatin, simvastatin, atorvastatin, cerivastatin, rosuvastatin, fluvastatin, pitavastatin, bervastatin, or dalvastatin, or a pharmaceutically acceptable salt thereof, more preferably atorvastatin, rosuvastatin or pitavastatin, or a pharmaceutically acceptable salt thereof, particularly rosuvastatin, or a pharmaceutically acceptable salt thereof.
(60) Use of an expression system according to any one of items 35 to 51 for preparing a compound of formula (I), wherein the formula (I) is as defined in any one of items 1 to 4.
(61) Use of the expression system according to item 57, wherein any one of the conditions of items 2 to 34, either alone or in combination, is further met.
(62) Use of an expression system according to any one of items 35 to 51 for preparing lovastatin, pravastatin, simvastatin, atorvastatin, cerivastatin, rosuvastatin, fluvastatin, pitavastatin, bervastatin, or dalvastatin, more preferably atorvastatin, rosuvastatin or pitavastatin, particularly rosuvastatin.
(63) Use of the expression system according to item 59, wherein any one of the conditions of items 2 to 34, either alone or in combination, is further met.
(64) Use of a microorganism for preparing a compound of formula (I) 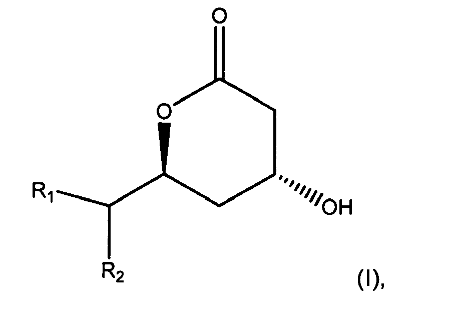 in which
   R1 independently from R2 denotes H, X, N₃, CN, NO₂, OH, (CH₂)ₙ-CH₃, O-(CH₂)ₙ-CH₃, S-(CH₂)ₙ-CH₃, NR³R⁴, OCO(CH₂)ₙCH₃, NR³CO(CH₂)ₙCH₃, CH2-R5, optionally substituted mono- or bicyclic aryl, heterocyclic or alicyclic group; and
   R2 independently from R1 denotes H, (CH₂)ₘ-CH₃, or aryl;
   or both of R1 and R2 denote either X, OH or O((CH₂)ₙCH₃);
   or R¹ and R² together denote =O, =CH-R5, or together form a ring -(CH₂)ₚ-, -(CH₂)ᵣ-(1,2-arylene)-(CH₂)ₛ-, wherein
   any one of CH₂ or CH₃ groups denoted above may optionally be further substituted by X, N₃, CN, NO₂, OH, (CH₂)ₙ-CH₃, aryl, O-(CH₂)ₙ-CH₃, OCO(CH₂)ₙCH₃, NR³R⁴, NR³CO(CH₂)ₙCH₃; or
   each CH₂ linking carbon atoms can be replaced by O, S or NR³; wherein
   R³ and R⁴ independently from each other, or together, denote H, (CH₂)ₘ-CH₃, or together form a ring -(CH₂)ₚ-, -(CH₂)ᵣ-(1,2-arylene)-(CH₂)ₛ-, -(CO)ᵣ-(1,2-arylene)-(CO)ₛ-;
   R5 denotes optionally substituted mono- or bicyclic aryl, heterocyclic or alicyclic group,
   X denotes F, Cl, Br or I;
   n represents an integer from 0 to 10;
   m represents an integer from 0 to 3;
   p represents an integer from 2 to 6;
   and at least one from r and s is 1;
   or a pharmaceutically acceptable salt, ester, or stereoisomer thereof, optionally with further processing of the compound of formula (I) to prepare a statin;
wherein the microorganism
(i) is selected from bacterial origin of the genera *Klebsiella Enteorobacter, Acinetobacter, Rhizobioum. Methylobacterium*, *Kluyvera*, *Gluconobacter, Pseudomonas, Erwinia, Rahnella and Deinococcus;* more particularly selected from the group of specific microorganisms *Klebsiella pneumoniae, Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans;* preferably selected from the group consisiting of: *Gluconobacter oxydans, Acinetobacter calcoaceticus* and *Kluyvera intermedium;* or
(ii) is *Escherichia coli* which is genetically engineered to be capable of expressing genes of the gene cluster for providing pyrroloquinoline quinine (PQQ).

### Detailed description of the Invention

Surprisingly we found a process for preparing a compound of formula (I) 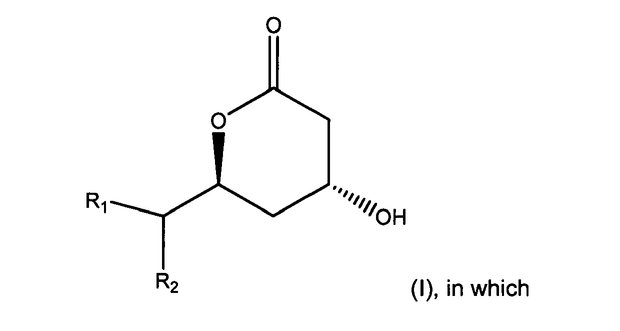 R1 independently from R2 denotes H, X, N₃, CN, NO₂, OH, (CH₂)ₙ-CH₃, O-(CH₂)ₙ-CH₃, S-(CH₂)ₙ-CH₃, NR³R⁴, OCO(CH₂)ₙCH₃, NR³CO(CH₂)ₙCH₃, CH2-R5, or Optionally substituted mono- or bicyclic aryl, heterocyclic or alicyclic group; and
R2 independently from R1 denotes H, (CH₂)ₘ-CH₃, or aryl;
or both of R1 and R2 denote either X, OH or O((CH₂)ₙCH₃);
or R¹ and R² together denote =O,=CH-R5, or together form a ring -(CH₂)ₚ-, -(CH₂)ᵣ-(1,2-arylene)-(CH₂)ₛ-, wherein
any one of CH₂ or CH₃ groups denoted above may optionally be further substituted by X, N₃, CN, NO₂ OH, (CH₂)ₙ-CH₃, aryl, O-(CH₂)ₙ-CH₃, OCO(CH₂)ₙCH₃, NR³R⁴, NR³CO(CH₂)ₙCH₃; or each CH₂ linking carbon atoms can be replaced by O, S or NR³; wherein
R³ and R⁴ independently from each other, or together, denote H, (CH₂)ₘ-CH₃, or together form a ring -(CH₂)ₚ-, -(CH₂)ᵣ-(1,2-arylene)-(CH₂)ₛ-, -(CO)ᵣ-(1,2-arylene)-(CO)ₛ-;
R5 denotes optionally substituted mono- or bicyclic aryl, heterocyclic or alicyclic group,
X denotes F, Cl, Br or I;
n represents an integer from 0 to 10;
m represents an integer from 0 to 3;
p represents an integer from 2 to 6;
and at least one from r and s is 1;
or a pharmaceutically acceptable salt, ester, or stereoisomer thereof,
wherein a compound of formula (II), 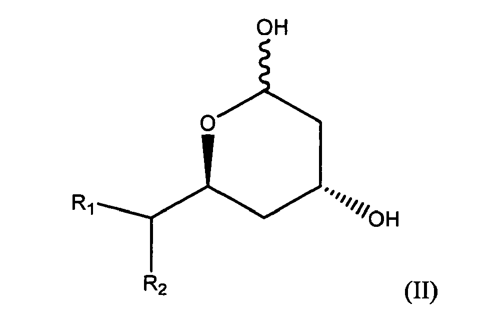 wherein R1 and R2 are defined as above, can be simply brought in contact with an enzyme capable of catalyzing oxidation or dehydrogenation, and optionally the product is salifyed, esterifyed or stereoselectively resolved.

The term "mono- or bicyclic aryl group" as used herein refers to any mono- or bicyclic, 5-, 6- or 7-membered aromatic or heteroaromatic ring, such as for example pyrolyl, furanyl, tiophenyl, phenyl, imidazolyl, pyridinyl, piridazinyl, indolyl, kinolinyl ftaliminyl and benzimidazolyl.

The term "aryl" as used herein, if not stated otherwise with respect to particular embodiments, includes reference to an aromatic ring system comprising 6, 7, 8, 9, 10, 11, 12, 13, 14, 15 or 16 ring carbon atoms. Aryl can be phenyl but may also be a polycyclic ring system, having two or more rings, at least one of which is aromatic. This term includes phenyl, naphthyl, fluorenyl, azulenyl, indenyl, anthryl and the like.

The term "mono- or bicyclic heterocyclic group" as used herein refers to any mono- or bicyclic, 5-, 6- or 7-membered saturated or unsaturated ring, wherein at least one carbon in the ring is replaced by an atom selected from the group of oxygen, nitrogen and sulphur. The non-limiting examples of mono- or bicyclic heterocyclic group are oksazolyl, tiazolyl, isotiazolyl, morfolinyl.

The term "heterocycle" as used herein includes, if not stated otherwise with respect to particular embodiments, a saturated (e.g. heterocycloalkyl) or unsaturated (e.g. heteroaryl) heterocyclic ring moiety having 3, 4, 5, 6, 7, 8, 9, 10, 11, 12, 13, 14, 15 or 16 ring atoms, at least one of which is selected from nitrogen and oxygen. In particular, heterocyclyl includes a 3- to 10-membered ring or ring system and more particularly a 5- or 6-or 7-membered ring, which may be saturated or unsaturated; examples thereof include oxiranyl, azirinyl, 1,2-oxathiolanyl, imidazolyl, thienyl, furyl, tetrahydrofuryl, pyranyl, thiopyranyl, thianthrenyl, isobenzofuranyl, benzofuranyl, chromenyl, 2H-pyrrolyl, pyrrolyl, pyrrolinyl, pyrrolidinyl, imidazolyl, imidazolidinyl, benzimidazolyl, pyrazolyl, pyrazinyl, pyrazolidinyl, thiazolyl, isothiazolyl, dithiazolyl, oxazolyl, isoxazolyl, pyridyl, pyrazinyl, pyrimidinyl, piperidyl, piperazinyl, pyridazinyl, morpholinyl, thiomorpholinyl, especially thiomorpholino, indolizinyl, isoindolyl, 3H-indolyl, indolyl, benzimidazolyl, cumaryl, indazolyl, triazolyl, tetrazolyl, purinyl, 4H-quinolizinyl, isoquinolyl, quinolyl, tetrahydroquinolyl, tetrahydroisoquinolyl, decahydroquinolyl, octahydroisoquinolyl, benzofuranyl, dibenzofuranyl, benzothiophenyl, dibenzothiophenyl, phthalazinyl, naphthyridinyl, quinoxalyl, quinazolinyl, quinazolinyl, cinnolinyl, pteridinyl, carbazolyl, β-carbolinyl, phenanthridinyl, acridinyl, perimidinyl, phenanthrolinyl, furazanyl, phenazinyl, phenothiazinyl, phenoxazinyl, ehromenyl, isochromanyl, chromanyl and the like.

More specifically, a saturated heterocyclic moiety may have 3, 4, 5, 6 or 7 ring carbon atoms and 1, 2, 3, 4 or 5 ring heteroatoms selected from nitrogen and oxygen. The group may be a polycyclic ring system but more often is monocyclic, for example including azetidinyl, pyrrolidinyl, tetrahydrofuranyl, piperidinyl, oxiranyl, pyrazolidinyl, imidazolyl, indolizidinyl, piperazinyl, thiazolidinyl, morpholinyl, thiomorpholinyl, quinolinidinyl and the like. Furthermore, the "heteroaryl" may include an aromatic heterocyclic ring system having 5, 6, 7, 8, 9, 10, 11, 12, 13, 14, 15 or 16 ring atoms, at least one of which is selected from nitrogen and oxygen. The group may be a polycyclic ring system, having two or more rings, at least one of which is aromatic, but is more often monocyclic. This term includes pyrimidinyl, furanyl, benzo[b]thiophenyl, thiophenyl, pyrrolyl, imidazolyl, pyrrolidinyl, pyridinyl, benzo[b]furanyl, pyrazinyl, purinyl, indolyl, benzimidazolyl, quinolinyl, phenothiazinyl, triazinyl, phthalazinyl, 2H-chromenyl, oxazolyl, isoxazolyl, thiazolyl, isoindolyl, indazolyl, purinyl, isoquinolinyl, quinazolinyl, pteridinyl and the like.

The term "mono- or bicyclic alicyclic group" as used herein refers to any mono- or bicyclic, 5-, 6- or 7-membered alicyclic ring.

The term "salifyed" or "a pharmaceutically acceptable salts" in a context of the compound of formula (I) or (II), API or statin, which can be optionally substituted, as used herein refers to the compound or statin in a form of a saft, such as potassium, sodium, calcium, magnesium, hydrochloride, hydrobromide, or the like, that is also substantially physiologically tolerated. The compound of formula (I) or (II), API or statin, can be salifyed or brought in the form of a salt by mixing the compound of formula (I) or (II), API or statin or intermediate thereof, with an acid or a base, optionally in an aqueous or organic solvent, or a mixture thereof. Preferably the solvent is afterwards removed.

The term "esterifying" or "esters" in a context of the compound of formula (I) or (II), API or statin, as used herein refers to the compound of formula (I) or (II), or statin, with at least one ester bond in their structure. Such ester bond or esterifying the compound can be achieved by coupling the compound of formula (I) or (II), API or statin or intermediate thereof, in the event the compound or statin contains hydroxyl group, with an carboxylic acid or a phosphate group containing compound. In the event that the compound of formula (I) or (II), API or statin or intermediate thereof, contain carboxylic or phosphate group, it can be achieved by coupling it with a hydroxylic group of another compound.

The term "stereoselectively resolved" is used herein to refer to any method known to the skilled person in the field of separating a mixture of stereoisomers, preparatory chemistry of stereospecific compounds, or analytics. The stereoisomers can be obtained for example by HPLC, wherein stereoselective column is used. Stereoselective columns are known in the art.

### Enzymes, Organisms

The term "an enzyme capable of catalyzing oxidation or dehydrogenation" as used herein refers to any enzyme that catalyzes oxidation or dehydrogenation. The enzyme recognises and uses e.g. the compound of formula (II) as a substrate. Combinations of enzymes, multiunit enzymes, wherein different units catalyse optionally different reactions, fused or joined enzymes, or enzymes coupled to another structural or a non-catalytic compound, unit, subunit or moiety, are also contemplated within the present invention as long as the requirement of being capable of catalyzing oxidation or dehydrogenation is fulfilled. The enzyme can be for example an enzyme found in the electron transfer chain of the prokaryote or eukaryote cells or in the biochemical pathways of alcohols, aldehydes or sugars in eukaryote or prokaryote cells. Enzymes that would normally act upon natural substrates were unexpectedly found to recognise and oxidise rather complex synthetic compounds, in particular convert lactols into lactones or possibly into esters. It is an important aspect of the present invention that the reactions, which are meant to be used for the an enzyme capable of catalyzing oxidation or dehydrogenation, do normally not occur in the nature, because the substrate is different from the natural occurring ones, like for example expempting from using the ethanol, methanol, acetaldehyde, acetic acid, naturally occurring sugars or naturally occurring amino acids, or acids obtained from the sugars, like for example gluconic acid. It was however surprisingly found that synthetic substrates as disclosed herein, even if being rather structurally complex, can yet be easily processed by using the enzyme capable of catalyzing oxidation or dehydrogenation in order to finally obtain API, particularly statin, or intermediates thereof, including e.g. a lactone compound of formula (I). Generally, enzyme can be chosen from oxydoreductases.

According to the enzyme nomenclature, the enzyme applicable in the present invention belongs primarily to EC 1.1 (oxidoreductases acting primarly on the CH-OH group of donors), more specifically to, but not limited to subclasses: EC 1.1.1 (with NAD⁺ or NADP⁺ as acceptor), EC 1.1.2 (with a cytochrome as acceptor), EC 1.1.3 (with oxygen as acceptor), EC 1.1.5 (with a quinine or flavine or similar compound as acceptor). Any of the oxidoreductases known in the art may be used for the reaction regardless of their sequence identity.

In the following, enzymes will be described in further detail, which are in principle applicable in the present invention - illustrative experimental examples will be described later, and if necessary suitable screeing and/or verification tests are also provided herein. Some of the particular enzymes had been described and optionally used prior to the invention, but in disctinctively other contexts or fields than the present invention. References, the disclosures of which are incorporated herein, will be cited and listed below.

Enzymes having activity of oxidation/dehydrogenation of sugars have been widely used in the industry. Typical examples of oxidative fermentations are traditional production of D-gluconate (gluconic acid), L-sorbose and others. These processes were developed as a practical industry based on empirically found properties of some microorganisms before the clarification of the molecular mechanisms of the responsible enzymes [Adachi, 2007].

Sugar oxidising enzymes had been used in food processing as additives, in dairy and the lactoperoxidase system for food preservation, in breadmaking, for producing dry egg powder, as antioxidants/preservatives (oxygen scavengers), for reducing alcohol wine, as glucose assays and fuel cells [Wong, 2008]. Sugar oxidising enzymes had been used also as amperometric biosensors, e.g. for measuring glucose concentration in blood [Igarashi, 2004], for detection of heavy metals [Lapenaite, 2003], for detection of formaldehyde in air [Acmann, 2008], for detection of phenolic compounds in flow injection analysis [Rose, 2001], as a ultrasensitive bienzyme sensor for adrenaline [Szeponik, 1997], for determination of xylose concentration [Smolander, 1992] etc.

A well known enzyme capable of oxidation of six-membered sugars is Glucose oxidase, Gox (EC 1.1.3.4), which is commercially available from Sigma as an extract from *Aspergillus niger.* This enzyme has a very narrow substrate specificity [Keilin, 1952]. It is produced naturally in some fungi and insects where its catalytic product, hydrogen peroxide, acts as an anti-bacterial and anti-fungal agent. Gox is generally regarded as safe, and Gox from *A*. *niger* is the basis of many industrial applications [Wong, 2008]. Gox-catalysed reaction has also been used in baking, dry egg powder production, wine production, gluconic acid production, etc. Its electrochemical activity makes it an important component in glucose sensors, especially in diabetics, as is also the case with PQQ dependent sugar dehydrogenase, and potentially in fuel cell applications. Glucose oxidase is capable of oxidising monosaccharides, nitroalkanes and hydroxyl compounds [Wilson, 1992]. Using the reaction rate of glucose as reference (100 %), only 2-deoxy-D-glucose (20-30 %), 4-O-methyl-D-glucose (15 %) and 6-deoxy-D-glucose (10 %) are oxidized by glucose oxidase from A. niger at a significant rate [Pazur, 1964; Leskovac, 2005]. The activities of glucose oxidase against other substrates are typically poor, with reaction rates lower than 2 % of glucose's [Keilin, 1948; Pazur, 1964; Leskovac, 2005].

In a preferred embodiment, the enzyme capable of catalyzing oxidation or dehydrogenation is a dehydrogenase. Particularly, the enzyme is capable of catalyzing sugar dehydrogenation, more particularly aldose dehydrogenation. Preferrably the enzyme is sugar dehydrogenase (EC 1.1). In a specific embodiment, the enzyme is an aldose dehydrogenase or a glucose dehydrogenase. The terms of the current art describing such enzymes may be different to the one provided by this invention, however it will be understood herein that substrate specificity and capability of the enzyme to catalyse the oxidation/dehydrogenation of compound (II) or other compounds contemplated herein are independent of terminology found in the current art. One example which can be found is the terminology for an enzyme found in E. coli (Ylil, Adh, Asd) which is termed "soluble glucose dehydrogenase" by some authors, "aldose sugar dehydrogenase" or "soluble aldose dehydrogenase" by others.

The natural substrate for the sugar dehydrogenases (aldose dehydrogenases) are various sugars that get oxidized. The broad range of sugars that aldose dehydrogenase can act upon encompasses pentoses, hexoses, disaccharides and trisaccharides. Preferably, the enzyme capable of catalyzing oxidation or dehydrogenation is specific for oxidation at position C1. In the case of the aldose 1-dehydrogenase the enzyme oxidizes the aldehyde or cyclic hemiacetal to lactone.

In agreement to the above sugar oxidoreductases are divided into classes, according to electron acceptors (in some cases these are the cofactors these enzymes use in order to become functional, i.e. FAD, NAD(P)⁺ or PQQ). In terms of substrate specificity, which may strongly vary between the subclasses of sugar oxidoreductases, the use of PQQ dependent dehydrogenases (EC 1.1.5) are preferred according to the present invention.

FAD- (flavoprotein dehydrogenases) and PQQ-dependent sugar dehydrogenases (quinoprotein dehydrogenases), EC 1.1.5, use flavin adenine dinucleotide (FAD) or pyrroloquinoline quinine (PQQ) cofactors respectively, and are located on the outer surface of the cytoplasmic membrane of bacteria, facing the periplasmic space with their active sites. These are often termed membrane sugar dehydrogenases. Alternatively, especially in the PQQ-dependent sugar dehydrogenases group, many enzymes are found in soluble form, located in the periplasmic space. There is no limitation according to this invention in nature of the used sugar dehydrogenase in regard to solubility, however it may be preferred, relating to cloning, expression procedures and molecular tools available, that soluble periplasmic sugar dehydrogenases are selected, i.e. water-soluble ones. Construction of expression strain for efficient periplasmic expression of such enzyme is technically easier and thus preferable. In the course of the present invention, examples are provided, e.g. using naturally occurring membrane bound sugar dehydrogenases, for conversion of compound (II) to compound (I) in industrially useful yields.

### Respiratory Chain; Electron Acceptors

The electrons generated by the oxidation process are transferred from substrates via the enzyme cofactor (electron carrier) to the terminal ubiquinol oxidase with ubiquinone as a mediator in the respiratory chain of host organisms. The final acceptor of electrons is oxygen which is reduced to water by the respiratory chain oxidoreductases.

A respiratory chain is a series of oxidoreductive enzymes, having ability to transfer electrons from a reduced molecule in a cascade of finely tuned stepwise reactions, which are concluded by reduction of oxygen. Each step uses a difference in redox potential for useful work, e.g. transfer of protons across the cytoplasmic membrane, reduction of other molecules etc. Electron carriers have a major role in the respiratory chain as well as in overall cell's oxidoreductive processes.

Electrons can enter the respiratory chain at various levels. At the level of a NADH dehydrogenase which oxidizes NADH/NADPH (obtained by various oxidoreductive processes in the cell) with transfer of electrons to ubiquinone pool and release of protons to extracellular space. Alternatively oxidoreductases can transfer electrons directly to ubiquinon via enzyme bound cofactors (FAD,PQQ). The ubiquinoles are furher oxydised by terminal oxidoreductases such as Cytochomes, Nirate reductsases etc., whereby the electrons are coupled with intracellular protons to reduce oxygen (forming water) and ubiquinole bound protons are released into extracellular space. Any system which is capable of translocation of protons exploiting redox potential is ofted known as a proton pump. A cross membrane proton potential is thereby established and is the driving force for function of ATP synthases. These levels have successively more positive redox potentials, i.e. successively decreased potential differences relative to the terminal electron acceptor. Individual bacteria often simultaneously use multiple electron transport chains. Bacteria can use a number of different electron donors, a number of different dehydrogenases, different oxidases and reductases, and different electron acceptors. E.g., *E*. *coli* (when growing aerobically using glucose as an energy source) uses two different NADH dehydrogenases and two different quinol oxidases, for a total of four different electron transport chains operating simultaneously.

It is therefore clear that an oxidoreductase (for example sugar dehydrogenase) can only be functional when a electron acceptor is provided. In case of natural systems this is provided by the respiratory chain, alternatively artificial electron acceptors with appropriate redox potential compared to substrate/enzyme/cofactor cascade can be used. In the latter option the disadvantage is that the artificial electron acceptor has to be provided in rather large quantities, in other words normally in equimolar amount to the substrate being oxidized.

In this aspect, the acceptor of electrons generated by the enzyme capable of catalyzing oxidation or dehydrogenation may be provided in the reaction mixture in order to promote electron flow and the oxidation or dehydrogenation of compound (II). The acceptor may be selected from but is not limited to: dichlorophenolindophenol (DCPIP), phenazine methosulfate (PMS), potassium ferricyanide (PF), potassium ferrioxalate, p-benzoquinone, phenyl-p-benzoquinone, duroquinone, silicomolybdate, vitamin K3, diaminodurene (DAD), *N,N,N',N*-tetramethyl-*p*-phenylenediamine (TMDP). Electron acceptor may also be oxygen. A person skilled in art will recognize and can e.g. use compounds listed as Hill reagents, dyes that act as artificial electron acceptors, changing colors when reduced, and find many additional candidate acceptors from literature.

### PQQ Dehydrogenases

After screening PQQ dependant aldose dehydrogenases we surprisingly found that all tested enzymes (e.g. Ylil aldose dehydrogenase from *E*. *coli,* GDH from *Acinetobacter calcoaceticus* (soluble or membrane-bound form), GDH from *Gluconobacter oxidans,* GDH from Kluyvera intermedium) could oxidize compound (II) to compound (I) - (R1 = H, R2 = O-CO-CH₃) in presence of an electron acceptor regardless of the acceptor being synthetic molecule or microorganism's respiratory chain. In fact all organisms tested, which contain PQQ dependent oxidoreductases, were found to successfully oxidize ((2S,4R)-4,6-dihydroxytetrahydro-2H-pyran-2-yl)methyl acetate to ((2S,4R)-4-hydroxy-6-oxotetrahydro-2H-pyran-2-yl)methyl acetate in our experiments. Several were tested successfully also for other compound (II) molecules which are exemplified below.

In this sense, the preferred aspect of this invention deals with PQQ dependent dehydrogenases (quinoproteins, EC 1.1.5), more specifically PQQ dependant sugar 1-dehydrogenases (EC 1.1.5.2).

For example Ylil is aldose sugar dehydrogenase from *E. coli,* which requires PQQ for its activity [Southall, 2006]. While *E. coli* lacks the ability to synthesize PQQ itself [Hommes, 1984; Matsushita, 1997], it shows positive chemotaxis effect towards PQQ, found in environment [de Jonge, 1998], and can use an externally supplied cofactor [Southall, 2006]. Ylil aldose sugar dehydrogenase is a soluble, periplasmic protein, containing N-terminal signal sequence necessary for translocation into the periplasm through the cytoplasmic membrane. Ylil aldose sugar dehydrogenase (Asd) fold contains six four stranded antiparallel β-sheets with PQQ-binding site lying on the surface of the protein in a shallow, solvent exposed cleft. Ylil protein is a monomer, each binding two calcium ions, one of them lying in the PQQ binding pocket, and the other compressed between two of the six strands that make up the propeller fold [Southall, 2006].

It had been shown [Southall, 2006] that D-glucose, D-galactose, D-fructose, D-arabinose, D-fucose, D-mannose, D-lyxose, D-xylose, D-ribose, xylitol, *myo*-inositol, L-sorbose, mannitol, 2-deoxy-D-glucose, glucosamine, N-acetylglucosamine, glucose 1-phosphate, glucose 6-phosphate, maltose, α-lactose, D-sucrose, D-cellobiose, melibiose and maltotriose are accepted natural substrates of Ylil aldose 1-dehydrogenase. Besides Ylil aldose sugar dehydrogenase, *E. coli* contains also membrane-bound glucose dehydrogenase (mGDH), which is also a quinoprotein involved within the respiratory chain in the periplasmic oxidation of alcohols and sugars [Yamada, 2003].

Another example of PQQ dependant sugar dehydrogenase is *Acinetobacter calcoaceticus* GDH. At least two distinct quinoprotein glucose dehydrogenase from *Acinetobacter calcoaceticus* are known: as usual membrane-bound form (mGDH) and as soluble form (sGDH), which contains a 24-amino-acid N-terminal signal sequence needed for translocation through the cytoplasmic membrane into the periplasm. Both forms are different in all characteristics, e.g. substrate specificity, molecular size, kinetics, optimum pH, immunoreactivity.

The substrate specificity of sGdh is different from that of mGdh. sGdh oxidizes preferably D-glucose, maltose and lactose and less successfully D-fucose, D-xylose, D-galactose, while mGdh is less reactive with disaccharides; it oxidises preferably D-glucose, D-fucose, D-xylose, D-galactose, D-ribose, yet less successfully maltose, and lactose [Adachi, 2007].

The two possible reaction mechanisms for s-GDH are: (A) The addition-elimination mechanism comprises general base-catalyzed proton abstraction followed by covalent addition of the substrate and subsequent elimination of the product. (B) Mechanism comprising general base catalyzed proton abstraction in concert with direct hydride transfer from substrate to PQQ, and tautomerization to PQQH₂ [Oubrie, 1999]. A similar mechanism is assumed to be the case for *E.coli* Ylil aldose dehydrogenase enzyme.

As the *E*. *coli* Ylil, the sGDH also requires calcium for dimerization and function [Olsthoorn, 1997]. The present structures confirm the presence of three calcium binding sites per monomer [Oubrie, 1999].

Current industrial application of PQQ dependent sugar dehydrogenases includes D-gluconate production (*Gluconobacter oxydans*) in classic fermentation processes as well as production of various natural sugars. *Gluconobacter oxydans* organism is well known for its important ability to incompletely oxidize natural carbon substrates such as D-sorbitol (producing L-sorbose for vitamin C synthesis), glycerol (producing dihydroxyacetone), D-fructose, and D-glucose (producing gluconic acid, 5-keto-, 2-keto- and 2,5-diketogluconic acid) for the use in biotechnological applications [Gupta, 2001].

As derived from the above description, PQQ dependent dehydrogenases are found in *Acinetobacter calcoaceticus,* an industrial microorganism used in vinegar production.

A more recent attention given to PQQ dependent sugar dehydrogenases is directed to development of different amperometric biosensors, e.g. for measuring glucose concentration in blood [D'Costa, 1986; Igarashi, 2004; Heller, 2008], for detection of heavy metals [Lapenaite, 2003], for detection of formaldehyde in air [Acmann, 2008], for detection of phenolic compounds in flow injection analysis [Rose, 2001], as a ultrasensitive bienzyme sensor for adrenaline [Szeponik, 1997], for determination of xylose concentration [Smolander, 1992], etc. PQQ dependent sugar dehydrogenases may have found its use also in nanotechnology as biofuel cells [Gao, 2010]. Soluble PQQ dependent glucose dehydrogenases have become the major group of enzymes used in biosensor systems for self monitoring of blood glucose, because these enzymes, unlike glucose oxidase, are independent of oxygen presence [Heller, 2008].

In the current art no proceess using PQQ dependant aldose dehydrogenases for production of unnatural compounds, especially in connection to active pharmaceutical compounds or their intermediates, exists or has been contemplated, but as disclosed and provided by the present invention such enzymes have turned out to be feasible and accomplishable to provide an effective and easy synthesis principle for useful unnatural compounds. Therefore the present invention opens a new field for use of these enzymes in further oxidoreductive reactions, used for purpose of synthesis of unnatural compounds especially belonging to classes of synthetic APIs and their intermediate compounds.

### Selection of Enzymes Particularly Useful for the Present Invention

With the information and experimental guidance provided herein, the skilled person will become aware and derive how to select the enzyme capable of catalyzing oxidation or dehydrogenation in order to convert e.g. the compound of formula (II) to the compound of formula (I) based on its substrate specificity or promiscuity, operational pH, temperature and ionic strength window, a need of additional ions or cofactors, or the like. Substrates and reaction conditions are normally chosen to give the optimal activity of the enzyme. However, the substrates and conditions to provide the least inhibitory effect on the cell that hosts the enzyme, or deteriorate stability of the product, can be leveraged against the substrates and conditions by which the optimal activity is reached. In principle, the substrates allowing an enhanced, preferably the best activity of the enzyme are preferred, or vica versa the enzyme having an enhanced, preferably the best specificity towards a desired compound substrate are preferred. It will be immediately apparent to the skilled person that reaction conditions include in one aspect that the temperature, pH, solvent composition, agitation and length of the reaction allow accumulation of the desired product. In addition to satisfy the enzyme activity, the skilled person will know with the disclosure provided herein to adapt the conditions in terms of applying proper pH, temperature and reaction time to prevent the product, e.g. lactone or ester, to deteriorate. If needed, specific cofactors, co-substrates and/or salts can be added to the enzyme in order to either allow or improve its activity. Cofactors are salts or chemical compounds. Often, said species are already included in the solvent mixture, especially if the enzyme is comprised within living whole cell, inactivated whole cell, homogenized whole cell, or cell free extract. Nevertheless, the cofactors, co-substrates and/or salts can be further added to the enzyme, solvent or reaction mixture. Depending on the enzyme, cupric, ferric, nickel, selenium, zinc, magnesium, calcium, molybdenum, or manganese ions, or nicotinamid adenine dinucleotide (NAD), nicotinamid adenine dinucleotide phosphate (NADP+), lipoamide, ascorbic acid, flavin mononucleotide, flavin adenine dinucleotide (FAD), coenzyme Q, coenzyme F420, pyrroloquinoline quinine, coenzyme B, glutathione, heme, tetrahydrobiopterin, or the like can be added to the enzyme, to the solvent or medium or to the reaction mixture comprising the enzyme. For example, with aldose-1-dehydrogenase, or preferably Ylil, calcium ions and pyrroloquinoline quinine or similar electron acceptor is added to the reaction mixture, enzyme or solvent or medium. Specifically, suitable conditions are exemplified in the examples hereinafter.

A dehydrogenase for use in the present invention may be particularly chosen among any enzyme that has oxidative activity towards above substrate (II). In general any sugar 1-dehydrogenase known in the art can be used regardless of their sequence identity to the enzymes listed below, notably dehydrogenases. As noted, it is beneficial to choose an enzyme capable of catalyzing oxidation or dehydrogenation specifically at position C1. In the case of the aldose 1-dehydrogenase the enzyme oxidizes the aldehyde or cyclic hemiacetal to lactone.

Special variants of the enzymes, like for example enzymes found in the termoresistant microorganism strains, are also contemplated within the present invention. The same applies to a modified or improved versions of the naturally occurring enzymes, whose amino acid sequence or structure has been changed to attain better substrate specificity, higher activity, activity over broader temperature or pH range, resistance to the presence of organic solvent or high ionic strength of the solvent, or the like.

We have surprisingly found that two distinct sugar dehydrogenases originating from taxonomically diverse micoroorganisms and having only 21,8% aminoacid sequence identity, prerformed equally successful in our experiments. Specifically, comparison was performed between SEQ ID NO. 02, representing amino acid sequence of GDH 01 aldose sugar dehydrogenase Ylil from *E. coli,* and SEQ ID NO. 06, representing amino acid sequence of GDH 02 glucose dehydrogenase GdhB from *A. calcoaceticus.* Sequence comparison algorithm was made with default settings in AlignX module, component of Vector NTI Advance 11.0 software (Invitrogen), using clustal W algoritm at default settings.

Owing to this surprising finding it is now reasonable to expect that proteins capable of converting compound (II) to compound (I) or similar reactions may be significantly diverse in ther aminoacid sequence. The yields of the reaction however may depend on each sugar dehydrogenase enzyme's substrate specificity.

Examples of suitable dehydrogenase enzyme include, but are not limited to enzymes in the sequence list, which are identified by their nucleotide sequences or respective codon optimized nucleotide sequences or amino acid sequences set forth in sequence listings.
GDH 01 is a dehydrogenase encoding gene comprised within nucleotide sequence of SEQ ID NO. 01 or an amino acid sequence of SEQ ID NO. 02.
GDH 02 is a dehydrogenase having a nucleotide sequence of SEQ ID NO. 03 or an amino acid sequence of SEQ ID NO. 04.
GDH 03 is a dehydrogenase having a nucleotide sequence of SEQ ID NO. 05 or an amino acid sequence of SEQ ID NO. 06.
GDH 04 is a dehydrogenase having a nucleotide sequence of SEQ ID NO. 07 or an amino acid sequence of SEQ ID NO. 08.
GDH 05 is a dehydrogenase having a nucleotide sequence of SEQ ID NO. 09 or an amino acid sequence of SEQ ID NO. 10.
GDH 06 is a dehydrogenase having a nucleotide sequence of SEQ ID NO. 23 or an amino acid sequence of SEQ ID NO. 24.
GDH 07 is a dehydrogenase having a nucleotide sequence of SEQ ID NO. 25 or an amino acid sequence of SEQ ID NO. 26.
GDH 08 is a dehydrogenase having a nucleotide sequence of SEQ ID NO. 27 or an amino acid sequence of SEQ ID NO. 28.
GDH 09 is a dehydrogenase having a nucleotide sequence of SEQ ID NO. 29 or an amino acid sequence of SEQ ID NO. 30.
GDH 10 is a dehydrogenase having a nucleotide sequence of SEQ ID NO. 31 or an amino acid sequence of SEQ ID NO. 32.
GDH 11 is a dehydrogenase having a nucleotide sequence of SEQ ID NO. 33 or an amino acid sequence of SEQ ID NO. 34.
GDH 12 is a dehydrogenase having a nucleotide sequence of SEQ ID NO. 35 or an amino acid sequence of SEQ ID NO. 36.
GDH 13 is a dehydrogenase having a nucleotide sequence of SEQ ID NO. 37 or an amino acid sequence of SEQ ID NO. 38.
GDH 14 is a dehydrogenase having a nucleotide sequence of SEQ ID NO. 39 or an amino acid sequence of SEQ ID NO. 40.
GDH 15 is a dehydrogenase having a nucleotide sequence of SEQ ID NO. 41 or an amino acid sequence of SEQ ID NO. 42.
GDH 16 is a dehydrogenase having a nucleotide sequence of SEQ ID NO. 43 or an amino acid sequence of SEQ ID NO. 44.
GDH 17 is a dehydrogenase having a nucleotide sequence of SEQ ID NO. 45 or an amino acid sequence of SEQ ID NO. 46.
GDH 18 is a dehydrogenase having a nucleotide sequence of SEQ ID NO. 47 or an amino acid sequence of SEQ ID NO. 48.
GDH 19 is a dehydrogenase having a nucleotide sequence of SEQ ID NO. 49 or an amino acid sequence of SEQ ID NO. 50.
GDH 20 is a dehydrogenase having a nucleotide sequence of SEQ ID NO. 51 or an amino acid sequence of SEQ ID NO. 52.
GDH 21 is a dehydrogenase having a nucleotide sequence of SEQ ID NO. 53 or an amino acid sequence of SEQ ID NO. 54.
GDH 22 is a dehydrogenase having a nucleotide sequence of SEQ ID NO. 55 or an amino acid sequence of SEQ ID NO. 56.
GDH 23 is a dehydrogenase having a nucleotide sequence of SEQ ID NO. 57 or an amino acid sequence of SEQ ID NO. 58.
GDH 24 is a dehydrogenase having a nucleotide sequence of SEQ ID NO. 59 or an amino acid sequence of SEQ ID NO. 60.
GDH 25 is a dehydrogenase having a nucleotide sequence of SEQ ID NO. 61 or an amino acid sequence of SEQ ID NO. 62.

Therefore a sugar dehydrogenase for use in the present invention may be any compound that has sugar 1- dehydrogenase activity toward compound (II). In one embodiment of the invention the sugar 1. dehydrogenase is a PQQ dependat sugar 1- dehydrogenase. Examples of sutable PQQ dependant sugar 1-dehydrogenase include but are not limited to: GDH 01, GDH 02, GDH 03, GDH 04, GDH 05, GDH 06, GDH 07, GDH 08, GDH 09, GDH 10, GDH 11, GDH 12, GDH 13, GDH 14, GDH 15, GDH 16, GDH 17, GDH 18, GDH 19, GDH 20, GDH 21, GDH 22, GDH 23, GDH 24 and GDH 25, wherein each enzyme is identified by it's corresponding nucleotide sequence or respective codon optimized nucleotide sequence or aminoacid sequence as set forth in sequence listing above.

The present invention provides sugar dehydrogenases having an amino acid sequence identitiy of at least 50 % thereof; preferably at least 70 % thereof, to any of dehydrogenases selected from GDH 01, GDH 02, GDH 03, GDH 04, GDH 05, GDH 06, GDH 07, GDH 08, GDH 09, GDH 10, GDH 11, GDH 12, GDH 13, GDH 14, GDH 15, GDH 16, GDH 17, GDH 18, GDH 19, GDH 20, GDH 21, GDH 22, GDH 23, GDH 24 and GDH 25. The amino acid sequence identities are determined by analysis with sequence comparison algorithm or by visual inspection. In one aspect, the sequence comparison IS made with default settings in AlignX module, component of Vector NTI Advance 11.0 software (Invitrogen), using clustal W algoritm at default settings.

A preferable sugar 1-dehydrogenase provided by this invention may be the sugar dehydrogenase originating from *Escherichia coli* identified as GDH01 in the above sequence listing and having corresponding nucleotide sequence SEQ ID NO. 01 and an amino acid sequence of SEQ ID NO. 02.
Equaly preferable sugar 1-dehydrogenase provided by this invention may be the sugar dehydrogenase originating from *Escherichia coli* identified as GDH02 in the above sequence listing and having corresponding nucleotide sequence SEQ ID NO. 03 and an amino acid sequence of SEQ ID NO. 04.

Another preferable sugar 1-dehydrogenase provided by this invention may be selected from the sugar dehydrogenase originating from *Acinetobacter calcoaceticus coli* identified as GDH03 in the above sequence listing and having corresponding nucleotide sequence SEQ ID NO. 05 and an amino acid sequence of SEQ ID NO. 06.

Yet another preferable sugar 1-dehydrogenase provided by this invention may be selected from the modified sugar dehydrogenase originating from *Acinetobacter calcoaceticus coli* identified as GDH04 in the above sequence listing and having corresponding nucleotide sequence SEQ ID NO. 07 and an amino acid sequence of SEQ ID NO. 08.

The most preferable sugar 1-dehydrogenase provided by this invention may be the sugar dehydrogenase from originating from *Escherichia coli* identified as GDH01 in the above sequence listing and having corresponding nucleotide sequence SEQ ID NO. 01 and an amino acid sequence of SEQ ID NO. 02. The said sugar 1-dehydrogenase is also described in the art and within this invention as PQQ dependant sugar dehydrogenase, PQQ dependant glucose dehydrogenase, PQQ dependant aldose dehydrogenase, aldose dehydrogenase, aldose dehydrogenase quinoprotein or glucose dehydrogenase quinoprotein. This particular enzyme is encoded by gene *ylil* naturally occurring in *E*. *coli* and encodes a protein termed Ylil, Asd or Adh.
The present invention illustratively makes use of sugar dehydrogenases having an amino acid sequence identitiy of at least 21,8% thereof; 50 % thereof; preferably at least 70 % thereof, to the aminoacid sequence SEQ ID NO. 02.

Within the present invention it is possible screen for enzymes/organisms capable of oxidizing lactols of formula (II) or, depending on the desired synthetic API or its precursor compound as product, another non-natural substrate. In one aspect of this invention a person skilled in art will find additional candidate enzymes from literature, which could be applicable for the desired type of enzymatic conversion.

Oxidation/dehydrogenization activity towards compound (II) may be screened among different microorganisms and/or enzymes. The term "analysed material" as used herein refers to any microorganism and/or enzyme that can be used in screening method to screen for and identify microorganism and/or enzyme able to convert compound (II) to compound (I) as the living whole cell catalyst, resting whole cell catalyst, cell free lysate, partially purified or purified enzyme, immobilized enzyme or any other form of catalyst as provided by this invention of any microorganism regardless of it being native or genetically modified microorganism. For practical purposes it will be understood hereinafter that a term "analysed material" includes all preparations of candidate catalyst as described above. Several methods are provided in this disclosure that allow screening for and identification of oxidation/dehydrogenation activity towards compound (II) and thus method for screening and identifying organisms and/or enzymes useful to carry out the present invention.

To successful perform said screening methods, "analysed material" may be obtained having regard to its cultivation properties. Cultivation may be performed to obtain biomass of "analysed material" in growth medium which satisfies the nutrient needs. Cultivation may be performed in liquid medium or on solid medium. Growth medium and conditions of cultivating may be chosen from but are not limited to Difco & BBL Manual, 2010 and to other protocols well known to person skilled in the art. Cultivated microorganisms may be prepared in different forms of catalyst as provided by this invention. In particular "analysed material" is brought in contact with compound (II) in such conditions that allow forming and accumulation of compound (I). These conditions include in one aspect that the "analysed material" is provided at sufficient load to be able to perform the oxidationldehydrogenization, in another aspect that the substrate and electron acceptors are present in the reaction in an amount that displays minimal inhibition of the activity of the catalyst, in another aspect that the temperature, pH, solvent composition, agitation and length of reaction allow accumulation of desired product, in another aspect that said conditions do not have detrimental effect on product stability. Specifically such conditions may be defined as indicated by, or as modified or varied from, values or conditions disclosed in examples. "Analysed material" may be able to intrinsically provide all cofactors needed for activity towards compound (I) (naming PQQ), or "analysed material" possess the capability of converting compound (II) to compound (I) when PQQ is provided externally as described in this invention. In all screening methods provided herein to PQQ may preferebly be added concentrations described and exemplified. It will become apparent to a person skilled in the art that quantification of PQQ presence can be determined by the above method and as described elsewhere in the present specification. In this case the material used for the method has to be depleted of PQQ or contain no PQQ already by it's nature.

One such method for screening of and identifying candidate catalysts is to bring into contact the "analysed material" with a compound (II). Converting of compound (II) to compound (I) should be performed at optimal reaction conditions as described above. Detection of substrates converting to product in presence of "analysed material" can be achieved by any of the well known chromatographic methods known in the art. The non-limiting examples include liquid HPLC, GC, TLC analysis etc. An exemplified but not limiting method for monitoring compound (I) and corresponding compound (II) is gas chromatography analysis (chromatographic column: DB-1 100 % dimethylpolysiloxane; temperature program: initial temperature: 50 °C, initial time: 5 min, temperature rate: 10 °C/min, final temperature: 215 °C, final time: 10 min; injector: split/spliteess injector; carrier gas: helium, initial flow: 10 mL/min; detector: flame ionization detector (FID), detector temperature: 230 °C). The prerequisite for carrying out such method is a presence of electron acceptor in the reaction mixture. For "analysed material" where natural electron acceptor is present (such as respiratory chain) no additional components are needed to be able to observe formation of compound (I) from compound (II) by using said chromatographic methods. In case the electron acceptor capable to relieve PQQ of it's electron pair is not available (such in cell free lysate), artificial electron acceptor such as DCPIP is needed. However the described method being analytical procedure and only small quantities of artificial electron acceptor being needed, the preferred way to carry out screening method with any kind of "analysed material" is in presence of artificial electron acceptor. Illustrative and preferred conditions for carrying out the above method are defined by values and conditions disclosed in examples.

Another screening method provided by this invention is a method performed in presence of alternative artificial electron acceptors with appropriate redox potential compared to a substrate/enzyme/cofactor cascade that can be used. In this sense, the present invention provides a screening method using artificial electron acceptor which changes its optically measurable property or properties (such as color, absorbance spectra, etc.) when reduced. Such artificial electron may be provided in the reaction mixture (a dye-linked system) in order to promote electron flow, hence being indicative of the oxidation or dehydrogenation of compound (II). The acceptor/indicator may be selected from but is not limited to: 2,6-dichlorophenol indophenol (DCPIP), phenazine methosulfate (PMS), potassium ferricyanide (PF), potassium ferrioxalate, p-benzoquinone, phenyl-*p*-benzoquinone, duroquinone, silicomolybdate, vitamin K3, diaminodurene (DAD), *N,N,N',N'*-tetramethyl-*p-*phenylenediamine (TMDP). A person skilled in the art will recognize compounds listed as Hill reagents, dyes that act as artificial electron acceptors, changing colors when reduced, and will find many additional candidate acceptors/indicators from literature. Preferably, 2,6-dichlorophenol indophenol (DCPIP) combined with phenazine methosulfate (PMS) may be used. An exemplified screening method contains following components in a reaction mixture: DCPIP combined with PMS as artificial electron acceptor, "analysed material" and compound (II). Oxidation/dehydrogenation activity of "analysed material" towards compound (II) is followed spectrophotometrically as reduction of absorbance of DCPIP which when oxidized is blue, turning color-less when reduced. When "analysed material" is capable of oxidation/dehydrogenization activity towards compound (II), electrons are transferred to artificial electron acceptor, which becomes reduced and thus reaction mixture turns color from blue to color-less.

One example of carrying out said method is to follow the following procedure: DCPIP and PMS are used in concentrations from about 0.01 mM to about 10mM for both said artificial electron acceptors.,in particular from about 0.05mM to about 5mM DCPIP combined with 0.01 mM to about 2mM DCPIP. Preferably the amount of DCPIP in a screening method is provided in concentration from 0.1mM to about 1mM combined with PMS in concentration from 0.05mM to about 0.5 mM. Most preferably the DCPIP combined with PMS is provided in the amount which allows observation of reduction of absorbance in timeline that can be spectrophotometrically followed. The compound (II) may be dissolved in appropriate aqueous solution and used in a screening method in concentrations from about 0.5 mM to about 1 M preferably from about 10 mM to about 500 mM, most preferably 20 mM to 200 mM. Compound (II) may be dissolved in distilled water or in suitable buffered solution. Suitable buffers for adjusting pH value are made with acids, bases, salts or mixtures thereof in particular phosphoric acid and sodium hydroxide may be used. The aqueous suspension, in which the screening method is performed, may be buffered to pH 5.5 to 9.0, preferably to 6.0 to 8.5, more preferably 7.0. to 8.0. "Analysed material" is added to reaction mixture in the said aqueous suspension (particularly in a concentration range from about 0.05 g/L to about 50 g/L), optionally in buffered solution (in particularly in phosphate buffer pH 6.0 to 8.5). Screening and identifying of catalysts capable of converting compound (II) to compound (I) can be observed spectrophotometrically following absorbance reduction in time line, may be at wavelength between 380 nm and 750 nm, perferably at wavelength between 450 nm and 650 nm, more preferably between 550 nm and 650 nm.

This invention also provides an aldose dehydrogenase activity unit. The aldose dehydrogenase activity unit is defined as absolute value of reduction in absorbance unit per minute per wet weight of cultured microorganisms used for preparation of any "analysed material" (abs[mAU min⁻¹ mg⁻¹]). For comparative studies cell density of tested microorganisms may be quantified as wet weight in mg per mL of sample, protein concentrations and/or other indirect or direct methods for quantification well known to person skilled in the art.

### PQQ

Pyrroloquinoline quinine (4,5-dihydro-4,5-dioxo-1H-pyyrolo-[2,3-f]quinoline-2,7,9-tricarboxylic acid: PQQ) is a molecule needed for functioning when using quinoproteins. PQQ, a redox cofactor, which is water soluble and heat-stable, is considered as the third type of coenzyme, after nicotinamide and flavin in biological oxidoreductions and was discovered by Hauge, 1964. To that time unknown redox cofactor was also found by Anthony and Zatman in alcohol dehydrogenase and was named by them as methoxantin [Anthony, 1967]. Later, PQQ has been reported to occur in dehydrogenase, oxidases, oxygenases, hydratases, and decarboxylases. The role of these quinoproteins is to catalyze the primary oxidation step of non-phosporylated substrates, such as alcohols, aldehydes, or aldoses. PQQ has been found in both prokaryotic (such as *Klebsiella pneumoniae, Acinetobacter calcoaceticus, Methylobacterium extorquens, Kluyvera intermedia, Gluconobacter oxydans, Pseudomonas aeruginosa, Erwinia amylovora, Rahnella aquatilis, Deinococcus radiodurans)* and eukaryotic organisms (such as *Polyporus versicolor*, *Rhus vernicifera*) [Goodwin, 1998; Hoelscher, 2006; Yang, 2010]

A generally accepted structure of PQQ is: 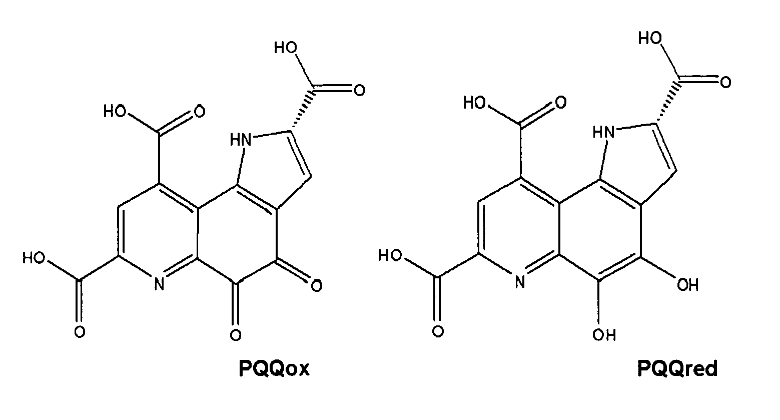 wherein the PQQox is the oxidized form of the cofactor and PQQred is the reduced from of the cofactor.

The number of genes involved in biosynthesis of PQQ varies between species, but in general it is known that for biosynthesis at least five or six genes are needed, usually clustered in the *pqq*ABCDE or *pqqABCDEF* operon. The number and organization of the genes is variable as it can be seen in following examples. In *Klebsiella pneumoniae,* the PQQ biosynthetic genes are clustered in the *pqqABCDEF* operon, while in *Pseudomonas aeruginosa* the *pqqF* is separated from the *pqqABCDE* operon. In *Acinetobacter calcoaceticus,* there is a *pqqABCDE* but no *pqqF* gene is known. A facultative methylotroph *Methylobacterium extorquens* AM1 contains a *pqqABC*/*DE* operon in which the *pqqC* and *pqqD* genes are fused, while the *pqqFG* genes form an operon with three others genes.

Although much is known about the enzymes that use PQQ as a cofactor, relatively little is known about its biosynthesis. However, backbone of PQQ is constructed from glutamate and tyrosine. Most probably these amino acids are encoded in the precursor peptide PqqA. The length of the small peptide varies between different organisms (from 23 amino acids in K. *pneumoniae* to 39 in *P. fluorescens,* respectively) and in all variants in the middle of the PqqA peptide motif Glu-X-X-X-Tyr is conserved. The PqqB protein might be involved in its transportation into the periplasm and thus is not directly required for PQQ biosynthesis. Residues of PqqC protein are highly conserved within PqqC proteins, which are responsible for catalyzing the final step in PQQ formation, from different bacteria. Although the alignment of protein sequences of PqqD proteins from different organisms shows strictly conserved residues, the function of PqqD is not fully resolved. In *Klebisella pneumoniae* it was shown that PqqE recognizes the PqqA, which links the C9 and C9a, afterwards it is accepted by PqqF which cuts out the linked amino acids. In the said organism it was shown that the next reaction (Schiff base) is spontaneous, following dioxygenation. The last cyclization and oxidation steps are catalysed by PqqC [Puehrunger, 2008].

When a comparison of PqqF and PqqG proteins derived from *Klebsiella pseudomonas* within a protein database was performed, it was purported that said proteins share similarity with a family divalent cation-containing endopeptidases that cleave small peptides [Meulenberg, 1992]. While the PqqF and PqqG proteins of *Methylobacterium extorquens* show some similarity to the two subunits of mitochondrial processing peptidases [Springer, 1996], the PqqF of *Klebsiella pneumoniae* is most closely related to the *Escherichia coli* peptidase pitrilysin encoded by *tldD* gene [Meulenberg, 1992]. It has been proposed and experimentally shown that PQQ gene clusters comprising only *pqqABCDE genes* and lacking *pqqF* may be used to provide compete PQQ biosynthetic maschinery in *E*. *coli*. (Kim C.H. et al., 2003, Yang X.-P. et al. 2010). The pitrilysin protease (encoded by *tldD* gene) is apparently complementig for the activity of pqqF gene found in some microorganisms.

While *E*. *coli* lacks the ability to synthesize PQQ itself [Hommes, 1984; Matsushita, 1997], it shows positive chemotaxis effect towards PQQ, found in environment [de Jonge, 1998], and can use an externally supplied cofactor [Southall, 2006]. Thus PQQ biosynthesis genes could be recombinantly expressed in *E*. *coli*, what is one of the aspects described in this invention.

In relation to the above, in general, there are at least three ways of providing PQQ to PQQ-dependent dehydrogenases *in situ:*
First, the PQQ can be added to the living or resting cells containing aldose dehydrogenase enzyme or to the cell free lysates or purified aldose dehydrogenase enzyme. The reconstitution of holo-enzyme form to the active apo-enzyme is almost instantaneous, which was shown in one aspect of our invention. In terms of benefit for industrial applications, it is however preferred that there is no need for external supply of expensive PQQ, by in situ generation of PQQ as described in more detail below. Further, there are yet some other possibilities of addition of PQQ which does not necessarily need to be purified in for of dietary complements, media components such as yeast extract etc. The fact that quinoproteins have a very high affinity towards PQQ [de Jonge, 1998] allows that equimolar quantities to the quinoproteins are used. In praxis this means concentrations at the nano molar to micro molar level. It will become apparent to a person skilled in the art that optimization of the amount of PQQ needed for optimal activity of the aldose dehydrogenase enzyme is easily performed in order to reduce the cost of the process. As an non- limiting example, increasing amounts (starting from 0.1nM) of PQQ are added to the aldose dehydrogenase catalyst and the added amount is optimal when the activity of said catalyst no more increases with additional PQQ provided.

In this sense the present invention provides a method of supplying the PQQ to the aldose dehydrogenase, more specifically to the living whole cell catalyst, resting or inactivated whole cell catalyst, cell free lysate or extract or any other form of catylst as provided by this invention in concentration from about 0.1 nM to about 5mM. In particular from about 1nM to about 100uM of PQQ can be provided. More preferably the PQQ is provided in concentration from 100nM to about 2uM. Most preferably the PQQ is provided in the minimal amount which allows maximal activity of the said catalyst. Practically this is in the range of 250nM to 1uM final concentration of PQQ. The PQQ can be obtained from any source and provided to the catalyst as solid matter or stock solution of PQQ.

Second, preferred option is, that the host organism for the production of appropriate dehydrogenase has intrinsic PQQ biosynthetic capability, in other words, contains functional genes for PQQ biosynthesis already integrated in its genetic material. Non-limiting examples are: *Klebsiella pneumoniae*, *Acinetobacter calcoaceticus*, *Methylobacterium extorquens*, *Kluyvera intermedia*, *Gluconobacter oxydans*, *Pseudomonas aeruginosa*, *Erwinia amylovora*, *Rahnella aquatilis, Deinococcus radiodurans* and others.
When using such microorganism as host for expression of homogenous or heterogeneous aldose dehydrogenases, the expressed enzymes are coupled with PQQ to form already their active form. Some of the said microorganisms have in addition to PQQ production ability, active PQQ dependant aldose dehydrogenase present, which are capable of converting compound (II) to compound (I). In several aspects of our invention this approach proved to be highly effective and successful as exemplified below.

In this aspect the present invention provides microorganisms with native ability to produce PQQ that can be used as hosts for homolohous or heterologous expression of PQQ dependant aldose dehydrogenases. Said microorganisms are preferably selected among bacteria, more preferably industrially culturable bacteria and particularly from *Klebsiella pneumoniae, Acinetobacter calcoaceticus, Methylobacterium extorquens, Kluyvera intermedia*, *Enterobacter*, *Gluconobacter oxydans*, *Pseudomonas aeruginosa*, *Erwinia amylovora*, *Rahnella aquatilis*, *Deinococcus radiodurans*. In the most prefereble imbodiment *Klebsiella pneumoniae, Acinetobacter calcoaceticus, Pseudomonas aeruginosa, Erwinia amylovora, Gluconobacter oxydans* may be used.

In similar yet different embodiment the present invention provides microorganisms with natural capability to convert compound (II) to compound (I). No genetic modifications are needed with provided organisms in order to obtain a catalyst capable of performing the desired oxidation. Therefore this invention provides microorganisms for the presently disclosed purpose and use, selected among bacterial origin, more particularly from genera: *Klebsiella Enteorobacter, Acinetobacter, Rhizobioum. Methylobacterium, Kluyvera, Gluconobacter, Pseudomonas, Erwinia, Rahnella and Deinococcus.* In a more particular sense the microorganisms may be selected from *Klebsiella pneumoniae*, *Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans*, most preferably from: *Gluconobacter oxydans*, *Acinetobacter calcoaceticus and Kluyvera intermedium.*

Described above are non-limiting examples of microorganisms with desired properties to carry out this invention. Further, methods are disclosed and provided which allow screening for and identification of such microorganisms.

The third option is especially applicable to microorganisms which do not have intrinsic capability of biosynthetis of PQQ, such as *Escherichia coli.* It is well known in the art that some microorganims such as *E. coli* and most of higher organisms have PQQ-dependent enzymes encoded in their genomes and expresses in certain conditions but lack biosynthesis of PQQ [Matshushita, 1997]. It is contemplated in the art that such microorganisms obtain the PQQ as an essential nutrient, or with other words, a vitamin. Ways to establish biosynthesis of PQQ in such organisms to be used for the present invention will be apparent to a person skilled in the art. Approaches of providing biosynthesis of PQQ to such organisms are described (see e.g. Goosen, 1988; Yoshida, 2001; Kim, 2003; Khairnar, 2003; Hoelscher, 2006; Yang, 2010). As also provided by the present invention, it can be established by cloning of PQQ biosynthesis gene cluster from microorganisms which do posses PQQ biosynthesis machinery to the plasmid vector or to the bacterial chromosome and then allowing expression of such genes in the host organisms. Non-limiting examples of microorganims suitable for this purpose include *Klebsiella pneumonia, Methylobacterium extorguens, Pseudomonas aeruginosa, Gluconobacter oxydans, Kluyvera intermedia, Erwinia amylovora* and others. A term "heterologous expression of PQQ gene cluster" will be immediately understood by a person skilled in the art, as a well established term describing the above procedures.

For use in the present invention any PQQ gene cluster may be used, providing that said gene cluster encodes functional proteins as described above with capability of biosynthesis of PQQ either alone or in concert with the host organism's enzymes.

In one embodiment of the invention the pQQ gene cluster can be obtained from any living organism producing PQQ. In a more particular embodiment of the invention, the PQQ gene cluster can be obtained from any microorganisms selected among bacterial, more particularly from genera: *Klebsiella Enteorobacter, Acinetobacter, Rhizobioum, Methylobacterium,Kluyvera, Gluconobacter, Pseudomonas, Erwinia, Rahnella and Deinococcus.* In a more particular sense the microorganisms may be selected from *Klebsiella pneumoniae, Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans,* most preferably from: *Gluconobacter oxydans, Acinetobacter calcoaceticus and Kluyvera intermedia.*

Examples of suitable PQQ gene clusters are included, but are not limited to nucleotide sequences of clusters or included genes in the sequence list, which are identified by their nucleotide sequences or amino acid sequences set forth in sequence listings. In general any of the PQQ clusters providing functional genes known in the art may be used for the reaction regardless of their sequence identity to the listed PQQ clusters, genes comprised within and proteins encoded by said genes.

PQQ 01 is a PQQ encoding gene cluster from *Gluconobacter oxydans* 621H comprised within nucleotide sequence of SEQ ID NO. 68 and allows expression of genes *pqqA, pqqB, pqqC, pqqD* and *pqqE* encoding proteins PqqA, PqqB, PqqC, PqqD and PqqE with aminoacid sequence SEQ ID NO. 12, 13, 14, 15, 16, respectively.

PQQ 02 is a PQQ encoding gene cluster from *Kluyvera intermedia* comprised within nucleotide sequence of SEQ ID NO. 69 and allows expression of genes *pqqA, pqqB, pqqC, pqqD* and *pqqE* encoding proteins PqqA, PqqB, PqqC, PqqD and PqqE with aminoacid sequence SEQ ID NO. 18, 19, 20, 21, 22, respectively.

PQQ 03 is a gene cluster *pqqABCDEF* from *Klebsiella pneumoniae* 324 having a nucleotide sequence of SEQ ID. NO 63. and allows expression of genes *pqqA, pqqB, pqqC, pqqD, pqqE* and *pqqF* encoding proteins PqqA, PqqB, PqqC, PqqD, PqqE and PqqF. The above sequence is available as part of the genome sequence with accession number CP000964 at NCBI genome database having location between 2602846 and 2599706.

PQQ 04 is a gene clusters *pqqABC*/*DE* and *pqqFG* from *Methylobacterium extorguens* AM1 having nucleotide sequences of SEQ ID. NO 64 and SEQ ID. NO 65, respectively and allows expression of genes *pqqA, pqqB, pqqC, pqqD, pqqE* and *pqqF* encoding proteins PqqA, PqqB, PqqC, PqqD, PqqE and PqqF. The above sequence is available as part of the genome sequence with accession number CP001510 at NCBI genome database having location between 1825235 and 1821763 (*pqqABC*/*DE*), 2401055 and 2403792 (*pqqEF*)*.*

PQQ 05 is a gene clusters *pqqABCDE* and *pqqF* from *Pseudomonas aeruginosa* PA7 having nucleotide sequences of SEQ ID. NO 66 and SEQ ID. NO 67, respectively and allows expression of genes *pqqA, pqqB, pqqC, pqqD, pqqE* and *pqqF* encoding proteins PqqA, PqqB, PqqC, PqqD, PqqE and PqqF. The above sequence is available as part of the genome sequence with accession number CP000744 at NCBI genome database having location between 3420385 and 3423578 (*pqqABCDE*), 3439512 and 3437221 (*pqqF).*

PQQ 06 is a gene cluster *pqqABCDEF* from *Erwinia amylovora* ATCC 49946 having a nucleotide sequence of SEQ ID. NO 70. and allows expression of genes *pqqA, pqqB, pqqC, pqqD, pqqE* and *pqqF* encoding proteins PqqA, PqqB, PqqC, PqqD, PqqE and PqqF. The above sequence is available as part of the genome sequence with accession number FN666575 at NCBI genome database having location between 597604 and 600850.

A person skiled in art would also recognize additional candidate gene clusters providing for PQQ synthesis, in publicly available databases (GenBank, Swiss-Prot/TrEMBL, RCSB PDB, BRENDA, KEGG, MetaCyc) using well established data mining tools.

The method for measuring activity of PQQ dependant aldose dehydrogenase provided by the present invention can be used, as exemplified by the invention herein, to screen for and identify organisms capable of producing PQQ regardless of their origin (native or genetically modified), and in addition allows, if desired, a semi quantitative method for estimating the quantity of produced PQQ. A PQQ dependant aldose dehydrogenase in any form, preferably expressed in E. coli (or any other microorganism unable to produce PQQ), can be used for reconstitution of active holo-enzyme. A calibration curve obtained by measuring activity of said PQQ dependant aldose dehydrogenase, supplemented with various quantities of PQQ, is compared to the activity of said PQQ dependant aldose dehydrogenase which was supplemented with analysed sample. Whithin the linear range of the method, the more PQQ is present in the analysed sample, the more activity is observed.

In a particular embodiment of this invention the PQQ gene clusters (or part thereof) are derived from *Kluyvera intermedia* or *Gluconobacter oxydans.* Particularly gene cluster from *Gluconobacter oxydans* 621H comprised within nucleotide sequence of SEQ ID NO. 68 may be used. Alternatively, a particular embodiment of this invention provides use of gene cluster from *Kluyvera intermedia* comprised within nucleotide sequence of SEQ ID NO. 69 The described gene cluster can be modified by methods known in the art, for example methods described in Sambrook and Russell, Molecular Cloning: A Laboratory Manual, 3'^" Ed., Cold Spring Harbor, NY 2001, in order to allow expression of genes encoded in said cluster in *E. coli.* Any of numerous strains of *E. coli* can be used: for example *E. coli* K12 strains such as JM109, DH5, DH10, HB101, MG4100 etc. or *E. coli* B strains such as BL21, Rossetta, Origami etc. Genes can be introduced into the said host strain by any genetic method known in the art, for example by transfection, transformation, electroporation, conjugal transfer and others. Said gene clusters may be maintained in the said host microorganism in any form known in the art, for example encoded in a autonomously replicating plasmid or integrated into host's genome. Expression of the genes encoded in said gene clusters can be obtained either by utilizing the activity of native promoters controlling the expression of said genes or by replacing the promoters by promoters which may be more suitable for expression in said host microorganism. Methods for making such modifications are well known in the art.

In one aspect the invention provides gene cluster from *Gluconobacter oxydans* 621H comprised within nucleotide sequence of SEQ ID NO. 68, which is carried on a autonomously replicating plasmid comprised within the host *E. coli strain.* In this particular aspect the genes encoded on the cluster are expressed under control of their corresponding native promoters.

In one aspect the invention provides gene cluster from gene cluster from *Kluyvera intermedia* comprised within nucleotide sequence of SEQ ID NO. 69, which is carried on a autonomously replicating plasmid comprised within the host *E. coli strain.* In this particular aspect the genes encoded on the cluster are expressed under control of their corresponding native promoters. In the same particular aspect the genes used for provision of PQQ synthesis in E. coli are pqqABCDE and function of pqqF is provided by intrinsic activity of *E. coli.*

According to our findings availability of PQQ during the heterologous expression of PQQ dependent dehydrogenases has little effect on their correct folding, transport, cleavage of leader sequence and other posttranslational modifications. This means that there is little difference in dehydrogenase activity regardless of when, i.e. at which time or during which period the PQQ is provided to the dehydrogenase, e.g. during or after the cultivation and induction of expression. Other parameters are more relevant when establishing enhanced or even maximal PQQ dependent aldose dehydrogenase activity in periplasmic space where optimal coupling to cell's native electrone acceptors (the respiratory chain) is allowed. One of these parameters are presence of appropriate leader sequence, directing the protein to the periplasm. Another such parameter is expression strength which can be controlled by temperature of cultivation, transcriptional promoter selected, codon usage in the PQQ dependent aldose dehydrogenase encoding gene, quantity of expression inducer etc. Yet another such parameter are intrinsic properties of selected PQQ dependent aldose dehydrogenase such as ability to fold correctly in heterologous host, toxicity to heterologous host, resistance to the host's degrading enzymes etc. All such parameters, which are useful for enhanced activity and optimization and methods to do so, will become apparent to persons skilled in the art.

### Further Exemplified and Modified or Alternative Embodiments of the Present Invention

Various further embodiments, modifications and alternatives to carry out the present invention will be become apparent from the above description.

Further exemplified, the present invention for example provides a particular process comprising the step of reacting a substrate (II) under dehydrogenase catalyzed oxidation conditions to form the corresponding lactone (I), wherein the dehydrogenase is selected in first embodiment from GDH 01 or GDH 02 or GDH 03 or GDH 04 or GDH 05, or any dehydrogenase having an amino acid sequence identity of at least 70 % to those, more preferably 90 % to those. In another embodiment the dehydrogenase is selected from GDH 06 or GDH 07 or GDH 08 or GDH 09 or GDH 10, or any dehydrogenase having an amino acid sequence identity of at least 70 % to those, more preferably 90 % to those.

In another specific aspect, this invention relates to a method of constructing and providing appropriate synthetic biological pathways, such as exemplified with *E. coli* as a host microorganism, wherein DERA (deoxyribose 5-phosphate aldolase), PQQ dependant dehydrogenase and PQQ biosynthetic pathway genes are expressed simultaniously. The respiratory chain of the host organism are established and provided also.

Ylil aldose dehydrogenase meets all preferred features and is thus most prefered enzyme used. The Ylil encompasses any aldose dehydrogenase having an amino acid sequence identity to at least 50 % of the Ylil described herein, preferably at least 70 %. The amino acid sequence identities are determined by the analysis with a sequence comparison algorithm or by a visual inspection. In one aspect the sequence comparison algorithm is made with AlignX algorithm of Vector NTI 9.0 (InforMax) with settings set to default.

A further special aspect, the present invention relates to a process of oxidation or dehydrogenation of compound (II) using an enzyme as described above, comprising the provision of microorganism or microorganism-derived material used as a living whole cell catalyst, a resting whole cell catalyst, a cell free lysate, a partially purified or purified enzyme, an immobilized enzyme or any other form of catalyst, wherein the enzyme capable of catalyzing oxidation or dehydrogenation reaction as described above is expressed in said microorganism naturally, i.e. it being the microorganism's natural property. In said aspect such organism when cultivated and used as catalyst in said reaction can convert compound (II) to corresponding lactone without the need for additional genetic modification of said microorganisms. Said microorganism can be selected from vide diversity of bacteria as exemplified below. An organism with described properties can be selected from bacteria, more particularly proteobacteria, actinomycetales, mixobacteriaceae,. More particularly said microorganism may be selected from Gamma proteobacteriaceae. Most preferably organism in this sense is selected from the group of Enterobacteriaceae, Rihzobium, Gluconobacter and Acinetobacter.

Given the disclosure provided herein it will be apparent to a skilled person how to search for an organism having a capability of oxidation or dehydrogenation of compound (II). One such method would be to provide to a culture of studied microorganism an amount of compound (II) and look for activity.

In a further particular embodiment, the definition of the compound of formula (II) and thus also of the compound of formula (I) can be limited in that R5 denotes the moiety selected from the formulae (III), (IV), (V), (VI), (VII), (VIII) and (IX).

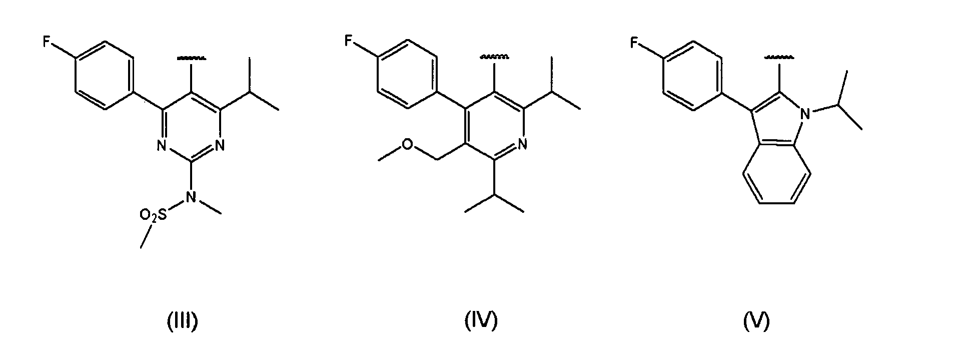

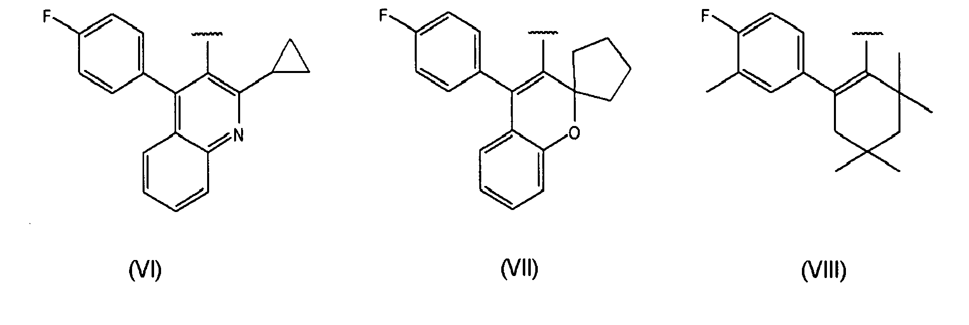

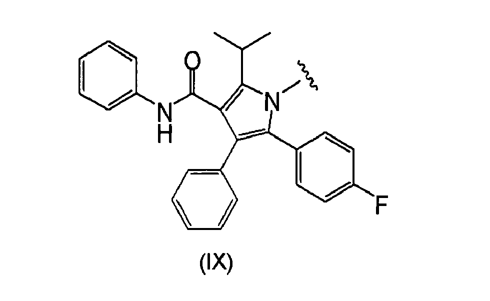

In yet another particular embodiment, the definition of formula (II) and thus also of formula (I) can be specified to the definition, wherein
R1 independently from R2 denotes H, X, N₃, CN, NO₂, OH, (CH₂)ₙ-CH₃, O-(CH₂)ₙ-CH₃, S-(CH₂)ₙ-CH₃, NR³R⁴, OCO(CH₂)ₙCH₃, or NR³CO(CH₂)ₙCH₃; and
R2 independently from R1 denotes H or (CH₂)ₘ-CH₃;
or both of R1 and R2 denote either X, OH or O(CH₂)ₙCH₃;
or R¹ and R² together denote =O, -(CH₂)ₚ-, -(CH₂)ᵣ-(1,2-arylene)-(CH₂)ₛ-, wherein R³ and R⁴ independently from each other, or together, denote H, (CH₂)ₘ-CH₃, or together form a ring -
(CH₂)ₚ-, -(CH₂)ᵣ-(1,2-arylene)-(CH₂)ₛ-, -(CO)ᵣ-(1,2-arylene)-(CO)ₛ-;
X denotes F, Cl, Br or I;
n represents an integer from 0 to 10;
m represents an integer from 0 to 3;
p represents an integer from 2 to 6;
and at least one from r and s is 1.

The compound of formula (I) obtained by the process of the present invention can be used as an intermediate for preparing a statin. The skilled person will know how to put the process step of obtaining said compound according to the present invention in the context of a statin synthesis. In principle, there are two basic ways to arrive at the statin. According to a first route, a lactone is prepared from the lactol and then coupled to the statin backbone. Alternatively, first the statin backbone containing the aldehyde side moiety is prepared, which is subsequently converted to lactol, for example by using 2-deoxyribose-5-phosphate aldolase (DERA) enzyme, and then oxidized to lactone. For the specific case of atorvastatin, as an example, one can refer to schemes 2 to 4 of the WO 2006134482.

In a specific embodiment an enzyme 2-deoxyribose-5-phosphate aldolase (DERA, EC 4.1.2.4) is used for preparing the compound of formula (II), which is subsequently converted to lactone by the enzyme capable of catalyzing oxidation or dehydrogenation. Multiple wild type, variants or mutant version of DERA enzyme are know in the art, including, but not limited to, J. Am. Chem. Soc. 116 (1994), p. 8422-8423, WO 2005/118794 or WO 2006/134482. In preferred embodiment, 2-deoxyribose-5-phosphate aldolase enzyme is used for a synthetic step just preceding the step of bringing in contact the compound of formula (II) with the enzyme capable of catalyzing oxidation or dehydrogenation.

In another preferred embodiment, DERA is used to prepare the compound of formula (II) at least in part simultaneously to conversion of said compound to the compound of the formula (I) by the enzyme capable of catalyzing oxidation or dehydrogenation. It will be immediately understood that the enzymes necessary to catalyse the reaction of preparing the compound of formula (II) and the reaction of oxidizing said compound to formula (I) can be used within the reaction mixture, or can be added to the reaction mixture, simultaneously or subsequently, at once, intermittently or continuously. The embodiment having the compound of formula (II), and thus the starting material for the reaction with the enzyme capable of catalyzing oxidation or dehydrogenation, prepared by DERA is advantageous, because this arrangement is well compatible and it allows using aqueous solvents in the preceding step and thus makes it unnecessary to purify the compound of formula (II) prior to offering it to the enzyme capable of catalyzing oxidation or dehydrogenation for conversion to lactone. The preferred embodiment is thus to bring the compound of formula (II) in contact with the enzyme capable of catalyzing oxidation or dehydrogenation without prior isolation or purification of said compound. In this event, the complete reaction mixture of the preceding step can be used for the subsequent reaction, which reduces the number of process steps and simplifies the process. With obviating a purification step yield is also increased. In addition, because of better enzyme specificity over chemical oxidants, to most extent only the compound of formula (II) gets oxidised to lactone, while the rest of the present compounds do not change, hence reducing a tendency of impurity generation and thus improving subsequent working-up or purification procedures. All of the substrate in any enzyme reaction according to the present invention can be added to the reaction at once or can be added continuously over longer period, or in one batch or intermittent batches.

Significant improvement can be achieved when the compound of formula (I) is prepared at least in part simultaneously to preparing the compound of formula (II). At the same time it solves multiple drawbacks of using the reaction with the DERA enzyme individually. For example, aldehydes used as a starting material in preparing the compound of formula (II) by using DERA enzyme tend to inactivate the DERA enzyme during the course of reaction and thus reduce enzyme's activity. In addition, the lactol of formula (II) that builds up in the reaction mixture is toxic to the living microorganism. Therefore, it is highly desired to shorten the reaction step with DERA and to consume the starting aldehyde and/or the lactol as soon as possible, which is achieved when both enzymatic reaction steps are performed at least in part simultaneously. Namely, when both reaction steps are performed at least in part simulateneously, preferably completely simultaneously, the toxic lactol immediately enters into the consequent reaction and is transformed to the non-toxic lactone. Moreover, since the second reaction step typically is not a rate limiting step, as confirmed in examples hereinafter, and proceeds faster than the first step with DERA, the steady state equilibrium of the first reaction shifts in a direction of the product. This leads to reduced time for completion of the first step and thus protects DERA from being inactivated. It also protects living cells from being disrupted by high concentrations of lactol.

Important aspect of present invention deals with the intrinsic capability of a microorganisms to transfer electrons produced by oxidation/dehydrogenation of (II), to oxygen (a terminal electron acceptor) via its respiratory chain. This drives the reaction of enzyme catalysed oxidation / dehydrogenation of compound (II) in a whole cell system. It will be immediately apparent that the capability of acting as an electron sink is a significant and beneficial property of whole cell systems as described hereby in the invention.

Use of whole cells therefore avoids use of additional electron acceptors such as DCIP and others described above. An additional benefit of using whole cell processes is ability to provide all aspects of described synthetic biological pathway, i.e. the DERA enzyme, PQQ and a PQQ dependant sugar dehydrogenase, in one organism. As productivity and yields of such process are industrially suitable as exemplified below, use of whole cells is preferred as costs can be controlled at significantly lower level compared to other approaches, e.g. free enzyme process, immobilized enzyme, cell free lysate etc. Also the possibility to perform both DERA and oxidation/ dehydrogenation step fully or partially simultaneously using one pot design leads to significant cost reductions when used in industrial scale.

In this sense of making use of whole cell system as electron sink, it is also advantageous to perform the process in the presence of oxygen, particularly where the oxygen is provided in quantity allowing at least 5%, preferably at least 15%, of dissolved oxygen at given process condition, wherein 100% dissolved oxygen is understood as saturated solution of oxygen at given process conditions and 0% is understood as oxygen free liquid and correlation between oxygen concentration and dissolved oxygen percent is linear between the 0% and 100% at said given process conditions. In this aspect process conditions are understood as liquid composition, temperature, pH, pressure, wherein the measurements in dynamic process are understood to be performed in a homogenous solid/liquid/gas multiphase system.

The presence of the oxygen in that amount makes the oxidation or dehydrogenation reaction irreversible, which secures the obtained lactone and further enhances shifting of the steady state equilibrium of the first reaction towards the product. This preferred embodiment thus increases yield and reduces time needed for the process.

The enzyme capable of catalyzing oxidation or dehydrogenation, and/or the DERA enzyme, i.e. respectively alone or in combination and optionally independently, can be comprised within single or multiple living whole cell(s), inactivated whole cell(s), homogenized whole cell(s), or cell free extract(s); or are respectively purified, immobilized and/or are in the form of an extracellularly expressed protein. Preferably one of the two enzymes, yet more preferably both enzymes are comprised within same living whole cell, same inactivated whole cell or same homogenized whole cell, more preferably are within same living whole cell or same inactivated whole cell, particularly are comprised within same living whole cell, because having the enzyme in a common whole cell or at least in the common inactivated whole cell, does not demand much handling with the enzyme prior it being used in the process, which reduces costs. Moreover, having the enzyme comprised in a living whole cell enables simple removal of the enzyme by filtration, which alleviates final purification steps at the industrial scale. In addition, it allows a reuse of the enzyme comprised within the living cell in subsequent batches.

Another advantage of using the enzyme in a whole cell or at least in the inactivated whole cell is possibility of providing PQQ cofactor intrinsically as described in detail above.

As an advantageous option, a whole cell system capable of translating 2-deoxyribose-5-phosphate aldolase (DERA) enzyme and an enzyme capable of catalyzing oxidation or dehydrogenation can be arranged to overexpress both of the genes needed for said translation. Measn for overexpression are known to the person skilled in the art, and are sometimes referred to elsewhere herein.

According to a further aspect of the present invention, an expression system is provided comprising one or more cell types, the respective cell type(s) being genetically engineered to express, in the totality of cell type(s), both the 2-deoxyribose-5-phosphate aldolase (DERA) enzyme and an enzyme capable of catalyzing oxidation or dehydrogenation.

An expression system can be made up of appropriate organisms or cells and optionally further factors and additives, wherein reference is made to the disclosure provided herein.

In one aspect, this invention provides a method of constructing or providing synthetic biological pathway for use in the present invention, examplified with *E. coli* as a host microorganism, wherein DERA (deoxyribose 5-phosphate aldolase), PQQ dependant dehydrogenase and PQQ biosynthetic pathway genes are expressed simultaniously. Providing the respiratory chain of the host organism, said synthetic biological pathway has a capability of carrying out production of compound (I) from simple molecules such as compound (X), shown below, and acetaldehyde. This approach is advantageous since this approach joins previously separate steps of production of compound (II), purification of compound (II), and oxidation of compound (II) to compound (I). Additionaly, the cultivation of organisms carrying having said synthetic biological pathway is performed in one industrial fermentation process which immediately provides material capable of converting molecules such as compound (IX) and acetaldehyde into compound of formula (I).

Another embodiment of the present invention is obtaining the compound of formula (I), or salts, esters or stereoisomers thereof, in a one-pot process by reacting the starting materials for the DERA enzyme reaction in the presence of 2-deoxyribose-5-phosphate aldolase (DERA) enzyme and an enzyme capable of catalyzing oxidation or dehydrogenation, and optionally salifying, esterifying or stereoselectively resolving the product. This embodiment contemplates to start from the compound of formula (X) 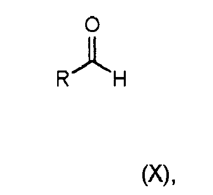 in which R denotes R1-CH-R2 moiety of formula (I), and R1 and R2 being as defined hereinabove; and subjecting said compound (X) to reaction with acetaldehyde in the presence of the two enzymes, namely aldolase (DERA) enzyme and the enzyme capable of catalyzing oxidation or dehydrogenation. This setup allows obtaining the compound of formula (I) in a single process step starting from relatively simple starting materials, e.g. acetaldehyde. The reaction is industrially suitable, as it proceeds to completion within few hours. It renders intermediate purification steps superfluous. In addition, it provides a possibility of having both enzymes added together - the product of the first enzymatic reaction forming a substrate of the second enzymatic reaction - preferably comprised within the same living whole cell, inactivated whole cell, homogenized whole cell, or cell free extract; or are purified, immobilized and/or are in the form of an extracellularly expressed protein, preferably are within the same living whole cell, inactivated whole cell or homogenized whole cell, more preferably are within the same living whole cell or inactivated whole cell, particularly are comprised within the same living whole cell.

This makes use of all the advantageous effects of combined enzymatic reactions, including, but not limited to the ones described herein. In one aspect, the total amount of substrates added to the mixture is such that the total amount of the substrate (X) added would be from about 20 mmol per liter of the reaction mixture to about 2 mol per liter of the reaction mixture, in particular from about 100 mmol per liter of the reaction mixture to about 1.5 mmol per liter of the reaction mixture, more particular from about 200 mmol per liter of the reaction mixture to about 700 mmol per liter of the reaction mixture. Acetaldehyde may be added by several means. In one aspect, acetaldehyde is added to the reaction mixture in one batch or more batches or alternatively continuously. Acetaldehyde may be premixed with the substrate of formula (X) and added to the reaction mixture. The total amount of acetaldehyde added to the reaction mixture is from about 0.1 to about 4 molar equivalents to the total amount of the acceptor substrate, in particular from about 2 to about 2.5 molar equivalents. In one aspect of the invention, the pH-value used for the reaction is from about 4 to about11. In one embodiment, the pH used for reaction is from about 5 to about 10. In another embodiment, the pH-value used for reaction is from about 5 to about 8. Specifically the pH-value will be maintained by a suitable buffer in a range from 52 to 7.5. Alternatively the pH-value as stated above may be controlled by, but not limited to, controlled addition of acid or base according to need as will be obvious to the person skilled in the art.

In one aspect the pH used for the reaction described by the present invention may be optimized so that the compromise between optimal enzyme activity and optimal substrate and/or product stability is taken. It is understood herein that optimal enzymatic activity for different enzymes described in this invention may not be identical to optimal conditions for substate/product stability. A person skilled in art may find it benneficial to sacrifice some enzyme activity by adjusting conditions to suite substrate and/or product stability (or vice versa) to obtain optimal product yields.

Specifically, aldolase enzyme, optionally at least in part together with the enzyme capable of catalyzing oxidation or dehydrogenation are prepared in an aqueous solution (particularly each in a concentration 0.1 g/L to 3 g/L), optionally in the presence of a salt (in particular NaCl in a concentration from 50 to 500 mM and CaCl₂ or alternative Calcium salt in a concentration from 0,01 to 10mM). The aqueous solution may contain organic solvents miscible with water (in particular dimethyl sulfoxide in a concentration from 2 to 15 % V/V), and may be buffered to pH 4 to 11. Some commonly used buffers can lower the yield of the aforementioned reaction that starts from the acetaldehyde by limiting the availability of aldolase-condensation intermediates, particularly first condensation reaction products as they may undergo a chemical reaction with a buffer. For example, bis-tris propane reacts with said intermediates ((S)-3-hydroxy-4,4-dimethoxybutanal) giving (*S,Z*)-2-(3-((1,3-dihydroxy-2-hydroxymethyl)propan-2-yl)(3-hydroxy-4,4-dimethoxybut-1-enyl)amino)propyl-amino)-2-(hydroxymethyl)propane-1,3-diol. Other buffers that may react similarly are bis-tris, tricin, tris, bicin or any other buffer having a primary, secondary or tertiary amino group. Thus suitable buffers for adjusting pH, if this adjustment is needed, are made with acids, bases, salts or mixtures thereof, in particular phosphoric acid and sodium hydroxide. In a particularly preferred embodiment, the buffer is a phosphate buffer. In particular, phosphate buffer, in a concentration 10 to 500 mM can be used. The aqueous solution can also be prepared by adding the aldolase enzyme, optionally at least in part together with the enzyme capable of catalyzing oxidation or dehydrogenation to water and maintaining the pH-value during the reaction by means of an automated addition of inorganic acids, bases, salts or mixtures thereof.

In one aspect according to the invention, the temperature used for the reaction starting from acetaldehyde is from about 10 to about 70 °C. In one embodiment, the temperature used for the reaction is from about 20 to about 50 °C. In another embodiment, the temperature used for the reaction is from about 25 to about 40 °C.

In one aspect the temperature used for the reaction described by this invention may be optimized so that the compromise between optimal enzyme activity and optimal substrate and/or product stability is taken. It is understood herein that optimal enzymatic activity for different enzymes described in this invention may not be identical to optimal conditions for substate/product stability. A person skilled in art may find it beneficial to sacrifice some enzyme activity by adjusting conditions to suite substrate and/or product stability (or vice versa) to obtain optimal product yields.

After the completion of the reaction, either enzyme can be removed from the reaction mixture, for example by the addition of at least about 1 vol. of acetonitrile to 1 vol. of reaction mixture. Alternatively the enzyme can be removed by any salting out method known in the art. In one embodiment the salting out is performed with the addition of ammonium sulfate of at least 5 % m/V. In the embodiment, where the enzyme is comprised within living or inactivated cells, the enzyme may be removed by filtrating or centrifuging the reaction mixture.

In another embodiment the product is removed by liquid/liquid extraction to any of a number of water immiscible or poorly miscible solvents. The solvent may be selected from but is not limited to: methylene chloride, ethyl acetate, diethyl ether, propionyl acetate, methyl *t*-butyl ether (MTBE), nitromethane, pentane, hexane, heptane, 1,2-dichloroethane, chloroform, carbon tetrachloride, *n*-butanol, *n*-pentanol, benzene, toluene, *o*-, *m*-, *p*-xylene, cyclohexane, petroleum ether, triethylamine. Prior the liquid/liquid extraction with chosen organic solvent the pH of water solution of the product may be adjusted to values between 1 and 12, preferably between 2 and 8, more preferably between 3 and 5. Drying of water residues in organic phase after extraction completion may be performed with but is not limited to adding salts listed: sodium sulfate, magnesium sulfate (monohydrate), calcium sulfate, calcium chloride, copper sulfate.

In general, the aldolase enzyme and/or enzyme capable of catalyzing oxidation or dehydrogenation used can be prepared by any means known in the art, for example by methods of protein expression described in Sambrook et al. (1989) Molecular cloning: A laboratory Manual 2nd Edition, New York: Cold Spring Harbor Laboratory Press, Cold Spring Harbor. Gene coding aldolase enzyme and/or enzyme capable of catalyzing oxidation or dehydrogenation can be cloned into an expression vector and the enzyme be expressed in a suitable expression host. Modified versions of known aldolase enzyme or enzyme capable of catalyzing oxidation or dehydrogenation may be necessary or may result depending on cloning conditions and are encompassed in the present invention.

### Cells and Organisms

One aspect of present invention provides a process of oxidation or dehydrogenation of compound (II) or other compounds recited herein using a microorganism in any form described herein having enzyme capable of catalyzing oxidation or dehydrogenation reaction natively expressed. In said aspect such organism when cultivated and used as catalyst can convert compound (II) to corresponding lactone without the need for additional genetic modification of said microorganisms. A methods of identifying such organisms is exemplified in hereby invention. Non-limiting examples of such organism can be selected from vide diversity of bacteria, more particularly *Escherichia, Corynebacterium, Pseudomonas, Streptomyces, Rhodococcus, Bacillus, Lactobacillus, Klebsiella, Enteorobacter, Acinetobacter, Rhizobioum. Methylobacterium, Kluyvera, Gluconobacter, Erwinia, Rahnella and Deinococcus.*

In referred embodiments, and in order to practice embodiments of the present invention in its best configuration, a specially adapted expression system capable of translating 2-deoxyribose-5-phosphate aldolase (DERA) enzyme and an enzyme capable of catalyzing oxidation or dehydrogenation, and overexpressing both of the genes needed for said translation, is provided. The term "overexpressing" as used herein refers to the expression under control of a strong promoter, or wherein the gene is expressed at high levels (compared to w.t. expression control) and is accumulated intracellularly or extracellularly. The process of obtaining such a modified expression is known to a person skilled in the art. For example, cloning methods described in Sambrook et al. (1989) Molecular cloning: A laboratory Manual 2nd Edition, New York: Cold Spring Harbor Laboratory Press, Cold Spring Harbor, can be used. The genes for the enzymes can be for example cloned on the same or different vector and transformed into a cell. In an alternative, the expression system comprises separate cells, wherein first cell overexpresses the gene for aldolase enzyme and second cell overexpresses the gene for the enzyme capable of catalyzing oxidation or dehydrogenation. The present specification illustratively, without limitation facing common general knowledge, provides an example of making such expression system. The expression system is particularly suited for preparing statin or intermediate thereof.

The skilled person is aware of all the possible cell systems for either preparing or hosting of either DERA enzyme or the enzyme capable of catalyzing oxidation or dehydrogenation, either alone or in combination, and optionally independent from each other. In general, the cell system would be prokaryotic or eukaryotic. In a specific embodiment, the enzyme can be prepared synthetically. The cell for preparing or hosting either of the enzymes can be a bacteria, yeast, insect cell or a mammalian cell. Preferably the cell is bacteria or yeast and more preferably is bacteria, because bacteria or yeast cell are easier cultivated and grown. The bacteria can be selected from the group of genera consisting of Escherichia, Corynebacterium, Pseudomonas, Streptomyces, Rhodococcus, Bacillus and Lactobacillus, preferably from Escherichia and Lactobacillus, more preferably Escherichia, particularly is Escherichia Coli. In case of yeast, the cell can be selected from the group of genera consisting of Saccharomyces, Pichia, Shizosaccharomyces and Candida, preferably Saccharomyces. The examples of mammalian cells are Chinese hamster ovary cell or a hepatic cell, preferably is Chinese hamster ovary cell.

Another embodiment of the invention is a process for the preparation of compound (I), in particular an industrial fermentative process, wherein the process comprises the step of cultivation of a microorganism capable of oxidation of compound (II), wherein the said microorganism is brought in contact with compound (II).

Yet another embodiment of this invention is a process for the preparation of compound(I), in particular an industrial fermentative process, wherein the process comprises the step of cultivation of a microorganism, capable of oxidation of compound (II), wherein said microorganism is brought in contact with another microorganism having ability of enzymatic production of (II), particularly by catalysis of DERA and wherein substrates which allow production of compound (II) are provided to the reaction mixture.

A preferred embodiment of his invention is a process for the preparation of compound (I), in particular an industrial fermentative process, wherein the process comprises the step of cultivation of a microorganism, capable both of production as well as oxidation/dehydrogenation of compound (II) and wherein substrates which allow production of compound (II) are provided to the reaction mixture.

In particular embodiments, the process according to the present invention comprises the following steps:
Step a1) If not already known or provided, as disclosed elsewhere hererin, this step includes identification of a microorganism capable of oxidation/dehydrogenation of compound (II) and/or generation of genetically modified strain of a microorganism to obtain capability of oxidation/dehydrogenation of compound (II) as described in this invention. Particularly organisms having sugar 1-dehydrogenase activity are prefered.
Step a2) If not already known or provided, as disclosed elsewhere hererin, this step includes identification of a microorganism capable of production of compound (II) and/or generation of genetically modified strain of a microorganism to obtain capability of production of compound (II) as described in this invention or is known in the present art. Particularly organisms having aldolase catalytic ability are prefered.

It is particularly prefered that a microorganism is identified and/or genetically modified in order to obtain both properties described in step a1) and step a2). In this sense the invention specifically relates to a genetically modified strain of a microorganism wherein the genetic material of the strain comprises at least one over expressed gene coding for and enzyme capable of catalysing aldol condensation to form compound (II), more specifically a gene encoding DERA enzyme.

Procedures to identify and/or generate genetically modified microorganisms as described in step a1) and step a2) are exemplified in detail in this invention, however a skilled person will immediately find alternative procedures which may lead to the same desired properties of said microorganisms. For example, cloning methods described in Sambrook et al. (1989) Molecular cloning: A laboratory Manual 2nd Edition, New York: Cold Spring Harbor Laboratory Press, Cold Spring Harbor, can be used. The genes for the enzymes can be for example cloned on the same or different vector and transformed into a cell. In an alternative, the expression system comprises separate cells, wherein first cell overexpresses the gene for aldolase enzyme and second cell overexpresses the gene for the enzyme capable of catalyzing oxidation or dehydrogenation. The present specification illustratively, without limitation and taking into account common general knowledge, provides an example of making such modified microorganism. The microorganism is particularly suited for preparing statin or intermediate thereof.

The skilled person is aware of all the possible cell systems for either preparing or hosting of either DERA enzyme or the enzyme capable of catalyzing oxidation or dehydrogenation or, optionally, the PQQ biosynthetic genes, either alone or in combination, and optionally independent from each other. In general, the cell system would be prokaryotic or eukaryotic. In a specific embodiment, the enzyme can be prepared synthetically. The cell for preparing or hosting either of the enzymes can be a bacteria, yeast, insect cell or a mammalian cell. Preferably the cell is bacteria or yeast and more preferably is bacteria, because bacteria or yeast cell are easier cultivated and grown.

The bacteria can be selected from the group of genera consisting of *Escherichia, Corynebacterium, Pseudomonas, Streptomyces, Rhodococcus, Bacillus, Lactobacillus, Klebsiella, Enteorobacter, Acinetobacter, Rhizobioum. Methylobacterium, Kluyvera, Gluconobacter, Erwinia, Rahnella and Deinococcus.* In a more particular sense the microorganisms may be selected from *Klebsiella pneumoniae, Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans, Corynebacterium glutamicum, Escherichia coli, Bacillus licheniformis, Lactobacillus lactis,* most preferably from: *Escherichia coli, Gluconobacter oxydans, Acinetobacter calcoaceticus and Kluyvera intermedium.* In case of yeast, the cell can be selected from the group of genera consisting of *Saccharomyces, Pichia, Shizosaccharomyces* and *Candida*, preferably *Saccharomyces and Pichia.*

It is particularly preferred that a microorganism is identified and/or genetically modified in order to obtain both properties described in step a1) and step a2). In this sense the invention specifically relates to a genetically modified strain of a microorganism wherein the genetic material of the strain comprises at least one over expressed gene coding for and enzyme capable of catalysing aldol condensation to form compound (II), more specifically a gene encoding DERA aldolase enzyme.

Accordingly, the present invention provides an exemplified method of constructing synthetic biological pathway, examplified with *E. coli* as a host microorganism, wherein DERA (deoxyribose 5-phosphate aldolase), PQQ dependant dehydrogenase and PQQ biosynthetic pathway genes are expressed simultaniously. Providing the respiratory chain of the host organism said synthetic biological pathway has a capability of carrying out production of compound (I) from simple molecules such as compound (X) and acetaldehyde.

One aspect described in this invention is to cultivate microorganisms described in step a1 and a2 simultaneously or independently.

### Step b) Preparation of seed medium

Cultivation of the microorganisms as described in the present invention can be carried out by methods known to a person skilled in art. Cultivation processes of various microorganisms are for example described in the handbook "Difco & BBL Manual, Manual of Microbiological Culture Media" (Zimbro M.J. et al., 2009, 2nd Edition. ISBN 0-9727207-1-5). Preferably the production of seed microorganism which can be used in the main fermentation process for the production of (I) starts from a colony of said microorganism. In this respect the process according to the present application comprises the preparation of frozen stock of described microorganism. This preparation of frozen stock may be carried out using method known in the state of art, such as using a liquid propagation medium. Preferably this frozen stock of microorganism is used to produce a vegetative seed medium by inoculation to a vegetative medium.

The seed medium may be transferred aseptically to a bioreactor. In principle the cultivating of seed microorganism can be carried out under the conditions (e.g. pH and temperature) as in the main fermentation process (described under step c).

### Step c) Main fermentation process

Preferably the main fermentation process using a microorganism as described in the present application is carried out in a bioreactor in particular under agitation and/or aeration. Preferably, cultivation of a microorganism used for the process for the production of compound (I) as described in the present application is carried out under submerged aerobic conditions in aqueous nutrient medium (production medium), containing sources of assimilable carbon, nitrogen, phosphate and minerals. Additional compounds may be added to the production medium during or after the cultivation process in order to obtain appropriate enzymatic activities. These may include expression inducers , sources of cofactors and or compounds allowing maintanence of genetic elements (such as antibiotics).

Preferably the main fermentation process comprises the inoculation of production medium with seed microorganism obtained in step b) in particular by asepticall transfer into the reactor. It is preferred to employ the vegetative form of the microorganism for inoculation. The addition of nutrient medium (production medium) in the main fermentation process into the reactor can be carried out once or more batch-wise or in a continuous way. Addition of nutrient medium (production medium) can be carried out before and/or during the fermentation process.

The preferred sources of carbon in the nutrient media can selected from dextrin, glucose, soluble starch, glycerol, lactic acid, maltose, fructose, molasses and sucrose as exemplified below.

The preferred sources of nitrogen in the nutrient media are ammonia solution, yeast extract, soy peptone, soybean meal, bacterial peptone, casein hydrolysate, L-lysine, ammonium sulphate, corn steep liquor and other.

Inorganic/mineral salts such as calcium carbonate, sodium chloride, sodium or potassium phosphate, magnesium, manganese, zinc, iron and other salts may also be added to the medium.

Further known additives for fermentative process may be added in particular in the main fermentation process. To prevent excessively foaming of the culture medium anti-foaming agents could be added, such as silicone oil, fatty oil, plant oil and the like. Particularly a silicone-based anti-foaming agent may be added during the fermentation process to prevent excessively foaming of the culture medium. Expression inducers such as isopropyl β-D-1-tiogalaktopyranoside (IPTG), Arabinose, Tetracycline, indoleacrilate etc. may be added to the culture medium. Additionally, cofactors such as PQQ (pyrroloquinoline quinone), NAD(P), FAD may be added to improved the activity of involved enzymes. In a particular aspect IPTG and PQQ may be used.

The fermentative process could be performed in aerobic conditions with agitation and aeration of production medium. Agitation and aeration of the culture mixture may be accomplished in a variety of ways. The agitation of production medium may be provided by a propeller or similar mechanical device and varied to various extents according to fermentation conditions and scale. The aeration rate can be varied in the range of 0.5 to 2.5 WM (gas volume flow per unit of liquid volume per minute (volume per volume per minute)) with respect to the working volume of the bioreactor.

The main fermentation process by the present process is carried out at a pH in the range of about 6.3 to 8.5 and temperature in the range of 18 to 37 °C. Preferably the pH is in the range of about 6.5 to 8.3 and the temperature is in the range of about 21 to 31 °C. Preferably, the cultures are incubated for 16 to about 300 hours, more preferably for about 30 to 70 hours.

It will be obvious to a person skilled in the art that different microorganisms may demand different growth conditions and that it is well described in the art how one can determine the optimal conditions for growth of specific organism. It will be also obvious to a person skilled in the art that different growth conditions may have a big effect on activity of the enzymes involved in the provided process. The present inventions provides detailed examples of methods which can be used to optimize the growth conditions to obtain maximal activities and reaction rates of said enzymes.

Another embodiment of in this invention encompasses cultivation of microorganisms described in step a) simulaniously or independatly. Likewise it is also possible to conduct reactions for preparation of compound (II) and compound (I) separately or simultanuosly, successively or in a one pot manner.

Specifically, dehydrogenase/oxidase enzyme is prepared after step c) in an aqueous solution (particularly in a concentration range from 0.1 g/L to 300 g/L) in present of salt (in particular NaCl in concentration range from 50 to 500 mM), diluted or concentrated to said concentration range. When supplemented with fresh medium or components of the medium allowing viability of the organism living whole cell catalyst is obtained. When prepared in buffered aqueous solution or in used medium, resting whole cell catalyst is obtained. The aqueous solution may be buffered to pH 4.0 to 11, preferably to pH 5.0. to 10.0, more preferably to 5.0 to pH 8.0. Most preferably the solution is buffered to about pH5.2 to obout pH 7,5. Suitable buffers can be prepared from: acids, bases, salts or mixtures thereof, and any other buffer system known in the art except those possessing primary, secondary or tertiary amino group. In particular, phosphate buffer, in concentration 10 to 500 mM may be used. The aqueous solution can be prepared by adding the said dehydrogenase/oxidase enzyme to water and maintaing pH during the reaction by means of automated addition of inorganic or organic acids, bases, salts of mixtures thereof.

### Step d) Enzymatic reaction

Several options to carry out this invention are provided. It is possible to conduct reactions for preparation of compound (II) and compound (I) separately or simultaneously, successively or in a one pot manner. In one embodiment, these variations are:

### 1. Compound (II) is added into the culture of microorganism having compound (II) oxidation/dehydrogenation capability.

After completion of preceding step of cultivating a microorganism capable of oxidation/dehydrogenation of compound (II) in main fermentation step, compound (II) is brought into contact with said microorganism. Said microorganism may intrinsically contain all needed cofactors or external PQQ addition may be used. PQQ may be added in concentration from about 0.1nM to about 5mM. In particular from about 1nM to about 100uM of PQQ can be provided. More preferably the PQQ is provided in concentration from 100nM to about 2µM. Most preferably the PQQ is provided in the minimal amount which allows maximal activity of the said catalyst. Practically this is in the range of 250nM to 1µM final concentration of PQQ. Optionally an artificial electron acceptor is added in equimolar concentration to compound II as described in this invention. Preferably the process is preformed by using microorganisms capability of accepting electrons formed during dehydrogenation / oxidation reaction into its intrinsic respiratory chain. Compound (II) may be provided as partiallly or fully isolated compound (II) obtained from previous reaction mixture containing DERA aldolase under aldolase-catalysed aldol condensation conditions or from other sources (organic synthesis). Compound (II) may be added in by any means and rates as described within this invention in a final concentration from about 20mM to about 1 M, preferably from about 50mM to about 700mM, most preferred concentrations are between 100mM and 500mM. During the process of dehydrogenation / ocxidation of compound (II) additional nutrients in order to support microorganisms viability may be added in similar way as described in step c). Practically it may be beneficial to provide additional carbon source to said reaction mixture as described within hereby invention, optionally independently or in addition also a nitrogen source. General reaction conditions which allow dehydrogenation/ oxidation of compound (II) in this specific aspect are defined as 'aldose dehydrogenation/oxidation conditions' as provided by this invention.

The compound (II) as substrate for oxidase/dehydrogenase to obtain compound (II) may be added to the reaction mixture in one batch or more batches. In one aspect, the total amount of substrate added to the mixture is such that the total amount of compound (II) added would be from about 20 mmol per liter of reaction mixture to about 1.5 mol per liter of reaction mixture, more particular from about 100 mmol per liter of reaction mixture to about 700 mmol per liter of reaction mixture. In preferred embodiment the substrates are added continuously to the reaction mixture by means of programmable pump at specific flow rate at any given time of the reaction. Optimally, the flow rate is determined as maximum flow rate where the substrate is not accumulating in the reaction mixture. In particular this allows minimal concentrations of undesired products. In another embodiment the inhibitory effect of substrate can be further minimized using correct addition strategy.

### 2. Sequential reaction using DERA aldolase and oxidation/dehydrogenase enzyme.

This aspect the invention provides a variant described as d1), however in this case the compound (II) is provided in situ directly in form of reaction mixture obtained by reacting DERA aldolase with compound (X) and acetaldehyde by methods known in the art. Preferably this approach uses the advantage of performing a one-pot reaction and thus significantly impacting the simplicity of the combined process. It is advantageous to use whole cell catalyst containing DERA aldolase in the proceeding step in accordance to 'aldolase-catalysed aldol condensation conditions"

### 3. Simultaneous reaction using DERA aldolase and oxidation/dehydrogenase enzyme within two separated catalysts.

Both catalysts are obtained by fermentation process simultaneously or independently (as described in step c) and catalysts are then transferred into a suitable vessel or reactor, preferably joined or left in same fermenter used for obtaining the catalyst. Another yet similar aspect of the process both enzymatic activities are present in a single microorganism which is preferably obtained as described in step c. In a specific embodiment a simultaneous process containing using DERA aldolase and enzyme capable of oxidation/dehydrogenation and thus providing compound (I). More perferably the acetyloxyacetaldehyde (CH₃CO₂CH₂CHO) as substrate for DERA aldolase to obtain compound (II) may be added to the reaction mixture continuously or alternatively the acetyloxyacetaldehyde (CH₃CO₂CH₂CHO) is added to the reaction mixture in one batch or more batches. In one aspect, the total amount of substrates added to the mixture is such that the total amount of acetyloxyacetaldehyde (CH₃CO₂CH₂CHO) added would be from about 20 mmol per liter of reaction mixture to about 1.5 mol per liter of reaction mixture, more particular from about 100 mmol per liter of reaction mixture to about 700 mmol per liter of reaction mixture. Acetaldehyde may be added by several means. In one aspect the acetaldehyde is added to the reaction mixture in one batch or more batches or alternatively continuously. Acetaldehyde may be premixed with acetyloxyacetaldehyde (CH₃CO₂CH₂CHO) and added to the reaction mixture. The total amount of acetaldehyde added to the reaction mixture is from about 0.1 to about 4 molar equivalents to total amount of acceptor substrate acetyloxyacetaldehyde (CH₃CO₂CH₂CHO), in particular from about 1 to about 3 molar equivalents, more preferably from about 2 to 2.5 molar equivalents. In particular, this allows minimal concentrations of undesired products. Optionally PQQ is added into the reaction mixture in concentrations from about 0.05 µM to about 10 mM, more preferably 0.1 uM to about 1 mM. In preferred embodiment the substrates are added continuously to the reaction mixture by means of programmable pump at specific flow rate at any given time of the reaction. The flow rate is determined as maximum flow rate where the substrates are not accumulating in the reaction mixture. In particular this allows minimal concentrations of undesired products. In another embodiment the inhibitory effect of substrates can be further minimized using correct addition strategy. In one aspect, the temperature used for dehydrogenase/oxidase-catalysed reaction is from about 10 °C to about 70 °C. in one embodiment, the temperature used for dehydrogenase/oxidase reaction is from about 20 to about 50 °C. In one embodiment the temperature used for dehydrogenase/oxidase reaction is from about 25 °C to about 40 °C. The reaction is industrially suitable, as it proceeds to completion within few hours.

The term "aldose dehydrogenation/oxidation conditions" as used herein refers to any dehydrogenation/oxidation conditions known in the art that can be catalysed by any dehydogenase/oxidase enzyme, as described herein. In particular the dehydrogenase/oxidase activity conditions are such that allow forming and accumulation of desired product. These conditions include in one aspect that the dehydrogenase/oxidase is an active enzyme provided at sufficient load to be able to perform the dehydrogenation/oxidation. In another aspect, that the compound (II) as substrate is present in the reaction mixture in an amount that displays minimal inhibition of the activity of the aldolase. Preferably, that the temperature, pH, solvent composition, agitation and length of reaction allow accumulation of desired product and thus forming from compound (II) corresponding compound (I), in another aspect that said conditions do not have detrimental effect stability. Specifically those conditions are defined by values disclosed in examples. An dehydrogenase/oxidase for use in the present invention may be any enzyme that has dehydrogenase/oxidase activity towards compound of formula (II). In a prefered embodiment, the dehydrogenase/oxidase enzymes include but are not limited to: GDH 01, GDH 02, GDH 03, GDH 04, GDH 05, GDH 06, GDH 07, GDH 08, GDH 09, GDH 10, GDH 11, GDH 12, GDH 13, GDH 14, GDH 15, GDH 16, GDH 17, GDH 18, GDH 19, GDH 20, GDH 21, GDH 22, GDH 23, GDH 24 and GDH 25, wherein each enzyme is identified by it's corresponding nucleotide sequence or respective codon optimized nucleotide sequence or aminoacid sequence as set forth in sequence listing above. The dehydrogenase/oxidase catalyst described herein can be used in any biologically active form provided in this invention. Generally, catalyst able to obtain compound (I), will be provided in a suitable vessel or reactor, and the compound (II) will be added batch-wise or continuously, or provided by, at least in part simultaneous production of compound (II), preferably in same vessel under "aldolase-catalysed aldol condensation conditions".

The term "aldolase-catalysed aldol condensation conditions" as used herein refers to any aldol condensation conditions known in the art that can be catalysed by any aldolase, as described for example in WO2008/119810 A2. In particular the aldolase-catalysed aldol condensation conditions are such that allow forming and accumulation of desired product, more preferably that the substituted acetaldehyde R₁CO₂CH₂CHO, more particularly acetyloxyacetaldehyde (CH₃CO₂CH₂CHO) as substrate and acetaldehyde are present in the reaction mixture in an amount that displays minimal inhibition of the activity of the aldolase, in another aspect that the temperature, pH, solvent composition, agitation and length of reaction allow accumulation of desired product and thus forming corresponding lactole (compound II). The DERA aldolase described therein can be used in any biologically active form provided in said invention. The substrates for DERA aldolase, the compounds of formula (X) are selected according to the corresponding compound (II). These products having a masked aldehyde group are key intermediates in WO2008/119810 A2 and WO2009/092702 A2 allowing further steps in preparation of statins, in particular, substrates yielding a product with aldehyde group are preferred. The compound (X) may be in particular acetyloxyacetaldehyde (CH₃CO₂CH₂CHO).

### Optional step e): Separation and/or purification of compound (I)

Isolation and/or purification of compound (I), which was produced in the main fermentation process from the said medium, may be carried out in a further separation step.

In the first step of the isolation, whole cell catalyst present in reaction mixture may be removed by any known type of filtration/flocculation/sedimentation/centrifugation procedure or further steps of the isolation are performed on whole cell containing reaction mixture. Preferably it is the object of this invention to omit the step of separating solid particles from the reaction mixture and perform direct extraction step immediately after completion of the reaction as described further on.

In one embodiment of the invention, the isolation of compound (I) may be carried out by adsorbtion to an adsorbent capable of binding compound (I) at significant levels and releasing compound (I) upon elution conditions such as replacement of the medium by a more nonpolar compound. Adsorbent may be selected from but not limited to: silica gel, zeolites, activated carbon, Amberlite™ XAD™ adsorbent resins, Amberlite™ and Amberlite™ FP ion exchange resins etc.

Alternatively liquid-liquid extraction may be carried out using water miscible solvents such as acetonitrile or methanol and supplementing the mixture with high concentration of salts, optionaly Sodium chloride in a so colled "salting out" extraction procedure. In this process separation of phases is observed and the compound (I) is preferentialy distributed in solvent rich phase.

In a preferred embodiment the extraction solvent for the liquid/liquid extraction is chosen from any a number of water immiscible or poorly miscible solvents. The solvent may be selected from but is not limited to: methylene chloride, diethyl ether, propionyl acetate, methyl t-butyl ether (MTBE), nitromethane, pentane, hexane, heptane, 1,2-dichloroethane, chloroform, carbon tetrachloride, n-butanol, n-pentanol, benzene, toluene, o-, m-, p-xylene, cyclohexane, petroleum ether, triethylamine. Prior the liquid/liquid extraction with chosen organic solvent the pH of water solution of the product may be adjusted to values between 1 and 9, preferably between 2 and 8, more preferably between 2 and 5. Drying of water residues in organic phase after extraction completion may be performed with but is not limited to adding salts listed: sodium sulfate, magnesium sulfate (monohydrate), calcium sulfate, calcium chloride, copper sulfate.

In most prefered embodiment of the purification process, the pH of the reaction mixture is first corrected to value from about 1 to 9, preferably from about 2 to 8, more preferably from about 2 to 5. Most preferably pH is corrected to about 3,5 using and alkaline compound such as NaOH, NaH₂CO₂ etc. Ethyl acetate is added at least 1 time by the addition of at least 1 volume of ethyl acetate to 1 volume of reaction mixture, preferably at lest 3 times by the addition of 1 volume of ethyl acetate to 1 volume of reaction mixture. Most preferably the steps of adding ethyl acetate and separationg the extract are carried out until no more than 5% of compound (I) is present in aqueous phase. Ethyl acetate fractions are collected, dried with any of the drying salts known in the art, preferably CaCl₂ or MgSO₄ or other methods of water stripping known in the art. In one aspect the obtained ethylacetate extract can be evaporated to yield isolated compound (I) in a form of yellow - amber oul at room temperature or can alternatively proceede into further steps in order tu yield pharmaceutically usefull compounds, preferably statins.

### Optional step f): Further processing

After obtaining the compound of formula (I), the compound of formula (I) can be further transformed to an API, preferably statin, or a pharmaceutically acceptable salt thereof, by subjecting said compound (I) to conditions sufficient to prepare the API, preferably statin. Thus, in an embodiment, a statin or salt, ester or stereoisomer thereof is prepared by (i) bringing in contact the compound of formula (II) as defined hereinabove with an enzyme capable of catalyzing oxidation or dehydrogenation, to prepare a compound of formula (I) as defined hereinabove, (ii) subjecting said compound (I) to conditions sufficient to prepare a statin; and (iii) optionally salifying, esterifying or stereoselectively resolving the product. Again, the compound of formula (II) can be prepared by using 2-deoxyribose-5-phosphate aldolase (DERA, EC 4.1.2.4) enzyme. The reaction setup can be arranged to introduce both enzymes substantially simultaneously or subsequently, at once or continuously, in one batch or in intermittent batches. Preferably the compounds of formula (II) and (I) are prepared at least in part simultaneously, more preferably substantially simultaneously. It is advantageous to use enzymes for preparing compounds of formula (I) and/or (II) in the case of enzymes, because the product immediately contains the correct spacious orientation of the substituents and no further purification or separation is needed. In the event that other stereoisomers are needed, the process can be combined with methods of stereospecific chemistry known to the skilled person. In preferred embodiment, the statin prepared is lovastatin, pravastatin, simvastatin, atorvastatin, cerivastatin, rosuvastatin, fluvastatin, pitavastatin, bervastatin, or dalvastatin, more preferably atorvastatin, rosuvastatin or pitavastatin, particularly is rosuvastatin.

The term "conditions sufficient to produce an API, preferably statin" as used herein refers to those means described in the art, including those means described herein for conversion of the compound of formula (I) further to the API, preferably statin. In a specific embodiment, where statin is prepared, the skilled person would choose the chemical route by selecting the proper R1 and R2, or R. In the event that R1, R2 and R are chosen to represent the statin skeleton, the compound of formula (I) is already a statin molecule ro an "advanced" intermediate thereof. Nevertheless, modifying the statin like for example by opening the lactone ring, forming the salt or the ester or resolving desired stereoisomers from the mixture of stereoisomers is also possible. In an alternative, if the statin is to be prepared by first providing a lactone and then coupling it to the statin skeleton, a reaction scheme 2 can be followed. After obtaining the compound of formula (I), its hydroxyl group in position 4 can be protected with the protecting group P (formula (XI)), which can be any conventionally used protecting group, in particular is silyl protecting group. Afterwards, the compound can be brought in the form of an aldehyde or its hydrate (formulas (XII) and (XIII), respectively). 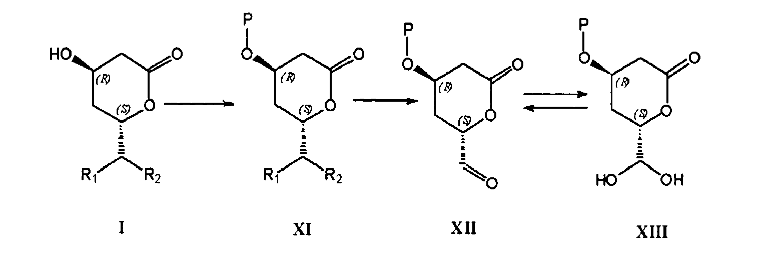 Compound of formula (XII), or hydrate thereof (XIII), obtained from I can be further used to prepare statin by reacting the compound of formula (XII), or hydrate thereof (XIII), under the condition of a Wittig coupling with a heterocylic or alicyclic derivative (statin skeleton) followed by hydrogenation when needed. In the specific embodiment related to preparing rosuvastatin, in the subsequent reaction step, (2*S*,4*R*)-4-(*P*-oxy)-6-oxo-tetrahydro-2*H*-pyran-2-carbaldehyde (XII) can be reacted under the conditions of a Wittig coupling (in the presence of a base) with a ((4-(4-fluorophenyl)-6-isopropyl-2-(N-methylmethylsulfonamido)pyrimidin-5-yl)methyl)triphenyl-phosphonium halide or any other ((4-(4-fluorophenyl)-6-isopropyl-2-(*N*-methylmethylsulfon-amido)pyrimidin-5-yl)methyl)phosphonium salt or alternatively di-*i*-propyl({4-(4-fluorophenyl)-6-isopropyl-2-[methyl(methylsulfonyl)amino]-5-pyrimidinyl}methylphosphonate or any other ({4-(4-fluorophenyl)-6-isopropyl-2-[methyl(methylsulfonyl)amino]-5-pyrimidinyl}methylphosphonate ester to give *N*-(5-((*E*)-2-((2*S*,4*R*)-4-(P-oxy)-6-oxo-tetrahydro-2*H*-pyran-2-yl)vinyl)-4-(4-fluorophenyl)-6-isopropylpyrimidin-2-yl)-*N*-methylmethanesulfonamide. As a base, lithium hexamethyldisilazane (LiHMDS), potassium hexamethyldisilazane (KHMDS), sodium hexamethyldisilazane (NaHMDS), lithium diisopropylamide (LDA), sodium hydride, butyllithium or Grignard reagents, preferably sodium hexamethyldisilazane may be used. When the source is the hydrate form XIII or a mixture of XII and the hydrate form XIII thereof, which is dissolved in ethers selected from THF, Et₂O, *i*-Pr₂O, *^{t}*BuMeO; hydrocarbons selected from: pentane, hexane, cyclohexane, methylcyclohexane, heptane; aromatic hydrocarbons selected from toluene or the chlorinated derivatives thereof; chlorinated hydrocarbons selected from: chloroform and dichloromethane or in mixtures of those solvents, water released from the hydrate should be removed prior to the addition to the formed ylide solution. The preferred solvents for the reaction are anhydrous toluene and dichloromethane. The reaction can be performed at temperatures between -80 °C and 90 °C preferably at 0 to 90 °C, more preferably at 80 - 90 °C. The reaction is accomplished in 1 - 12 hours. Isolation of the crude product with extraction can be performed with AcOEt, ethers or alkanes, preferably with *^{t}*BuMeO. The protecting group may be removed and the lactone opened to produce a rosuvastatin free acid or a salt thereof, optionally an amine, which may be converted to hemicalcium salt. The deprotection can be performed at temperatures between 0 °C to 80 °C. Preferably at 20 or 40 °C in a suitable solvent, preferably a solvent selected from alcohols, acetic acid, THF, acetonitrile, methyltetrahydrofuran, dioxane, CH₂Cl₂, more preferably in alcohols and a mixture of THF/AcOH. The usual deprotecting reagents may be used, such as tetra-*n*-butylammonium fluoride, ammonium fluoride, AcCl, FeCl₃, TMSCl/HF·2H₂O, chloroethylchloroformate (CEC), Ph₃PCH₂COMeBr. The opening of the lactone preferably takes place in a 4:1 to 2:1 mixture of THF/H₂O as well as in pure THF at temperatures between 20 °C to 60 °C with a suitable alkali such as NaOH, KOH, ammonia or amines. The hydrolysis is accomplished in 30 minutes (at 60 °C) to 2 hours (at 20 °C). After the hydrolysis step, evaporation of THF can be conducted at temperatures between 10 °C to 50 °C under the reduced pressure, and conversion to the calcium salt, preferably by the addition of Ca(OAc)_{2·}×H₂O, which can be added in one portion or dropwise in 5 to 60 minutes, can be performed at temperatures between 0 °C to 40 °C. After the addition of Ca(OAc)_{2·}×H₂O, the resulting suspension can be stirred at temperatures between 0 °C to 40 °C from 30 minutes to 2 hours. The details of such reaction are known in the art, including in, but not limited to, WO2008/119810.

The API, preferably statin, obtained by any of the aforementioned embodiments, can be formulated in a pharmaceutical formulation. The methods for preparing a pharmaceutical formulation with the API, preferably statin, are known to the person skilled in the art. Generally, one can chose among preparing formulations such as powder, granulate, tablet, capsule, suppository, solution, ointment, suspension, foam, patch, infusion, solution for injection, or the like. The formulation can be changed in order to modify specific aspects of the API like for example release, stability, efficacy or safety. Depending on the API, the skilled person knows how to select proper administration route for said API. Based on that, he can choose proper formulation. Then, the skilled person is in a position to select from the excipients needed for formulating the pharmaceutical formulation. Besides the API, which can be optionally combined with at least one another API, the skilled person can select from excipients and additives to formulate the pharmaceutical formulation. Suitable excipients may be, for example, binder, diluent, lubricant, disintegrant, filler, glidant, solvent, pH modifying agent, ionic strength modifying agent, surfactant, buffer agent, anti-oxidant, colorant, stabilizer, plasticizer, emulsifier, preservatives, viscosity-modifying agent, passifier, flavouring agent, without being limited thereto, which can be used alone or in combination. The method can involve, depending on the selected formulation, mixing, grinding, wet granulation, dry granulation, tabletting, dissolving, lyophilisation, filling into capsules, without being limited thereto.

### Application to APIs and intermediates thereof

In further aspects of the present invention, an enzyme capable of catalyzing oxidation or dehydrogenation can be generally used for preparing an API or intermediate thereof being compatible with the enzymatic system(s) disclosed herein.

This aspect of the invention preferably relates to synthetic API or intermediate thereof respectively categorisable as substituted or unsubstituted dideoxyaldose sugars, lactols (optionally containing multiple hydroxyl groups) and synthetic non-natural alcohols as possible substrates, and (optionally further hydroxylated) lactons or esters as possible products. From the disclosure of the present invention and its technical character, it will be understood that compounds naturally occurring or arising in the biochemical pathways of sugars (e.g. glycolysis, pentose phosphate pathway, glycogenesis), fatty acids, or cellular respiratory chain, would be exempted from being regarded as either substrate for the enzyme capable of catalyzing oxidation or dehydrogenation or the API or intermediate thereof according to the present invention. In the same manner, naturally occurring amino acids, vitamins or cofactors are also exempted from the definition of the API or intermediate thereof according to the present invention. The substrates or products of such pathways in the nature include methanol, ethanol, formaldehyde, acetaldehyde, methanoic, acetic acid, monosaccharide, disaccharide, trisaccharide, glucuronic acid, especially methanol, ethanol, formaldehyde, acetaldehyde, methanoic, acetic acid, monosaccharide, glucuronic acid, and particularly ethanol, acetic acid and monosaccharide. In a preferred embodiment, the enzyme capable of catalyzing oxidation or dehydrogenation is used for preparing the compound of formula (I), wherein the formula (I) is as defined hereinabove. In this aspect, it is the most efficient to use the enzyme to act upon the compound of formula (II), wherein the formula (II) is as defined hereinabove. Specific alternatives of the use will be immediately apparent to the skilled person when other embodiments, aspects, or preferred features of the invention disclosed hereinabove are taken into account.

To give an illustrative further example, a possible useful further definition of the substrate and thus starting compound useful for the preparation of appropriate APIs or intermediates thereof is given by the following formula XIV, hence leading to a corresponding product compound defined by formula XV. 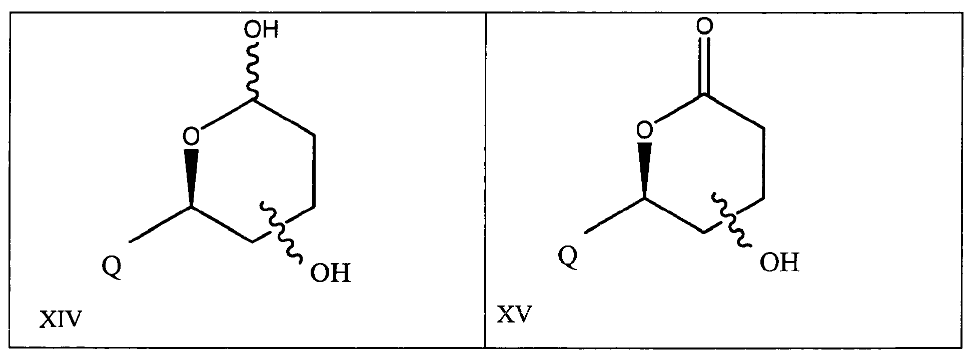 wherein Q is any desired structural moiety, for example selected from the groups of R1, R2 and R5 defined hereinabove, optionally with an intermediate linker molecule between R1, R2 or R5 and the lactol/lactone ring. As shown in the structural formulae, the non-lactol hydroxyl group not being oxidyzed can be positioned at any position of the lactol/lactone ring.

In a preferred embodiment, the enzyme capable of catalyzing oxidation or dehydrogenation is used for preparing an API or intermediate thereof simultaneously or subsequently with a DERA enzyme.

### Examples

The following examples are merely illustrative examples of the present invention and they should not be considered a limiting the scope of the invention in any way, as these examples and other equivalents thereof will become apparent o those versed in the art in the light of the present disclosure, and the accompanying claims.

### Example 1: Preparation of aldose dehydrogenases comprised within the whole cell

Several aldose dehydrogenases have been prepared as descried in the following:
First, a water-soluble aldose dehydrogenase, a pyrroloquinoline quinone (PQQ) dependent dehydrogenase encoded by gene ylil (E. coli GeneBank # ECK0827, locus tag b0837) was prepared. For oxidation studies said periplasmic enzyme found in *Escherichia coli* was used.

The genomic DNA from *E. coli* DH5α was isolated using Wizard Genomic DNA Purification Kit (Promega, Madison, WI, USA) according to manufacturer's instructions. Isolation of genomic DNA was made using overnight culture of *E. coli* grown on LB medium at 37 °C. Amplification of gene *ylil* was performed by PCR using oligonucleotide primers GCGCCATATGCATCGACAATCCTTT (SEQ ID NO. 71) and GCGCGCTCAGGCTAATTGCGTGGGCTAACTTTAAG (SEQ ID NO. 72). Amplification was performed by *Pfx*50 DNA polymerase (Invitrogen, Calsbad, CA, USA) as follows: an initial denaturation at 94 °C for 10 min, followed by 30 cycles of 45 s at 94 °C, 45 s at 52 °C, and 150 s at 68 °C. Final elongation was performed 420 s at 68 °C. A 1.2-kb DNA fragment containing *ylil* (SEQ ID NO. 01) was separated by agarose gel electrophoresis and purified. The product was ligated into plasmid pGEM T-Easy (Promega, Madison, WI, USA) in a T4 ligase reaction. The plasmid construct was cleaved with restriction endonucleases *Nde*I and *Sal*I, the resulting fragments were separated on agarose gel electrophoresis and 1.2 kb fragment containing *ylil* was purified. An expression vector pET30a(+) (Novagen Inc., Madison, WI, USA) was cleaved using the same aforementioned restriction endonucleases and purified. The 1.2 kb fragment containing *ylil* gene was assembled with the cleaved expression vector in a T4 ligase reaction. *E. coli* JM109 cells were transformed with the obtained ligation reaction and kanamycin resistant colonies were cultured. Afterwards plasmid DNA was isolated. The resulting construct was designated pET30/Ylil and sequenced for confirmation of the gene sequence. The cloning procedure was performed to allow expression of protein having sequence (SEQ ID NO. 02) including leader sequence for Ylil translocation to periplasm. The aldose dehydrogenase expressing organism was prepared by transforming BL21 (DE3) competent cells with said plasmid.

Expression of aldose dehydrogenase (Procedure 1A): VD medium (30 mL; 10 g/L bacto yeast extract, 5 g/L glycerol, 5 g/L NaCl, 4 g/L NaH2PO4·2H2O, pH was adjusted with 1 M NaOH to pH=7.0) supplemented with kanamycin (25 µg/mL) was inoculated with a single colony *E. coli* BL21 DE(3) pET30/Ylil from a freshly streaked VD agar plate and pre-cultured to late log phase (37 °C, 250 rpm, 8 h).

The pre-culture cells (inoculum size 10 %, v/v) were then transferred to fresh VD medium (100 mL; 10 g/L bacto yeast extract, 5 g/L glycerol, 5 g/L NaCl, 4 g/L NaH2PO4·2H2O, pH was adjusted with 1 M NaOH to pH=7.0) supplemented with kanamycin (25 µg/mL). For the induction of protein expression 0.1 mM isopropyl-β-D-thiogalactopyranoside (IPTG, purchased by Sigma Aldrich, Germany) was added. The cells were cultured in a rotary shaker at 25 °C, 250 rpm for 16 h.

Preparation of living whole cell catalysts (Procedure 1 B): After expression (as described in Procedure 1A) cells were harvested by centrifugation (10 000 g, 5 min, 4 °C). Supernatant was discarded after centrifugation and pellet was resuspended in fresh VD medium (10 g/L bacto yeast extract, 5 g/L glycerol, 5 g/L NaCl, 4 g/L NaH2PO4·2H2O, pH was adjusted with 1M NaOH to pH=6.0). Cells were resuspended in one tenth of initial culture volume. The aldose dehydogenase comprised within the living whole cell catalysts was thus obtained in fresh medium.

Alternatively (Procedure 1C), after discarding supernatant (in regard to Procedure 1A) the pellet was resuspended in phosphate buffer (50 mM KH₂PO₄, 150 mM NaCl, pH 6.0). Cells were resuspended in one tenth of initial culture volume. The aldose dehydrogenase expressed in periplasm comprised within the resting whole cell catalysts was thus obtained. Additionally, a construct encoding *ylil* gene without the leader sequence for translocation to periplasm was prepared. Such protein is translated and expressed in cytoplasm.

The genomic DNA from *E. coli* DH5α was isolated using Wizard Genomic DNA Purification Kit (Promega, Madison, WI, USA) according to manufacturer's instructions. Isolation of genomic DNA was made using overnight culture of *E. coli* grown on LB medium at 37 °C. Amplification of gene *ylil* was performed by PCR using oligonucleotide primers GCGCCATATGGCTCCTGCAACGGTAAAT (SEQ ID NO. 73) and GCGCGCTCAGGCTAATTGCGTGGGCTAACTTTAAG (SEQ ID NO. 72). Amplification was performed by *Pfx*50 DNA polymerase (Invitrogen, Calsbad, CA, USA) as follows: 94 °C for 10 min, followed by 30 cycles of 45 s at 94 °C, 45 s at 50 °C, and 150 s at 68 °C. Final elongation was performed 420 s at 68 °C. A 1.2-kb DNA fragment containing *ylil* (SEQ ID NO. 03) was separated by agarose gel electrophoresis and purified. The product was ligated into plasmid pGEM T-Easy (Promega, Madison, WI, USA) in a T4 ligase reaction. The plasmid construct was cleaved with restriction endonucleases *Nde*I and *Sal*I, the resulting fragments were separated on agarose gel electrophoresis and 1.2 kb fragment containing *ylil* was purified. An expression vector pET30a(+) (Novagen Inc., Madison, WI, USA) was cleaved using the same aforementioned restriction endonucleases and purified. The 1.2 kb fragment containing *yli*l gene was assembled with the cleaved expression vector in a T4 ligase reaction. *E. coli* JM1 09 cells were transformed with the obtained ligation reaction and kanamycin resistant colonies were cultured. Afterwards plasmid DNA was isolated. The resulting construct was designated pET30/YliI_C and sequenced for confirmation of the gene sequence. The cloning procedure was performed with a target to provide protein Ylil expressed in cytoplasm with coding sequence (SEQ ID NO. 04) The aldose dehydrogenase expressing organism was prepared by transforming BL21 (DE3) competent cells with said plasmid. The procedure of expression of *E. coli* Ylil_C was undertaken as described in Procedure 1A. After expression cells were harvested and whole cell catalysts were obtained as described in Procedure 1B to obtain living whole cell catalysts. To obtain resting whole cell catalysts Procedure 1C was undertaken.

Another water-soluble aldose dehydrogenase, found in *Acinetobacter calcoaceticus*, was used for alternative oxidation studies. A nucleotide sequence encoding a gene and leader sequence for transportation to periplasm (PQQ GdhB, *A. calcoaceticus* GeneBank #X15871) was optimized for expression in *E. coli* and DNA was chemically synthesized (Geneart, Regensburg, Germany) (SEQ ID. NO. 5). *E. coli* JM109 cells were transformed with artificial plasmid bearing nucleotide sequence *gdhB* and kanamycin resistant colonies were cultured. Afterwards plasmid DNA was isolated and the construct was cleaved with restriction endonucleases *Nde*I and *Hin*dIII, the resulting fragments were separated on agarose gel electrophoresis and 1.5 kb fragment was purified (SEQ ID NO. 05). An expression vector pET30a(+) (Novagen Inc., Madison, WI, USA) was cleaved using the same aforementioned restriction endonucleases and purified. The 1.5 kb fragment containing *gdhB* gene was assembled in the cleaved expression vector in a T4 ligase reaction. *E*. *coli* JM109 cells were transformed with the obtained ligation reaction and kanamycin resistant colonies were cultured. Afterwards plasmid DNA was isolated. The resulting construct was designated pET30/GdhB and sequenced for confirmation of the gene sequence. The cloning procedure was performed with a target to allow expression of protein having sequence SEQ ID NO. 06. The aldose dehydrogenase from *A. calcoaceticus* expressing organism was prepared by transforming BL21 (DE3) competent cells with said plasmid.
The procedure of expression of *E*. *coli* GdhB was undertaken as described in Procedure 1A. After expression cells were harvested and whole cell catalyst was obtained as described in Procedure 1B to obtain living whole cell catalysts. To obtain resting whole cell catalyst Procedure 1C was undertaken.

Yet another water-soluble aldose dehydrogenase found in *Acinetobacter calcoaceticus* (PQQ GdhB, *A. calcoaceticus* GeneBank #X15871) with altered sequence and hence its increased thermal stability properties was used [Igarashi, 2003]. A nucleotide sequence encoding a gene and leader sequence for transportation to periplasm (PQQ GdhB, *A. calcoaceticus* GeneBank #X15871) was optimized for expression in *E. coli* and DNA was chemically synthesized (Geneart, Regensburg, Germany) (SEQ ID NO. 07).

*E*. *coli* JM109 cells were transformed with artificial plasmid bearing nucleotide sequence *gdhB_therm* and ampicillin resistant colonies were cultured. Afterwards plasmid DNA was isolated and the construct was cleaved with restriction endonucleases *Nde*I and *Hin*dIII, the resulting fragments were separated on agarose gel electrophoresis and 1.5 kb fragment was purified (SEQ ID NO. 07). An expression vector pET30a(+) (Novagen Inc., Madison, WI, USA) was cleaved using the same aforementioned restriction endonucleases and purified. The 1.5 kb fragment containing *gdhB_therm* gene was assembled in the cleaved expression vector in a T4 ligase reaction. *E*. *coli* JM109 cells were transformed with the obtained ligation reaction and kanamycin resistant colonies were cultured. Afterwards plasmid DNA was isolated. The resulting construct was designated pET30/GdhB_therm and sequenced for confirmation of the gene sequence. The alternative aldose dehydrogenase expressing organism was prepared by transforming BL21 (DE3) competent cells with said plasmid. The cloning procedure was performed with a target to allow expression of protein having sequence SEQ ID NO. 08. SEQ ID NO. 08 which compared to SEQ ID NO 06 has altered sequence coding Ser residue at position 231 to Lys residue.

The procedure of expression of *E. coli* GdhB_therm was undertaken as described in Procedure 1A. After expression cells were harvested and whole cell catalysts were obtained as described in Procedure 1B to obtain living whole cell catalysts. To obtain resting whole cell catalysts Procedure 1C was undertaken.

Where stated, *E*. *coli* BL21(DE3) having an expression plasmid vector pET30a(+) (Novagen Inc., Madison, WI, USA) was used as negative control. The control cells were prepared by transforming *E. coli* BL21 (DE3) competent cells with said plasmid and kanamycin resistant colonies were cultured. The procedure of cultivation of *E. coli* BL21(DE3) pET30 was undertaken regarding to Procedure 1A. After expression cells were harvested and negative control cells were obtained as described in Procedure 1B to obtain negative control for living whole cell catalysts. To obtain negative control cells for resting whole cell catalysts Procedure 1C was undertaken.

### Example 2: Preparation of aldose dehydrogenase comprised within cell free lysate

To obtain cell free lysate after expression of *E*. *coli* Ylil, *E. coli* Ylil_C, *E. coli* GdhB or *E. coli* GdhB_therm (as described in Example 1, more particularly after undertaking Procedure 1A) cells were harvested by centrifugation (10 000 g, 5 min, 4°C). Supernatant was discarded after centrifugation and pellet was resuspended in phosphate buffer (50 mM KH₂PO₄, 150 mM NaCl, pH 7.0) in one tenth of initial volume. Cells were supplemented with 1 mg/mL lysozyme solution. Lysis was performed at 37°C, 1h. After lysis cell debris were removed by sedimentation (10 min, 20 000 g, 4 °C) to obtain a clear aqueous solution. Aldose dehydrogenase comprised within a cell free extract was thus obtained.

Alternatively the pellet was resuspended in a lytic buffer (50 mM NaH2PO4, pH 7.0, 300 mM NaCl, 2 mM DTT) using 200 g of pellet per 1L of said buffer. Cells were sonified (3 x 15 s) using Branson digital sonifier and cell debris were removed by sedimentation (10 min, 20 000 g, 4 °C) to obtain a clear aqueous solution. Aldose dehydrogenase comprised within a cell free extract was thus obtained.

In the same manner as above, cell free lysates were prepared from whole cell *Klyuvera intermedia* and *Gluconobacter oxydans* (cultivations and preparations of whole cell catalysts are described in Example 9 and Example 10, respectively).

Culture of *E*. *coli* BL21(DE3) pET30, used as negative control was treated by the same procedures.

### Example 3: Preparation of periplasmic cell fraction containing aldose dehydrogenase

To obtain specific release of periplasmic proteins after expression of *E*. *coli* Ylil, *E*. *coli* GdhB or *E*. *coli* GdhB_therm (as described in Example 1, more particularly after undertaking Procedure 1A) the following procedure was used. Cells from freshly expressed culture were pelleted by centrifugation and the growth medium was completely removed. The cell pellet was washed three times in 20 mM Tris-HCl (pH 7.5). The cell pellet was then centrifuged (10 000 g, 5 min, 4 °C) before resuspended in one tenth of initial culture volume of hypertonic solution containing 20 mM Tris HCl (pH 7.5), 20 % sucrose, and 0.5 mM EDTA. The cell suspension was incubated on ice for 10 min. After centrifugation (10 min, 12 000 g, 4 °C) cell pellet was resuspended gently by pipetting into hypotonic solution (50 mM Tris-HCl pH 7.0) in the same volume as hypertonic solution. Cells were incubated on ice for additional 10 min. Cells were pelleted by centrifugation (10 min, 20 000 g, 4 °C) and the supernatant was removed and labelled as periplasmic fraction.

Culture of *E*. *coli* BL21(DE3) pET30, used as negative control was treated by the same procedure.

### Example 4: Preparation of deoxyribose-5-phosphate aldolase enzyme (DERA) comprised within the whole cell

The aldolase gene *deoC* (*E*. *coli* GeneBank #EG10221, locus tag b4381) comprised within whole cell catalyst can be obtained by a number of procedures, for example as described in WO2009/092702. Nevertheless, we provide a nonlimiting example of preparation of aldolase enzyme (DERA) comprised within the whole cell.

The genomic DNA from *E*. *coli* DH5α was isolated using Wizard Genomic DNA Purification Kit (Promega, Madison, WI, USA) according to manufacturer's instructions. Isolation of genomic DNA was made using overnight culture of *E*. *coli* grown on LB medium at 37 °C. Amplification of gene *deoC* was performed by PCR using oligonucleotide primers CCGGCATATGACTGATCTGAAAGCAAGCAG (SEQ ID NO. 74) and CCGCTCAGCTCATTAGTAGCTGCTGGCGCTC (SEQ ID NO. 75). Amplification was performed by *Pfx*50 DNA polymerase (Invitrogen, Calsbad, CA, USA) as follows: an initial denaturation at 95°C for 10 min, followed by 30 cycles of 45 s at 94°C, 45 s at 60°C, and 60 s at 68°C. Final elongation was performed 420 s at 68°C. The resulting fragments were separated by agarose gel electrophoresis and purified. A 0.9-kb DNA fragment containing *deoC* (SEQ ID NO. 09) was separated by agarose gel electrophoresis and purified. The product was ligated into plasmid pGEM T-Easy (Promega, Madison, WI, USA) in a T4 ligase reaction. Thus obtained plasmid construct was cleaved with restriction endonucleases *Nde*I and *Blp*I, the resulting fragments were separated on agarose gel electrophoresis and 0.9 kb fragment containing *deoC* was purified. An expression vector pET30a(+) (Novagen Inc., Madison, WI, USA) was cleaved using the same aforementioned restriction endonucleases and purified. The 0.9-kb fragment containing *deoC* gene was assembled with the cleaved expression vector in a T4 ligase reaction. *E*. *coli* JM109 cells were transformed with the obtained ligation reaction and kanamycin resistant colonies were cultured. Afterwards plasmid DNA was isolated. The resulting construct was designated pET30/DeoC and sequenced for confirmation of the gene sequence. The cloning procedure was performed with a target to allow expression of protein having sequence SEQ ID NO. 10. The DERA aldolase expressing organism was prepared by transforming BL21 (DE3) competent cells with said plasmid.

Expression of *deoC*: VD medium (50 mL; 10 g/L bacto yeast extract, 5 g/L glycerol, 5 g/L NaCl, 4 g/L NaH₂PO₄·2H₂O, pH=7.0) supplemented with kanamycin (25 µg/mL) was inoculated with a single colony *E*. *coli* BL21 DE(3) pET30/DeoC from a freshly streaked plate and cultured overnight (37 °C, 250 rpm).

The procedure of expression of *E*. *coli* DeoC was undertaken as described in Procedure 1A, with a sole difference of IPTG inducer concentration being 0.5 mM. After expression cells were harvested and whole cell catalysts were obtained as described in Procedure 1B to obtain living whole cell catalysts. To obtain resting whole cell catalysts Procedure 1C was undertaken.

### Example 5: Preparation of aldolase enzyme (DERA) and aldose dehydrogenase enzyme comprised within a whole cell culture of a single microorganism

A gene *ylil* having additional ribosomal binding site (RBS) was added into the construct pET30/DeoC resulting in construct bearing two different coding sequences organised in a single operon and under transcriptional control of IPTG inducible promoter.

The plasmid DNA from *E*. *coli* JM109 pET30/Ylil (as described in Example 1) was isolated using Wizard Plus SV Minipreps DNA Purification Kit (Promega, Madison, WI, USA) according to manufacturer's instructions. Isolation of plasmid DNA was made using overnight culture of *E*. *coli* grown on LB medium at 37°C. The said isolated plasmid pET30/Ylil was used as a template in a PCR reaction to amplify sequence containing gene *ylil* and RBS derived from expression vector pET30a(+) upstream of the coding region. PCR reaction was performed using oligonucleotide primers GCAGGCTGAGCTTAACTTTAAGAAGGAGATATACATATG (SEQ ID NO. 76) and GCGCGCTCAGCCTAATTGCGTGGGCTAACTTTAAG (SEQ ID NO. 77). Amplification of this fragment was performed by PCR using *Pfu* ULTRA II Fusion HS DNA 200 polymerase (Agilent, Santa Clara, CA, USA) as follows: an initial denaturation at 98°C for 3 min, followed by 30 cycles of 20 s at 98°C, 20 s at 55°C, and 90 s at 72°C. Final elongation was performed 180 s at 72°C. The resulting fragments were separated by agarose gel electrophoresis and 1.2 kb fragment containing *ylil* and RBS site was purified. The product was transferred to plasmid pGEM T-Easy (Promega, Madison, WI, USA) and designated pGEM/RBS_ylil. The construct was cleaved with restriction endonuclease *Blp*I, then the resulting fragments were separated on agarose gel electrophoresis and 1.2 kb fragment containing *ylil* and RBS site was purified.

The construct pET30a/DeoC (construction is described in Example 4) was cleaved using the same aforementioned restriction endonuclease (*Blp*I) and purified. Shrimp Alkaline Phosphatase (SAP, purchased by Promega, Madison, WI, USA) was used to dephosphorylate the 5' phosphorylated ends of cleaved pET30a/DeoC according to manufacturer's instructions. Thus self-ligation of pET30a/DeoC was prevented. The fragments were assembled in a T4 ligase reaction. *E*. *coli* JM109 cells were transformed with the obtained ligation reaction and kanamycin resistant colonies were cultured and plasmid DNA was isolated. The resulting construct was designated pET30/DeoC_RBS_Ylil and sequenced for confirmation of the gene sequences. The organism expressing DERA aldolase and aldose dehydrogenase was prepared by transforming BL21 (DE3) competent cells with said plasmid.

Expression of *E. coli* Ylil and DERA comprised within a single whole cell culture was undertaken as described in Procedure 1A. Corresponding to Procedures 1B and 1C living whole cell catalysts and resting whole cell catalysts, respectively, were prepared.

### Example 6: Preparation of E. coli able to synthesize pyrroloquinone quinone (PQQ)

*Gluconobacter oxydans* (ATCC 621H) gene cluster *pqqABCDE* (*pqqA, pqqB, pqqC, pqqD, pqqE,* GeneBank #CP000009, approximate location in the genome 1080978...1084164), which is involved in pyrroloquinoline quinone (PQQ) biosynthesis was expressed in *E*. *coli.*

The genomic DNA from *Gluconobacter oxydans* (ATCC 621H) was isolated using Wizard Genomic DNA Purification Kit (Promega, Madison, WI, USA) according to manufacturer's instructions. Isolation of genomic DNA was made using culture of *G. oxydans* grown on mannitol medium (10 g/L bacto yeast extract, 3 g/L peptone, 5 g/L mannitol, pH 7.0) 48 h at 26°C. The said isolated genome was used as template for amplification of gene cluster *pqqABCDE* and its own promoter. Amplification was performed by PCR using oligonucleotide primers GCGCGGTACCGCACATGTCGCGGATGTTCAGGTGTTC (SEQ ID NO. 78) and GCGCGGATCCGGGCGGAGAGTTTGGAGAACCTCTTCA (SEQ ID NO. 79) and using *Pfu* ULTRA II Fusion HS DNA 200 polymerase (Agilent, Santa Clara, CA, USA) as follows: an initial denaturation at 95°C for 2 min, followed by 30 cycles of 20 s at 95°C, 20 s at 65 °C, and 120 s at 72 °C. Final elongation was performed 180 s at 72 °C. A 3.4-kb DNA fragment of *G. oxydans* bearing the *pqqABCDE* operon and parts of the upstream and downstream sequences was separated by agarose gel electrophoresis and purified. The 3.4-kb product (SEQ ID NO. 11) was ligated to plasmid pGEM T-Easy (Promega, Madison, WI, USA). *E*. *coli* JM109 cells were transformed with the obtained ligation reaction and ampicillin resistant colonies were cultured and plasmid DNA was isolated. The resulting plasmid construct was designated pGEMpqqA-E and sequenced for confirmation of the gene sequences. The cloning procedure was performed with a target to allow expression of genes under control of their native promoters and thus production of proteins PqqA, PqqB, PqqC, PqqD and PqqE derived from *G*. *oxidans* (SEQ ID NO. 12, 13, 14, 15, 16, respectively).

Alternatively, *Kluyvera intermedia* (ATCC 33421, formerly *Enterobacter intermedium*) gene cluster *pqqABCDE* (*pqq*A*, pqq*B*, pqq*C*, pqq*D*, pqq*E*,* GenBank: AY216683.1), which is involved in pyrroloquinoline quinone (PQQ) biosynthesis was expressed in *E*. *coli.*

The genomic DNA from *Kluyvera intermedia* (ATCC 33421) was isolated using Wizard Genomic DNA Purification Kit (Promega, Madison, WI, USA) according to manufacturer's instructions. Isolation of genomic DNA was made using culture of *K. intermedia* grown on VD medium overnight at 37°C. The said isolated genome was used as template for amplification of gene cluster *pqqABCDE* and its own promoter. Amplification was performed by PCR using oligonucleotide primers GCGCGGATCCACGCAGCATCGGGCCGTTCT (SEQ ID NO. 80) and GCGCGGTACCAAGCTTCGCTGCCGCAAAACA (SEQ ID NO. 81) and using *Pfu* ULTRA II Fusion HS DNA 200 polymerase (Agilent, Santa Clara, CA, USA) as follows: an initial denaturation at 94°C for 7 min, followed by 30 cycles of 45 s at 94°C, 45 s at 65 °C, and 480 s at 72 °C. Final elongation was performed 180 s at 72 °C. A 4.3-kb DNA fragment containing the *pqqABCDE* operon from *K. intermedia* was separated by agarose gel electrophoresis and purified. The 4.3-kb product (SEQ ID NO. 17) was ligated to plasmid pGEM T-Easy (Promega, Madison, WI, USA). *E*. *coli* JM109 cells were transformed with the obtained ligation reaction and ampicillin resistant colonies were cultured and plasmid DNA was isolated. The resulting plasmid construct was designated pGEM/KI_pqqA-E and sequenced for confirmation of the gene sequences.

The cloning procedure was performed with a target to allow expression of genes under control of their native promoters and thus production of proteins PqqA, PqqB, PqqC, PqqD and PqqE derived from *K*. *intermedia* (SEQ ID NO. 18, 19, 20, 21, 22, respectively).

Synthesis of PQQ in *E*. *coli*: (Procedure 6D): LB medium (30 mL; 20 g/L Bacto LB broth) supplemented with ampicillin (100 µg/mL) was inoculated with a single colony *E*. *coli* JM109 pGEM/pqqA-E from a freshly streaked plate and pre-cultured to late log phase (37 °C, 250 rpm, 8 h). The pre-culture cells were pelleted by centrifugation (10 000 g, 10 min) and washed three times with 50 mM phosphate buffer (pH 7.0). Pre-culture cells were resuspended in the same aforementioned buffer at the same volume as in the original pre-culture. The suspension (inoculum size 5%, v/v) was then transferred to glucose minimal medium (100 mL, 5 g/L D-glucose, 2 g/L sodium citrate 10 g/L K2HP04, 3.5 g/L (NH4)2SO4, pH 7.0). The culture was grown at 37°C, 250 rpm, 48 h. The cell culture was supplemented with 1 mg/mL lysozyme solution. Lysis was performed at 37°C, 1 h. After lysis cell debris were removed by sedimentation (10 min, 20 000 g, 4°C) to obtain a clear aqueous solution. PQQ comprised within a cell free extract was thus obtained.

Method for confirming PQQ production in *E*. *coli* is described in Example 13.

### Example 7: Preparation of aldose dehydrogenase enzyme comprised within a whole cell culture of a single microorganism able to synthesize pyrroloquinone quinone (PQQ)

The expression plasmid bearing gene *yli*l and gene cluster *pqqA*-E was constructed.

Construct pET301Ylil (preparation is described in Example 1) was digested with restriction endonuclease *Sph*I. Shrimp alkaline phosphatase (SAP, purchased by Promega, Madison, WI, USA) was used to dephosphorylate the 5' phosphorylated ends of cleaved pET30a/Ylil according to manufacturer's instructions. Thus self-ligation of pET30a/Ylil was prevented. A double stranded linker GCGCAAGCATGCGGATCCGGTACCAAGCTTGCATGCACACTA (SEQ ID NO. 82) (ordered at Invitrogen, Calsbad, CA, USA) containing double restriction recognition sites *Sph*I and recognition sites for restriction endonucleases *Bam*HI, *Kpn*I and *Hin*dIII was following cleaving with *Sph*I introduced into the *Sph*I site on the digested construct pET30/Ylil.

The cleaved linker and cleaved construct were assembled in a T4 ligase reaction. The introduction of the linker inserts additional restriction endonucleases recognition sites into the plasmid. The construct pGEM/pqqA-E (according to Example 6) was cleaved with restriction endonucleases *Bam*HI and *Kpn*I, then the resulting fragments were separated on agarose gel electrophoresis and 3.4 kb fragment containing gene operon *pqqABCDE* was purified. Construct pET30/Ylil with linker was cleaved using the aforementioned restriction endonucleases and purified. The fragments (cleaved pET30/Ylil with linker and *pqq*A-E, respectively) were assembled in a T4 ligase reaction. *E. coli* JM109 cells were transformed with the obtained ligation reaction and kanamycin resistant colonies were cultured and plasmid DNA was isolated. The resulting plasmid construct was designated pET30/Ylil+pqqA-E and sequenced for confirmation of the gene sequences.

The organism able to express aldose dehydrogenase and meanwhile synthesize pyrroloquinone quinine was prepared by transforming BL21 (DE3) competent cells with said plasmid.

Expression of *E*. *coli* Ylil and comprised within a whole cell culture of a single microorganism able to produce PQQ was undertaken as described in Procedure 1A. Corresponding to Procedures 1B and 1C living whole cell catalyst and resting whole cell catalyst, respectively, were prepared. Cell free lysate was prepared following procedure described in Example 2.

Method for confirming oxidation capability and PQQ production in *E. coli* is described in Example 13.

### Example 8: Preparation of aldolase enzyme (DERA) and aldose dehydrogenase enzyme comprised within a whole cell culture of a single microorganism able to synthesize pyrroloquinone quinone (PQQ)

An artificial construct possessing genes *deo*C, *yli*l and *pqq* operon was constructed. Construct pET30/DeoC (preparation is described in Example 4) was digested with restriction endonuclease *Sph*I*.* Shrimp alkaline phosphatase (SAP, purchased by Promega, Madison, WI, USA) was used to dephosphorylate the 5' phosphorylated ends of cleaved pET30a/DeoC according to manufacturer's instructions. Thus self-ligation of pET30a/DeoC was prevented. A double stranded linker GCGCAAGCATGCGGATCCGGTACCAAGCTTGCATGCACACTA (SEQ ID NO. 82) (ordered at Invitrogen, Calsbad, CA, USA) containing double restriction recognition sites *Sph*I and recognition sites for restriction endonucleases *Bam*HI, *Kpn*I and *Hind*III was following cleaving with *Sph*I introduced into the *Sph*I site on the digested construct pET30/DeoC.

The cleaved linker and cleaved construct were assembled in a T4 ligase reaction. The introduction of the linker inserts additional restriction endonucleases recognition sites.

The construct pGEM/RBS_ylil (Example 5) was cleaved with restriction endonuclease *Blp*I, then the resulting fragments were separated on agarose gel electrophoresis and 1.2 kb fragment containing *ylil* and RBS site was purified.

The aforementioned construct pET30a/DeoC with linker was cleaved using the same restriction endonuclease (*Blp*I) and purified. Shrimp alkaline phosphatase (SAP, purchased by Promega, Madison, WI, USA) was used to dephosphorylate the 5' phosphorylated ends of cleaved plasmid construct pET30a/DeoC with linker according to manufacturer's instructions. Thus self-ligation of pET30a/DeoC with linker was prevented. The fragments were assembled in a T4 ligase reaction. E. coli JM109 cells were transformed with the obtained ligation reaction and kanamycin resistant colonies were cultured and plasmid DNA was isolated. Intermediate construct was designed pET30/DeoC_RBS_Ylil with linker.

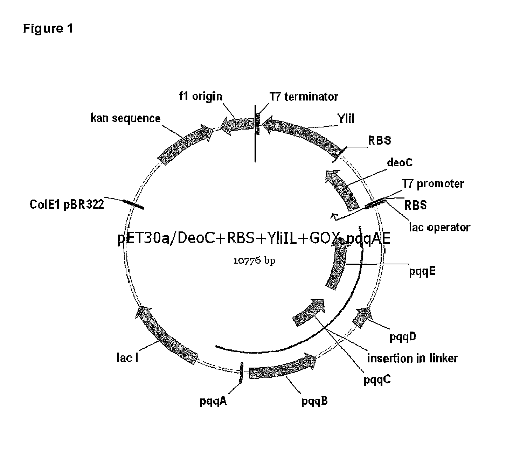

The construct pGEM/pqqA-E (according to Example 6) was cleaved with restriction endonucleases *Bam*HI and *Kpn*I, then the resulting fragments were separated on agarose gel electrophoresis and 3.4 kb fragment containing gene operon *pqqABCDE* was purified. Construct pET30/DeoC_RBS_Ylil with linker was cleaved using the aforementioned restriction endonucleases and purified. The fragments (cleaved pET30/DeoC_RBS_Ylil with linker and *pqqA*-E, respectively) were assembled in a T4 ligase reaction. *E*. *coli* JM109 cells were transformed with the obtained ligation reaction and kanamycin resistant colonies were cultured and plasmid DNA was isolated. The resulting plasmid construct was designated

pET30/DeoC_RBS_Ylil+pqqA-E and sequenced for confirmation of the gene sequences and orientation in plasmid construct. Physical map of described artificial plasmid bearing the said genes is presented in Figure 1. Specifically, Fig. 1 shows a physical map of inserted genes *deoC. ylil.* gene cluster *pqqABCDE* and additional double stranded sequences presenting ribosomal binding site (RBS) and linker sequence bearing additional recognition sites for restriction endonucleases into expression plasmid pET30a (Novagen Inc.. Madison. WI. USA). Artificial construct was designated pET30/DeoC_RBS_Ylil+pqqA-E.

The organism able to synthesize pyrroloquinone quinine and meanwhile expressing DERA aldolase and aldose dehydrogenase was prepared by transforming BL21 (DE3) competent cells with said plasmid.

Expression of *E*. *coli* Ylil and DERA comprised within a single whole cell culture able to produce PQQ was undertaken as described in Procedure 1A, with a sole difference of IPTG inducer concentration being 0.5 mM. Corresponding to Procedures 1B and 1C living whole cell catalysts and resting whole cell catalysts, respectively, were prepared. Cell free lysate was prepared following procedure the described in Example 2.

### Example 9: Preparation of Kluyvera intermedia whole cell catalyst

Cultivation of *Kluyvera intermedia* (ATCC 33421, formerly *Enterobacterintermedium*): VD medium (30 mL; 10 g/L bacto yeast extract, 5 g/L glycerol, 5 g/L NaCl, 4 g/L NaH2PO4·2H2O, pH was adjusted with 1 M NaOH to pH=7,0) was inoculated with a single colony of *Kluyvera intermedia* from a freshly streaked VD agar plate and pre-cultured to late log phase (30 °C, 250 rpm, 8 h).

The pre-culture cells (inoculum size 10 %, v/v) were then transferred to fresh VD medium (100 mL; 10 g/L bacto yeast extract, 5 g/L glycerol, 5 g/L NaCl, 4 g/L NaH2PO4·2H2O, pH was adjusted with 1 M NaOH to pH=7,0) .The cells were maintained at 30°C, 250 rpm for 16h.

Preparation of whole cell catalysts (Procedure 1B): After cultivation cells were harvested by centrifugation (10 000 g, 5 min, 4°C). Supernatant was discarded after centrifugation and pellet was resuspended in fresh VD medium (10 g/L bacto yeast extract, 5 g/L glycerol, 5 g/L NaCl, 4 g/L NaH2PO4·2H2O, pH was adjusted with 1M NaOH to pH=6.0). Cells were resuspended in one tenth of initial culture volume. The aldose dehydogenase comprised within the living whole cell was thus obtained in fresh medium.

Alternatively (Procedure 1C), after discarding supernatant after centrifugation pellet was resuspended in phosphate buffer (50 mM KH₂PO₄, 150 mM NaCl, pH 6.0). Cells were resuspended in one tenth of initial culture volume. The aldose dehydrogenase comprised within the resting whole cell culture was thus obtained.

Cell free lysate was prepared by following the procedure described in Example 2.

### Example 10: Preparation of Gluconobacter oxydans whole cell catalyst

Cultivation of *Gluconobacter oxydans* (ATCC #621H): Mannitol medium (100 mL; 10 g/L bacto yeast extract, 3 g/L peptone, 5 g/L mannitol, pH was adjusted with 1 M NaOH to pH=7,0) was inoculated with a single colony *Gluconobacter oxydans* from a freshly streaked mannitol agar plate and cultured. The cells were maintained at 26°C, 250 rpm for 48 h.

Preparation of whole cell catalysts (Procedure 1B): After cultivation cells were harvested by centrifugation (10 000 g, 5 min, 4°C). Supernatant was discarded after centrifugation and pellet was resuspended in fresh VD medium (10 g/L bacto yeast extract, 5 g/L glycerol, 5 g/L NaCl, 4 g/L NaH2PO4·2H2O, pH was adjusted with 1M NaOH to pH=6.0). Cells were resuspended in one tenth of initial culture volume. The aldose dehydogenase comprised within the living whole cell was thus obtained in fresh medium.

Alternatively (Procedure 1C), after discarding supernatant after centrifugation pellet was resuspended in phosphate buffer (50 mM KH₂PO₄, 150 mM NaCl, pH 6,0). Cells were resuspended in one tenth of initial culture volume. The aldose dehydrogenase comprised within the resting whole cell culture was thus obtained.

Cell free lysate was prepared following the procedure described in Example 2.

### Example 11: A method for determination of compound (II) oxidation/dehydrogenation activity using DCPIP as electron acceptor

This method is useful for determining aldose dehydrogenase activity (or activity of other dehydrogenases and/or oxidases). Accordingly the same method is used for screening and identifying enzymes and/or organisms capable of carrying out the reaction of oxidation/dehydrogenation of compound (II) resulting in compound (I).

Oxidation/dehydrogenation activity towards compound (II) can be measured using a living whole cell catalyst (regard to Procedure 1B), resting whole cell catalyst (regard to Procedure 1C), a lysate (preparation is described in Example 2) or a periplasmic fraction (Example 3) of any microorganism regardless of it being native or genetically modified microorganism. For practical purposes it will be understood hereinafter that a term "analysed material" includes all preparations of catalysts as described in previous examples. The results obtained using different preparations are given separately in provided tables below.

For comparative studies cell density of tested microorganisms was quantified as wet weight in mg per mL of sample. The cells in a sample were separated from the broth by centrifugation (10 000 g, 10 min) and wet pellet was weighted. When lysate or periplasmic fraction was used as a testing fraction the data of wet cell weight of whole cell derived from was taken into account.

Where stated, 1 mL of "analysed material" was supplemented with 0.25 µM PQQ (Sigma Aldrich, Germany) and incubated at room temperature for 10 minutes. Prior to measurement pH value of "analysed material" was adjusted to 8.0 with 1 M NaOH.

Screening method was performed in a 96-well microplate in order to screen for and identify "analysed material" useful for converting ((2S,4R)-4,6-dihydroxytetrahydro-2H-pyran-2-yl)methyl acetate to ((2S,4R)-4-hydroxy-6-oxotetrahydro-2H-pyran-2-yl)methyl acetate in presence of artificial electron acceptor 2,6-dichlorobenzenone-indophenole (DCPIP) combined with phenazine methosulfate (PMS).

The reaction mixture (total volume was 200 µL) contained phosphate buffer pH 8.0, 50 mM substrate ((2S,4R)-4,6-dihydroxytetrahydro-2H-pyran-2-yl)methyl acetate), 0.2 mM DCPIP (Sigma Aldrich, Germany), 0.04 mM PMS (Sigma Aldrich, Germany). 50 ul of "analysed material" (50 µL) was added to the reaction mixture. Where needed (due to rapid completion of the reaction), the "analysed material" was diluted in phosphate buffer pH 8.0. All tested "analysed materials" were made in triplicates. Useful range in which the assay is linear was found to be between 10 and 400 mAU/sec.

Method was performed with spectrophotometer Spectra Max Pro M2 (Molecular Devices, USA) photometrically at 600 nm. Absorbance at 600 nm was measured every 15 seconds for 30 minutes at 28°C. Results were collected and analysed using software SoftMax Pro Data Acquisition & Analysis Software.

Activity of "analysed material" and thus capability of carrying out the reaction of converting compound (II) to compound (I), specifically of converting ((2S,4R)-4,6-dihydroxytetrahydro-2H-pyran-2-yl)methyl acetate to ((2S,4R)-4-hydroxy-6-oxotetrahydro-2H-pyran-2-yl)methyl acetate was defined as absolute value of reduction in absorbance unit per minute per wet weight of culture cells used for preparation of any "analysed material" according to procedures 1C, 2 or 3 present in the reaction mixture (abs[mAU min⁻¹ mg⁻¹]). Data are average values of three parallel measurements and are shown in a table below.

| | | resting whole cell catalyst (Procedure 1B) | | lysate (Example 2) | | periplasm fraction (Example 3) | |
|---|---|---|---|---|---|---|---|
| "analysed material" | | without PQQ | with PQQ | without PQQ | with PQQ | without PQQ | with PQQ |
| 1 | *E. coli* BL21(DE3) pET30 (Example 1) | 8 | 184 | 9 | 80 | 15 | 65 |
| 2 | *E*. *coli* BL21(DE3) pET30/Ylil (Example 1) | 7 | 978 | 7 | 950 | 10 | 965 |
| 3 | *E. coli* BL21(DE3) pET30/Ylil_C (Example 1) | 15 | 352 | 14 | 456 | 15 | 67 |
| 4 | *E. coli* BL21(DE3) pET30/GdhB (Example 1) | 10 | 877 | 8 | 911 | 9 | 920 |
| 5 | *E. coli* BL21(DE3) pET30/GdhB_therm (Example 1) | 15 | 812 | 9 | 897 | 8 | 887 |
| 6 | *E*. *coli* BL21(DE3) pET30/DeoC (Example 4) | 9 | 175 | 15 | 158 | 8 | 45 |
| 7 | *E. c*o*li* BL21(DE3) pET30/DeoC_RBS_Ylil (Example 5) | 8 | 811 | 10 | 797 | 11 | 688 |
| 8 | *E*. *coli* BL21 (DE3) pET30/Ylil+pqqA-E (Example 6) | 957 | 961 | 932 | 952 | 865 | 877 |
| 9 | *E*. *coli* BL21(DE3) pET30/DeoC_RBS_Ylil+pqqA-E (Example 8) | 889 | 905 | 970 | 954 | 943 | 922 |
| 10 | *Kluyvera intermedia* (Example 9) | 875 | 880 | 10 | 11 | N.A. | N.A. |
| 11 | *Gluconobacter oxydans* (Example 10) | 640 | 680 | 11 | 13 | N.A. | N.A. |

For negative controls, at least one component (except electron acceptor DCPIP combined with PMS) of reaction mixture was replaced with phosphate buffer pH 8.0. No discoloration was observed in all negative control wells even after prolonged incubation. When aldose dehydrogenase clear aqueous solution was replaced with phosphate buffer, no discoloration was observed. When any of the substrates (((2S,4R)-4,6-dihydroxytetrahydro-2H-pyran-2-yl)methyl acetate, glucose, galacatose or glycerol) were replaced with phosphate buffer, no discoloration was observed. When PQQ was not provided to the reaction mixture by any means described in this invention (except in mixtures 8,9,10 and 11 where PQQ is provided intrinsically), no discoloration was observed.

### Example 12: Determination of properties of aldose dehydrogenase contained in E. coli in a presence of DCPIP

For determination of activity of E. coli aldose dehydrogenases on other substrates and different concentrations, additional experiments were performed.

50 µL of "analysed material" (more particularly *E. coli* BL21 pET30/Ylil reconstituted with PQQ and undertaking procedure 1B), 0.2 mM DCPIP, 0.04 mM PMS were placed in 96-well microtiter plate wells along with different concentrations (2.5 - 10 g/L) of substrates (((2S,4R)-4,6-dihydroxytetrahydro-2H-pyran-2-yl)methyl acetate, D-glucose and D-galactose). Final volume of reaction mixture was 200 µL, all components were dissolved in phosphate buffer pH 8.0.

Discoloration of DCPIP was observed in these wells. The discoloration rate was about two times faster when ((2S,4R)-4,6-dihydroxytetrahydro-2H-pyran-2-yl)methyl acetate was used as substrate than with control wells containing D-glucose or D-galactose as a substrate.

Discoloration was faster when 10 g/L of ((2S,4R)-4,6-dihydroxytetrahydro-2H-pyran-2-yl)methyl acetate used as substrate in contrast to 2.5 g/L said substrate. A slight substrate inhibition to the reaction rate was observed only when concentration of ((2S,4R)-4,6-dihydroxytetrahydro-2H-pyran-2-yl)methyl acetate in the reaction mixture was raised above 40g/L

To estimate possible influence of the product ((2S,4R)-4-hydroxy-6-oxotetrahydro-2H-pyran-2-yl)methyl acetate on the reaction rate, various amounts of said product were added to the reaction mixture before the initiation of the reaction. Slight reduction in reaction rates were observed only when more than 35g/L of ((2S,4R)-4-hydroxy-6-oxotetrahydro-2H-pyran-2-yl)methyl acetate was added in addition of 10g/L of ((2S,4R)-4,6-dihydroxytetrahydro-2H-pyran-2-yl)methyl acetate.

To investigate substrate specificity and pH optima of *E*. *coli* aldose dehydrogenase the following experiments were performed:
One reaction mixture (total volume was 200 µL) contained phosphate buffer pH 8.0, 50 mM substrate (((2S,4R)-4,6-dihydroxytetrahydro-2H-pyran-2-yl)methyl acetate, D-glucose or glycerol), 0.2 mM DCPIP, 0.04 mM PMS. 50 µL of "analysed material" (described in detail futher on ), supplemented with 1 µM PQQ (Sigma Aldrich, Germany) was added to the mixture. The catalyst *E*. *coli* BL21 pET30/Ylil culture obtained either as resting whole cell catalyst (obtained undertaking Procedure 1C) or as lysate (as described in Example 2). The initial pH value of reaction mixture was 8.0. All tested solutions were made in triplicates.

The second reaction mixture was prepared identically to the above with a sole difference: the pH value of assembled mixture being 6.0. (all compounds of reaction mixture were dissolved in phosphate buffer pH 6.0 and the initial pH value of reaction mixture was thus 6.0) All tested solutions were made in triplicates.

Differentiation of aldose dehydrogenases and thus converting substrates at different optimums in said reaction mixtures was determined with spectrophotometer Spectra Max Pro M2 (Molecular Devices, USA) photometrically at 600 nm. Absorbance at 600 nm was measured every 15 seconds for 30 minutes at 28°C. Results were collected and analysed using software SoftMax Pro Data Acquisition & Analysis Software. Experiment was performed as described in Example 11. In the table below are shown activities of "analysed material" (abs[mAU min⁻¹ mg⁻¹]).

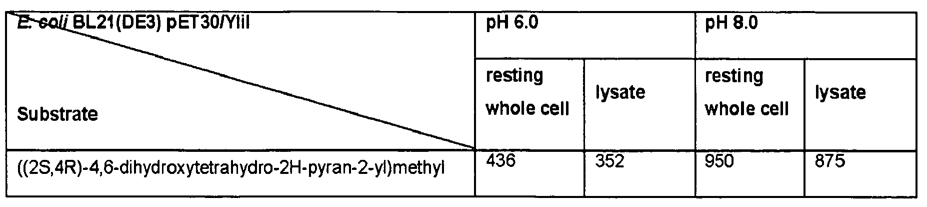

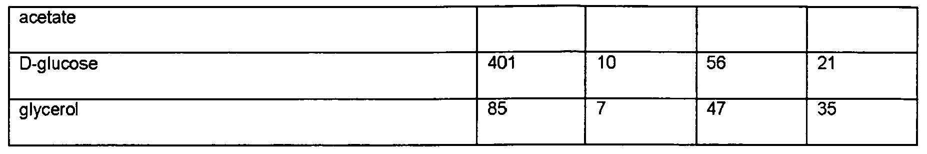

The results show that the overexpressed Ylil aldose dehydrogenase is performing better at pH8 when the substrate is ((2S,4R)-4,6-dihydroxytetrahydro-2H-pyran-2-yl)methyl acetate and has significantly lower affinity for glucose at ph 8.0. The activity in this case is comparable for both whole cell catalyst and cell free lysate. Activity towards ((2S,4R)-4,6-dihydroxytetrahydro-2H-pyran-2-yl)methyl acetate surprisingly surpasses activity towards glucose at any conditions tested.

On the other hand significant activity is obtained for glucose at pH 6.0 but only with the whole cell preparation of the catalyst. Given that similar activities are obtained when negative control catalyst whole cell *E*. *coli* BL21 pET30 shows similar activity profiles on glucose, this activity is most likely derived from membrane bound PQQ dependant glucose dehydrogenase which is natively expressed in *E*. *coli.*

Glycerol is a bad substrate for aldose dehydrogenases tested here.

### Example 13: Reconstitution of holo-enzyme aldose dehydrogenase with PQQ from various sources and assay with DCPIP as electron acceptor

Three different possibilities of providing PQQ to PQQ-dependent aldose dehydrogenases *in situ* and thus obtaining an active aldose dehydrogenase, were tested. For determination we used method with artificial electron acceptor (DCPIP combined with PMS) as described in Example 11.

To the whole cell catalysts *E*. *coli* BL21(DE3) pET30 or to whole cell catalysts *E*. *coli* BL21 (DE3) pET30/Ylil, *E. coli* BL21 (DE3) pET30/Ylil_C, *E. coli* BL21 (DE3) pET30/GdhB, *E*. *coli* BL21 (DE3) pET30/GdhB_therm, *E. coli* BL21(DE3) pET30/DeoC, *E. coli* BL21 (DE3) pET30/DeoC_RBS_Ylil prepared according to Procedure 1B or 1C PQQ could be supplied as:
- Procedure 13E: 1 µM PQQ (Sigma Aldrich, Germany) and phosphate buffer pH 6.0 (5 mL) was added to living whole cell catalysts or to resting whole cell catalysts (5 mL) and incubated 10 min at room temperature.
- Procedure 13F: 5 mL of supernatant of cultivated *E*. *coli* JM109 pGEM/pqqA-E (lysate was obtained as described in Procedure 6D) supplemented whole cell catalysts or resting cell catalysts (5 mL) and incubated 10 min at room temperature.
- Procedure 13G: 5 mL of supernatant of cultivated *Klyuvera intermedia* or *Gluconobacter oxydans* (cultivation was described in Example 9 and Example 10, respectively) supplemented whole cell catalysts or resting cell catalysts (5 mL). Supernatant was obtained after centrifugation of cultures (10 000 g, 5 min, 4 °C) and incubated 10 min at room temperature.

Living whole cell catalysts or resting whole cell catalysts *E*. *coli* BL21 (DE3) pET30/Ylil+pqqA-E and *E. coli* BL21(DE3) pET30/DeoC_RBS_Ylil+pqqA-E which have undertaken Procedures 1B or 1C (5 mL) were supplemented with phosphate buffer pH=6.0 in same volume ratio and incubated 10 min at room temperature.

All reaction mixtures were tested in a 96-well microplate for their ability of converting ((2S,4R)-4,6-dihydroxytetrahydro-2H-pyran-2-yl)methyl acetate to ((2S,4R)-4-hydroxy-6-oxotetrahydro-2H-pyran-2-yl)methyl acetate in presence of artificial electron acceptor DCPIP undertaking procedure as described in Example 11.

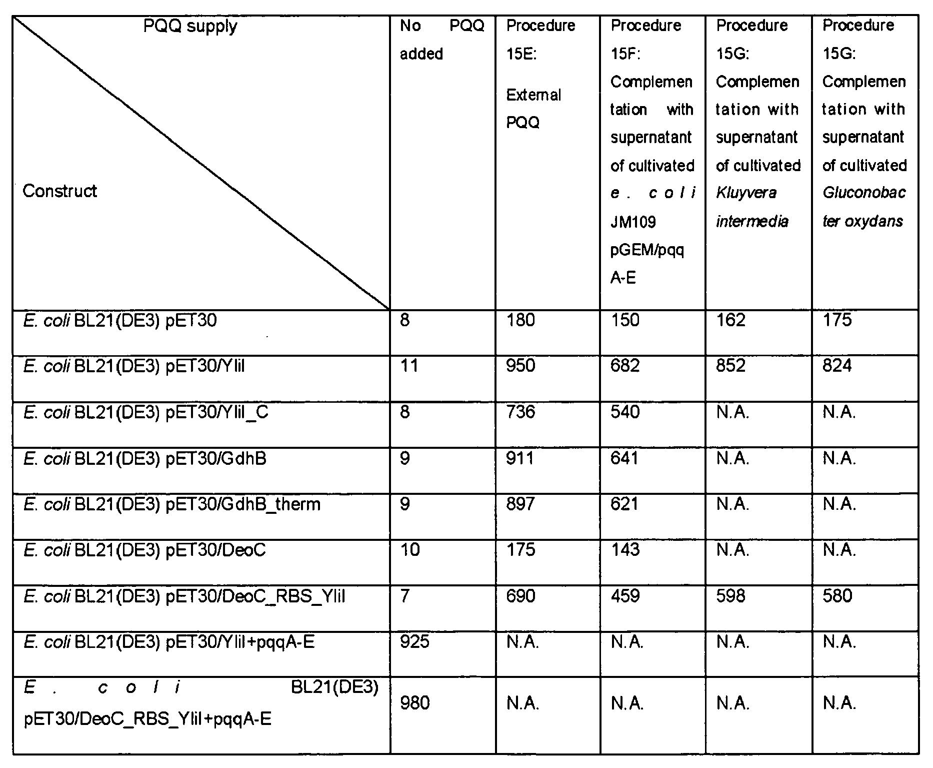

Addition of 1 µM PQQ (Sigma Aldrich, Germany) at the time of induction of expression of plasmids pET30, pET30/Ylil, pET30/Ylil_C, pET30/GdhB, pET30a/GdhB_therm in *E. coli* BL21(DE3) cells (following Procedure 1A) revealed similar results as addition immediately before the reaction.

### Example 14: Oxidation of lactol ((2S,4R)-4,6-dihydroxytetrahydro-2H-pyran-2-yl)methyl acetate with various aldose dehydrogenases comprised within living whole cell catalysts

Various living whole cell catalysts with aldose dehydrogenases (variety of them is described in Examples 1, 7, 8, 9 and 10, respectively) were tested for bioconversion of ((2S,4R)-4,6-dihydroxytetrahydro-2H-pyran-2-yl)methyl acetate to ((2S,4R)-4-hydroxy-6-oxotetrahydro-2H-pyran-2-yl)methyl acetate.

Living whole cell catalysts *E*. *coli* BL21 (DE3) pET30a, *E*. *coli* BL21 (DE3) pET30a/Ylil, *E*. *coli* BL21(DE3) pET30a/Ylil_C, *E. coli* BL21(DE3) pET30a/GdhB, *E*. *coli* BL21(DE3) pET30a/GdhB_therm (preparation is described in Example 1) were prepared as described in Procedure 1B. 9 mL samples of aforementioned living whole cell catalysts were transferred to 100 mL Erlenmeyer flasks, where the reaction was performed. Reaction mixture was supplemented with 1 µM PQQ (10 µL of 1 mM pre-prepared stock of PQQ, Sigma).

Living whole cell catalyst *E*. *coli* BL21 (DE3) pET30a/Ylil+pqqA-E (preparation is described in Example 7) were prepared as described in Procedure 1B. 9 mL samples of aforementioned cells were transferred to 100 mL Erlenmeyer flasks, where the reaction was performed.

Living whole cell catalysts *Kluyvera intermedia* (Example 9) and *Gluconobacter oxydans* (Example 10) were prepared as described in Procedure 1B. 9 mL samples of aforementioned cells were transferred to 100 mL Erlenmeyer flasks, where the reaction was performed.

((2*S*,4*R*)-4,6-dihydroxytetrahydro-2H-pyran-2-yl)methyl acetate was dissolved in phosphate buffer (50 mM KH₂PO₄, 150 mM NaCl, pH 6.0) to concentration of 2 mol/L. All samples of concentrated living whole cell catalyst were supplemented with 1 mL 2 mol/L ((2S,4R)-4,6-dihydroxytetrahydro-2H-pyran-2-yl)methyl acetate. Reaction was performed at 37°C, 250 rpm for 6 hours on a rotary shaker. At time 0', 60', 120', 180', 240', 300' and 360', samples were taken. Each sample was diluted 50-times with acetonitrile for GC-MS analysis. Results at time 0', 180' and 360' are shown in the table below.

The major product of the reaction had identical retention time and ion distribution as ((2S,4R)-4-hydroxy-6-oxotetrahydro-2H-pyran-2-yl)methyl acetate obtained by chemical oxidation as described in the art.

After 360 min reaction mixture was extracted five times with equal volume of ethyl acetate and all organic fractions were collected and dried over rotavapor. Finally a yellow oil was obtained.

The structure of resulting product was confirmed by NMR and is identical to what is reported in the art. ¹H NMR (300 MHz, acetone-d₆) δ 4.88 (m, 1H), 4.45 (d, *J*= 3,0 Hz, 1H), 4.38 (hex, *J* = 3,0 Hz, 1H), 4.23 (dd, *J* = 3.5 Hz, *J* = 12.0 Hz, 1H), 4.16 (dd, *J* = 5.5 Hz, *J* = 12.1Hz, 1H), 2.68 (dd, *J* = 4.3 Hz, *J* = 17.5 HZ, 1H), 2,51 (dddd, *J* = 0.8 Hz, *J* = 2.0 Hz, *J* = 3.3 Hz, *J* = 17.5 Hz, 1H), 2.03 (s, 3H), 1.91 (m, 2H), ¹³C NMR (75 MHz, acetone-d₆) δ 170.8, 169.7, 74.2, 66.5, 62.7, 39.1, 32.3, 20.6.

| | Time [min] | | | | | |
|---|---|---|---|---|---|---|
| | 0' | | 180' | | 360' | |
| Living whole cell catalyst | ((2S,4R)-4,6-dihydroxytetr ahydro-2H-pyran-2-yl)methyl acetate | **((2*S*,4*R*)-4-hydroxy-6-oxotetrahydr o-2H-pyran-2-yl)methyl acetate** | ((2S,4R)-4,6-dihydroxytetr ahydro-2H-pyran-2-yl)methyl acetate | **((2*S*,4*R*)-4-hydroxy-6-oxotetrahydr o-2H-pyran-2-yl)methyl acetate** | ((2S,4R)-4,6-dihydroxytetr ahydro-2H-pyran-2-yl)methyl acetate | **((2*S*,4*R*)-4-hydroxy-6-oxotetrahydr o-2H-pyran-2-yl)methyl acetate** |
| *E. coli* BL21 (DE3) pET30 | 38.20 g/L | 0.00 g/L | 19.17 g/L | 4.26 g/L | 10.12 g/L | 12.01 g/L |
| *E. coli* BL21 (DE3) pET30/Ylil | 38.20 g/L | 0.00 g/L | 13.01 g/L | 15.88 g/L | 1.65 g/L | 25.07 g/L |
| *E. coli* BL21 (DE3) pET30/Ylil_C | 38.20 g/L | 0.00 g/L | 15.12 g/L | 8.74 g/L | 6.98 g/L | 17.65 g/L |
| *E. coli* BL21(DE3) pET30/GdhB | 38.20 g/L | 0.00 g/L | 12.88 g/L | 14.01 g/L | 1.15 g/L | 23.15 g/L |
| *E. coli* BL21 (DE3) pET30/GdhB_therm | 38.20 g/L | 0.00 g/L | 12.10 g/L | 14.52 g/L | 1.01 g/L | 24.95 g/L |
| *E. c*o*li* BL21 (DE3) pET30/Ylil+pqqA-E | 38.20 g/L | 0.00 g/L | 12.55 g/L | 14.87 g/L | 1.15 g/L | 22.55 g/L |
| *Kluyvera intermedia* | 38.20 g/L | 0.00 g/L | 12.11 g/L | 13.85 g/L | 1.70 g/L | 23.45 g/L |
| *Gluconobacter oxydans* | 38.20 g/L | 0.00 g/L | 13 66 g/L | 13.12 g/L | 1.23 g/L | 25.56 g/L |

*E*. *coli* BL21 (DE3) pET30 was used as negative control. Reaction was held at the same conditions as described above and at the end of the reaction no ((2S.4R)-4-hydroxy-6-oxotetrahydro-2H-pyran-2-yl)methyl acetate was observed, however all of the provided ((2*S*,4*R*)-4.6-dihydroxytetrahydro-2H-pyran-2-yl)methyl acetate remained untouched.

### Example 15: Sequential reaction using DERA aldolase and aldose dehydrogenaze (Ylil) for the production ((2S.4R)-4-hydroxy-6-oxotetrahydro-2H-pyran-2-yl) methyl acetate from acetyloxyacetaldehyde and acetaldehyde

Living whole cell catalyst *E. coli* BL21 (DE3) pET30/DeoC was prepared as described in Example 4 according to Procedure 1B. 5 mL of living whole cell catalyst was transferred to 100 mL Erlenmayer flask.

Stock solution of acetyloxyacetaldehyde and acetaldehyde was prepared. 600.5 mg acetyloxyacetaldehyde (chemical synthesis of said compound was performed in our laboratory and revealed 85 % purity) and 467.2 mg acetaldehyde (purchased by Fluka, USA) were dissolved in ice cold phosphate buffer pH 6.0 to final volume 10 mL.

At time 0' 1 mL of said stock solution of acetyloxyacetaldehyde and acetaldehyde was added into reaction mixture. Reaction was performed at 37 °C, 250 rpm for 3 hours on a rotary shaker.

5 mL of living whole cell catalyst *E*. *coli* BL21 (DE3) pET30/Ylil (preparation is described in Example 1 according to Procedure 1B) supplemented with 1 µM PQQ (Sigma Aldrich. Germany) was added to the same Erlenmayer flask after 3 hours. The oxidation reaction was left for additional 3 hours at 37°C, 250 rpm on a rotary shaker.

At time 0', 60', 120', 180', 240', 300' and 360' samples were taken. Each sample was diluted 50-times with acetonitrile for GC-MS analysis. The major product of the reaction had identical retention time and ion distribution as ((2S,4R)-4-hydroxy-6-oxotetrahydro-2H-pyran-2-yl)methyl acetate obtained by chemical oxidation as described in the art. After 360' 12,30 g/L of ((2*S*.4*R*)-4-hydroxy-6-oxotetrahydro-2H-pyran-2-yl)methyl acetate was observed which represents 64 % molar yield.

### Example 16: Simultaneous reaction using DERA aldolase and aldose dehydrogenaze (Ylil) for the production ((2S.4R)-4-hydroxy-6-oxotetrahydro-2H-pyran-2-yl)methyl acetate from acetyloxyacetaldehyde and acetaldehyde

Living whole cell catalysts *E*. *coli* BL21 (DE3) pET30/Ylil and *E*. *coli* BL21 (DE3) pET30/DeoC (preparation is described in Examples 1 and 4, respectively) were prepared as described in Procedure 1B. Both living whole cell catalysts were transferred to 100ml Erlenmayer flask in the same volume ratio (5 mL). Reaction mixture was supplemented with 1 µM PQQ (Sigma Aldrich, Germany).

Stock solution of acetyloxyacetaldehyde and acetaldehyde was prepared. 1.201 g acetyloxyacetaldehyde (chemical synthesis of said compound was performed in our laboratory and revealed 85 % purity) and 934.4 mg acetaldehyde (purchased by Fluka. USA) were dissolved in ice cold phosphate buffer pH 6.0 to final volume 10 mL.

At time 0' 1 mL of said stock solution of acetyloxyacetaldehyde and acetaldehyde was added into reaction mixture. Final concentrations of acetyloxyacetaldehyde and acetaldehyde were 100 mM and 210 mM. respectively. Reaction was performed at 37°C, 250 rpm for 6 hours.

At time 0', 60', 120', 180', 240', 300' and 360' samples were taken. Each sample was diluted 50-times with acetonitrile for GC-MS analysis. The major product of the reaction had identical retention time and ion distribution as ((2S,4R)-4-hydroxy-6-oxotetrahydro-2H-pyran-2-yl)methyl acetate obtained by chemical oxidation as described in the art.

After 360' 9.70 g/L of ((2S,4R)-4-hydroxy-6-oxotetrahydro-2H-pyran-2-yl)methyl acetate was observed which represents 51 % molar yield. After 360' no lactol ((2S,4R)-4,6-dihydroxytetrahydro-2H-pyran-2-yl)methyl acetate was observed, however substrates (acetaldehyde and acetyloxyacetaldehyde) and intermediate ((*S*)-(4-hydroxyoxtetan-2-yl)methyl acetate) were still present at the time. At the same time, an identically prepared reaction having only DERA aldolase present in the reaction produced only 3.36g/L of lactol ((2S,4R)-4,6-dihydroxytetrahydro-2H-pyran-2-yl)methyl acetate.

These indications show that the reaction step by the aldose dehydrogenase (Ylil) is faster than the step catalyzed by the aldolase enzyme (DERA) and that presence of aldose dehydrogenase shifts the steady state equilibrium of the DERA reaction step towards the product and simultaneous reaction with aldose dehydrogenasee (Ylil) in fact improves overall rates toward the lactone (compound I).

### Example 17: Simultaneous reaction comprising DERA aldolase and aldose dehydrogenase (Ylil) within a living whole cell catalyst of a single microorganism capably of synthesis pf PQQ for the production of ((2S,4R)-4-hydroxy-6-oxotetrahydro-2H-pyran-2-yl)methyl acetate from acetyloxyacetaldehyde and acetaldehyde

The below example provides a synthetic biological pathway provided within a living microorganism which is capable of producing highly enantiomericaly pure ((2S,4R)-4-hydroxy-6-oxotetrahydro-2H-pyran-2-yl)methyl acetate from simple and inexpensive molecules: acetyloxyacetaldehyde and acetaldehyde.

Living whole cell catalyst *E. coli* BI21(DE3) pET30/DeoC_RBS_Ylil+pqqA-E (preparation is described in Example 8) was prepared as described in Procedure 1A. Said whole cell catalyst was concentrated by centrifugation (5 000 g, 10 min) and pellet was resuspended in one tenth of initial volume of the same supernatant. Exceeded supernatant was discarded. 10 mL of said concentrated living whole cell catalyst was transferred to 100 ml Erlenmayer flask.

Stock solution of acetyloxyacetaldehyde and acetaldehyde was prepared. 1.201 g acetyloxyacetaldehyde (chemical synthesis of said compound was performed in our laboratory and revealed 85 % purity) and 934.4 g acetaldehyde (purchased by Fluka. USA) were dissolved in ice cold phosphate buffer pH 6.0 to final volume 10 mL.

At time 0' 1 mL of said stock solution of acetyloxyacetaldehyde and acetaldehyde was added into reaction mixture. Final concentrations of acetyloxyacetaldehyde and acetaldehyde in reaction mixture were 100 mM and 210 mM. respectively. Reaction was performed at 37 °C. 250 rpm for 6 hours.

At time 0', 60', 120', 180', 240', 300' and 360' samples were taken. Each sample was diluted 50-times with acetonitrile for GC-MS analysis. The major product of the reaction had identical retention time and ion distribution as ((2S,4R)-4-hydroxy-6-oxotetrahydro-2H-pyran-2-yl)methyl acetate obtained by chemical oxidation as described in the art.

After 360' 7.80 g/L of ((2S,4R)-4-hydroxy-6-oxotetrahydro-2H-pyran-2-yl)methyl acetate was observed which represents 41 % molar yield

### Example 18: Simultaneous reaction comprising DERA aldolase and living whole cell catalyst Kluyvera intermedia for the production ((2S,4R)4-hydroxy-6-oxotetrahydro-2H-pyran-2-yl)methyl acetate from acetyloxvacetaldehyde and acetaldehyde

Living whole cell catalysts *E*. *coli* BL21(DE3) pET30/Ylil and *Kluyvera intermedia* (preparation is described in Examples 1 and 9, respectively) were prepared as described in Procedure 1B. Both living whole cell catalysts were transferred to 100 mL Erlenmayer flask in the same volume ratio (5 mL).

Stock solution of acetyloxyacetaldehyde and acetaldehyde was prepared. 1.201 g acetyloxyacetaldehyde (chemical synthesis of said compound was performed in our laboratory and revealed 85 % purity) and 934.4 mg acetaldehyde (purchased by Fluka. USA) were dissolved in ice cold phosphate buffer pH 6.0 to final volume 10 mL.

At time 0' 1 mL of said stock solution of acetyloxyacetaldehyde and acetaldehyde was added into reaction mixture. Final concentrations of acetyloxyacetaldehyde and acetaldehyde in reaction mixture were 100 mM and 210 mM, respectively. Reaction was performed at 37 °C, 250 rpm for 6 hours.

At time 0', 60', 120', 180', 240', 300' and 360' samples were taken. Each sample was diluted 50-times with acetonitrile for GC-MS analysis. The major product of the reaction had identical retention time and ion distribution as ((2S,4R)-4-hydroxy-6-oxotetrahydro-2H-pyran-2-yl)methyl acetate obtained by chemical oxidation as described in the art.

After 360' 10.40 g/L of ((2*S*,4*R*)-4-hydroxy-6-oxotetrahydro-2H-pyran-2-yl)methyl acetate was observed which represents 54 % molar yield.

### Example 19: Simultaneous reaction using DERA aldolase and aldose dehydrogenaze (Ylil) for the production of ((4R,6S)-6-(chloromethyl)-4-hydroxytetrahydro-2H-pyran-2 one)

Living whole cell catalysts *E. coli* BL21 (DE3) pET30/Ylil and *E. coli* BL21 (DE3) pET30/DeoC (preparation is described in Examples 1 and 4, respectively) were prepared as described in Procedure 1B. 6 mL of said living whole cell catalyst *E. coli* BL21 (DE3) pET30/DeoC and 3 mL of *E. coli* BL21 (DE3) pET30/Ylil were transferred to 50 mL polystyrene conical tubes (BD Falcon, USA). Reaction mixture was supplemented with 1 µM PQQ (Sigma Aldrich, Germany).

Stock solution of chloroacetaldehyde and acetaldehyde was prepared. 817 µL of chloroacetaldehyde solution (50 wt. % in H₂O, purchased by Aldrich, USA) and 500.6 mg acetaldehyde (purchased by Fluka. USA) were dissolved in ice cold phosphate buffer pH 6.0 to final volume 5 mL.

At time 0' 1 mL of said stock solution of chloroacetaldehyde and acetaldehyde was added into reaction mixture - total reaction volume was thus 10 mL and starting concentrations of chloroacetaldehyde and acetaldehyde in reaction mixture were 100 mM and 225 mM, respectively. Reaction was performed for 2 hours at 37°C, 200 rpm of shaking in water bath.

The progress of the reaction was monitored with gas chromatography. After 120 min reaction mixture was extracted three times with equal volume of ethyl acetate and all organic fractions were collected and dried over rotavapor. 66 mg (36.2 % yield) of dried product (4*R*,6*S*)-6-(chloromethyl)-4-hydroxytetrahydro-2*H*-pyran-2-one remained and was analyzed with NMR. ¹H NMR (300 MHz, CDCl₃) δ 5.01 (m, 1H), 4.47 (m, 1H), 3.80 (m, 1H), 3.68 (m, 1H), 2.69 (d, *J* = 3.6 Hz, 2H), 2.09 (m, 1H), 1.97 (m, 1H). ¹³C NMR (75 MHz, CDCl₃) δ 170.2, 74.8, 62.5, 46.6, 38.5, 32.8.

### Example 20: Simultaneous reaction using DERA aldolase and aldose dehydrogenaze (Ylil) for the production of ((4R,6S)-6-(dimethoxymethyl)-4-hydroxytetrahydro-2H-pyran-2-one)

Living whole cell catalysts *E. coli* BL21 (DE3) pET30/Ylil and *E. coli* BL21 (DE3) pET30/DeoC (preparation is described in Examples 1 and 4, respectively) were prepared as described in Procedure 1B. 6 mL of said living whole cell catalyst *E. coli* BL21 (DE3) pET30/DeoC and 3 mL of *E*. *coli* BL21 (DE3) pET30/Ylil were transferred to 50 mL polystyrene conical tubes (BD Falcon, USA). Reaction mixture was supplemented with 1 µM PQQ (Sigma Aldrich. Germany).

Stock solution of dimethoxyacetaldehyde and acetaldehyde was prepared. 868 µL of 2,2-dimethoxyacetaldehyde solution (60 wt. % in H₂O, purchased by Fluka. USA) and 500.6 mg acetaldehyde (purchased by Fluka, USA) were dissolved in ice cold phosphate buffer pH 6.0 to final volume 5 mL.

At time 0' 1 mL of said stock solution of dimethoxyacetaldehyde and acetaldehyde was added into reaction mixture - total reaction volume was thus 10 mL and starting concentrations of dimethoxyacetaldehyde and acetaldehyde in reaction mixture were 100 mM and 225 mM, respectively. Reaction was performed for 2 hours at 37°C, 200 rpm of shaking in water bath.

The progress of the reaction was monitored with gas chromatography. After 120 min reaction mixture was extracted three times with equal volume of ethyl acetate and all organic fractions were collected and dried over rotavapor. 53 mg (25.3 % yield) of dried product (4R,6S)-6-(dimethoxymethyl)-4-hydroxytetrahydro-2H-pyran-2-one remained and was analyzed with NMR. ¹H NMR (300 MHz, CDCl₃) δ 4.74 (quint, *J* = 3.5 Hz, 1H), 4.43 (m, 2H), 3.49 (s, 3H), 3.47 (s, 3H), 3.10 (br s, 1H), 2.72 (dd, *J* = 3.5 Hz, *J* = 17.8 Hz, 2H), 2.62 (m, 2H),1.99 (m, 2H).

### Example 21: Simultaneous reaction using DERA aldolase and aldose dehydrogenaze (Ylil) for the production of ((4R,6S)-6-benzyloxymethyl)-4-hydroxytetrahydro-2H-pyran 2-one)

Living whole cell catalysts *E. coli* BL21 (DE3) pET30/Ylil and *E. coli* BL21 (DE3) pET30/DeoC (preparation is described in Examples 1 and 4, respectively) were prepared as described in Procedure 1B. 6 mL of said living whole cell catalyst *E. coli* BL21 (DE3) pET30/DeoC and 3 mL of *E*. *coli* BL21 (DE3) pET30/Ylil were transferred to 50 mL polystyrene conical tubes (BD Falcon, USA). Reaction mixture was supplemented with 1 µM PQQ (Sigma Aldrich, Germany).

Stock solution of benzyloxyacetaldehyde and acetaldehyde was prepared. 774.1 mg of benzyloxyacetaldehyde (purchased by Fluka, USA) and 500.6 mg acetaldehyde (purchased by Fluka, USA) were dissolved in ice cold phosphate buffer pH 6.0 to final volume 5 mL.

At time 0' 1 mL of said stock solution of benzyloxyacetaldehyde and acetaldehyde was added into reaction mixture - total reaction volume was thus 10 mL and starting concentrations of benzyloxyacetaldehyde and acetaldehyde in reaction mixture were 100 mM and 225 mM, respectively. Reaction was performed for 2 hours at 37°C. 200 rpm of shaking in water bath.

The progress of the reaction was monitored with gas chromatography. After 120 min reaction mixture was extracted three times with equal volume of ethyl acetate and all organic fractions were collected and dried over rotavapor. 51 mg (19.6 % yield) of dried product (4R,6S)-6-(benzyloxymethyl)-4-hydroxytetrahydro-2*H*-pyran-2-one remained. The crude product was dissolved in dichloromethane and reacted with TBDMSCI and imidazole for 24 h. Solution was concentrated and purified by chromatography to give TBDMSCI protected compound ((4*R*,6*S*)-6-(benzyloxymethyl)-4-hydroxytetrahydro-2H-pyran-2-one), which was analyzed with NMR. ¹H NMR (500 MHz, CDCl₃) δ 7.36 (m, 5H), 5.17 (s, 2H), 4.93 (quint, *J* = 4.7 Hz, 1H), 4.38 (dd, *J* = 3.4 Hz, *J* = 11.8 Hz, 1H), 4.35 (quint, *J* = 3.3 Hz, 1H), 4.27 (dd, *J* = 4.7 Hz, *J* = 11.8 Hz, 1H), 2.58 (d, *J* = 3.3 Hz, 2H), 1.85 (m, 2H), 0.88 (s, 9H), 0.09 (s, 3H), 0.08 (s, 3H), ¹³C NMR (125 MHz, CDCl₃) δ 154.8, 134.8, 128.7, 128.6, 128.4, 73.2, 70.0, 68.8, 63.2, 39.0, 32.2, 25.6, 17.8, -5.0.

### Example 22: Simultaneous reaction using DERA aldolase and aldose dehydrogenaze (Ylil) for the production of ((4R,6R)-4-hydroxy-6-methyltetrahydro-2H-pyran-2-one)

Living whole cell catalysts *E. coli* BL21 (DE3) pET30/Ylil and *E. coli* BL21 (DE3) pET30/DeoC (preparation is described in Examples 1 and 4, respectively) were prepared as described in Procedure 1B. 6 mL of said living whole cell catalyst *E. coli* BL21 (DE3) pET30/DeoC and 3 mL of *E. coli* BL21 (DE3) pET30/Ylil were transferred to 50 mL polystyrene conical tubes (BD Falcon, USA). Reaction mixture was supplemented with 1 µM PQQ (Sigma Aldrich, Germany).

Stock solution of acetaldehyde was prepared. 445 mg of acetaldehyde (purchased by Fluka, USA) was dissolved in ice cold phosphate buffer pH 6.0 to final volume 5 mL.

At time 0' 1 mL of said stock solution of acetaldehyde was added into reaction mixture - total reaction volume was thus 10 mL and its final concentration in reaction mixture was 200 mM. Reaction was performed for 2 hours at 37°C, 200 rpm of shaking in water bath.

The progress of the reaction was monitored with gas chromatography. After 120 min reaction mixture was extracted three times with equal volume of ethyl acetate and all organic fractions were collected and dried over rotavapor. 99 mg (34.1 % yield) of dried product (4*R*,6*R*)-4-hydroxy-6-methyltetrahydro-2*H*-pyran-2-one remained and was analyzed with NMR. ¹H NMR (300 MHz, acetone-d₆) δ 4.76 (dq, *J*_{d} = 11.2 Hz, *J*_{q} = 3.2 Hz, 1H), 4.41-4.15 (m, 2H), 2.63 (dd, *J* = 4.3 Hz, *J* = 17.0 Hz, 1H), 2.46 (ddd, *J* = 1.7 Hz, *J* = 3.3 Hz, *J* = 17.0 Hz, 1H), 1.92 (m, 1H), 1.71 (dd, *J* = 3.0 Hz, *J* = 14.3 Hz, 1H), 1.29 (d, *J* = 6.4 Hz, 3H). ¹³C NMR (75 MHz, acetone-d₆) δ 170.6, 72.7, 63.1, 39.1, 38.2, 21.8.

### Example 23: High cell density production of living whole cell catalysts and one pot sequential production of ((2S,4R)-4-hdroxy-6-oxotetrahydro-2H-pyran-2-yl)methyl acetate from acetyloxyacetaldehyde and acetaldehyde

The high cell density culture of living whole cell catalysts *E. coli* BL21(DE3) pET30/Ylil+pqqA-E and *E. coli* BL21 (DE3) pET30a/DeoC (preparation is described in Example 7 and Example 4, respectively) were prepared in "fed batch" bioprocess using laboratory bioreactors Infors ISF100 with maximal volume of 2L. The reactors were stirred, aerated, temperature and pH controlled as described bellow. After consumption of initial substrates provided in the medium. ammonia and glucose are fed continuously to the process as nitrogen and carbon source, respectively.

The composition and preparation of the media was as follows:
13.3 g/L KH2PO4, 1.7 g/L citric acid, 60 mg/L Fe(III)citrate, 40 g/L D-glucose, 8 mg/L Zn(CH3COO)2 2H2O, 1 g/L (NH4)2HPO4, 2.7 g/L MgSO4 7H20 and 10 mL/L mineral solution.
Mineral solution was pre-prepared as follows:
1.5 g/L MnCl2 4H20, 0.3 g/L H3BO3, 0.25 g/L NaMoO4 2H2O, 0.25 g/L CoCl2 6H20, 0.15 g/L CuCl2 2H20, 0.84 g/L EDTA, 1 g/L Na2PO4 2H20

To prevent precipitation, the initial medium was prepared according to a special protocol: KH2PO4, Fe(III)citrate, mineral solution, Zn(CH3COO)2 2H2O and (NH4)2HPO4 were sequentially added as solutions to about half of the final volume. After autoclaving (20 min at 121°C). sterile solutions of glucose, MgSO4 7H2O and kanamycin (25 mg/mL) were added after prior adjustment of the pH to 6.8 with 12.5 % (v/v) ammonium hydroxide solution. Sterile distilled water was added to adjust the final volume (1L) in the bioreactor. The above said solutions were sterilized separately by filtration (0.2 µm).

Feeding solutions were 12.5 % (v/v) ammonium hydroxide solution, the silicone antifoam compound BC SIMETHICONE ANTIFOAM C100EP (Basildon Chemical. UK) and 50 % (w/v) glucose.

Inoculums for both cultures were provided as 50mL of shake flask culture in exponential growth phase. VD medium (50 mL; 10 g/L Bacto yeast extract, 5 g/L glycerol, 5 g/L NaCl, 4 g/L NaH2PO4*2H2PO. pH was adjusted with 1 M NaOH to 7.0) was inoculated with a single colony of said whole cell catalyst from a freshly streaked VD agar plate and pre-cultured to late exp. phase (37 °C, 250 rpm. 8h).

The initial process parameters at inoculation were as follows: 25°C, air flow rate 1.5 Umin (1.5 WM), stirrer speed 800 rpm, pH 6.8. During cultivation, the dissolved oxygen concentration was kept at ≥ 20 % of saturation by a pO2/agitation rate control loop and a pO2/air flow ratio control loop. towards the end of bioprocess approaching to 0 % and bioreactor capabilities reaching maximum (stirrer speed 2000 rpm, aeration 3L/min).

The pH was kept at 6.8 during the whole process using a pH sensor controlled external pump which provides pulses of Ammonia solution to the bioreactor whenever the pH drops below 6.8.

After depletion of glucose present in the initial medium, a distinctive rise in pO₂ and pH level is observed (about 10-12h into the process). At this time feeding with 50 % (w/v) glucose started and was manually regulated as approximation as exponential curve (feeding started with 0.1 mL/min and ended with 0.6 mL/min in 24 h period. The process can be controlled by substrate supply; when glucose concentration is kept at dynamic zero, the glucose solution flow controls respiration rate of the culture. Technical limitations in regards to oxygen supply and heat transfer can be successfully overcome this way.

Induction for expression of protein Ylil in the culture of *E*. *coli* BL21 (DE3) pET30/Ylil+pqqA-E. was performed by adding 0.05 mM IPTG (Sigma Aldrich, Germany) 6 hours after start of the feeding phase.

Induction for expression of protein DeoC in the culture of coli BL21 (DE3) pET30a/DeoC, was performed by adding 0.1 mM IPTG (Sigma Aldrich, Germany) 6 hours after start of the feeding phase.

The overall length of the process is 34 - 42h and wet weight of biomass at level between 200 and 240g/L is obtained.

The high density culture of *E*. *coli* BL21 (DE3) pET30/Ylil+pqqA-E was cooled down to 15°C and kept in the reactor with light steering and aeration (400 rpm, 0.5 L/min) until used for the reaction (5h).

The high density culture of *E*. *coli* BL21(DE3) pET30a/DeoC was kept in the bioreactor and strirred with 800 rpm. Temperature was raised to 37°C and 56.6 g of acetyloxyacetaldehyde and 120 mL of acetaldehyde (45.4 g) diluted in water were added with programmable pump to the reaction mixture. The whole quantity of acetyloxyacetaldehyde was added in with the constant flow rate in 30 minutes. Acetaldehyde was added continuously in 3 hours time span as described in the table below:

| Time [min] | 0 | 5 | 10 | 15 | 20 | 25 | 30 | 35 | 45 | 60 | 75 | 90 | 105 | 120 | 135 | 150 | 165 | 180 |
|---|---|---|---|---|---|---|---|---|---|---|---|---|---|---|---|---|---|---|
| Volume of added acetaldehyde [mL] | 0 | 15 | 27 | 38 | 47 | 54 | 60 | 65 | 73 | 82 | 90 | 96 | 102 | 106 | 110 | 114 | 117 | 120 |
| Flow [mL/min] | 3000 | 2400 | 2 200 | 1 800 | 1 400 | 1 200 | 1 000 | 0 800 | 0600 | 0533 | 0400 | 0400 | 0267 | 0 267 | 0 267 | 0 200 | 0 200 | 0 |

During the reaction the pH was kept at 5.8 using 12.5 % (v/v) ammonium hydroxide solution and bioreactor's sensor dependant pH correction function. After 3h or the reaction, some of the reaction mixture was removed from the reactor leaving 1 L of the reaction mixture steering with 800 rpm at 37°C. 0.5 L of high density culture of *E. coli* BL21 (DE3) pET30/Ylil+pqqA-E. preheated to 37°C was added to the reaction mixture and aeration (1L/min) was provided. The reaction mixture was left for 6 hours. and during that time. 0.1 mL/min of glycerol was added to the mixture and pH was maintained at 5.8 using 12.5 % (v/v) ammonium hydroxide solution.

After the 6 hours the reaction was stopped. pH lowered to 3.5 using 5M HCl solution and the whole volume of reaction mixture was transferred to simple glass vessel and mixed with 1.5 L of ethylacetate to perform a "whole broth" extraction process. The organic phase was collected and another 1.5L of ethyl acetate were added to the aqueous phase. The whole procedure was repeated 5 times. The collected organic phase fractions were joined, 200 g of anhydrous sodium sulphate was added (in order to bind the ethyl acetate dissolved water) and filtered off. The solvent was then removed by low pressure evaporation at 37 °C. The remaining substance (82.6g) was yellow to amber oil with consistency of honey at RT. Subsequent analysis using 1H NMR and GC-MS confirmed the structure of ((2S,4R)-4-hydroxy-6-oxotetrahydro-2H-pyran-2-yl)methyl acetate and the chromatographic purity was 72%. Overall molar yield of the reaction was estimated at 59%.

### REFERENCES

1. Achmann, S. et al. Direct detection of formaldehyde in air by a novel NAD+- and glutathione-independent formaldehyde dehydrogenase-based biosensor. Talanta 75, 786-91 (2008).
2. Adachi, O. et al. Biooxidation with PQQ-and FAD-Dependent Dehydrogenases. Modern Biooxidation : Enzymes, Reactions and Applications, Wiley-VCH, Weinheim 1-41(2007).
3. Adachi, O. et al. New quinoproteins in oxidative fermentation. Biochimica et Biophysica Acta (BBA)-Proteins & Proteomics 1647, 10-17(2003).
4. Ameyama, M. et al. Method of enzymatic determination of pyrroloquinoline quinone. Analytical biochemistry 151, 263-7(1985).
5. Anthony C, Zatman LJ (1967). "The microbial oxidation of methanol. The prosthetic group of the alcohol dehydrogenase of Pseudomonas sp. M27: a new oxidoreductase prosthetic group". Biochem J 104 (3): 960-9. PMID 6049934. PMID 6049934
6. Anthony, C. Pyrroloquinoline quinone (PQQ) and quinoprotein enzymes. Antioxidants and Redox Signaling 3, 757-774(2001).
7. Anthony, C. The quinoprotein dehydrogenases for methanol and glucose. Archives of biochemistry and biophysics 428, 2-9(2004).
8. Cline, A. & Hu, A. Enzymatic characterization and comparison of three sugar dehydrogenases from a pseudomonad. Journal of Biological Chemistry 240, 4493(1965).
9. Cozier, G.E., Salleh, R.A. & Anthony, C. Characterization of the membrane quinoprotein glucose dehydrogenase from Escherichia coli and characterization of a site-directed mutant in which histidine-262 has been changed to tyrosine. Biochemical Journal 340, 639(1999).
10. D'Costa, E. J., I. J. Higgins & A. P. F. Turner. 1986. Quinoprotein glucose dehydrogenase and its application in an amperometric glucose sensor. Biosensors. 2 71-87
11. Duine, J.A. Quinoproteins: enzymes containing the quinonoid cofactor pyrroloquinoline quinone, topaquinone or tryptophan-tryptophan quinone. European Journal of Biochemistry 200, 271-284(1991).
12. Durand, F. et al. Designing a highly active soluble PQQ-glucose dehydrogenase for efficient glucose biosensors and biofuel cells. Biochemical and biophysical research communications 402, 750-4(2010).
13. Gao, F.,Viry, L., Maugey, M., Poulin, P., Mano, N., Engineering hybrid nanotube wires for high-power biofuel cells, Nat.Commun.1 (2010), doi:10.1038/ncomms1000
14. Goodwin P.M, Anthony C., "The biochemistry, physisiology and genetics of PQQ and PQQ-containing enzymes," Adv. Microb. Physiol. (1998) 40:1-80
15. Goosen N. et al., "Acinetobacter calcoaceticus Genes Involved in Biosynthesis of the Coenzyme Pyrrolo-Quinoline-Quinone: Nucleotide Sequence and Expression in Escherichia coli K-12," J Bacteriol. (1989) 171:447-455
16. Gupta, A. et al. Gluconobacter oxydans: its biotechnological applications. Journal of molecular microbiology and biotechnology 3, 445-56(2001).
17. Hauge JG (1964). "Glucose dehydrogenase of bacterium anitratum: an enzyme with a novel prosthetic group". J Biol Chem 239: 3630-9. PMID 14257587.PMID 14257587
18. Heller, A. and Feldman, B., Electrochemical glucose sensors and their applications in diabetes management, Chem. Rev. 108 (2008), pp. 2482-2505. Full Text via CrossRef | View Record in Scopus | Cited By in Scopus (77)
19. Hoelscher T., Goerisch H., "Knockout and Overexpression of Pyrroloquinoline Quinone Biosynthetic Genes in Gluconobacteroxydans 621H," J Bacteriology (2006) 188;21:7668-7676
20. Hommes, R. W. J., Postma, P. W., Neijssel, 0. M., Tempest, D. W.,Dokter, P. & Duine, J.A. (1984). Evidence for a glucose dehydrogenase apo-enzyme in several strains of Escherichia cofi.FEMS Microbiol Lett 24,329-333.
21. Igarashi, S. et al. Molecular engineering of PQQGDH and its applications. Archives of biochemistry and biophysics 428, 52-63(2004).
22. Igarashi, S., Hirokawa, T. & Sode, K. Engineering PQQ glucose dehydrogenase with improved substrate specificity. Site-directed mutagenesis studies on the active center of PQQ glucose dehydrogenase. Biomolecular engineering 21, 81-9(2004).
23. Jonge, R.D., Mattos, M. D. & Stock, J. Pyrroloquinoline quinone, a chemotactic attractant for Escherichia coli. Journal of 178, 1224-1226(1996).
24. Keilin, D. & Hartree, E.F. Properties of glucose oxidase (notatin): Addendum. Sedimentation and diffusion of glucose oxidase (notatin). The Biochemical journal 42, 221-9(1948).
25. Keilin, D. & Hartree, E.F. Specificity of glucose oxidase (notatin). The Biochemical journal 50, 331-41 (1952).
26. Keilin, D. & Hartree, E.F. The use of glucose oxidase (notatin) for the determination of glucose in biological material and for the study of glucose-producing systems by manometric methods. The Biochemical journal 42, 230-8(1948).
27. Khairnar N.P. et al., "Pyrroloquinoline-quinone synthesized in Escherichia coli by pyrroloquinoline-quinone synthase of Deinococcus radiodurans plays a role beyond mineral phosphate solubilization," Biochemical and Biophysical Research Communications (2003) 312:303-308
28. Kim C.H. et al., "Cloning and Expression of Pyrroloquinoline Quinone (PQQ) Genes from a Phosphate-Solubilizing Bacterium Enterobacter intermedium," Current Microbiology (2003) 47:457-461
29. Kujawa, M. et al. Properties of pyranose dehydrogenase purified from the litter-degrading fungus Agaricus xanthoderma. The FEBS journal 274, 879-94(2007).
30. Lapenaite, I., Kurtinaitiene, B. & Pliu 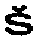 kys, L. Application of PQQ-GDH Based Polymeric Layers in Design of Biosensors for Detection of Heavy Metals. (2003)
31. Lapenaite, I., Ramanaviciene, A. & Ramanavicius, A. Current trends in enzymatic determination of glycerol. Critical Reviews in Analytical Chemistry 36, 13-25(2006).
32. Leskovac V, Trivic S, Wohlfahrt G, Kandrac J, Pericin D (2005)Glucose oxidase from Aspergillus niger the mechanism of action with molecular oxygen, quinones, and one-electron acceptors. Int J Biochem 37:731-750
33. Lidstrom, M.E. Genetics of bacterial quinoproteins. Methods in enzymology. San Diego CA 258, 217-227(1995).
34. Linton, J., Woodard, S. & Gouldney, D. The consequence of stimulating glucose dehydrogenase activity by the addition of PQQ on metabolite production by Agrobacterium radiobacter NCIB 11883. Applied Microbiology and Biotechnology 25, 357-361(1987).
35. Magnusson, O.T. et al. The structure of a biosynthetic intermediate of pyrroloquinoline quinone (PQQ) and elucidation of the final step of PQQ biosynthesis. Journal of the American Chemical Society 126, 5342-3(2004).
36. Martin, E.J.S. Assay for D-allose using a NAD cofactor coupled D-allose dehydrogenase. US Patent 5,567,605 (1996).
37. Matsushita, K. et al. Escherichia coli is unable to produce pyrroloquinoline quinone (PQQ). Microbiology (Reading, England) 143 (Pt 1, 3149-56(1997).
38. Meulenberg J.J., "nucleotide sequence and structure of the Klebsiella pneumoniae pqq operon," Mol. Gen. Genet. (1992) 232:284-294
39. Mitchell, R. & Duke, F. Kinetics and equilibrium constants of the gluconic acid-gluconolactone equilibrium. Ann. NY Acad. Sci 172, 129-138(1970).
40. Olsthoorn, a J. & Duine, J. a On the mechanism and specificity of soluble, quinoprotein glucose dehydrogenase in the oxidation of aldose sugars. Biochemistry 37, 13854-61 (1998).
41. Olsthoorn, a J., Otsuki, T. & Duine, J. a Ca2+ and its substitutes have two different binding sites and roles in soluble, quinoprotein (pyrroloquinoline-quinone-containing) glucose dehydrogenase. European journal of biochemistry / FEBS 247, 659-65(1997).
42. Oubrie, a & Dijkstra, B.W. Structural requirements of pyrroloquinoline quinone dependent enzymatic reactions. Protein science : a publication of the Protein Society 9, 1265-73(2000).
43. Oubrie, a et al. Structure and mechanism of soluble quinoprotein glucose dehydrogenase. The EMBO journal 18, 5187-94(1999).
44. Oubrie, a Structure and mechanism of soluble glucose dehydrogenase and other PQQ-dependent enzymes. Biochimica et Biophysica Acta (BBA)-Proteins & Proteomics 1647, 143-151 (2003).
45. Pazur JH, Kleppe K (1964) The oxidation of glucose and related compounds by glucose oxidase from Aspergillus niger. Biochemistry 3:578-583
46. Puehringer S., Metlitzky M. et al., "The pyrroloquinoline quinine biosynthesis pathway revisited: A structural approach,", BMC Biochemistry (2008) 9:8
47. Rose, a, Scheller, F. & Wollenberger, U. Quinoprotein glucose dehydrogenase modified thick-film electrodes for the amperometric detection of phenolic compounds in flow injection analysis. Fresenius' journal of 369, 145-52(2001).
48. Schie, B. J. van et al. Energy transduction by electron transfer via a pyrrolo-quinoline quinone-dependent glucose dehydrogenase in Escherichia coli, Pseudomonas aeruginosa, and Acinetobacter calcoaceticus (var. Iwoffi). Journal of bacteriology 163, 493-9(1985).
49. Schmid, R. & Urlacher, V. B. Modern biooxidation: enzymes, reactions and applications. Engineering (Vch Verlagsgesellschaft Mbh: 2007).
50. Sierks, M.R. et al. Active site similarities of glucose dehydrogenase, glucose oxidase, and glucoamylase probed by deoxygenated substrates. Biochemistry 31, 8972-7(1992).
51. Smolander, M., Livio, H.-L., Rasanen, L., Mediated amperometric determination of xylose and glucose with an immobilized aldose dehydrogenase electrode, Biosensors and Bioelectronics, Volume 7, Issue 9, 1992, Pages 637-643.
52. Sode, K. et al. Effect of PQQ glucose dehydrogenase overexpression in Escherichia coli on sugar-dependent respiration. Journal of biotechnology 43, 41-4(1995).
53. Sode, K. et al. Thermostable chimeric PQQ glucose dehydrogenase. FEBS letters 364, 325-7(1995).
54. Sode, K., Ootera, T., Shirahane, M., Witarto, A.B., Igarashi, S., Yoshida, H., Increasing the thermal stability of the water-soluble pyrroloquinoline quinone glucose dehydrogenase by single amino acid replacement, Enzyme Microb. Technol. 26 (2000) 491-496.
55. Southall, S. M. et al. Soluble aldose sugar dehydrogenase from Escherichia coli: a highly exposed active site conferring broad substrate specificity. The Journal of biological chemistry 281, 30650-9(2006).
56. Springer A.L. et al., "Characterisation and nucleotide sequence of pqqE and pqqF in Methylobacterium extorquens AM1," J Bacteriol. (1996) 178:2154-2157
57. Szeponik, J. et al. Ultrasensitive bienzyme sensor for adrenaline. Biosensors and 12, 947-52(1997).
58. Tanaka, S. et al. Increasing stability of water-soluble PQQ glucose dehydrogenase by increasing hydrophobic interaction at dimeric interface. BMC biochemistry 6, 1(2005).
59. Volc, J. et al. Pyranose 2-dehydrogenase, a novel sugar oxidoreductase from the basidiomycete fungus Agaricus bisporus. Archives of microbiology 167, 119-25(1997).
60. Wilson, G.S. et al. Progress toward the Development of an Implantable Sensor for Glucose. 1617, 1613-1617(1992).
61. Wong, C.M., Wong, K.H. & Chen, X.D. Glucose oxidase: natural occurrence, function, properties and industrial applications. Applied microbiology and biotechnology 78, 927-38(2008).
62. Yamada, M. et al. Escherichia coli PQQ-containing quinoprotein glucose dehydrogenase: its structure comparison with other quinoproteins. Biochimica et Biophysica Acta (BBA)-Proteins & Proteomics 1647, 185-192(2003).
63. Yang X.-P. et al., "Pyrroloquinoline quinine biosynthesis in Escherichia coli through expression of the Gluconobacter oxydans pqqABCDE gene cluster," J Ind Microbiol Biotechnol (2010) 37:575-580
64. Yoshida H. et al., "Secretion of water soluble pyrroloquinoline quinine glucose dehydrogenase by recombinant Pichia pastoris," Enzy Microbial Tech (2002) 30:312-318
65. Zheng, Y.J. & Bruice, T.C. Conformation of coenzyme pyrroloquinoline quinone and role of Ca2+ in the catalytic mechanism of quinoprotein methanol dehydrogenase. Proceedings of the National Academy of Sciences of the United States of America 94, 11881-6(1997).
66. Gijsen, H. Unprecedented asymmetric aldol reactions with three aldehyde substrates catalyzed by 2-deoxyribose-5-phosphate aldolase. Journal of the American Chemical 8422-8423(1994)

### Patent references cited:

WO2009156083, JP2009232872, EP2251420, US2009148874, MX2007000560, JP2006314322, JP2006217811, WO 2006/134482 WO2008/119810, WO 2005/118794, WO 2006/134482, WO2009/092702

## Claims

**1.** A process for preparing a compound of formula (I) 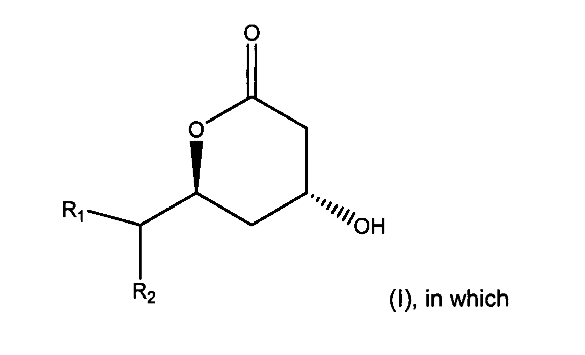 R1 independently from R2 denotes H, X, N₃, CN, NO₂, OH, (CH₂)ₙ-CH₃, O-(CH₂)ₙ-CH₃, S-(CH₂)ₙ-CH₃, NR³R⁴, OCO(CH₂)ₙCH₃, NR³CO(CH₂)ₙCH₃, CH2-R5, optionally substituted mono- or bicyclic aryl, heterocyclic or alicyclic group; and
R2 independently from R1 denotes H, (CH₂)ₘ-CH₃, or aryl;
or both of R1 and R2 denote either X, OH or O((CH₂)ₙCH₃);
or R¹ and R² together denote =O =CH-R5, or together form a ring -(CH₂)ₚ-, -(CH₂)ᵣ-(1,2-arylene)-(CH₂)ₛ-, wherein
any one of CH₂ or CH₃ groups denoted above may optionally be further substituted by X, N₃, CN, NO₂, OH, (CH₂)ₙ-CH₃, aryl, O-(CH₂)ₙ-CH₃, OCO(CH₂)ₙCH₃, NR³R⁴, NR³CO(CH₂)ₙCH₃; or
each CH₂ linking carbon atoms can be replaced by O, S or NR³; wherein
R³ and R⁴ independently from each other, or together, denote H, (CH₂)ₘ-CH₃, or together form a ring -(CH₂)ₚ-, -(CH₂)ᵣ-(1,2-ary)ene)-(CH₂)ₛ-, -(CO)ᵣ-(1,2-arylene)-(CO)ₛ-;
R5 denotes optionally substituted mono- or bicyclic aryl, heterocyclic or alicyclic group,
X denotes F, Cl, Br or I;
n represents an integer from 0 to 10;
m represents an integer from 0 to 3;
p represents an integer from 2 to 6;
and at least one from r and s is 1;
or a pharmaceutically acceptable salt, ester, or stereoisomer thereof,
the process comprising bringing in contact a compound of formula (II), 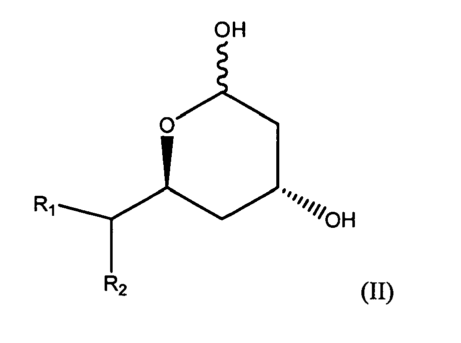 wherein R1 and R2 are defined as above, with an enzyme capable of catalyzing oxidation or dehydrogenation, and optionally salifying, esterifying or stereoselectively resolving the product.

**2.** The process according to claim 1, wherein
R5 denotes a moiety selected from the formula (III), (IV), (V), (VI), (VII), (VIII) and (IX);

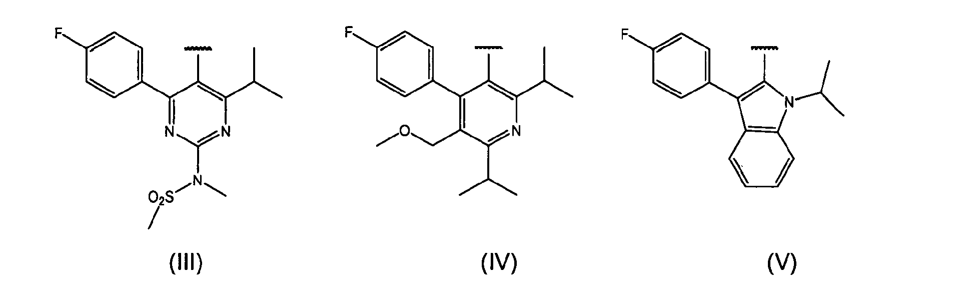

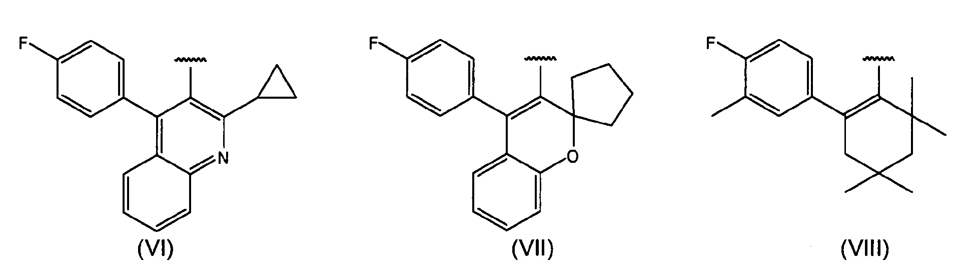

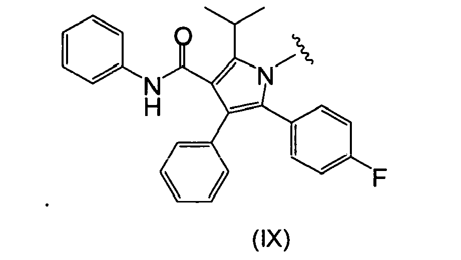

**3.** The process for preparing a compound of formula (I) according to claims 1 or 2, in which
R1 independently from R2 denotes H, X, N₃, CN, NO₂, OH, (CH₂)ₙ-CH₃, O-(CH₂)ₙ-CH₃, S-(CH₂)ₙ-CH₃, NR³R⁴, OCO(CH₂)ₙCH₃, or NR³CO(CH₂)ₙCH₃, optionally substituted mono- or bicyclic aryl, heterocyclic or alicyclic group; and
R2 independently from R1 denotes H, (CH₂)ₘ-CH₃, or aryl;
or both of R1 and R2 denote X, OH or O(CH₂)ₙCH₃;
or R¹ and R² together denote =O, or together form a ring -(CH₂)ₚ-, -(CH₂)ᵣ-(1,2-arylene)-(CH₂)ₛ-, wherein
any one of CH₂ or CH₃ groups denoted above may optionally be further substituted by X, N₃, CN, NO₂, OH, (CH₂)ₙ-CH₃, aryl, O-(CH₂)ₙ-CH₃, OCO(CH₂)ₙCH₃, NR³R⁴, NR³CO(CH₂)ₙCH₃; or
each CH₂ linking carbon atoms can be replaced by O, S or NR³; wherein
R³ and R⁴ independently from each other, or together, denote H, (CH₂)ₘ-CH₃, or together form a ring -(CH₂)ₚ-, -(CH₂)ᵣ-(1,2-arylene)-(CH₂)ₛ-, -(CO)ᵣ-(1,2-arylene)-(CO)ₛ-;
X denotes F, Cl, Br or I;
n represents an integer from 0 to 10;
m represents an integer from 0 to 3;
p represents an integer from 2 to 6;
and at least one from r and s is 1.

**4.** The process according to any one of the previous claims, wherein the enzyme capable of catalyzing oxidation or dehydrogenation is an oxidase or a dehydrogenase, preferably is an aldose dehydrogenase, more preferably is aldose 1-dehydrogenase (EC 1.1.5.2).

**5.** The process according to any one of the previous claims, wherein the enzyme capable of catalyzing oxidation or dehydrogenation is selected from a group of pyrroloquinoline quinine (PQQ) dependent dehydrogenases and water-soluble aldose dehydrogenases, preferably is Ylil aldose dehydrogenase.

**6.** The process according to any one of the previous claims, wherein the enzyme capable of catalyzing oxidation or dehydrogenation is selected from the group consisting of dehydrogenases encoded by dehydrogenase-encoding genes comprised within, or constituted by, any one of nucleotide sequences of SEQ ID NOS. 01, 03, 05, 07, 09, 23, 25, 27, 29, 31, 33, 35, 37, 39, 41, 43, 45, 47, 49, 51, 53, 55, 57, 59 and 61; or dehydrogenases defined by any one of amino acid sequences comprised within, or constituted by, SEQ ID NOS. 02, 04, 06, 08, 10, 24, 26, 28, 30, 32, 34, 36, 38, 40, 42, 44, 46, 48, 50, 52, 54, 56, 58, 60 and 62;
or any dehydrogenase having a nucleotide sequence identity or an amino acid sequence identity respectively of at least 50 % to said sequences, more preferably 70 % to said sequences, provided that the resulting sequence variants maintain dehydrogenase activity.

**6.** The process according to any one of the previous claims, wherein 2-deoxyribose-5-phosphate aldolase (DERA, EC 4.1.2.4) enzyme is used for preparing the compound of formula (II), preferably wherein the 2-deoxyribose-5-phosphate aldolase enzyme is used for a synthetic step preceding, or alternatively simultaneously at least in an overlapping time period with, bringing in contact the compound of formula (II) with the enzyme capable of catalyzing oxidation or dehydrogenation.

**7.** The process according to any one of the previous claims, wherein the enzyme capable of catalyzing oxidation or dehydrogenation, optionally also the DERA enzyme independently, are comprised within living whole cell, inactivated whole cell, homogenized whole cell, or cell free extract; or are purified, immobilized and/or are in the form of an extracellularly expressed protein, preferably are within living whole cell, inactivated whole cell or homogenized whole cell.

**8.** A reaction system comprising a 2-deoxyribose-5-phosphate aldolase (DERA) enzyme and an enzyme capable of catalyzing oxidation or dehydrogenation, and the reaction system being capable of, or being arranged for, converting a compound of formula (IX), 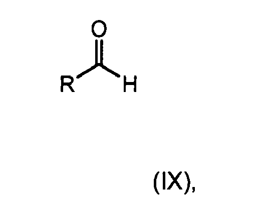 in which R denotes R1-CH-R2 moiety of formula (I), R1 and R2 being as defined in any one of tems 1 to 4, with acetaldehyde into a compound of formula (I) 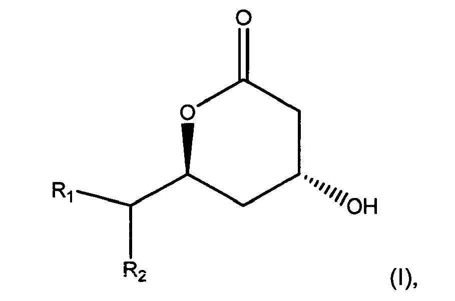 in which R1 and R2 are as defined above.

**9.** The process according to claim 7 or the reaction system according to claim 8, wherein both said enzymes, i.e. DERA and the enzyme capable of catalyzing oxidation or dehydrogenation, are expressed by one or more cells, preferably by the same cell, wherein the type of cell is selected from the group consisting of bacteria, yeast, insect cell and mammalian cells, preferably is selected from yeast and particularly from bacteria, more preferably from bacteria.

**10.** The process according to claim 7 or the reaction system according to claim 8, further providing for the presence of Pyrroloquinoline quinine (PQQ), which preferably is accomplished by a measure selected from the group consisting of:
(i) PQQ is added from externally;
(ii) a host organism is used which, beyond providing for the presence of dehydrogenase activity, further has intrinsic PQQ biosynthetic capability; and
(iii) a microorganism is used, which does not have intrinsic capability of biosynthetis of PQQ, but which is genetically engineered to express PQQ-synthesis related gene cluster, preferably providing for an expression of a PQQ-synthesis encoding gene comprised within, or constituted by, any one of the nucleotide sequences of SEQ ID NOS. 11, 17, 63, 64, 65, 66, 67, 68, 69, 70; or expression of a PQQ-synthesis gene encoding any one of the aminoacid sequence of SEQ ID NOS. 12, 13, 14, 15, 16, 18, 19, 20, 21 and 22; or by expression of a PQQ-synthesis encoding gene having a nucleotide sequence identity or an amino acid sequence identity respectively of at least 50 % to said sequences, more preferably 70 % to said sequences, provided that the resulting sequence variants maintain activity to produce PQQ.

**11.** The process according to any one of the previous claims, further comprising subjecting said compound (I) to conditions sufficient to prepare a statin or a pharmaceutically acceptable salt thereof, optionally salifying, esterifying or stereoselectively resolving the statin product, wherein the statin preferably is lovastatin, pravastatin, simvastatin, atorvastatin, cerivastatin, rosuvastatin, fluvastatin, pitavastatin, bervastatin, or dalvastatin, or a pharmaceutically acceptable salt thereof, more preferably atorvastatin, rosuvastatin or pitavastatin, or a pharmaceutically acceptable salt thereof, particularly rosuvastatin, or a pharmaceutically acceptable salt thereof.

**12.** A process for preparing a pharmaceutical composition, the process comprising carrying out a process according to the process of claim 11, and
formulating said statin, or a pharmaceutically acceptable salt thereof, with at least one pharmaceutically acceptable excipient to obtain said pharmaceutical composition,
preferably wherein said statin is selected from the group of lovastatin, pravastatin, simvastatin, atorvastatin, cerivastatin, rosuvastatin, fluvastatin, pitavastatin, bervastatin and dalvastatin, and pharmaceutically acceptable salts thereof, more preferably selected from atorvastatin, rosuvastatin and pitavastatin or pharmaceutically acceptable salts thereof.

**13.** An expression system capable of translating 2-deoxyribose-5-phosphate aldolase (DERA) enzyme and an enzyme capable of catalyzing oxidation or dehydrogenation, and overexpressing both of the genes needed for said translation.

**14.** The expression system capable of translating 2-deoxyribose-5-phosphate aldolase (DERA) enzyme and an enzyme capable of catalyzing oxidation or dehydrogenation, wherein said translation is arranged in one or more cell types, the respective cell type(s) being genetically engineered to express, in the totality of cell type(s) and preferably within the same cell type, both said 2-deoxyribose-5-phosphate aldolase (DERA) enzyme and said enzyme capable of catalyzing oxidation or dehydrogenation.

**15.** The expression system according to claim 13 or 14, wherein the enzyme capable of catalyzing oxidation or dehydrogenation is selected from the group consisting of dehydrogenases encoded by dehydrogenase-encoding genes comprised within, or constituted by, any one of nucleotide sequences of SEQ ID NOS. 01, 03, 05, 07, 09, 23, 25, 27, 29, 31, 33, 35, 37, 39, 41, 43, 45, 47, 49, 51, 53, 55, 57, 59 and 61; or dehydrogenases defined by any one of amino acid sequences comprised within, or constituted by, SEQ ID NOS. 02, 04, 06, 08, 10, 24, 26, 28, 30, 32, 34, 36, 38, 40, 42, 44, 46, 48, 50, 52, 54, 56, 58, 60 and 62;
or any dehydrogenase having nucleotide sequence identity or an amino acid sequence identity respectively of at least 70 % to said sequences, more preferably 90 % to to said sequences, provided that the resulting sequence variants maintain dehydrogenase activity.

**16.** The expression system, according to any one of claims 13 to 15, further providing for an expression of a PQQ-synthesis encoding gene comprised within, or constituted by, any one of the nucleotide sequences of SEQ ID NOS. 11, 17, 63, 64, 65, 66, 67, 68, 69, 70; or expression of a PQQ-synthesis gene encoding any one of the aminoacid sequence of SEQ ID NOS. 12, 13, 14, 15, 16, 18, 19, 20, 21 and 22;
or by expression of a PQQ-synthesis encoding gene having a nucleotide sequence identity or an amino acid sequence identity respectively of at least 50 % to said sequences, more preferably 70 % to said sequences, provided that the resulting sequence variants maintain activity to produce PQQ.

**17.** Use of the reaction system according to claim 8 or 9, or of the expression system according to claim 13 or 14, respectively for preparing a compound of formula (I), wherein the formula (I) is as defined in any one of claims 1 to 3, and optionally for further preparation of a statin or a pharmaceutically acceptable salt thereof.

**18.** Use of an enzyme capable of catalyzing oxidation or dehydrogenation for preparing a synthetic API or intermediate thereof.

**19.** Use of an aldose dehydrogenase enzyme for preparing statin or a pharmaceutically acceptable salt thereof, preferably lovastatin, pravastatin, simvastatin, atorvastatin, cerivastatin, rosuvastatin, fluvastatin, pitavastatin, bervastatin, or dalvastatin, or a pharmaceutically acceptable salt thereof, more preferably atorvastatin, rosuvastatin or pitavastatin, or a pharmaceutically acceptable salt thereof, particularly rosuvastatin or a pharmaceutically acceptable salt thereof.

**20.** Use of a microorganism for preparing a compound of formula (I) 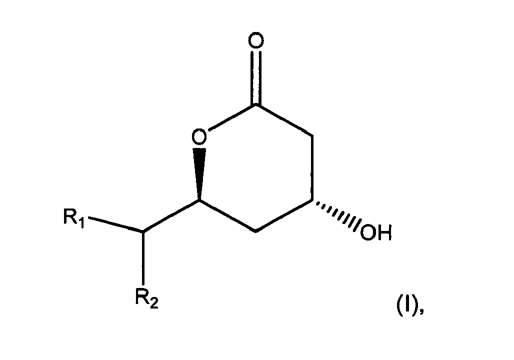 in which
R1 independently from R2 denotes H, X, N₃, CN, NO₂, OH, (CH₂)ₙ-CH₃, O-(CH₂)ₙ-CH₃, S-(CH₂)ₙ-CH₃, NR³R⁴, OCO(CH₂)ₙCH₃, NR³CO(CH₂)ₙCH₃, CH2-R5, optionally substituted mono- or bicyclic aryl, heterocyclic or alicyclic group; and
R2 independently from R1 denotes H, (CH₂)ₘ-CH₃, or aryl;
or both of R1 and R2 denote either X, OH or O((CH₂)ₙCH₃);
or R¹ and R² together denote =O, =CH-R5, or together form a ring -(CH₂)ₚ-, -(CH₂)ᵣ-(1,2-arylene)-(CH₂)ₛ-, wherein
any one of CH₂ or CH₃ groups denoted above may optionally be further substituted by X, N₃, CN, NO₂, OH, (CH₂)ₙ-CH₃, aryl, O-(CH₂)ₙ-CH₃, OCO(CH₂)ₙCH₃, NR³R⁴, NR³CO(CH₂)ₙCH₃; or
each CH₂ linking carbon atoms can be replaced by O, S or NR³; wherein
R³ and R⁴ independently from each other, or together, denote H, (CH₂)ₘ-CH₃, or together form a ring -(CH₂)ₚ-, -(CH₂)ᵣ-(1,2-arylene)-(CH₂)ₛ-, -(CO)ᵣ-(1,2-arylene)-(CO)ₛ-;
R5 denotes optionally substituted mono- or bicyclic aryl, heterocyclic or alicyclic group,
X denotes F, Cl, Br or I;
n represents an integer from 0 to 10;
m represents an integer from 0 to 3;
p represents an integer from 2 to 6;
and at least one from r and s is 1;
or a pharmaceutically acceptable salt, ester, or stereoisomer thereof, optionally with further processing of the compound of formula (I) to prepare a statin;
wherein the microorganism
(i) is selected from bacterial origin of the genera proteobacteria, actinomycetales, mixobacteriaceae, *Klebsiella Enteorobacter, Acinetobacter, Rhizobioum. Methylobacterium, Kluyvera, Gluconobacter, Pseudomonas, Erwinia, Rahnella and Deinococcus;* more particularly selected from the group of specific microorganisms *Klebsiella pneumoniae, Acinetobacter calcoaceticus, Methylobacterium extorquens, Kluyvera intermedia, Enterobacter, Gluconobacter oxydans, Pseudomonas aeruginosa, Erwinia amylovora, Rahnella aquatilis, Deinococcus radiodurans;* preferably selected from the group consisiting of: *Gluconobacter oxydans, Acinetobacter calcoaceticus* and *Kluyvera intermedium;* or
(ii) is *Escherichia coli* which is genetically engineered to be capable of expressing genes of the gene cluster for providing pyrroloquinoline quinine (PQQ).
